# EUROPEAN PATENT APPLICATION

(11) **EP 1 941 906 A1**
(43) Date of publication of application: **09.07.2008**
(21) Application number: 06810619.4
(22) Date of filing: 27.09.2006
(51) Int. Cl.: A61K 45/00, A61K 38/43, A61P 1/16, A61P 35/00, G01N 33/15, G01N 33/50

(54) **HEPATIC FIBROSIS INHIBITOR**

(30) Priority: 27.10.2005 WO PCT/JP2005/002015
(71) Applicant: Stelic Institute Of Regenerative Medicine, Stelic Institute & Co., Tokyo 106-0044 (JP)
(72) Inventor: YONEYAMA, Hiroyuki, Tokyo 106-0044 (JP)
(74) Representative: Grünecker, Kinkeldey, Stockmair & Schwanhäusser Anwaltssozietät
(86) International application number: PCT/JP2006/319128
(87) International publication number: WO 2007/049424

(57) **Abstract**

The present invention relates to hepatic fibrosis-suppressing agents that are suitable for treating or preventing fibrotic liver diseases such as cirrhosis, which comprise as an active ingredient a substance that inhibits the production or accumulation of chondroitin sulfate proteoglycans including chondroitinase ABC and ADAMTS-4; and methods of screening for the agents.

The present inventors discovered for the first time that hepatic fibrosis could be efficiently suppressed by suppressing the production or accumulation of chondroitin sulfate proteoglycans. Specifically, fibrosis of liver tissues can be suppressed by administering chondroitinase ABC, a chondroitin sulfate proteoglycan-degrading enzyme, or by using siRNA to suppress the expression ofC4ST-1, C6ST-1, or C6ST-2, a sulfotransferase for chondroitin sulfate proteoglycans. Compounds such as nucleic acids that are used as siRNA can be used as effective agents for suppressing hepatic fibrosis. Furthermore, hepatic fibrosis-suppressing agents can be found by screening for compounds that suppress the production or accumulation of chondroitin sulfate proteoglycans.

SEQUENCE LISTING

<110>  STELIC INSTITUTE OF REGENERATIVE MEDICINE
       STELIC INSTITUTE & CO.

<120>  Agents for suppressing hepatic fibrosis

<130>  EP57687HV163pau

<140>  EP 06 810 619.4
<141>  2006-09-27

<150>  PCT/JP2006/319128
<151>  2006-09-27

<150>  PCT/JP2005/020154
<151>  2005-10-27

<160>  68

<170>  PatentIn version 3.3

<210>  1
<211>  7382
<212>  DNA
<213>  Mus musculus


<220>
<221>  CDS
<222>  (133)..(6531)

<400>  1
ccccccgcgc agagcatccc tgctgcagcg cagcaagacc cggggctggc agccccagga      60

atccctagct gcttcgcagg gataaaggac tgaagttctt ggaggagcga gtccaactct     120

tcaagctgaa ct atg acc act tta ctc ttg gtc ttt gtg act ctg agg gtc     171
              Met Thr Thr Leu Leu Leu Val Phe Val Thr Leu Arg Val
              1               5                   10

atc gct gca gtg atc tca gaa gaa gtt cca gac cat gac aac tca ctg       219
Ile Ala Ala Val Ile Ser Glu Glu Val Pro Asp His Asp Asn Ser Leu
    15                  20                  25

agc gtg agc atc cct caa cca tcc cca ttg aag gtc ctc cta ggg tct       267
Ser Val Ser Ile Pro Gln Pro Ser Pro Leu Lys Val Leu Leu Gly Ser
30                  35                  40                  45

tcc ctc acc atc ccc tgc tac ttc atc gac ccc atg cat cct gtg acc       315
Ser Leu Thr Ile Pro Cys Tyr Phe Ile Asp Pro Met His Pro Val Thr
                50                  55                  60

act gcc ccc tcc act gcc ccc ctc acc cca aga atc aag tgg agc cgt       363
Thr Ala Pro Ser Thr Ala Pro Leu Thr Pro Arg Ile Lys Trp Ser Arg
            65                  70                  75

gtt tcc aag gaa aag gag gtg gta ctg ctg gtg gcc act gaa gga cag       411
Val Ser Lys Glu Lys Glu Val Val Leu Leu Val Ala Thr Glu Gly Gln
        80                  85                  90

gtt cga gtc aac agc atc tac caa gac aag gtg tcg ctc ccc aac tat       459
Val Arg Val Asn Ser Ile Tyr Gln Asp Lys Val Ser Leu Pro Asn Tyr
    95                  100                 105

cca gcc atc ccc agc gat gct acc ttg gag atc cag aac ctt cgc tcc       507
Pro Ala Ile Pro Ser Asp Ala Thr Leu Glu Ile Gln Asn Leu Arg Ser
110                 115                 120                 125

aat gac tct ggg atc tac cgc tgt gaa gtg atg cac ggc atc gag gac 555
Asn Asp Ser Gly Ile Tyr Arg Cys Glu Val Met His Gly Ile Glu Asp
                130                 135                 140

agc gaa gcc acc ctg gag gtc ata gtg aaa ggt att gtg ttc cac tac 603
Ser Glu Ala Thr Leu Glu Val Ile Val Lys Gly Ile Val Phe His Tyr
            145                 150                 155

aga gct att tcc aca cgc tac acc ctg gac ttt gat cga gca cag cgg 651
Arg Ala Ile Ser Thr Arg Tyr Thr Leu Asp Phe Asp Arg Ala Gln Arg
        160                 165                 170

gct tgc cta cag aac agc gcc atc atc gcc acc cca gaa caa ctg cag 699
Ala Cys Leu Gln Asn Ser Ala Ile Ile Ala Thr Pro Glu Gln Leu Gln
    175                 180                 185

gct gcc tat gag gat ggc ttc cac cag tgc gat gca ggc tgg ctg gct 747
Ala Ala Tyr Glu Asp Gly Phe His Gln Cys Asp Ala Gly Trp Leu Ala
190                 195                 200                 205

gac cag aca gtc aga tac ccc atc cac acg ccc cgg gaa ggt tgc tat 795
Asp Gln Thr Val Arg Tyr Pro Ile His Thr Pro Arg Glu Gly Cys Tyr
                210                 215                 220

ggt gac aag gac gag ttc cct gga gtg aga acc tac gga atc cgg gac 843
Gly Asp Lys Asp Glu Phe Pro Gly Val Arg Thr Tyr Gly Ile Arg Asp
            225                 230                 235

acc aat gag acc tat gat gtg tac tgc ttc gct gag gag atg gag ggt 891
Thr Asn Glu Thr Tyr Asp Val Tyr Cys Phe Ala Glu Glu Met Glu Gly
        240                 245                 250

gag gtc ttt tat gcg aca tcc cca gag aaa ttc acc ttc cag gag gca 939
Glu Val Phe Tyr Ala Thr Ser Pro Glu Lys Phe Thr Phe Gln Glu Ala
    255                 260                 265

gcc aac gag tgc cgg agg ctg ggg gca cgc ttg gcc acc aca ggc cag 987
Ala Asn Glu Cys Arg Arg Leu Gly Ala Arg Leu Ala Thr Thr Gly Gln
270                 275                 280                 285

ctc tac ctg gcc tgg cag ggc ggt atg gac atg tgc agc gct ggc tgg 1035
Leu Tyr Leu Ala Trp Gln Gly Gly Met Asp Met Cys Ser Ala Gly Trp
                290                 295                 300

ctg gcg gac cgc agc gtg cgc tac ccc atc tcc aag gct cgg ccc aac 1083
Leu Ala Asp Arg Ser Val Arg Tyr Pro Ile Ser Lys Ala Arg Pro Asn
            305                 310                 315

tgc ggg ggc aac ctc ctg ggt gta agg act gtc tat cta cac gcc aac 1131
Cys Gly Gly Asn Leu Leu Gly Val Arg Thr Val Tyr Leu His Ala Asn
        320                 325                 330

cag aca ggc tat cct gat ccc tca tcc cga tat gac gcc atc tgt tac 1179
Gln Thr Gly Tyr Pro Asp Pro Ser Ser Arg Tyr Asp Ala Ile Cys Tyr
    335                 340                 345

aca ggt gaa gat ttt gta gac atc cca gaa aac ttc ttc ggg gtg ggt 1227
Thr Gly Glu Asp Phe Val Asp Ile Pro Glu Asn Phe Phe Gly Val Gly
350                 355                 360                 365

ggt gaa gac gac atc acc atc cag acg gtg acc tgg cca gac ctg gag 1275
Gly Glu Asp Asp Ile Thr Ile Gln Thr Val Thr Trp Pro Asp Leu Glu
                370                 375                 380

ctg ccc ttg ccc cgt aat gtc aca gag gga gag gcc ctg ggc agc gtg 1323
Leu Pro Leu Pro Arg Asn Val Thr Glu Gly Glu Ala Leu Gly Ser Val
            385                 390                 395

atc ctc acg gca aag ccc atc ttt gac ctg tcc ccc act atc tca gag 1371

Ile Leu Thr Ala Lys Pro Ile Phe Asp Leu Ser Pro Thr Ile Ser Glu
        400                 405                 410

ccg ggg gag gcc ctc aca ctt gcc cct gaa gtg ggg agc aca gcc ttc      1419
Pro Gly Glu Ala Leu Thr Leu Ala Pro Glu Val Gly Ser Thr Ala Phe
    415                 420                 425

cca gag gct gag gag aga act gga gaa gcc acc aga ccc tgg ggc ttt      1467
Pro Glu Ala Glu Glu Arg Thr Gly Glu Ala Thr Arg Pro Trp Gly Phe
430                 435                 440                 445

cct gca gaa gtc aca cgt ggg ccg gac tct gcc act gcc ttt gcc agt      1515
Pro Ala Glu Val Thr Arg Gly Pro Asp Ser Ala Thr Ala Phe Ala Ser
                450                 455                 460

gag gac ctg gta gtg cga gtg acc atc tct cca ggt gca gct gaa gtc      1563
Glu Asp Leu Val Val Arg Val Thr Ile Ser Pro Gly Ala Ala Glu Val
            465                 470                 475

cct ggt cag ccc cac ttg cca ggg gga gtt gta ttc cac tat cgc cca      1611
Pro Gly Gln Pro His Leu Pro Gly Gly Val Val Phe His Tyr Arg Pro
        480                 485                 490

ggc tcc acc aga tac tct ctg aca ttt gag gag gca cag cag gcc tgc      1659
Gly Ser Thr Arg Tyr Ser Leu Thr Phe Glu Glu Ala Gln Gln Ala Cys
    495                 500                 505

atg cac acc ggg gcc atc att gcc tct cct gag cag ctc caa gct gcc      1707
Met His Thr Gly Ala Ile Ile Ala Ser Pro Glu Gln Leu Gln Ala Ala
510                 515                 520                 525

tat gag gca ggc tat gag cag tgt gat gct ggc tgg ctg cag gac cag      1755
Tyr Glu Ala Gly Tyr Glu Gln Cys Asp Ala Gly Trp Leu Gln Asp Gln
                530                 535                 540

acc gtc aga tac ccc att gtg agc cca cga acc cca tgt gtg ggt gac      1803
Thr Val Arg Tyr Pro Ile Val Ser Pro Arg Thr Pro Cys Val Gly Asp
            545                 550                 555

aaa gac agc agc cca gga gtc agg acc tac cgc gtg cgc cca tca tca      1851
Lys Asp Ser Ser Pro Gly Val Arg Thr Tyr Arg Val Arg Pro Ser Ser
        560                 565                 570

gaa acc tat gat gtc tac tgc tac gtg gac aag ctt gag ggg gaa gtg      1899
Glu Thr Tyr Asp Val Tyr Cys Tyr Val Asp Lys Leu Glu Gly Glu Val
    575                 580                 585

ttc ttc gcc aca cgc ctc gag cag ttc acc ttc cag gaa gcg cgg gcc      1947
Phe Phe Ala Thr Arg Leu Glu Gln Phe Thr Phe Gln Glu Ala Arg Ala
590                 595                 600                 605

ttc tgt gcg gct caa aat gcc acc ctg gcc tcc acc ggc cag ctc tat      1995
Phe Cys Ala Ala Gln Asn Ala Thr Leu Ala Ser Thr Gly Gln Leu Tyr
                610                 615                 620

gct gcc tgg agc cag ggt ctg gac aag tgc tat gct ggc tgg ctg gca      2043
Ala Ala Trp Ser Gln Gly Leu Asp Lys Cys Tyr Ala Gly Trp Leu Ala
            625                 630                 635

gat ggc acc ctc cga tac ccc atc atc acc cct cgg cct gcc tgt ggt      2091
Asp Gly Thr Leu Arg Tyr Pro Ile Ile Thr Pro Arg Pro Ala Cys Gly
        640                 645                 650

ggg gac aaa cct ggc gtg aga act gtc tac ctc tac ccc aac caa acc      2139
Gly Asp Lys Pro Gly Val Arg Thr Val Tyr Leu Tyr Pro Asn Gln Thr
    655                 660                 665

ggc ctc cct gac cca ctg tca aag cac cat gcc ttc tgc ttc cga ggt      2187
Gly Leu Pro Asp Pro Leu Ser Lys His His Ala Phe Cys Phe Arg Gly

670                 675                 680                 685

gtg tca gtg gcg ccc tct cca gga gaa gaa gag ggt agt aca ccc aca     2235
Val Ser Val Ala Pro Ser Pro Gly Glu Glu Glu Gly Ser Thr Pro Thr
                690                 695                 700

tca ccc tct gac ata gag gac tgg atc gtc act cag gtg ggg cct ggt     2283
Ser Pro Ser Asp Ile Glu Asp Trp Ile Val Thr Gln Val Gly Pro Gly
            705                 710                 715

gtg gat gct gtc ccc ttg gag cca aag aca aca gaa gtg cca tat ttc     2331
Val Asp Ala Val Pro Leu Glu Pro Lys Thr Thr Glu Val Pro Tyr Phe
        720                 725                 730

acc act gag cca aga aaa cag act gaa tgg gag cca gcc tac acc cca     2379
Thr Thr Glu Pro Arg Lys Gln Thr Glu Trp Glu Pro Ala Tyr Thr Pro
    735                 740                 745

gtg ggc aca tcc cca cag cca ggg atc cct ccc aca tgg ctt ccc acc     2427
Val Gly Thr Ser Pro Gln Pro Gly Ile Pro Pro Thr Trp Leu Pro Thr
750                 755                 760                 765

ctc cca gca gca gag gaa cac aca gaa agc ccc tct gcc tct gaa gag     2475
Leu Pro Ala Ala Glu Glu His Thr Glu Ser Pro Ser Ala Ser Glu Glu
                770                 775                 780

ccc tct gcc tca gca gtc cct tcc acc tca gag gag cca tac aca tct     2523
Pro Ser Ala Ser Ala Val Pro Ser Thr Ser Glu Glu Pro Tyr Thr Ser
            785                 790                 795

tca ttt gca gtg ccg agc atg aca gag ctg cca ggc tct ggg gag gcg     2571
Ser Phe Ala Val Pro Ser Met Thr Glu Leu Pro Gly Ser Gly Glu Ala
        800                 805                 810

tcg ggc gca cct gac ctc agt ggt gac ttc aca ggc agt gga gat gct     2619
Ser Gly Ala Pro Asp Leu Ser Gly Asp Phe Thr Gly Ser Gly Asp Ala
    815                 820                 825

tca gga cgc ctt gac tcc agt ggg cag cct tca ggg ggc att gaa agt     2667
Ser Gly Arg Leu Asp Ser Ser Gly Gln Pro Ser Gly Gly Ile Glu Ser
830                 835                 840                 845

ggc ctt ccc tca ggt gac ctt gac tcc agt ggc ctc agc ccc aca gtg     2715
Gly Leu Pro Ser Gly Asp Leu Asp Ser Ser Gly Leu Ser Pro Thr Val
                850                 855                 860

agc tca ggc ctg cct gta gaa agt ggt tct gcc tca gga gat gga gaa     2763
Ser Ser Gly Leu Pro Val Glu Ser Gly Ser Ala Ser Gly Asp Gly Glu
            865                 870                 875

gtc ccc tgg tcc cat act ccc aca gtt ggc agg ttg ccc tct gga ggt     2811
Val Pro Trp Ser His Thr Pro Thr Val Gly Arg Leu Pro Ser Gly Gly
        880                 885                 890

gag agc ccc gaa ggc tct gcc tct gcc tct gga aca gga gac ctt agt     2859
Glu Ser Pro Glu Gly Ser Ala Ser Ala Ser Gly Thr Gly Asp Leu Ser
    895                 900                 905

ggg ctg cct tca gga gga gaa att aca gaa act tct act tct ggg gca     2907
Gly Leu Pro Ser Gly Gly Glu Ile Thr Glu Thr Ser Thr Ser Gly Ala
910                 915                 920                 925

gaa gaa acc agt gga ctt cct tct gga gga gac ggt cta gaa act tct     2955
Glu Glu Thr Ser Gly Leu Pro Ser Gly Gly Asp Gly Leu Glu Thr Ser
                930                 935                 940

acc tct gga gta gat gat gtc agt gga att cct act gga aga gaa ggt     3003
Thr Ser Gly Val Asp Asp Val Ser Gly Ile Pro Thr Gly Arg Glu Gly
            945                 950                 955

cta gag act tct gcc tct gga gta gag gac ctc agt gga ctt cct tct 3051
Leu Glu Thr Ser Ala Ser Gly Val Glu Asp Leu Ser Gly Leu Pro Ser
        960                 965                 970

gga gaa gaa ggt tca gaa aca tct acc tct gga ata gag gac atc agt 3099
Gly Glu Glu Gly Ser Glu Thr Ser Thr Ser Gly Ile Glu Asp Ile Ser
    975                 980                 985

gta ctt cca act gga gga gaa agt cta gaa acc tct gct tct gga gtg 3147
Val Leu Pro Thr Gly Gly Glu Ser Leu Glu Thr Ser Ala Ser Gly Val
990                 995                 1000                1005

gga gac ttg agt gga ctt ccc tca gga gga gaa agt cta gaa aca 3192
Gly Asp Leu Ser Gly Leu Pro Ser Gly Gly Glu Ser Leu Glu Thr
                1010                1015                1020

tct gct tca ggt gca gag gat gtc act cag ctt cct act gaa aga 3237
Ser Ala Ser Gly Ala Glu Asp Val Thr Gln Leu Pro Thr Glu Arg
                1025                1030                1035

gga ggt cta gag act tct gcc tct gga gta gaa gac atc act gtt 3282
Gly Gly Leu Glu Thr Ser Ala Ser Gly Val Glu Asp Ile Thr Val
                1040                1045                1050

ctt cct act gga aga gaa agt cta gaa act tct gcc tct gga gta 3327
Leu Pro Thr Gly Arg Glu Ser Leu Glu Thr Ser Ala Ser Gly Val
                1055                1060                1065

gag gat gtc agt gga ctt cct tct gga aga gaa ggt cta gag act 3372
Glu Asp Val Ser Gly Leu Pro Ser Gly Arg Glu Gly Leu Glu Thr
                1070                1075                1080

tct gcc tct gga ata gag gac att agt gtg ttt cct act gaa gca 3417
Ser Ala Ser Gly Ile Glu Asp Ile Ser Val Phe Pro Thr Glu Ala
                1085                1090                1095

gaa ggt ctg gac act tct gcc tct ggg gga tat gtt agt ggg att 3462
Glu Gly Leu Asp Thr Ser Ala Ser Gly Gly Tyr Val Ser Gly Ile
                1100                1105                1110

cct tct gga gga gat ggt aca gaa acc tct gct tct gga gta gag 3507
Pro Ser Gly Gly Asp Gly Thr Glu Thr Ser Ala Ser Gly Val Glu
                1115                1120                1125

gat gtg agt ggt ctt cca tct gga gga gag ggt cta gaa act tct 3552
Asp Val Ser Gly Leu Pro Ser Gly Gly Glu Gly Leu Glu Thr Ser
                1130                1135                1140

gcc tct gga gtg gaa gat ctt ggt cct tct act aga gat agt cta 3597
Ala Ser Gly Val Glu Asp Leu Gly Pro Ser Thr Arg Asp Ser Leu
                1145                1150                1155

gag aca tct gct tca gga gta gat gtt act ggg ttt cct tct gga 3642
Glu Thr Ser Ala Ser Gly Val Asp Val Thr Gly Phe Pro Ser Gly
                1160                1165                1170

aga ggg gac cca gag acc tct gtt tct ggg gta ggt gat gac ttc 3687
Arg Gly Asp Pro Glu Thr Ser Val Ser Gly Val Gly Asp Asp Phe
                1175                1180                1185

agt gga ctt cct tct gga aaa gaa ggc ctg gag acc tca gct tct 3732
Ser Gly Leu Pro Ser Gly Lys Glu Gly Leu Glu Thr Ser Ala Ser
                1190                1195                1200

gga gct gag gac ctc agt ggc ttg ccc tct gga aaa gaa gac ttg 3777
Gly Ala Glu Asp Leu Ser Gly Leu Pro Ser Gly Lys Glu Asp Leu
                1205                1210                1215

gta ggg tct gct tct ggg gcc ttg gac ttt ggc aaa cta cct cct 3822
Val Gly Ser Ala Ser Gly Ala Leu Asp Phe Gly Lys Leu Pro Pro
                1220                1225                1230

gga act cta gga agt ggt caa act cca gaa gta aat ggc ttt ccc 3867
Gly Thr Leu Gly Ser Gly Gln Thr Pro Glu Val Asn Gly Phe Pro
                1235                1240                1245

tct gga ttt agt ggt gag tat tct gga gca gac att gga agt ggc 3912
Ser Gly Phe Ser Gly Glu Tyr Ser Gly Ala Asp Ile Gly Ser Gly
                1250                1255                1260

cca tcc tct ggc ctg cct gac ttt agt gga ctt cca tct ggc ttt 3957
Pro Ser Ser Gly Leu Pro Asp Phe Ser Gly Leu Pro Ser Gly Phe
                1265                1270                1275

cca act gtc tcc ctt gtg gac agt acc tta gtg gaa gtg atc aca 4002
Pro Thr Val Ser Leu Val Asp Ser Thr Leu Val Glu Val Ile Thr
                1280                1285                1290

gcc acc act tcc agt gaa ctg gaa gga agg ggg acc att ggc atc 4047
Ala Thr Thr Ser Ser Glu Leu Glu Gly Arg Gly Thr Ile Gly Ile
                1295                1300                1305

agt ggt tca gga gaa gta tca ggg ctg ccc ctg ggt gaa ttg gac 4092
Ser Gly Ser Gly Glu Val Ser Gly Leu Pro Leu Gly Glu Leu Asp
                1310                1315                1320

agt agt gcg gac att agt ggt ctc cct tca gga act gaa ctc agt 4137
Ser Ser Ala Asp Ile Ser Gly Leu Pro Ser Gly Thr Glu Leu Ser
                1325                1330                1335

ggc caa gca tct gga tct cct gat agc agt gga gaa aca tct gga 4182
Gly Gln Ala Ser Gly Ser Pro Asp Ser Ser Gly Glu Thr Ser Gly
                1340                1345                1350

ttt ttt gat gtt agt gga cag cca ttt ggg tct tct ggc gtc agc 4227
Phe Phe Asp Val Ser Gly Gln Pro Phe Gly Ser Ser Gly Val Ser
                1355                1360                1365

gag gaa aca tct ggg att cct gaa atc agt ggg cag cca tca ggg 4272
Glu Glu Thr Ser Gly Ile Pro Glu Ile Ser Gly Gln Pro Ser Gly
                1370                1375                1380

act cct gac acc act gcg aca tct gga gtg act gag ctt aat gaa 4317
Thr Pro Asp Thr Thr Ala Thr Ser Gly Val Thr Glu Leu Asn Glu
                1385                1390                1395

ctg tcc tct gga caa cca gat gtc agt gga gat ggg tct gga att 4362
Leu Ser Ser Gly Gln Pro Asp Val Ser Gly Asp Gly Ser Gly Ile
                1400                1405                1410

ctc ttt ggc agt gga cag tcc tct ggt ata aca tct gtg agt gga 4407
Leu Phe Gly Ser Gly Gln Ser Ser Gly Ile Thr Ser Val Ser Gly
                1415                1420                1425

gaa acc tct ggg att tct gat ctc agt ggg cag ccc tca ggg ttc 4452
Glu Thr Ser Gly Ile Ser Asp Leu Ser Gly Gln Pro Ser Gly Phe
                1430                1435                1440

cca gtg ttc agt gga aca gca acc aga acc cct gac ctg gct tct 4497
Pro Val Phe Ser Gly Thr Ala Thr Arg Thr Pro Asp Leu Ala Ser
                1445                1450                1455

ggc acc ata agt ggc agt gga gag tct tct ggc att aca ttt gtg 4542
Gly Thr Ile Ser Gly Ser Gly Glu Ser Ser Gly Ile Thr Phe Val
                1460                1465                1470

gac acc agt ttt gtt gaa gtg acc cct acc aca ttt agg gaa gaa 4587

Asp Thr Ser Phe Val Glu Val Thr Pro Thr Thr Phe Arg Glu Glu
                1475                1480                1485

gaa ggg tta gga tct gtg gaa ctc agt ggc ttt cct tct ggg gag 4632
Glu Gly Leu Gly Ser Val Glu Leu Ser Gly Phe Pro Ser Gly Glu
                1490                1495                1500

acg gaa ctg tct ggc aca tct ggg acg gtg gac gtc agt gaa caa 4677
Thr Glu Leu Ser Gly Thr Ser Gly Thr Val Asp Val Ser Glu Gln
                1505                1510                1515

tct tct gga gca att gat tcc agt gga ctc aca tcc ccc act cca 4722
Ser Ser Gly Ala Ile Asp Ser Ser Gly Leu Thr Ser Pro Thr Pro
                1520                1525                1530

gag ttc agt ggc ctc cca agt gga gta gct gag gtc agt ggt gaa 4767
Glu Phe Ser Gly Leu Pro Ser Gly Val Ala Glu Val Ser Gly Glu
                1535                1540                1545

ttc tct gga gtt gag act ggg agc agc ttg ccc tca gga gca ttt 4812
Phe Ser Gly Val Glu Thr Gly Ser Ser Leu Pro Ser Gly Ala Phe
                1550                1555                1560

gat ggc agt gga ctt gtc tca ggt ttc ccc act gtg tct ctt gta 4857
Asp Gly Ser Gly Leu Val Ser Gly Phe Pro Thr Val Ser Leu Val
                1565                1570                1575

gac aga act ttg gtg gaa tct ata act cag gct cct act gct caa 4902
Asp Arg Thr Leu Val Glu Ser Ile Thr Gln Ala Pro Thr Ala Gln
                1580                1585                1590

gaa gct gga gaa gga cct tcg ggc att ttg gaa ttc agt ggt gcc 4947
Glu Ala Gly Glu Gly Pro Ser Gly Ile Leu Glu Phe Ser Gly Ala
                1595                1600                1605

cat tct ggg aca cca gac ata tct ggg gag ctt tct ggg tct ctg 4992
His Ser Gly Thr Pro Asp Ile Ser Gly Glu Leu Ser Gly Ser Leu
                1610                1615                1620

gac cta agc aca ttg cag tct ggg cag atg gaa acc agc acg gag 5037
Asp Leu Ser Thr Leu Gln Ser Gly Gln Met Glu Thr Ser Thr Glu
                1625                1630                1635

aca cca agc tct cca tat ttt agt gga gac ttt tcc agc acc act 5082
Thr Pro Ser Ser Pro Tyr Phe Ser Gly Asp Phe Ser Ser Thr Thr
                1640                1645                1650

gat gta agt gga gaa tcc ata gct gcc aca act ggc agt ggg gaa 5127
Asp Val Ser Gly Glu Ser Ile Ala Ala Thr Thr Gly Ser Gly Glu
                1655                1660                1665

agc tct ggg ctt ccg gaa gtt act tta aac acc tca gag tta gtg 5172
Ser Ser Gly Leu Pro Glu Val Thr Leu Asn Thr Ser Glu Leu Val
                1670                1675                1680

gag ggt gtg act gaa ccc act gtt tcc cag gaa ctt ggc cat ggt 5217
Glu Gly Val Thr Glu Pro Thr Val Ser Gln Glu Leu Gly His Gly
                1685                1690                1695

cct tct atg aca tac atc tcc cgg ctc tct gag gcc agt ggg gac 5262
Pro Ser Met Thr Tyr Ile Ser Arg Leu Ser Glu Ala Ser Gly Asp
                1700                1705                1710

gcc tca gca tcc ggg gac ctt ggt ggc gct gta aca aac ttc cca 5307
Ala Ser Ala Ser Gly Asp Leu Gly Gly Ala Val Thr Asn Phe Pro
                1715                1720                1725

ggg tct gga gta gaa gct tca gtc cca gaa gcc agc agt gac ctg 5352
Gly Ser Gly Val Glu Ala Ser Val Pro Glu Ala Ser Ser Asp Leu

1730                1735                1740

tct gct tac cct gag  gct ggt gtg gga gtg  tct gct gcc cct gag      5397
Ser Ala Tyr Pro Glu  Ala Gly Val Gly Val  Ser Ala Ala Pro Glu
                1745                1750                1755

gcc agc agt aaa ctg  tct gag ttc cca gat  ctg cat gga atc act      5442
Ala Ser Ser Lys Leu  Ser Glu Phe Pro Asp  Leu His Gly Ile Thr
                1760                1765                1770

tct gcc ttc cat gaa  aca gat ctg gaa atg  aca acc cca agc aca      5487
Ser Ala Phe His Glu  Thr Asp Leu Glu Met  Thr Thr Pro Ser Thr
                1775                1780                1785

gag gta aac agc aac  cca tgg acc ttt cag  gaa ggc acc agg gag      5532
Glu Val Asn Ser Asn  Pro Trp Thr Phe Gln  Glu Gly Thr Arg Glu
                1790                1795                1800

gga tcg gcc gct ccg  gaa gtg agt gga gaa  tct agc act acc tcc      5577
Gly Ser Ala Ala Pro  Glu Val Ser Gly Glu  Ser Ser Thr Thr Ser
                1805                1810                1815

gac ata gac aca ggc  act tca ggg gtg cct  tct gcc aca ccc atg      5622
Asp Ile Asp Thr Gly  Thr Ser Gly Val Pro  Ser Ala Thr Pro Met
                1820                1825                1830

gct tct gga gac agg  act gaa atc agc gga  gaa tgg tct gat cac      5667
Ala Ser Gly Asp Arg  Thr Glu Ile Ser Gly  Glu Trp Ser Asp His
                1835                1840                1845

acc tca gag gtg aat  gtt gcc atc agc agc  acc atc aca gag tcc      5712
Thr Ser Glu Val Asn  Val Ala Ile Ser Ser  Thr Ile Thr Glu Ser
                1850                1855                1860

gag tgg gcc cag cct  acc cgg tac cct aca  gag aca ctt caa gaa      5757
Glu Trp Ala Gln Pro  Thr Arg Tyr Pro Thr  Glu Thr Leu Gln Glu
                1865                1870                1875

atc gaa tcc cca aat  ccc tca tac tca gga  gag gag acc cag aca      5802
Ile Glu Ser Pro Asn  Pro Ser Tyr Ser Gly  Glu Glu Thr Gln Thr
                1880                1885                1890

gca gaa aca acc atg  tcc ctg aca gat gcc  ccc acc ctc tct tct      5847
Ala Glu Thr Thr Met  Ser Leu Thr Asp Ala  Pro Thr Leu Ser Ser
                1895                1900                1905

tca gaa ggg tca ggg  gag acg gag tca acc  gtt gca gac cag gag      5892
Ser Glu Gly Ser Gly  Glu Thr Glu Ser Thr  Val Ala Asp Gln Glu
                1910                1915                1920

caa tgt gag gag ggg  tgg acc aag ttc cag  ggt cac tgt tac cgc      5937
Gln Cys Glu Glu Gly  Trp Thr Lys Phe Gln  Gly His Cys Tyr Arg
                1925                1930                1935

cac ttt cct gac cga  gag acc tgg gtg gat  gcg gag aga cgg tgt      5982
His Phe Pro Asp Arg  Glu Thr Trp Val Asp  Ala Glu Arg Arg Cys
                1940                1945                1950

cgg gag cag cag tca  cat ctg agc agc att  gtc act cct gag gaa      6027
Arg Glu Gln Gln Ser  His Leu Ser Ser Ile  Val Thr Pro Glu Glu
                1955                1960                1965

cag gag ttc gtc aac  aaa aat gct caa gac  tac cag tgg atc ggt      6072
Gln Glu Phe Val Asn  Lys Asn Ala Gln Asp  Tyr Gln Trp Ile Gly
                1970                1975                1980

ctg aat gac agg act  atc gaa ggg gac ttc  cgc tgg tct gac gga      6117
Leu Asn Asp Arg Thr  Ile Glu Gly Asp Phe  Arg Trp Ser Asp Gly
                1985                1990                1995

cac tct ctg caa ttt gag aag tgg cgt cca aac cag cct gac aac 6162
His Ser Leu Gln Phe Glu Lys Trp Arg Pro Asn Gln Pro Asp Asn
                2000                2005                2010

ttc ttt gcc acc gga gag gac tgt gtg gtg atg atc tgg cat gag 6207
Phe Phe Ala Thr Gly Glu Asp Cys Val Val Met Ile Trp His Glu
                2015                2020                2025

aga ggc gaa tgg aac gac gtc ccc tgc aat tac cag ctg ccc ttc 6252
Arg Gly Glu Trp Asn Asp Val Pro Cys Asn Tyr Gln Leu Pro Phe
                2030                2035                2040

acg tgt aaa aag ggc acc gtg gcc tgt gga gac ccc cca gtg gtg 6297
Thr Cys Lys Lys Gly Thr Val Ala Cys Gly Asp Pro Pro Val Val
                2045                2050                2055

gag cat gct aga acc ctc ggg cag aag aaa gat cgc tac gag atc 6342
Glu His Ala Arg Thr Leu Gly Gln Lys Lys Asp Arg Tyr Glu Ile
                2060                2065                2070

agc tcc ctg gtg cgg tac cag tgc act gag ggc ttt gtc cag cgc 6387
Ser Ser Leu Val Arg Tyr Gln Cys Thr Glu Gly Phe Val Gln Arg
                2075                2080                2085

cac gtg ccc acc atc cgg tgc cag ccc agc ggg cac tgg gaa gag 6432
His Val Pro Thr Ile Arg Cys Gln Pro Ser Gly His Trp Glu Glu
                2090                2095                2100

cct cga atc acc tgc aca gac ccc aac acc tac aag cac agg cta 6477
Pro Arg Ile Thr Cys Thr Asp Pro Asn Thr Tyr Lys His Arg Leu
                2105                2110                2115

cag aag cgg acg atg aga ccc aca cgg agg agc cgc ccc agc atg 6522
Gln Lys Arg Thr Met Arg Pro Thr Arg Arg Ser Arg Pro Ser Met
                2120                2125                2130

gcc cac tga gaggagcttc cataatgtgc ccaggatgct gagcccagcg 6571
Ala His

gccagccagg ctgaccgtgc atcccaccca catggtgtct tcttgtcgct ttttgtcata 6631

taaggaatcc attaaagaag gaaaaaaaac ccacattgtg tgtgccccac tccccgcatc 6691

acccaaactg catctaattt atccccttct cccccccccc caaaaaaaaa aagaaaagaa 6751

atgcccaagc aagtcactgt aaccccgtgg tacctcagct ttgagacttt tttgatttcc 6811

ccacccgtgc cctttcccag ggaccagtgc aggcacaggg tgagaaggta aggggttaaa 6871

ttaaataaag aagattcttt tttgttgttg tttcctgact ttgcccaaga acagtacaat 6931

ggttggttac ttcgcctcca gggagagcta ggggcccaga ggaggagctg aaagaagaca 6991

ggaccaggaa gggaggagta gggcggtcac agagcttgga ggactcagaa gaggcaaagt 7051

acagcttagt gtgttggaca aaaggaattg aggtctgtgc catctgtgag ggaaggacct 7111

ggggcaggga caggctggct gaagagaagc agagccctct cctagggacc tttcatctgg 7171

gtcaaccaac ctagcaggtg tcatttctcg gctggactgg gtaggggcac ttccttccca 7231

ggggtctccg ccgtcccctt ccctctcccc accccgggac ggtgcaggca ccagtgttcc 7291

gtgcacctat ttatattttt gaaaactaaa gattatgata attataataa taaagacatt 7351

ggaagagatc taaaaaaaaa aaaaaaaaaa a 7382

<210>  2
<211>  2132
<212>  PRT
<213>  Mus musculus

<400>  2

Met Thr Thr Leu Leu Leu Val Phe Val Thr Leu Arg Val Ile Ala Ala
1               5                   10                  15

Val Ile Ser Glu Glu Val Pro Asp His Asp Asn Ser Leu Ser Val Ser
            20                  25                  30

Ile Pro Gln Pro Ser Pro Leu Lys Val Leu Leu Gly Ser Ser Leu Thr
        35                  40                  45

Ile Pro Cys Tyr Phe Ile Asp Pro Met His Pro Val Thr Thr Ala Pro
    50                  55                  60

Ser Thr Ala Pro Leu Thr Pro Arg Ile Lys Trp Ser Arg Val Ser Lys
65                  70                  75                  80

Glu Lys Glu Val Val Leu Leu Val Ala Thr Glu Gly Gln Val Arg Val
                85                  90                  95

Asn Ser Ile Tyr Gln Asp Lys Val Ser Leu Pro Asn Tyr Pro Ala Ile
            100                 105                 110

Pro Ser Asp Ala Thr Leu Glu Ile Gln Asn Leu Arg Ser Asn Asp Ser
        115                 120                 125

Gly Ile Tyr Arg Cys Glu Val Met His Gly Ile Glu Asp Ser Glu Ala
    130                 135                 140

Thr Leu Glu Val Ile Val Lys Gly Ile Val Phe His Tyr Arg Ala Ile
145                 150                 155                 160

Ser Thr Arg Tyr Thr Leu Asp Phe Asp Arg Ala Gln Arg Ala Cys Leu
                165                 170                 175

Gln Asn Ser Ala Ile Ile Ala Thr Pro Glu Gln Leu Gln Ala Ala Tyr
            180                 185                 190

Glu Asp Gly Phe His Gln Cys Asp Ala Gly Trp Leu Ala Asp Gln Thr
        195                 200                 205

Val Arg Tyr Pro Ile His Thr Pro Arg Glu Gly Cys Tyr Gly Asp Lys
    210                 215                 220

Asp Glu Phe Pro Gly Val Arg Thr Tyr Gly Ile Arg Asp Thr Asn Glu
225                 230                 235                 240

Thr Tyr Asp Val Tyr Cys Phe Ala Glu Glu Met Glu Gly Glu Val Phe

245                 250                 255

Tyr Ala Thr Ser Pro Glu Lys Phe Thr Phe Gln Glu Ala Ala Asn Glu
            260                 265                 270

Cys Arg Arg Leu Gly Ala Arg Leu Ala Thr Thr Gly Gln Leu Tyr Leu
        275                 280                 285

Ala Trp Gln Gly Gly Met Asp Met Cys Ser Ala Gly Trp Leu Ala Asp
    290                 295                 300

Arg Ser Val Arg Tyr Pro Ile Ser Lys Ala Arg Pro Asn Cys Gly Gly
305                 310                 315                 320

Asn Leu Leu Gly Val Arg Thr Val Tyr Leu His Ala Asn Gln Thr Gly
                325                 330                 335

Tyr Pro Asp Pro Ser Ser Arg Tyr Asp Ala Ile Cys Tyr Thr Gly Glu
            340                 345                 350

Asp Phe Val Asp Ile Pro Glu Asn Phe Phe Gly Val Gly Gly Glu Asp
        355                 360                 365

Asp Ile Thr Ile Gln Thr Val Thr Trp Pro Asp Leu Glu Leu Pro Leu
    370                 375                 380

Pro Arg Asn Val Thr Glu Gly Glu Ala Leu Gly Ser Val Ile Leu Thr
385                 390                 395                 400

Ala Lys Pro Ile Phe Asp Leu Ser Pro Thr Ile Ser Glu Pro Gly Glu
                405                 410                 415

Ala Leu Thr Leu Ala Pro Glu Val Gly Ser Thr Ala Phe Pro Glu Ala
            420                 425                 430

Glu Glu Arg Thr Gly Glu Ala Thr Arg Pro Trp Gly Phe Pro Ala Glu
        435                 440                 445

Val Thr Arg Gly Pro Asp Ser Ala Thr Ala Phe Ala Ser Glu Asp Leu
    450                 455                 460

Val Val Arg Val Thr Ile Ser Pro Gly Ala Ala Glu Val Pro Gly Gln
465                 470                 475                 480

Pro His Leu Pro Gly Gly Val Val Phe His Tyr Arg Pro Gly Ser Thr
                485                 490                 495

Arg Tyr Ser Leu Thr Phe Glu Glu Ala Gln Gln Ala Cys Met His Thr
            500                 505                 510

Gly Ala Ile Ile Ala Ser Pro Glu Gln Leu Gln Ala Ala Tyr Glu Ala
        515                 520                 525

Gly Tyr Glu Gln Cys Asp Ala Gly Trp Leu Gln Asp Gln Thr Val Arg
    530                 535                 540

Tyr Pro Ile Val Ser Pro Arg Thr Pro Cys Val Gly Asp Lys Asp Ser
545                 550                 555                 560

Ser Pro Gly Val Arg Thr Tyr Arg Val Arg Pro Ser Ser Glu Thr Tyr
                565                 570                 575

Asp Val Tyr Cys Tyr Val Asp Lys Leu Glu Gly Glu Val Phe Phe Ala
            580                 585                 590

Thr Arg Leu Glu Gln Phe Thr Phe Gln Glu Ala Arg Ala Phe Cys Ala
        595                 600                 605

Ala Gln Asn Ala Thr Leu Ala Ser Thr Gly Gln Leu Tyr Ala Ala Trp
    610                 615                 620

Ser Gln Gly Leu Asp Lys Cys Tyr Ala Gly Trp Leu Ala Asp Gly Thr
625                 630                 635                 640

Leu Arg Tyr Pro Ile Ile Thr Pro Arg Pro Ala Cys Gly Gly Asp Lys
                645                 650                 655

Pro Gly Val Arg Thr Val Tyr Leu Tyr Pro Asn Gln Thr Gly Leu Pro
            660                 665                 670

Asp Pro Leu Ser Lys His His Ala Phe Cys Phe Arg Gly Val Ser Val
        675                 680                 685

Ala Pro Ser Pro Gly Glu Glu Glu Gly Ser Thr Pro Thr Ser Pro Ser
    690                 695                 700

Asp Ile Glu Asp Trp Ile Val Thr Gln Val Gly Pro Gly Val Asp Ala
705                 710                 715                 720

Val Pro Leu Glu Pro Lys Thr Thr Glu Val Pro Tyr Phe Thr Thr Glu
                725                 730                 735

Pro Arg Lys Gln Thr Glu Trp Glu Pro Ala Tyr Thr Pro Val Gly Thr
            740                 745                 750

Ser Pro Gln Pro Gly Ile Pro Pro Thr Trp Leu Pro Thr Leu Pro Ala
        755                 760                 765

Ala Glu Glu His Thr Glu Ser Pro Ser Ala Ser Glu Glu Pro Ser Ala
    770                 775                 780

Ser Ala Val Pro Ser Thr Ser Glu Glu Pro Tyr Thr Ser Ser Phe Ala
785                 790                 795                 800

Val Pro Ser Met Thr Glu Leu Pro Gly Ser Gly Glu Ala Ser Gly Ala
                805                 810                 815

Pro Asp Leu Ser Gly Asp Phe Thr Gly Ser Gly Asp Ala Ser Gly Arg
            820                 825                 830

Leu Asp Ser Ser Gly Gln Pro Ser Gly Gly Ile Glu Ser Gly Leu Pro
        835                 840                 845

Ser Gly Asp Leu Asp Ser Ser Gly Leu Ser Pro Thr Val Ser Ser Gly
    850                 855                 860

Leu Pro Val Glu Ser Gly Ser Ala Ser Gly Asp Gly Glu Val Pro Trp
865                 870                 875                 880

Ser His Thr Pro Thr Val Gly Arg Leu Pro Ser Gly Gly Glu Ser Pro
                885                 890                 895

Glu Gly Ser Ala Ser Ala Ser Gly Thr Gly Asp Leu Ser Gly Leu Pro
            900                 905                 910

Ser Gly Gly Glu Ile Thr Glu Thr Ser Thr Ser Gly Ala Glu Glu Thr
        915                 920                 925

Ser Gly Leu Pro Ser Gly Gly Asp Gly Leu Glu Thr Ser Thr Ser Gly
    930                 935                 940

Val Asp Asp Val Ser Gly Ile Pro Thr Gly Arg Glu Gly Leu Glu Thr
945                 950                 955                 960

Ser Ala Ser Gly Val Glu Asp Leu Ser Gly Leu Pro Ser Gly Glu Glu
                965                 970                 975

Gly Ser Glu Thr Ser Thr Ser Gly Ile Glu Asp Ile Ser Val Leu Pro
            980                 985                 990

Thr Gly Gly Glu Ser Leu Glu Thr Ser Ala Ser Gly Val Gly Asp Leu
        995                 1000                1005

Ser Gly Leu Pro Ser Gly Gly Glu Ser Leu Glu Thr Ser Ala Ser
    1010                1015                1020

Gly Ala Glu Asp Val Thr Gln Leu Pro Thr Glu Arg Gly Gly Leu
    1025                1030                1035

Glu Thr Ser Ala Ser Gly Val Glu Asp Ile Thr Val Leu Pro Thr
    1040                1045                1050

Gly Arg Glu Ser Leu Glu Thr Ser Ala Ser Gly Val Glu Asp Val
    1055                1060                1065

Ser Gly Leu Pro Ser Gly Arg Glu Gly Leu Glu Thr Ser Ala Ser
    1070                1075                1080

Gly Ile Glu Asp Ile Ser Val Phe Pro Thr Glu Ala Glu Gly Leu
    1085                1090                1095

Asp Thr Ser Ala Ser Gly Gly Tyr Val Ser Gly Ile Pro Ser Gly
    1100                1105                1110

Gly Asp Gly Thr Glu Thr Ser Ala Ser Gly Val Glu Asp Val Ser
    1115                1120                1125

Gly Leu Pro Ser Gly Gly Glu Gly Leu Glu Thr Ser Ala Ser Gly
    1130                1135                1140

Val Glu Asp Leu Gly Pro Ser Thr Arg Asp Ser Leu Glu Thr Ser
    1145                1150                1155

Ala Ser Gly Val Asp Val Thr Gly Phe Pro Ser Gly Arg Gly Asp
    1160                1165                1170

Pro Glu Thr Ser Val Ser Gly Val Gly Asp Asp Phe Ser Gly Leu
    1175                1180                1185

Pro Ser Gly Lys Glu Gly Leu Glu Thr Ser Ala Ser Gly Ala Glu
    1190                1195                1200

Asp Leu Ser Gly Leu Pro Ser Gly Lys Glu Asp Leu Val Gly Ser
    1205                1210                1215

Ala Ser Gly Ala Leu Asp Phe Gly Lys Leu Pro Pro Gly Thr Leu
    1220                1225                1230

Gly Ser Gly Gln Thr Pro Glu Val Asn Gly Phe Pro Ser Gly Phe
    1235                1240                1245

Ser Gly Glu Tyr Ser Gly Ala Asp Ile Gly Ser Gly Pro Ser Ser
    1250                1255                1260

Gly Leu Pro Asp Phe Ser Gly Leu Pro Ser Gly Phe Pro Thr Val
    1265                1270                1275

Ser Leu Val Asp Ser Thr Leu Val Glu Val Ile Thr Ala Thr Thr
    1280                1285                1290

Ser Ser Glu Leu Glu Gly Arg Gly Thr Ile Gly Ile Ser Gly Ser
    1295                1300                1305

Gly Glu Val Ser Gly Leu Pro Leu Gly Glu Leu Asp Ser Ser Ala
    1310                1315                1320

Asp Ile Ser Gly Leu Pro Ser Gly Thr Glu Leu Ser Gly Gln Ala

1325                    1330                    1335

Ser Gly Ser Pro Asp Ser Ser Gly Glu Thr Ser Gly Phe Phe Asp
    1340                    1345                    1350

Val Ser Gly Gln Pro Phe Gly Ser Ser Gly Val Ser Glu Glu Thr
    1355                    1360                    1365

Ser Gly Ile Pro Glu Ile Ser Gly Gln Pro Ser Gly Thr Pro Asp
    1370                    1375                    1380

Thr Thr Ala Thr Ser Gly Val Thr Glu Leu Asn Glu Leu Ser Ser
    1385                    1390                    1395

Gly Gln Pro Asp Val Ser Gly Asp Gly Ser Gly Ile Leu Phe Gly
    1400                    1405                    1410

Ser Gly Gln Ser Ser Gly Ile Thr Ser Val Ser Gly Glu Thr Ser
    1415                    1420                    1425

Gly Ile Ser Asp Leu Ser Gly Gln Pro Ser Gly Phe Pro Val Phe
    1430                    1435                    1440

Ser Gly Thr Ala Thr Arg Thr Pro Asp Leu Ala Ser Gly Thr Ile
    1445                    1450                    1455

Ser Gly Ser Gly Glu Ser Ser Gly Ile Thr Phe Val Asp Thr Ser
    1460                    1465                    1470

Phe Val Glu Val Thr Pro Thr Thr Phe Arg Glu Glu Glu Gly Leu
    1475                    1480                    1485

Gly Ser Val Glu Leu Ser Gly Phe Pro Ser Gly Glu Thr Glu Leu
    1490                    1495                    1500

Ser Gly Thr Ser Gly Thr Val Asp Val Ser Glu Gln Ser Ser Gly
    1505                    1510                    1515

Ala Ile Asp Ser Ser Gly Leu Thr Ser Pro Thr Pro Glu Phe Ser
    1520                    1525                    1530

Gly Leu Pro Ser Gly Val Ala Glu Val Ser Gly Glu Phe Ser Gly
    1535                    1540                    1545

Val Glu Thr Gly Ser Ser Leu Pro Ser Gly Ala Phe Asp Gly Ser
    1550                    1555                    1560

Gly Leu Val Ser Gly Phe Pro Thr Val Ser Leu Val Asp Arg Thr
    1565                    1570                    1575

Leu Val Glu Ser Ile Thr Gln Ala Pro Thr Ala Gln Glu Ala Gly
    1580                    1585                    1590

Glu Gly Pro Ser Gly Ile Leu Glu Phe Ser Gly Ala His Ser Gly
    1595                1600                1605

Thr Pro Asp Ile Ser Gly Glu Leu Ser Gly Ser Leu Asp Leu Ser
    1610                1615                1620

Thr Leu Gln Ser Gly Gln Met Glu Thr Ser Thr Glu Thr Pro Ser
    1625                1630                1635

Ser Pro Tyr Phe Ser Gly Asp Phe Ser Ser Thr Thr Asp Val Ser
    1640                1645                1650

Gly Glu Ser Ile Ala Ala Thr Thr Gly Ser Gly Glu Ser Ser Gly
    1655                1660                1665

Leu Pro Glu Val Thr Leu Asn Thr Ser Glu Leu Val Glu Gly Val
    1670                1675                1680

Thr Glu Pro Thr Val Ser Gln Glu Leu Gly His Gly Pro Ser Met
    1685                1690                1695

Thr Tyr Ile Ser Arg Leu Ser Glu Ala Ser Gly Asp Ala Ser Ala
    1700                1705                1710

Ser Gly Asp Leu Gly Gly Ala Val Thr Asn Phe Pro Gly Ser Gly
    1715                1720                1725

Val Glu Ala Ser Val Pro Glu Ala Ser Ser Asp Leu Ser Ala Tyr
    1730                1735                1740

Pro Glu Ala Gly Val Gly Val Ser Ala Ala Pro Glu Ala Ser Ser
    1745                1750                1755

Lys Leu Ser Glu Phe Pro Asp Leu His Gly Ile Thr Ser Ala Phe
    1760                1765                1770

His Glu Thr Asp Leu Glu Met Thr Thr Pro Ser Thr Glu Val Asn
    1775                1780                1785

Ser Asn Pro Trp Thr Phe Gln Glu Gly Thr Arg Glu Gly Ser Ala
    1790                1795                1800

Ala Pro Glu Val Ser Gly Glu Ser Ser Thr Thr Ser Asp Ile Asp
    1805                1810                1815

Thr Gly Thr Ser Gly Val Pro Ser Ala Thr Pro Met Ala Ser Gly
    1820                1825                1830

Asp Arg Thr Glu Ile Ser Gly Glu Trp Ser Asp His Thr Ser Glu
    1835                1840                1845

Val Asn Val Ala Ile Ser Ser Thr Ile Thr Glu Ser Glu Trp Ala
    1850                    1855                    1860

Gln Pro Thr Arg Tyr Pro Thr Glu Thr Leu Gln Glu Ile Glu Ser
    1865                    1870                    1875

Pro Asn Pro Ser Tyr Ser Gly Glu Glu Thr Gln Thr Ala Glu Thr
    1880                    1885                    1890

Thr Met Ser Leu Thr Asp Ala Pro Thr Leu Ser Ser Ser Glu Gly
    1895                    1900                    1905

Ser Gly Glu Thr Glu Ser Thr Val Ala Asp Gln Glu Gln Cys Glu
    1910                    1915                    1920

Glu Gly Trp Thr Lys Phe Gln Gly His Cys Tyr Arg His Phe Pro
    1925                    1930                    1935

Asp Arg Glu Thr Trp Val Asp Ala Glu Arg Arg Cys Arg Glu Gln
    1940                    1945                    1950

Gln Ser His Leu Ser Ser Ile Val Thr Pro Glu Glu Gln Glu Phe
    1955                    1960                    1965

Val Asn Lys Asn Ala Gln Asp Tyr Gln Trp Ile Gly Leu Asn Asp
    1970                    1975                    1980

Arg Thr Ile Glu Gly Asp Phe Arg Trp Ser Asp Gly His Ser Leu
    1985                    1990                    1995

Gln Phe Glu Lys Trp Arg Pro Asn Gln Pro Asp Asn Phe Phe Ala
    2000                    2005                    2010

Thr Gly Glu Asp Cys Val Val Met Ile Trp His Glu Arg Gly Glu
    2015                    2020                    2025

Trp Asn Asp Val Pro Cys Asn Tyr Gln Leu Pro Phe Thr Cys Lys
    2030                    2035                    2040

Lys Gly Thr Val Ala Cys Gly Asp Pro Pro Val Val Glu His Ala
    2045                    2050                    2055

Arg Thr Leu Gly Gln Lys Lys Asp Arg Tyr Glu Ile Ser Ser Leu
    2060                    2065                    2070

Val Arg Tyr Gln Cys Thr Glu Gly Phe Val Gln Arg His Val Pro
    2075                    2080                    2085

Thr Ile Arg Cys Gln Pro Ser Gly His Trp Glu Glu Pro Arg Ile
    2090                    2095                    2100

Thr Cys Thr Asp Pro Asn Thr Tyr Lys His Arg Leu Gln Lys Arg
    2105                2110                2115

Thr Met Arg Pro Thr Arg Arg Ser Arg Pro Ser Met Ala His
    2120                2125                2130

<210>  3
<211>  2291
<212>  DNA
<213>  Mus musculus

<220>
<221>  CDS
<222>  (257)..(1363)

<400>  3
aagaggtaat ccagaactca aggagcgcgg gcgcagcagc tgtgagcatt aggtgctgtg      60

gccaagcgct gggacaagga tctgccgcta ccagcgcatc ctctctcctc tccagctctg     120

tcccgcactc gctgcaccct tccccaggcc accgcgcttc ctatgtgatc ttccggggca     180

acgcggagcc ctttctcaca gctcagcagt gaatttccac cccgaaaact gcagtaagcc     240

gcctttcaag gacaag atg ttg ata aat atg aaa ggc atc tta tgg atg tgc     292
                  Met Leu Ile Asn Met Lys Gly Ile Leu Trp Met Cys
                  1               5                   10

tca acc tta tta cta acc cat gca cta cat caa gcc aaa atg gaa acc       340
Ser Thr Leu Leu Leu Thr His Ala Leu His Gln Ala Lys Met Glu Thr
        15                  20                  25

agc cca cct gtt aaa ggc tct ctg tct gga aaa gtg gtc cta cct tgt       388
Ser Pro Pro Val Lys Gly Ser Leu Ser Gly Lys Val Val Leu Pro Cys
    30                  35                  40

cat ctt tca acc tta cct acc tta cca ccc aat tac aac acg agt gaa       436
His Leu Ser Thr Leu Pro Thr Leu Pro Pro Asn Tyr Asn Thr Ser Glu
45                  50                  55                  60

ttt ctc aga atc aaa tgg tct aag atg gaa gtg gac aaa aat gga aaa       484
Phe Leu Arg Ile Lys Trp Ser Lys Met Glu Val Asp Lys Asn Gly Lys
                65                  70                  75

gat ata aag gag acg act gtc ttg gtg gcc cag aac gga aat atc aag       532
Asp Ile Lys Glu Thr Thr Val Leu Val Ala Gln Asn Gly Asn Ile Lys
            80                  85                  90

att ggt cag gac tac aag ggg cga gtg tcc gtg cct aca cat ccc gat       580
Ile Gly Gln Asp Tyr Lys Gly Arg Val Ser Val Pro Thr His Pro Asp
        95                  100                 105

gat gta ggt gat gct tcc ctc acc atg gtc aaa ctc cgg gct agt gat       628
Asp Val Gly Asp Ala Ser Leu Thr Met Val Lys Leu Arg Ala Ser Asp
    110                 115                 120

gca ggc gtc tac cga tgt gat gtc atg tat ggg att gaa gac act cag       676
Ala Gly Val Tyr Arg Cys Asp Val Met Tyr Gly Ile Glu Asp Thr Gln
125                 130                 135                 140

gac acc atg tca ctg gct gtg gat ggt gtt gtg ttt cac tac agg gca       724
Asp Thr Met Ser Leu Ala Val Asp Gly Val Val Phe His Tyr Arg Ala
                145                 150                 155

gcc acc agc agg tac act ctg aac ttt gcc gct gct caa cag gct tgt       772
Ala Thr Ser Arg Tyr Thr Leu Asn Phe Ala Ala Ala Gln Gln Ala Cys

160                 165                 170

ttg gat atc ggg gcg gtc ata gca agc cca gag cag ctg ttt gcc gcc     820
Leu Asp Ile Gly Ala Val Ile Ala Ser Pro Glu Gln Leu Phe Ala Ala
    175                 180                 185

tat gag gat gga ttt gag cag tgt gat gca gga tgg ctg tct gat caa     868
Tyr Glu Asp Gly Phe Glu Gln Cys Asp Ala Gly Trp Leu Ser Asp Gln
    190                 195                 200

act gtc aga tat ccc ata cgg gct ccc cga gag ggc tgt tac gga gac     916
Thr Val Arg Tyr Pro Ile Arg Ala Pro Arg Glu Gly Cys Tyr Gly Asp
205                 210                 215                 220

atg atg ggg aag gaa ggg gtt cgg acc tat gga ttc cgc tct ccc cag     964
Met Met Gly Lys Glu Gly Val Arg Thr Tyr Gly Phe Arg Ser Pro Gln
                225                 230                 235

gaa acc tat gat gtg tat tgt tat gtg gat cat ctg gat ggc gat gtg     1012
Glu Thr Tyr Asp Val Tyr Cys Tyr Val Asp His Leu Asp Gly Asp Val
            240                 245                 250

ttc cac atc act gct ccc agt aag ttc acc ttc gag gag gcc gaa gca     1060
Phe His Ile Thr Ala Pro Ser Lys Phe Thr Phe Glu Glu Ala Glu Ala
    255                 260                 265

gag tgt aca agc agg gat gcg agg ctg gcg act gtt gga gaa ctt cag     1108
Glu Cys Thr Ser Arg Asp Ala Arg Leu Ala Thr Val Gly Glu Leu Gln
    270                 275                 280

gca gct tgg aga aat ggc ttt ggc caa tgc gat tac ggc tgg ctg tcg     1156
Ala Ala Trp Arg Asn Gly Phe Gly Gln Cys Asp Tyr Gly Trp Leu Ser
285                 290                 295                 300

gat gcc agc gtg cgg cac cct gtg act gtg gcc agg gcc cag tgt gga     1204
Asp Ala Ser Val Arg His Pro Val Thr Val Ala Arg Ala Gln Cys Gly
                305                 310                 315

gga ggt cta ctt ggg gtg aga acc ctg tat cgt ttt gag aac cag aca     1252
Gly Gly Leu Leu Gly Val Arg Thr Leu Tyr Arg Phe Glu Asn Gln Thr
            320                 325                 330

tgc ttc cct ctc cct gat agc aga ttt gat gcc tac tgc ttt aaa cgt     1300
Cys Phe Pro Leu Pro Asp Ser Arg Phe Asp Ala Tyr Cys Phe Lys Arg
        335                 340                 345

aag tgt ttg ata tct ctt tta aaa ata cag att aaa aag ctg cag caa     1348
Lys Cys Leu Ile Ser Leu Leu Lys Ile Gln Ile Lys Lys Leu Gln Gln
    350                 355                 360

ata tat acc cac tga ggttccagtg gatgtcggga tggattctgt attctgtact     1403
Ile Tyr Thr His
365

aagtgcatgg aggacctaca gtgcagtgac acaaacagac ttaataccac cattatggtt     1463

aacacagtag ggagtttggc aagtgggatt ttttttggga ggtggggaga taacaacgtc     1523

agagtggggt cttaactgca ccccagaatt ccaaaaatca cagaacacac cctgaattga     1583

aatttcttat taattgaagc ctttcaatgc aatttcactc ttaatataac aatgttaaga     1643

caacagcagc tgagttaaac ctttgaatcc tgttttattt tttgatatga ttcttattat     1703

aaaaaggaga caatttaagc ttaagtcatt gcaggtgagt atttgtgctg aaattaataa     1763

tgattgtgtt caattcctac ctaaaaatgc atcatactat caaataatac gattcacata     1823

tctcattttg tttcaagtaa tagaataggc gccttttggc gattaatctt gctttgaatt     1883

aattaaagta caatacagcc tccttgcatg aattggctat gctcagttag atttgccttg 1943

taaaattaat tttaggtagc attcattaca tatatatata tgtatatata tatattagac 2003

attcttaaat tattttaaaa tattagtcac atacagtttt atagttgtag agtgcttaac 2063

taaactaata actagtcaat attttattgt aatatgtact ttaggatcgg taagatgtta 2123

gctaacaaat gcctccatac tttagcaaca gaggccaagt ctgacatcga gtatcagaac 2183

aatgaaaagc caatgtaaca catgaagtct tttaaaccag ctttctaatt ccttttgtta 2243

gatcaatggg taattaaaat agcttgaaaa tgtaaaaaaa aaaaaaaa 2291

<210> 4
<211> 368
<212> PRT
<213> Mus musculus

<400> 4

Met Leu Ile Asn Met Lys Gly Ile Leu Trp Met Cys Ser Thr Leu Leu
1               5                   10                  15

Leu Thr His Ala Leu His Gln Ala Lys Met Glu Thr Ser Pro Pro Val
            20                  25                  30

Lys Gly Ser Leu Ser Gly Lys Val Val Leu Pro Cys His Leu Ser Thr
        35                  40                  45

Leu Pro Thr Leu Pro Pro Asn Tyr Asn Thr Ser Glu Phe Leu Arg Ile
    50                  55                  60

Lys Trp Ser Lys Met Glu Val Asp Lys Asn Gly Lys Asp Ile Lys Glu
65                  70                  75                  80

Thr Thr Val Leu Val Ala Gln Asn Gly Asn Ile Lys Ile Gly Gln Asp
                85                  90                  95

Tyr Lys Gly Arg Val Ser Val Pro Thr His Pro Asp Asp Val Gly Asp
            100                 105                 110

Ala Ser Leu Thr Met Val Lys Leu Arg Ala Ser Asp Ala Gly Val Tyr
        115                 120                 125

Arg Cys Asp Val Met Tyr Gly Ile Glu Asp Thr Gln Asp Thr Met Ser
    130                 135                 140

Leu Ala Val Asp Gly Val Val Phe His Tyr Arg Ala Ala Thr Ser Arg
145                 150                 155                 160

Tyr Thr Leu Asn Phe Ala Ala Ala Gln Gln Ala Cys Leu Asp Ile Gly
                165                 170                 175

Ala Val Ile Ala Ser Pro Glu Gln Leu Phe Ala Ala Tyr Glu Asp Gly
            180                 185                 190

Phe Glu Gln Cys Asp Ala Gly Trp Leu Ser Asp Gln Thr Val Arg Tyr
        195                 200                 205

Pro Ile Arg Ala Pro Arg Glu Gly Cys Tyr Gly Asp Met Met Gly Lys
    210                 215                 220

Glu Gly Val Arg Thr Tyr Gly Phe Arg Ser Pro Gln Glu Thr Tyr Asp
225                 230                 235                 240

Val Tyr Cys Tyr Val Asp His Leu Asp Gly Asp Val Phe His Ile Thr
                245                 250                 255

Ala Pro Ser Lys Phe Thr Phe Glu Glu Ala Glu Ala Glu Cys Thr Ser
            260                 265                 270

Arg Asp Ala Arg Leu Ala Thr Val Gly Glu Leu Gln Ala Ala Trp Arg
        275                 280                 285

Asn Gly Phe Gly Gln Cys Asp Tyr Gly Trp Leu Ser Asp Ala Ser Val
    290                 295                 300

Arg His Pro Val Thr Val Ala Arg Ala Gln Cys Gly Gly Gly Leu Leu
305                 310                 315                 320

Gly Val Arg Thr Leu Tyr Arg Phe Glu Asn Gln Thr Cys Phe Pro Leu
                325                 330                 335

Pro Asp Ser Arg Phe Asp Ala Tyr Cys Phe Lys Arg Lys Cys Leu Ile
            340                 345                 350

Ser Leu Leu Lys Ile Gln Ile Lys Lys Leu Gln Gln Ile Tyr Thr His
        355                 360                 365

<210>  5
<211>  2583
<212>  DNA
<213>  Mus musculus

<220>
<221>  CDS
<222>  (162)..(2339)

<400>  5
cggcagtggc tggcaagaaa caattctgca aaaataatca tacccagcct ggcaattgtc      60

tgctcctcgg tccattgctc cgccgccgtc cacagtcgct tgcaagggga aggcactgaa     120

tttaccgcgg ccagaacatc cctcccagcc ggcagtttac a atg ctg cga act aag     176
                                              Met Leu Arg Thr Lys
                                              1               5

gat ctc atc tgg act ttg ttt ttc ctg gga act gca gtt tcc ctg cag       224
Asp Leu Ile Trp Thr Leu Phe Phe Leu Gly Thr Ala Val Ser Leu Gln
                10                  15                  20

gta gat att gtt ccc agc caa gga gaa atc agc gtt gga gag tcc aaa 272
Val Asp Ile Val Pro Ser Gln Gly Glu Ile Ser Val Gly Glu Ser Lys
            25                  30                  35

ttc ttc ctg tgt caa gtg gca gga gat gcc aaa gat aag gac atc tcc 320
Phe Phe Leu Cys Gln Val Ala Gly Asp Ala Lys Asp Lys Asp Ile Ser
        40                  45                  50

tgg ttc tcc ccc aat ggg gag aag ctg agc cca aac cag cag cgg atc 368
Trp Phe Ser Pro Asn Gly Glu Lys Leu Ser Pro Asn Gln Gln Arg Ile
    55                  60                  65

tca gtg gtg tgg aat gat gac gac tcc tct acc ctc acc atc tac aac 416
Ser Val Val Trp Asn Asp Asp Asp Ser Ser Thr Leu Thr Ile Tyr Asn
70                  75                  80                  85

gcc aac atc gac gat gcc ggc ata tac aag tgt gtg gtc acc gct gag 464
Ala Asn Ile Asp Asp Ala Gly Ile Tyr Lys Cys Val Val Thr Ala Glu
                90                  95                  100

gac ggc acc cag tct gag gcc act gtc aac gtg aag atc ttc cag aag 512
Asp Gly Thr Gln Ser Glu Ala Thr Val Asn Val Lys Ile Phe Gln Lys
            105                 110                 115

ctc atg ttc aag aat gca cca acc cca cag gag ttt aag gaa ggg gag 560
Leu Met Phe Lys Asn Ala Pro Thr Pro Gln Glu Phe Lys Glu Gly Glu
        120                 125                 130

gat gct gtg att gtc tgt gat gtg gtc agc tcc ctg cct cca acc atc 608
Asp Ala Val Ile Val Cys Asp Val Val Ser Ser Leu Pro Pro Thr Ile
    135                 140                 145

atc tgg aaa cac aaa ggc cga gat gtc att ctg aaa aaa gac gtc cgg 656
Ile Trp Lys His Lys Gly Arg Asp Val Ile Leu Lys Lys Asp Val Arg
150                 155                 160                 165

ttc ata gtc ctg tcc aac aac tac ctg cag atc agg ggc atc aag aaa 704
Phe Ile Val Leu Ser Asn Asn Tyr Leu Gln Ile Arg Gly Ile Lys Lys
                170                 175                 180

aca gat gag ggt act tac cgc tgt gag ggc agg atc ctg gcc cgc ggg 752
Thr Asp Glu Gly Thr Tyr Arg Cys Glu Gly Arg Ile Leu Ala Arg Gly
            185                 190                 195

gaa atc aac ttc aag gac att cag gtc att gtg aat gta cca ccc act 800
Glu Ile Asn Phe Lys Asp Ile Gln Val Ile Val Asn Val Pro Pro Thr
        200                 205                 210

gtc cag gcc aga cag agc atc gtg aat gcc act gcc aac ctg ggc cag 848
Val Gln Ala Arg Gln Ser Ile Val Asn Ala Thr Ala Asn Leu Gly Gln
    215                 220                 225

tct gtc acc ctg gtg tgt gat gct gat gga ttt cca gag ccc acc atg 896
Ser Val Thr Leu Val Cys Asp Ala Asp Gly Phe Pro Glu Pro Thr Met
230                 235                 240                 245

agc tgg aca aag gat ggg gaa ccc ata gag aac gag gag gaa gat gag 944
Ser Trp Thr Lys Asp Gly Glu Pro Ile Glu Asn Glu Glu Glu Asp Glu
                250                 255                 260

aga agc aga tct tca gtg agt gac agc tcc gag gtg acc atc agg aat 992
Arg Ser Arg Ser Ser Val Ser Asp Ser Ser Glu Val Thr Ile Arg Asn
            265                 270                 275

gtg gat aaa aac gac gag gcc gaa tac gtc tgc atc gca gag aac aag 1040
Val Asp Lys Asn Asp Glu Ala Glu Tyr Val Cys Ile Ala Glu Asn Lys
        280                 285                 290

gct ggc gag cag gat gcc tcc atc cac ctc aag gtc ttt gca aag ccc 1088

Ala Gly Glu Gln Asp Ala Ser Ile His Leu Lys Val Phe Ala Lys Pro
    295                 300                 305

aaa atc acc tat gta gag aat cag acg gcc atg gaa cta gag gag caa      1136
Lys Ile Thr Tyr Val Glu Asn Gln Thr Ala Met Glu Leu Glu Glu Gln
310                 315                 320                 325

gtc act ctg acc tgt gaa gcc tcc gga gac ccc atc ccg tcc atc acg      1184
Val Thr Leu Thr Cys Glu Ala Ser Gly Asp Pro Ile Pro Ser Ile Thr
                330                 335                 340

tgg aga aca tcc acc cgg aac atc agc agt gaa gaa cag gat ctg gat      1232
Trp Arg Thr Ser Thr Arg Asn Ile Ser Ser Glu Glu Gln Asp Leu Asp
            345                 350                 355

ggg cac atg gtg gtt cgc agc cat gct cgt gtg tcc tcc ttg acc ctg      1280
Gly His Met Val Val Arg Ser His Ala Arg Val Ser Ser Leu Thr Leu
        360                 365                 370

aag agc atc cag tac aga gat gct gga gaa tac atg tgc act gcc agc      1328
Lys Ser Ile Gln Tyr Arg Asp Ala Gly Glu Tyr Met Cys Thr Ala Ser
    375                 380                 385

aac acc atc ggc cag gac tcc cag tcc atc gac ctc gaa ttt caa tat      1376
Asn Thr Ile Gly Gln Asp Ser Gln Ser Ile Asp Leu Glu Phe Gln Tyr
390                 395                 400                 405

gct ccc aag ctc cag ggc cct gtg gct gtc tac acc tgg gaa ggg aac      1424
Ala Pro Lys Leu Gln Gly Pro Val Ala Val Tyr Thr Trp Glu Gly Asn
                410                 415                 420

caa gtg aac atc acc tgt gag gtc ttt gcc tat ccc agt gcc aca atc      1472
Gln Val Asn Ile Thr Cys Glu Val Phe Ala Tyr Pro Ser Ala Thr Ile
            425                 430                 435

tcc tgg ttc cga gat ggt cag ttg ctg cca agc tcc aac tac agc aat      1520
Ser Trp Phe Arg Asp Gly Gln Leu Leu Pro Ser Ser Asn Tyr Ser Asn
        440                 445                 450

atc aag atc tac aac acc cca tct gct agc tat ctg gag gtg acc cca      1568
Ile Lys Ile Tyr Asn Thr Pro Ser Ala Ser Tyr Leu Glu Val Thr Pro
    455                 460                 465

gat tca gaa aat gac ttt gga aac tac aac tgt aca gca gtg aac cgt      1616
Asp Ser Glu Asn Asp Phe Gly Asn Tyr Asn Cys Thr Ala Val Asn Arg
470                 475                 480                 485

att gga cag gag tcc ttg gaa ttc atc ctt gtt caa gca gac aca ccg      1664
Ile Gly Gln Glu Ser Leu Glu Phe Ile Leu Val Gln Ala Asp Thr Pro
                490                 495                 500

tct tct cca tcc att gac cgg gtg gaa cca tac tcc agc aca gcg cag      1712
Ser Ser Pro Ser Ile Asp Arg Val Glu Pro Tyr Ser Ser Thr Ala Gln
            505                 510                 515

gtg cag ttt gat gag cca gag gcc aca ggg ggc gtg ccc att ctc aag      1760
Val Gln Phe Asp Glu Pro Glu Ala Thr Gly Gly Val Pro Ile Leu Lys
        520                 525                 530

tac aag gct gag tgg aag tcg ctg ggc gaa gaa tcc tgg cat ttc acg      1808
Tyr Lys Ala Glu Trp Lys Ser Leu Gly Glu Glu Ser Trp His Phe Thr
    535                 540                 545

tgg tat gat gcc aaa gaa gcc aac atg gag ggc att gtc acc atc atg      1856
Trp Tyr Asp Ala Lys Glu Ala Asn Met Glu Gly Ile Val Thr Ile Met
550                 555                 560                 565

ggc ctg aaa cct gag acg acg tac tcg gat cga ctg gcg gcc ctc aac      1904
Gly Leu Lys Pro Glu Thr Thr Tyr Ser Asp Arg Leu Ala Ala Leu Asn

570                    575                    580

ggc aag ggg ctg ggc gag atc atg cag cca tct gag tcc aag aca cag        1952
Gly Lys Gly Leu Gly Glu Ile Met Gln Pro Ser Glu Ser Lys Thr Gln
            585                 590                 595

cca gtt ccg gaa ctc agt gca ccc aag ctg gaa ggg cag atg gga gag        2000
Pro Val Pro Glu Leu Ser Ala Pro Lys Leu Glu Gly Gln Met Gly Glu
        600                 605                 610

gac ggg aac tcc atc aag gtg aac ctg atc aag cag gat gac gga ggc        2048
Asp Gly Asn Ser Ile Lys Val Asn Leu Ile Lys Gln Asp Asp Gly Gly
    615                 620                 625

tcc ccc atc aga cac tat ctg gtc aag tac aga gcg ctc gcc tct gag        2096
Ser Pro Ile Arg His Tyr Leu Val Lys Tyr Arg Ala Leu Ala Ser Glu
630                 635                 640                 645

tgg aaa ccg gaa atc agg ctc cca tcc ggc agt cac cac gtc atg ctc        2144
Trp Lys Pro Glu Ile Arg Leu Pro Ser Gly Ser His His Val Met Leu
                650                 655                 660

aag tcc ctg gac tgg aac gca gag tat gaa gtc tat gtg gta gct gaa        2192
Lys Ser Leu Asp Trp Asn Ala Glu Tyr Glu Val Tyr Val Val Ala Glu
            665                 670                 675

aac cag caa gga aaa tcc aag gca gct cac ttt gtg ttc agg acc tca        2240
Asn Gln Gln Gly Lys Ser Lys Ala Ala His Phe Val Phe Arg Thr Ser
        680                 685                 690

gcc cag ccc acg gcc atc cca gcc acc ctg ggc gga agc tcc acc tcc        2288
Ala Gln Pro Thr Ala Ile Pro Ala Thr Leu Gly Gly Ser Ser Thr Ser
    695                 700                 705

tac acc ttg gtc tca ttg ctt ttc tct gcg gtg act ctt ctt ctg ctc        2336
Tyr Thr Leu Val Ser Leu Leu Phe Ser Ala Val Thr Leu Leu Leu Leu
710                 715                 720                 725

tga tagaaacttg aacatgcatg tgtgcgagct ctcatgtgcg catgcgcaca        2389

cgcacacaca cgcacacgca cacgcacacg cacgcacacg cacacacaca cacacacaga        2449

cacacacacg cacacacaca cgcacatgca cacgcgcaca cacgcacacg cacacgcaca        2509

cgcacgcaca cacacacaca cacacaccat tgtatttgat taaaaagccc agttcctata        2569

aaaaaaaaaa aaaa                                                          2583

<210>  6
<211>  725
<212>  PRT
<213>  Mus musculus

<400>  6

Met Leu Arg Thr Lys Asp Leu Ile Trp Thr Leu Phe Phe Leu Gly Thr
1               5                   10                  15

Ala Val Ser Leu Gln Val Asp Ile Val Pro Ser Gln Gly Glu Ile Ser
            20                  25                  30

Val Gly Glu Ser Lys Phe Phe Leu Cys Gln Val Ala Gly Asp Ala Lys
        35                  40                  45

Asp Lys Asp Ile Ser Trp Phe Ser Pro Asn Gly Glu Lys Leu Ser Pro

50                  55                  60

Asn Gln Gln Arg Ile Ser Val Val Trp Asn Asp Asp Asp Ser Ser Thr
65                  70                  75                  80

Leu Thr Ile Tyr Asn Ala Asn Ile Asp Asp Ala Gly Ile Tyr Lys Cys
                85                  90                  95

Val Val Thr Ala Glu Asp Gly Thr Gln Ser Glu Ala Thr Val Asn Val
            100                 105                 110

Lys Ile Phe Gln Lys Leu Met Phe Lys Asn Ala Pro Thr Pro Gln Glu
        115                 120                 125

Phe Lys Glu Gly Glu Asp Ala Val Ile Val Cys Asp Val Val Ser Ser
    130                 135                 140

Leu Pro Pro Thr Ile Ile Trp Lys His Lys Gly Arg Asp Val Ile Leu
145                 150                 155                 160

Lys Lys Asp Val Arg Phe Ile Val Leu Ser Asn Asn Tyr Leu Gln Ile
                165                 170                 175

Arg Gly Ile Lys Lys Thr Asp Glu Gly Thr Tyr Arg Cys Glu Gly Arg
            180                 185                 190

Ile Leu Ala Arg Gly Glu Ile Asn Phe Lys Asp Ile Gln Val Ile Val
        195                 200                 205

Asn Val Pro Pro Thr Val Gln Ala Arg Gln Ser Ile Val Asn Ala Thr
    210                 215                 220

Ala Asn Leu Gly Gln Ser Val Thr Leu Val Cys Asp Ala Asp Gly Phe
225                 230                 235                 240

Pro Glu Pro Thr Met Ser Trp Thr Lys Asp Gly Glu Pro Ile Glu Asn
                245                 250                 255

Glu Glu Glu Asp Glu Arg Ser Arg Ser Ser Val Ser Asp Ser Ser Glu
            260                 265                 270

Val Thr Ile Arg Asn Val Asp Lys Asn Asp Glu Ala Glu Tyr Val Cys
        275                 280                 285

Ile Ala Glu Asn Lys Ala Gly Glu Gln Asp Ala Ser Ile His Leu Lys
    290                 295                 300

Val Phe Ala Lys Pro Lys Ile Thr Tyr Val Glu Asn Gln Thr Ala Met
305                 310                 315                 320

Glu Leu Glu Glu Gln Val Thr Leu Thr Cys Glu Ala Ser Gly Asp Pro
                325                 330                 335

Ile Pro Ser Ile Thr Trp Arg Thr Ser Thr Arg Asn Ile Ser Ser Glu
            340                 345                 350

Glu Gln Asp Leu Asp Gly His Met Val Val Arg Ser His Ala Arg Val
        355                 360                 365

Ser Ser Leu Thr Leu Lys Ser Ile Gln Tyr Arg Asp Ala Gly Glu Tyr
    370                 375                 380

Met Cys Thr Ala Ser Asn Thr Ile Gly Gln Asp Ser Gln Ser Ile Asp
385                 390                 395                 400

Leu Glu Phe Gln Tyr Ala Pro Lys Leu Gln Gly Pro Val Ala Val Tyr
                405                 410                 415

Thr Trp Glu Gly Asn Gln Val Asn Ile Thr Cys Glu Val Phe Ala Tyr
            420                 425                 430

Pro Ser Ala Thr Ile Ser Trp Phe Arg Asp Gly Gln Leu Leu Pro Ser
        435                 440                 445

Ser Asn Tyr Ser Asn Ile Lys Ile Tyr Asn Thr Pro Ser Ala Ser Tyr
    450                 455                 460

Leu Glu Val Thr Pro Asp Ser Glu Asn Asp Phe Gly Asn Tyr Asn Cys
465                 470                 475                 480

Thr Ala Val Asn Arg Ile Gly Gln Glu Ser Leu Glu Phe Ile Leu Val
                485                 490                 495

Gln Ala Asp Thr Pro Ser Ser Pro Ser Ile Asp Arg Val Glu Pro Tyr
            500                 505                 510

Ser Ser Thr Ala Gln Val Gln Phe Asp Glu Pro Glu Ala Thr Gly Gly
        515                 520                 525

Val Pro Ile Leu Lys Tyr Lys Ala Glu Trp Lys Ser Leu Gly Glu Glu
    530                 535                 540

Ser Trp His Phe Thr Trp Tyr Asp Ala Lys Glu Ala Asn Met Glu Gly
545                 550                 555                 560

Ile Val Thr Ile Met Gly Leu Lys Pro Glu Thr Thr Tyr Ser Asp Arg
                565                 570                 575

Leu Ala Ala Leu Asn Gly Lys Gly Leu Gly Glu Ile Met Gln Pro Ser
            580                 585                 590

Glu Ser Lys Thr Gln Pro Val Pro Glu Leu Ser Ala Pro Lys Leu Glu
        595                 600                 605

Gly Gln Met Gly Glu Asp Gly Asn Ser Ile Lys Val Asn Leu Ile Lys
    610                 615                 620

Gln Asp Asp Gly Gly Ser Pro Ile Arg His Tyr Leu Val Lys Tyr Arg
625                 630                 635                 640

Ala Leu Ala Ser Glu Trp Lys Pro Glu Ile Arg Leu Pro Ser Gly Ser
                645                 650                 655

His His Val Met Leu Lys Ser Leu Asp Trp Asn Ala Glu Tyr Glu Val
            660                 665                 670

Tyr Val Val Ala Glu Asn Gln Gln Gly Lys Ser Lys Ala Ala His Phe
        675                 680                 685

Val Phe Arg Thr Ser Ala Gln Pro Thr Ala Ile Pro Ala Thr Leu Gly
    690                 695                 700

Gly Ser Ser Thr Ser Tyr Thr Leu Val Ser Leu Leu Phe Ser Ala Val
705                 710                 715                 720

Thr Leu Leu Leu Leu
                725

<210>  7
<211>  3153
<212>  DNA
<213>  Mus musculus

<220>
<221>  CDS
<222>  (151)..(2802)

<220>
<221>  misc_feature
<222>  (3043)..(3043)
<223>  n is a, c, g, or t

<220>
<221>  misc_feature
<222>  (3048)..(3048)
<223>  n is a, c, g, or t

<400>  7
gaggctcccg gcgagctggc gcccctgtct gggtcccgcg cgcccggccc tgctcgcgcc      60

cgcgcatcgc gccgcagtct cggtctgcgg ctgcgggacg tgacggcgtg cgcggagggg     120

acctcgcaag ttcttccatc agtgtgcaga atg ata cca ctg ctt ctg tcc ctg      174
                                 Met Ile Pro Leu Leu Leu Ser Leu
                                 1               5

ctg gcc gct ctg gtc ctg acc caa gcc cct gcc gcc ctc gct gat gac      222
Leu Ala Ala Leu Val Leu Thr Gln Ala Pro Ala Ala Leu Ala Asp Asp
    10                  15                  20

ctg aaa gaa gac agc tcg gag gat cga gcc ttc cgc gtg cgc atc ggt      270
Leu Lys Glu Asp Ser Ser Glu Asp Arg Ala Phe Arg Val Arg Ile Gly
25                  30                  35                  40

gcc gcg cag ctg cgg ggc gtg ctg ggc ggt gcc ctg gcc atc cca tgc 318
Ala Ala Gln Leu Arg Gly Val Leu Gly Gly Ala Leu Ala Ile Pro Cys
                45                  50                  55

cac gtc cac cac ctg cgg ccg ccg cgc agc cgc cgg gcc gcg ccg ggt 366
His Val His His Leu Arg Pro Pro Arg Ser Arg Arg Ala Ala Pro Gly
            60                  65                  70

ttt ccc cgg gtc aag tgg acc ttc ctg tcc ggg gac cgg gag gta gag 414
Phe Pro Arg Val Lys Trp Thr Phe Leu Ser Gly Asp Arg Glu Val Glu
        75                  80                  85

gtt ctg gtg gct cgc ggg ctg cgc gtc aag gta aac gaa gcc tac cgg 462
Val Leu Val Ala Arg Gly Leu Arg Val Lys Val Asn Glu Ala Tyr Arg
    90                  95                  100

ttc cgc gtg gcg ctg cct gcc tac ccc gca tcg ctc acg gat gtg tct 510
Phe Arg Val Ala Leu Pro Ala Tyr Pro Ala Ser Leu Thr Asp Val Ser
105                 110                 115                 120

cta gta ttg agc gaa ctg cgg ccc aat gat tcc ggg gtc tat cgc tgc 558
Leu Val Leu Ser Glu Leu Arg Pro Asn Asp Ser Gly Val Tyr Arg Cys
                125                 130                 135

gag gtc cag cac ggt atc gac gac agc agt gat gct gtg gag gtc aag 606
Glu Val Gln His Gly Ile Asp Asp Ser Ser Asp Ala Val Glu Val Lys
            140                 145                 150

gtc aaa ggg gtc gtc ttc ctc tac aga gag ggc tct gcg cgc tat gct 654
Val Lys Gly Val Val Phe Leu Tyr Arg Glu Gly Ser Ala Arg Tyr Ala
        155                 160                 165

ttc tcc ttc gct gga gcc cag gaa gcc tgc gct cgc ata gga gcc cga 702
Phe Ser Phe Ala Gly Ala Gln Glu Ala Cys Ala Arg Ile Gly Ala Arg
    170                 175                 180

atc gcc acc ccg gag cag ctc tat gct gcc tac ctc ggc ggc tat gag 750
Ile Ala Thr Pro Glu Gln Leu Tyr Ala Ala Tyr Leu Gly Gly Tyr Glu
185                 190                 195                 200

cag tgt gat gca ggc tgg ctg tcc gac caa act gtg agg tac ccc atc 798
Gln Cys Asp Ala Gly Trp Leu Ser Asp Gln Thr Val Arg Tyr Pro Ile
                205                 210                 215

cag aac cca cga gag gcc tgc tct gga gac atg gat ggc tat cct ggc 846
Gln Asn Pro Arg Glu Ala Cys Ser Gly Asp Met Asp Gly Tyr Pro Gly
            220                 225                 230

gtg cgg aac tac gga gtg gtg ggt cct gat gat ctc tat gat gtc tac 894
Val Arg Asn Tyr Gly Val Val Gly Pro Asp Asp Leu Tyr Asp Val Tyr
        235                 240                 245

tgt tat gcc gaa gac cta aat gga gaa ctg ttc cta ggc gcc cct ccc 942
Cys Tyr Ala Glu Asp Leu Asn Gly Glu Leu Phe Leu Gly Ala Pro Pro
    250                 255                 260

agc aag ctg aca tgg gag gag gct cgg gac tac tgt ctg gaa cgt ggt 990
Ser Lys Leu Thr Trp Glu Glu Ala Arg Asp Tyr Cys Leu Glu Arg Gly
265                 270                 275                 280

gca cag atc gct agc aca ggc cag ctg tac gca gcc tgg aat ggt ggc 1038
Ala Gln Ile Ala Ser Thr Gly Gln Leu Tyr Ala Ala Trp Asn Gly Gly
                285                 290                 295

ctg gac aga tgt agc cct ggc tgg ctg gct gat ggc agc gtg cgc tat 1086
Leu Asp Arg Cys Ser Pro Gly Trp Leu Ala Asp Gly Ser Val Arg Tyr
            300                 305                 310

ccc atc atc aca ccc agc caa cgc tgt ggg ggc ggc ctg cca gga gtc 1134
Pro Ile Ile Thr Pro Ser Gln Arg Cys Gly Gly Gly Leu Pro Gly Val
        315                 320                 325

aag acc ctc ttc ctc ttt ccc aac cag act ggc ttc ccc agc aag cag 1182
Lys Thr Leu Phe Leu Phe Pro Asn Gln Thr Gly Phe Pro Ser Lys Gln
    330                 335                 340

aac cgc ttc aat gtc tac tgc ttc cga gac tct gcc cat ccc tct gct 1230
Asn Arg Phe Asn Val Tyr Cys Phe Arg Asp Ser Ala His Pro Ser Ala
345                 350                 355                 360

tcc tct gag gcc tct agc cca gcc tca gat gga ctt gag gcc att gtc 1278
Ser Ser Glu Ala Ser Ser Pro Ala Ser Asp Gly Leu Glu Ala Ile Val
                365                 370                 375

aca gtg aca gaa aag ctg gag gaa ctg cag ctg cct cag gaa gcg atg 1326
Thr Val Thr Glu Lys Leu Glu Glu Leu Gln Leu Pro Gln Glu Ala Met
            380                 385                 390

gag agc gag tct cgt ggg gcc atc tac tcc atc ccc atc tca gaa gat 1374
Glu Ser Glu Ser Arg Gly Ala Ile Tyr Ser Ile Pro Ile Ser Glu Asp
        395                 400                 405

ggg gga gga gga agc tcc acc cca gaa gac cca gca gag gcc ccc agg 1422
Gly Gly Gly Gly Ser Ser Thr Pro Glu Asp Pro Ala Glu Ala Pro Arg
    410                 415                 420

act ccg cta gaa tcg gaa acc caa tcc att gca cca cct acc gag tcc 1470
Thr Pro Leu Glu Ser Glu Thr Gln Ser Ile Ala Pro Pro Thr Glu Ser
425                 430                 435                 440

tca gaa gag gaa ggc gta gcc ctg gag gaa gaa gaa aga ttc aaa gac 1518
Ser Glu Glu Glu Gly Val Ala Leu Glu Glu Glu Glu Arg Phe Lys Asp
                445                 450                 455

ttg gag gct ctg gag gaa gag aag gag cag gag gac ctg tgg gtg tgg 1566
Leu Glu Ala Leu Glu Glu Glu Lys Glu Gln Glu Asp Leu Trp Val Trp
            460                 465                 470

ccc aga gag ctc agc agc cct ctc cct act ggc tca gaa aca gag cat 1614
Pro Arg Glu Leu Ser Ser Pro Leu Pro Thr Gly Ser Glu Thr Glu His
        475                 480                 485

tca ctc tcc cag gtg tcc cca cca gcc cag gca gtt cta cag ctg gat 1662
Ser Leu Ser Gln Val Ser Pro Pro Ala Gln Ala Val Leu Gln Leu Asp
    490                 495                 500

gcg tca cct tct cct ggg cct cca agg ttc cgt gga ccg cct gca gag 1710
Ala Ser Pro Ser Pro Gly Pro Pro Arg Phe Arg Gly Pro Pro Ala Glu
505                 510                 515                 520

act ttg ctc ccc ccg agg gag tgg agc gcc aca tct act cct ggt ggg 1758
Thr Leu Leu Pro Pro Arg Glu Trp Ser Ala Thr Ser Thr Pro Gly Gly
                525                 530                 535

gca aga gaa gta ggg ggg gaa act ggg agc cct gag ctc tct ggg gtt 1806
Ala Arg Glu Val Gly Gly Glu Thr Gly Ser Pro Glu Leu Ser Gly Val
            540                 545                 550

cct cga gag agc gag gag gca ggg agc tcc agc ttg gag gat ggc cct 1854
Pro Arg Glu Ser Glu Glu Ala Gly Ser Ser Ser Leu Glu Asp Gly Pro
        555                 560                 565

tcc cta ctt cca gct aca tgg gcc cct gtg ggt ccc agg gag ctg gag 1902
Ser Leu Leu Pro Ala Thr Trp Ala Pro Val Gly Pro Arg Glu Leu Glu
    570                 575                 580

acc ccc tca gaa gag aag tct gga aga act gtc ctg gca ggc acc tca 1950

Thr Pro Ser Glu Glu Lys Ser Gly Arg Thr Val Leu Ala Gly Thr Ser
585                 590                 595                 600

gtg cag gcc cag cca gtg ctg ccc acc gac agt gcc agc cac ggt gga      1998
Val Gln Ala Gln Pro Val Leu Pro Thr Asp Ser Ala Ser His Gly Gly
                605                 610                 615

gtg gct gtg gct ccc tca tca ggt gac tgt atc ccc agc ccc tgc cac      2046
Val Ala Val Ala Pro Ser Ser Gly Asp Cys Ile Pro Ser Pro Cys His
            620                 625                 630

aat ggt ggg aca tgc ttg gag gag aag gag ggt ttc cgc tgc cta tgt      2094
Asn Gly Gly Thr Cys Leu Glu Glu Lys Glu Gly Phe Arg Cys Leu Cys
        635                 640                 645

ttg cca ggc tat ggg ggg gac ctg tgc gat gtt ggc ctt cat ttc tgc      2142
Leu Pro Gly Tyr Gly Gly Asp Leu Cys Asp Val Gly Leu His Phe Cys
    650                 655                 660

agc cct ggc tgg gag gcc ttc cag gga gcc tgc tac aag cac ttt tcc      2190
Ser Pro Gly Trp Glu Ala Phe Gln Gly Ala Cys Tyr Lys His Phe Ser
665                 670                 675                 680

aca cga agg agt tgg gag gag gca gaa agt cag tgc cga gcg cta ggt      2238
Thr Arg Arg Ser Trp Glu Glu Ala Glu Ser Gln Cys Arg Ala Leu Gly
                685                 690                 695

gct cat ctg acc agc atc tgc acc cct gag gag caa gac ttt gtc aat      2286
Ala His Leu Thr Ser Ile Cys Thr Pro Glu Glu Gln Asp Phe Val Asn
            700                 705                 710

gat cga tac cgg gag tac cag tgg att ggg ctc aat gac agg acc atc      2334
Asp Arg Tyr Arg Glu Tyr Gln Trp Ile Gly Leu Asn Asp Arg Thr Ile
        715                 720                 725

gag ggt gac ttc ttg tgg tca gat ggt gcc cct ctg ctc tat gaa aac      2382
Glu Gly Asp Phe Leu Trp Ser Asp Gly Ala Pro Leu Leu Tyr Glu Asn
    730                 735                 740

tgg aac cct ggg cag cct gac agc tac ttc ctg tct ggg gag aac tgt      2430
Trp Asn Pro Gly Gln Pro Asp Ser Tyr Phe Leu Ser Gly Glu Asn Cys
745                 750                 755                 760

gtg gtc atg gtg tgg cat gac cag gga cag tgg agt gat gtg ccc tgc      2478
Val Val Met Val Trp His Asp Gln Gly Gln Trp Ser Asp Val Pro Cys
                765                 770                 775

aac tac cat cta tcc tac acc tgc aag atg ggg ctt gtg tcc tgt ggg      2526
Asn Tyr His Leu Ser Tyr Thr Cys Lys Met Gly Leu Val Ser Cys Gly
            780                 785                 790

cct cca cca cag cta ccc ctg gct caa ata ttt ggt cgc cct cgg ctg      2574
Pro Pro Pro Gln Leu Pro Leu Ala Gln Ile Phe Gly Arg Pro Arg Leu
        795                 800                 805

cgc tac gcg gtg gat act gtg ctt cga tat cga tgc cga gac ggg ctg      2622
Arg Tyr Ala Val Asp Thr Val Leu Arg Tyr Arg Cys Arg Asp Gly Leu
    810                 815                 820

gct cag cgc aac ctg ccg ttg atc cgc tgc cag gag aat ggg ctt tgg      2670
Ala Gln Arg Asn Leu Pro Leu Ile Arg Cys Gln Glu Asn Gly Leu Trp
825                 830                 835                 840

gag gcc cct cag att tcc tgt gta ccc cgg agg cct ggc cgt gct ctg      2718
Glu Ala Pro Gln Ile Ser Cys Val Pro Arg Arg Pro Gly Arg Ala Leu
                845                 850                 855

cgc tcc atg gac gcc cca gaa gga cca cgg gga cag ctc tcg agg cac      2766
Arg Ser Met Asp Ala Pro Glu Gly Pro Arg Gly Gln Leu Ser Arg His

860                    865                    870

agg aag gca ccg ttg aca ccg ccc tcc agt ctc tag ggagcctgga          2812
Arg Lys Ala Pro Leu Thr Pro Pro Ser Ser Leu
        875                    880

agactgctgc ccccagcagg accctctcac atcaactgcc agtgctcttc cccatgatag   2872

ggggtgacgt gagaggggtg ggactgaaat tcagaggaca gcgctcgaag gggtttctgg    2932

gaaacacttg ggtggctccg ccccctcaca caagggcctc aggttttacc cggtaagtcc   2992

ctaagtgcct caactgccct ctcatgtcag ctgcctcctt gtccctcgat ntcgtnaggg   3052

gacactgtgc tattcgatct tgattgtcga agagttttta ggatggagta ccagcaaaac   3112

caggtggaaa taaagttgtc tgaacccaaa gaaaaaaaaa a                       3153

<210>  8
<211>  883
<212>  PRT
<213>  Mus musculus

<400>  8

Met Ile Pro Leu Leu Leu Ser Leu Leu Ala Ala Leu Val Leu Thr Gln
1               5                   10                  15

Ala Pro Ala Ala Leu Ala Asp Asp Leu Lys Glu Asp Ser Ser Glu Asp
            20                  25                  30

Arg Ala Phe Arg Val Arg Ile Gly Ala Ala Gln Leu Arg Gly Val Leu
        35                  40                  45

Gly Gly Ala Leu Ala Ile Pro Cys His Val His His Leu Arg Pro Pro
    50                  55                  60

Arg Ser Arg Arg Ala Ala Pro Gly Phe Pro Arg Val Lys Trp Thr Phe
65                  70                  75                  80

Leu Ser Gly Asp Arg Glu Val Glu Val Leu Val Ala Arg Gly Leu Arg
                85                  90                  95

Val Lys Val Asn Glu Ala Tyr Arg Phe Arg Val Ala Leu Pro Ala Tyr
            100                 105                 110

Pro Ala Ser Leu Thr Asp Val Ser Leu Val Leu Ser Glu Leu Arg Pro
        115                 120                 125

Asn Asp Ser Gly Val Tyr Arg Cys Glu Val Gln His Gly Ile Asp Asp
    130                 135                 140

Ser Ser Asp Ala Val Glu Val Lys Val Lys Gly Val Val Phe Leu Tyr
145                 150                 155                 160

Arg Glu Gly Ser Ala Arg Tyr Ala Phe Ser Phe Ala Gly Ala Gln Glu
                165                 170                 175

Ala Cys Ala Arg Ile Gly Ala Arg Ile Ala Thr Pro Glu Gln Leu Tyr
            180                 185                 190

Ala Ala Tyr Leu Gly Gly Tyr Glu Gln Cys Asp Ala Gly Trp Leu Ser
        195                 200                 205

Asp Gln Thr Val Arg Tyr Pro Ile Gln Asn Pro Arg Glu Ala Cys Ser
    210                 215                 220

Gly Asp Met Asp Gly Tyr Pro Gly Val Arg Asn Tyr Gly Val Val Gly
225                 230                 235                 240

Pro Asp Asp Leu Tyr Asp Val Tyr Cys Tyr Ala Glu Asp Leu Asn Gly
                245                 250                 255

Glu Leu Phe Leu Gly Ala Pro Pro Ser Lys Leu Thr Trp Glu Glu Ala
            260                 265                 270

Arg Asp Tyr Cys Leu Glu Arg Gly Ala Gln Ile Ala Ser Thr Gly Gln
        275                 280                 285

Leu Tyr Ala Ala Trp Asn Gly Gly Leu Asp Arg Cys Ser Pro Gly Trp
    290                 295                 300

Leu Ala Asp Gly Ser Val Arg Tyr Pro Ile Ile Thr Pro Ser Gln Arg
305                 310                 315                 320

Cys Gly Gly Gly Leu Pro Gly Val Lys Thr Leu Phe Leu Phe Pro Asn
                325                 330                 335

Gln Thr Gly Phe Pro Ser Lys Gln Asn Arg Phe Asn Val Tyr Cys Phe
            340                 345                 350

Arg Asp Ser Ala His Pro Ser Ala Ser Ser Glu Ala Ser Ser Pro Ala
        355                 360                 365

Ser Asp Gly Leu Glu Ala Ile Val Thr Val Thr Glu Lys Leu Glu Glu
    370                 375                 380

Leu Gln Leu Pro Gln Glu Ala Met Glu Ser Glu Ser Arg Gly Ala Ile
385                 390                 395                 400

Tyr Ser Ile Pro Ile Ser Glu Asp Gly Gly Gly Gly Ser Ser Thr Pro
                405                 410                 415

Glu Asp Pro Ala Glu Ala Pro Arg Thr Pro Leu Glu Ser Glu Thr Gln
            420                 425                 430

Ser Ile Ala Pro Pro Thr Glu Ser Ser Glu Glu Glu Gly Val Ala Leu
        435                 440                 445

Glu Glu Glu Glu Arg Phe Lys Asp Leu Glu Ala Leu Glu Glu Glu Lys
    450                 455                 460

Glu Gln Glu Asp Leu Trp Val Trp Pro Arg Glu Leu Ser Ser Pro Leu
465                 470                 475                 480

Pro Thr Gly Ser Glu Thr Glu His Ser Leu Ser Gln Val Ser Pro Pro
                485                 490                 495

Ala Gln Ala Val Leu Gln Leu Asp Ala Ser Pro Ser Pro Gly Pro Pro
            500                 505                 510

Arg Phe Arg Gly Pro Pro Ala Glu Thr Leu Leu Pro Pro Arg Glu Trp
        515                 520                 525

Ser Ala Thr Ser Thr Pro Gly Gly Ala Arg Glu Val Gly Gly Glu Thr
    530                 535                 540

Gly Ser Pro Glu Leu Ser Gly Val Pro Arg Glu Ser Glu Glu Ala Gly
545                 550                 555                 560

Ser Ser Ser Leu Glu Asp Gly Pro Ser Leu Leu Pro Ala Thr Trp Ala
                565                 570                 575

Pro Val Gly Pro Arg Glu Leu Glu Thr Pro Ser Glu Glu Lys Ser Gly
            580                 585                 590

Arg Thr Val Leu Ala Gly Thr Ser Val Gln Ala Gln Pro Val Leu Pro
        595                 600                 605

Thr Asp Ser Ala Ser His Gly Gly Val Ala Val Ala Pro Ser Ser Gly
    610                 615                 620

Asp Cys Ile Pro Ser Pro Cys His Asn Gly Gly Thr Cys Leu Glu Glu
625                 630                 635                 640

Lys Glu Gly Phe Arg Cys Leu Cys Leu Pro Gly Tyr Gly Gly Asp Leu
                645                 650                 655

Cys Asp Val Gly Leu His Phe Cys Ser Pro Gly Trp Glu Ala Phe Gln
            660                 665                 670

Gly Ala Cys Tyr Lys His Phe Ser Thr Arg Arg Ser Trp Glu Glu Ala
        675                 680                 685

Glu Ser Gln Cys Arg Ala Leu Gly Ala His Leu Thr Ser Ile Cys Thr
    690                 695                 700

Pro Glu Glu Gln Asp Phe Val Asn Asp Arg Tyr Arg Glu Tyr Gln Trp
705                 710                 715                 720

Ile Gly Leu Asn Asp Arg Thr Ile Glu Gly Asp Phe Leu Trp Ser Asp

                725                 730                 735

Gly Ala Pro Leu Leu Tyr Glu Asn Trp Asn Pro Gly Gln Pro Asp Ser
            740                 745                 750

Tyr Phe Leu Ser Gly Glu Asn Cys Val Val Met Val Trp His Asp Gln
        755                 760                 765

Gly Gln Trp Ser Asp Val Pro Cys Asn Tyr His Leu Ser Tyr Thr Cys
    770                 775                 780

Lys Met Gly Leu Val Ser Cys Gly Pro Pro Pro Gln Leu Pro Leu Ala
785                 790                 795                 800

Gln Ile Phe Gly Arg Pro Arg Leu Arg Tyr Ala Val Asp Thr Val Leu
                805                 810                 815

Arg Tyr Arg Cys Arg Asp Gly Leu Ala Gln Arg Asn Leu Pro Leu Ile
            820                 825                 830

Arg Cys Gln Glu Asn Gly Leu Trp Glu Ala Pro Gln Ile Ser Cys Val
        835                 840                 845

Pro Arg Arg Pro Gly Arg Ala Leu Arg Ser Met Asp Ala Pro Glu Gly
    850                 855                 860

Pro Arg Gly Gln Leu Ser Arg His Arg Lys Ala Pro Leu Thr Pro Pro
865                 870                 875                 880

Ser Ser Leu

<210> 9
<211> 3977
<212> DNA
<213> Mus musculus

<220>
<221> CDS
<222> (232)..(2784)

<400> 9
gaattcgcgg ccgcgtcgac cggatggcgc agttgaactg cgctgctgag ctcgcggccg 60

cctgcgcgcg ctggggggac tcgtctgggc tactccccca cctcgcgttg gacgaccggt 120

cctggacaca ccgcttgcga ggcaagttga acattgcaga gaaggatctt aaggctacac 180

ccgacttgcc acacttgcca tccagctgaa gaaccaaagg ctgttggaga g atg gca 237
Met Ala
1

gtg aca tcc cac cac atg gtc cct gtg ttt gtc ctg atg agc gcc tgc 285
Val Thr Ser His His Met Val Pro Val Phe Val Leu Met Ser Ala Cys
        5                   10                  15

ctg gcc acc gca ggt cca gag ccc agc acc cgg tgt gaa ctg tca ccg 333

Leu Ala Thr Ala Gly Pro Glu Pro Ser Thr Arg Cys Glu Leu Ser Pro
    20                  25                  30

atc agt gcc tct cat cca gtc cag gcc ctg atg gag agc ttc acc gtt      381
Ile Ser Ala Ser His Pro Val Gln Ala Leu Met Glu Ser Phe Thr Val
35                  40                  45                  50

ctg tct ggc tgt gcc agc aga ggc acc act ggg ctg cca agg gag gtt      429
Leu Ser Gly Cys Ala Ser Arg Gly Thr Thr Gly Leu Pro Arg Glu Val
                55                  60                  65

cac atc cta aac ctc cgc agt aca gac caa gga cta ggc cag ccg cag      477
His Ile Leu Asn Leu Arg Ser Thr Asp Gln Gly Leu Gly Gln Pro Gln
            70                  75                  80

aga gag gtt acc ctg cat ctg aac ccc att gcc tcc gtg cac act cac      525
Arg Glu Val Thr Leu His Leu Asn Pro Ile Ala Ser Val His Thr His
        85                  90                  95

cac aag cct gtt gtg ttc ctg ctc aac tcc cca cag ccc ctg gtg tgg      573
His Lys Pro Val Val Phe Leu Leu Asn Ser Pro Gln Pro Leu Val Trp
    100                 105                 110

cat gtg aag aca gag aga ctg gct gct ggt gtc ccc aga ctc ttc ctg      621
His Val Lys Thr Glu Arg Leu Ala Ala Gly Val Pro Arg Leu Phe Leu
115                 120                 125                 130

gtt tcg gag ggt tct gtg gtc cag ttt tca tca gga aac ttc tcc ttg      669
Val Ser Glu Gly Ser Val Val Gln Phe Ser Ser Gly Asn Phe Ser Leu
                135                 140                 145

aca gca gaa aca gag gaa agg agt ttc cct caa gaa aat gag cat ctg      717
Thr Ala Glu Thr Glu Glu Arg Ser Phe Pro Gln Glu Asn Glu His Leu
            150                 155                 160

cta cac tgg gcc caa aag gaa tat gga gca gtg act tca ttc acc gaa      765
Leu His Trp Ala Gln Lys Glu Tyr Gly Ala Val Thr Ser Phe Thr Glu
        165                 170                 175

ctt aag ata gca aga aat atc tat att aaa gtg gga gaa gat caa gtg      813
Leu Lys Ile Ala Arg Asn Ile Tyr Ile Lys Val Gly Glu Asp Gln Val
    180                 185                 190

ttc cct ccc acg tgt aac ata ggg aaa aat ttt ctc tcg ctc aat tac      861
Phe Pro Pro Thr Cys Asn Ile Gly Lys Asn Phe Leu Ser Leu Asn Tyr
195                 200                 205                 210

ctt gcg gag tac ctt caa ccc aaa gcc gcc gaa ggt tgt gtc ctg gcc      909
Leu Ala Glu Tyr Leu Gln Pro Lys Ala Ala Glu Gly Cys Val Leu Ala
                215                 220                 225

agt cag ccc cac gag aag gaa gtg cat atc att gag tta atc tcc ccc      957
Ser Gln Pro His Glu Lys Glu Val His Ile Ile Glu Leu Ile Ser Pro
            230                 235                 240

aac tcc aat cct tac agc acc ttc cag gtg gat ata ata att gac ata     1005
Asn Ser Asn Pro Tyr Ser Thr Phe Gln Val Asp Ile Ile Ile Asp Ile
        245                 250                 255

cga cct gct cga gag gat cct gag gtg gtc aaa aac ctc gtc ctg atc     1053
Arg Pro Ala Arg Glu Asp Pro Glu Val Val Lys Asn Leu Val Leu Ile
    260                 265                 270

ttg aag tgc aaa aaa tct gtc aac tgg gtg atc aag tct ttt gac gtc     1101
Leu Lys Cys Lys Lys Ser Val Asn Trp Val Ile Lys Ser Phe Asp Val
275                 280                 285                 290

aag gga aac ttg aaa gtt att gct cct gac agt att ggc ttt gga aaa     1149
Lys Gly Asn Leu Lys Val Ile Ala Pro Asp Ser Ile Gly Phe Gly Lys

                295                 300                 305

gag agt gaa cga tcc atg aca gtg acc aaa ttg gta aga aat gac tac 1197
Glu Ser Glu Arg Ser Met Thr Val Thr Lys Leu Val Arg Asn Asp Tyr
            310                 315                 320

cct tcc acc caa gag aat ctg atg aag tgg gca ttg gac aat ggc tac 1245
Pro Ser Thr Gln Glu Asn Leu Met Lys Trp Ala Leu Asp Asn Gly Tyr
        325                 330                 335

agc cca gtg acg tca tac acc ata gct cct gtg gcc aat aga ttt cat 1293
Ser Pro Val Thr Ser Tyr Thr Ile Ala Pro Val Ala Asn Arg Phe His
    340                 345                 350

ctt cgg ctt gag aac aac gag gag atg aga gat gag gaa gtc cac acc 1341
Leu Arg Leu Glu Asn Asn Glu Glu Met Arg Asp Glu Glu Val His Thr
355                 360                 365                 370

att cct cct gag ctt cgg atc ctg ctg ggc cct gac cac ctg cct gcc 1389
Ile Pro Pro Glu Leu Arg Ile Leu Leu Gly Pro Asp His Leu Pro Ala
                375                 380                 385

ctg gac agc cca ccc ttc caa ggg gaa atc cca aat gga ggt ttc ccc 1437
Leu Asp Ser Pro Pro Phe Gln Gly Glu Ile Pro Asn Gly Gly Phe Pro
            390                 395                 400

ttt cca ttc ccg gat atc ccc agg aga ggc tgg aag gag gga gaa gat 1485
Phe Pro Phe Pro Asp Ile Pro Arg Arg Gly Trp Lys Glu Gly Glu Asp
        405                 410                 415

agg atc ccc cgg cca aag gaa ccc atc att ccc aga gtt caa ttg ctt 1533
Arg Ile Pro Arg Pro Lys Glu Pro Ile Ile Pro Arg Val Gln Leu Leu
    420                 425                 430

cca gac cac aga gag cca gaa gaa gtg caa ggg ggc gtg aat atc gcc 1581
Pro Asp His Arg Glu Pro Glu Glu Val Gln Gly Gly Val Asn Ile Ala
435                 440                 445                 450

ctg tca gtc aaa tgt gac aat gaa aag atg gtg gta gct gta gac aaa 1629
Leu Ser Val Lys Cys Asp Asn Glu Lys Met Val Val Ala Val Asp Lys
                455                 460                 465

gat tct ttc cag acc aat ggc tac tcg ggg atg gag ctc acc ctg ttg 1677
Asp Ser Phe Gln Thr Asn Gly Tyr Ser Gly Met Glu Leu Thr Leu Leu
            470                 475                 480

gat cct tcc tgc aaa gcc aag atg aat ggt acc cac ttt gtt ctg gag 1725
Asp Pro Ser Cys Lys Ala Lys Met Asn Gly Thr His Phe Val Leu Glu
        485                 490                 495

tct ccg ctg aat ggc tgt ggt act aga cat cgg agg tca gcc cca gat 1773
Ser Pro Leu Asn Gly Cys Gly Thr Arg His Arg Arg Ser Ala Pro Asp
    500                 505                 510

ggt gtg gtt tac tat aac tct att gtg gtg cag gct cca tcc cct ggg 1821
Gly Val Val Tyr Tyr Asn Ser Ile Val Val Gln Ala Pro Ser Pro Gly
515                 520                 525                 530

gat agc agt ggc tgg cca gac ggc tac gaa gat ttg gag tcg ggt gat 1869
Asp Ser Ser Gly Trp Pro Asp Gly Tyr Glu Asp Leu Glu Ser Gly Asp
                535                 540                 545

aat gga ttt cct gga gac aca gat gaa gga gaa act gcc ccc ctg agc 1917
Asn Gly Phe Pro Gly Asp Thr Asp Glu Gly Glu Thr Ala Pro Leu Ser
            550                 555                 560

cgt gct gga gtg gta gtg ttt aac tgc agc ttg cgg cag ctg agg agt 1965
Arg Ala Gly Val Val Val Phe Asn Cys Ser Leu Arg Gln Leu Arg Ser
        565                 570                 575

ccc agt ggc ttc cag gac cag ctc gat gga aat gct acc ttc aat atg 2013
Pro Ser Gly Phe Gln Asp Gln Leu Asp Gly Asn Ala Thr Phe Asn Met
    580                 585                 590

gag ctg tat aac aca gac ctc ttt ctg gtg ccc tcc ccg ggg gtc ttc 2061
Glu Leu Tyr Asn Thr Asp Leu Phe Leu Val Pro Ser Pro Gly Val Phe
595                 600                 605                 610

tct gtg gca gag aat gag cat gta tat gtt gag gtg tct gtc act aag 2109
Ser Val Ala Glu Asn Glu His Val Tyr Val Glu Val Ser Val Thr Lys
                615                 620                 625

gct gac caa gat ctg gga ttt gcc atc caa acc tgc ttt atc tct cca 2157
Ala Asp Gln Asp Leu Gly Phe Ala Ile Gln Thr Cys Phe Ile Ser Pro
            630                 635                 640

tac tca aac cca gac aga atg tct gat tac acc atc atc gag aac atc 2205
Tyr Ser Asn Pro Asp Arg Met Ser Asp Tyr Thr Ile Ile Glu Asn Ile
        645                 650                 655

tgt ccg aag gat gac tct gtg aag ttc tac agc tcc aag aga gtg cac 2253
Cys Pro Lys Asp Asp Ser Val Lys Phe Tyr Ser Ser Lys Arg Val His
    660                 665                 670

ttc ccc atc cca cat gct gaa gtg gac aag aag cgg ttc agc ttt gtg 2301
Phe Pro Ile Pro His Ala Glu Val Asp Lys Lys Arg Phe Ser Phe Val
675                 680                 685                 690

ttc aag tcc gtg ttc aac acc tcc ctg ctc ttc ctg cac tgc gag ctg 2349
Phe Lys Ser Val Phe Asn Thr Ser Leu Leu Phe Leu His Cys Glu Leu
                695                 700                 705

acg ctg tgc tct agg aac aag ggc tcc cag aag ctg cca aag tgt gtg 2397
Thr Leu Cys Ser Arg Asn Lys Gly Ser Gln Lys Leu Pro Lys Cys Val
            710                 715                 720

act cct gat gac gcc tgc acc tct cta gat gcc acc atg atc tgg acc 2445
Thr Pro Asp Asp Ala Cys Thr Ser Leu Asp Ala Thr Met Ile Trp Thr
        725                 730                 735

atg atg cag aat aag aag aca ttc acc aag ccc ctg gcc gtg gtc ctc 2493
Met Met Gln Asn Lys Lys Thr Phe Thr Lys Pro Leu Ala Val Val Leu
    740                 745                 750

caa gta gat tat aaa gaa aat gtt cca aac atg aag gag tcc agt ccg 2541
Gln Val Asp Tyr Lys Glu Asn Val Pro Asn Met Lys Glu Ser Ser Pro
755                 760                 765                 770

gtt cct cct cct cca cag att ttc cac ggc ctg gac acg ctc acc gtg 2589
Val Pro Pro Pro Pro Gln Ile Phe His Gly Leu Asp Thr Leu Thr Val
                775                 780                 785

atg ggc att gcg ttt gca gca ttt gtg atc gga gca ctc ctg acg ggg 2637
Met Gly Ile Ala Phe Ala Ala Phe Val Ile Gly Ala Leu Leu Thr Gly
            790                 795                 800

gcc ttg tgg tat atc tac tcc cac aca ggg gag acg gca cga agg cag 2685
Ala Leu Trp Tyr Ile Tyr Ser His Thr Gly Glu Thr Ala Arg Arg Gln
        805                 810                 815

caa gtc cct acc tcg cca cca gcc tcg gag aac agc agc gca gcc cac 2733
Gln Val Pro Thr Ser Pro Pro Ala Ser Glu Asn Ser Ser Ala Ala His
    820                 825                 830

agc ata ggg agc act cag agc act ccc tgc tct agc agc agc acg gcc 2781
Ser Ile Gly Ser Thr Gln Ser Thr Pro Cys Ser Ser Ser Ser Thr Ala
835                 840                 845                 850

tag gtggacagat ggacgccgct gcctaggtgg acagacggat gcctgccacc 2834
gccggggcag ggccagatgc cgatgctggg tgtccagact cagaaggctt gatctgggtt 2894
ccctgtaaag agagagtgaa tttcagtata cagacagcct gctctaccca cccctccctt 2954
accacggccc acgtaaatgt gaccctggac atctgtcaca cgaaagctaa gctggtggcc 3014
ttccctgcca gcccctcgca ggatgggggt ttcaatgtga aacatatgcc agtttttgtt 3074
cttttttttt ttttattttt tatgctgctt tgtccaggtg tccaaacatc catcatttgg 3134
ggtcgtatgt tttaaagagt aaaggagacg gtgaagggat gtcagctaga gcgtagagcc 3194
agggagagac agccagggat tctcgcctag ctgaaccaag atgtaaaata ggagaaatgc 3254
tccctccata gccaatctgc atctcgctct ggatgcccac tcctgacctg ccacgcccta 3314
catggtcccc tgtgtagctt gctagccttg ggggcctgtc acttgccaaa gtaacattcc 3374
tctcttgggc caaaatgaat gctgactcct gatactccgc acaagcacac caactccaaa 3434
agtgccttaa agccaggttt gtgtctcctt tcaggagaga gcgtgtaacg tcagatttgt 3494
gcctgtctcg aacattctgt cctaatcata cttaaacatc aggcaggtgg ggttcagtgg 3554
taagcgaagg gattattagc aaggtaatgc ctgtgtatgg tcatataggc acaaatgcag 3614
aacacccagc acattggaga gacgaccact tcctccttta aaatgtcatt gcttccagag 3674
tcggaacctg agttatatgt ggtagattat attgactcca accatcagcc tttctgatga 3734
gcctttagag aagcctagag agcccgggca tctgatgatg tcacagtgct gcctcagatg 3794
atacagagac agggagtttc aggtgaactt cttcagcaga ataaggtgat gccactctga 3854
cctttgctga gctcagtgac tcttcagtgt taaaactgaa cccagagggg attctccatg 3914
ccagatccgc tgcaaattaa ataataccaa ttttctagag tccgtcgacg cggccgcgaa 3974
ttc 3977

<210> 10
<211> 850
<212> PRT
<213> Mus musculus

<400> 10

Met Ala Val Thr Ser His His Met Val Pro Val Phe Val Leu Met Ser
1               5                   10                  15

Ala Cys Leu Ala Thr Ala Gly Pro Glu Pro Ser Thr Arg Cys Glu Leu
            20                  25                  30

Ser Pro Ile Ser Ala Ser His Pro Val Gln Ala Leu Met Glu Ser Phe
        35                  40                  45

Thr Val Leu Ser Gly Cys Ala Ser Arg Gly Thr Thr Gly Leu Pro Arg
    50                  55                  60

Glu Val His Ile Leu Asn Leu Arg Ser Thr Asp Gln Gly Leu Gly Gln
65                  70                  75                  80

Pro Gln Arg Glu Val Thr Leu His Leu Asn Pro Ile Ala Ser Val His
                85                  90                  95

Thr His His Lys Pro Val Val Phe Leu Leu Asn Ser Pro Gln Pro Leu
            100                 105                 110

Val Trp His Val Lys Thr Glu Arg Leu Ala Ala Gly Val Pro Arg Leu
        115                 120                 125

Phe Leu Val Ser Glu Gly Ser Val Val Gln Phe Ser Ser Gly Asn Phe
    130                 135                 140

Ser Leu Thr Ala Glu Thr Glu Glu Arg Ser Phe Pro Gln Glu Asn Glu
145                 150                 155                 160

His Leu Leu His Trp Ala Gln Lys Glu Tyr Gly Ala Val Thr Ser Phe
                165                 170                 175

Thr Glu Leu Lys Ile Ala Arg Asn Ile Tyr Ile Lys Val Gly Glu Asp
            180                 185                 190

Gln Val Phe Pro Pro Thr Cys Asn Ile Gly Lys Asn Phe Leu Ser Leu
        195                 200                 205

Asn Tyr Leu Ala Glu Tyr Leu Gln Pro Lys Ala Ala Glu Gly Cys Val
    210                 215                 220

Leu Ala Ser Gln Pro His Glu Lys Glu Val His Ile Ile Glu Leu Ile
225                 230                 235                 240

Ser Pro Asn Ser Asn Pro Tyr Ser Thr Phe Gln Val Asp Ile Ile Ile
                245                 250                 255

Asp Ile Arg Pro Ala Arg Glu Asp Pro Glu Val Val Lys Asn Leu Val
            260                 265                 270

Leu Ile Leu Lys Cys Lys Lys Ser Val Asn Trp Val Ile Lys Ser Phe
        275                 280                 285

Asp Val Lys Gly Asn Leu Lys Val Ile Ala Pro Asp Ser Ile Gly Phe
    290                 295                 300

Gly Lys Glu Ser Glu Arg Ser Met Thr Val Thr Lys Leu Val Arg Asn
305                 310                 315                 320

Asp Tyr Pro Ser Thr Gln Glu Asn Leu Met Lys Trp Ala Leu Asp Asn
                325                 330                 335

Gly Tyr Ser Pro Val Thr Ser Tyr Thr Ile Ala Pro Val Ala Asn Arg
            340                 345                 350

Phe His Leu Arg Leu Glu Asn Asn Glu Glu Met Arg Asp Glu Glu Val
        355                 360                 365

His Thr Ile Pro Pro Glu Leu Arg Ile Leu Leu Gly Pro Asp His Leu
    370                 375                 380

Pro Ala Leu Asp Ser Pro Pro Phe Gln Gly Glu Ile Pro Asn Gly Gly
385                 390                 395                 400

Phe Pro Phe Pro Phe Pro Asp Ile Pro Arg Arg Gly Trp Lys Glu Gly
                405                 410                 415

Glu Asp Arg Ile Pro Arg Pro Lys Glu Pro Ile Ile Pro Arg Val Gln
            420                 425                 430

Leu Leu Pro Asp His Arg Glu Pro Glu Glu Val Gln Gly Gly Val Asn
        435                 440                 445

Ile Ala Leu Ser Val Lys Cys Asp Asn Glu Lys Met Val Val Ala Val
    450                 455                 460

Asp Lys Asp Ser Phe Gln Thr Asn Gly Tyr Ser Gly Met Glu Leu Thr
465                 470                 475                 480

Leu Leu Asp Pro Ser Cys Lys Ala Lys Met Asn Gly Thr His Phe Val
                485                 490                 495

Leu Glu Ser Pro Leu Asn Gly Cys Gly Thr Arg His Arg Arg Ser Ala
            500                 505                 510

Pro Asp Gly Val Val Tyr Tyr Asn Ser Ile Val Val Gln Ala Pro Ser
        515                 520                 525

Pro Gly Asp Ser Ser Gly Trp Pro Asp Gly Tyr Glu Asp Leu Glu Ser
    530                 535                 540

Gly Asp Asn Gly Phe Pro Gly Asp Thr Asp Glu Gly Glu Thr Ala Pro
545                 550                 555                 560

Leu Ser Arg Ala Gly Val Val Val Phe Asn Cys Ser Leu Arg Gln Leu
                565                 570                 575

Arg Ser Pro Ser Gly Phe Gln Asp Gln Leu Asp Gly Asn Ala Thr Phe
            580                 585                 590

Asn Met Glu Leu Tyr Asn Thr Asp Leu Phe Leu Val Pro Ser Pro Gly
        595                 600                 605

Val Phe Ser Val Ala Glu Asn Glu His Val Tyr Val Glu Val Ser Val
    610                 615                 620

Thr Lys Ala Asp Gln Asp Leu Gly Phe Ala Ile Gln Thr Cys Phe Ile

625                 630                 635                 640

Ser Pro Tyr Ser Asn Pro Asp Arg Met Ser Asp Tyr Thr Ile Ile Glu
                645                 650                 655

Asn Ile Cys Pro Lys Asp Asp Ser Val Lys Phe Tyr Ser Ser Lys Arg
            660                 665                 670

Val His Phe Pro Ile Pro His Ala Glu Val Asp Lys Lys Arg Phe Ser
        675                 680                 685

Phe Val Phe Lys Ser Val Phe Asn Thr Ser Leu Leu Phe Leu His Cys
    690                 695                 700

Glu Leu Thr Leu Cys Ser Arg Asn Lys Gly Ser Gln Lys Leu Pro Lys
705                 710                 715                 720

Cys Val Thr Pro Asp Asp Ala Cys Thr Ser Leu Asp Ala Thr Met Ile
                725                 730                 735

Trp Thr Met Met Gln Asn Lys Lys Thr Phe Thr Lys Pro Leu Ala Val
            740                 745                 750

Val Leu Gln Val Asp Tyr Lys Glu Asn Val Pro Asn Met Lys Glu Ser
        755                 760                 765

Ser Pro Val Pro Pro Pro Pro Gln Ile Phe His Gly Leu Asp Thr Leu
    770                 775                 780

Thr Val Met Gly Ile Ala Phe Ala Ala Phe Val Ile Gly Ala Leu Leu
785                 790                 795                 800

Thr Gly Ala Leu Trp Tyr Ile Tyr Ser His Thr Gly Glu Thr Ala Arg
                805                 810                 815

Arg Gln Gln Val Pro Thr Ser Pro Pro Ala Ser Glu Asn Ser Ser Ala
            820                 825                 830

Ala His Ser Ile Gly Ser Thr Gln Ser Thr Pro Cys Ser Ser Ser Ser
        835                 840                 845

Thr Ala
    850

<210>  11
<211>  1767
<212>  DNA
<213>  Mus musculus

<220>
<221>  CDS
<222>  (207)..(1271)

<400> 11
gactaatgtt ggggtaaaca cagaaagccc attataacat gataagttca ttaaaagagg 60

tcagaatatc gtgcttcaga tcacagaagc ggtaacgagc agcaccctgc tcagaggaga 120

agtgagggga gacaggggca ggagacccag aatctcacgt aacctttaaa cttcttcaga 180

aggtcgtgaa aatacatgag ataatc atg aag gca act ctc atc ttc ttc ctt 233
Met Lys Ala Thr Leu Ile Phe Phe Leu
1               5

ctg gca caa gtc tct tgg gct gga cca ttt gaa cag aga ggc tta ttt 281
Leu Ala Gln Val Ser Trp Ala Gly Pro Phe Glu Gln Arg Gly Leu Phe
10                  15                  20                  25

gac ttc atg cta gaa gat gag gct tct ggc ata atc cct tat gac cct 329
Asp Phe Met Leu Glu Asp Glu Ala Ser Gly Ile Ile Pro Tyr Asp Pro
                30                  35                  40

gac aat ccc ctg ata tct atg tgc ccc tac cga tgc cag tgt cat ctt 377
Asp Asn Pro Leu Ile Ser Met Cys Pro Tyr Arg Cys Gln Cys His Leu
            45                  50                  55

cga gtg gtg cag tgt tct gat ctg ggt ttg gac aaa gtg ccc tgg gat 425
Arg Val Val Gln Cys Ser Asp Leu Gly Leu Asp Lys Val Pro Trp Asp
        60                  65                  70

ttt cca ccc gac aca acc ttg cta gac ctg caa aac aac aaa att aca 473
Phe Pro Pro Asp Thr Thr Leu Leu Asp Leu Gln Asn Asn Lys Ile Thr
    75                  80                  85

gag atc aaa gaa ggg gcc ttc aag aac ctg aag gac ttg cat acc ttg 521
Glu Ile Lys Glu Gly Ala Phe Lys Asn Leu Lys Asp Leu His Thr Leu
90                  95                  100                 105

atc ctt gtc aac aac aag atc agc aaa atc agt cca gag gca ttc aaa 569
Ile Leu Val Asn Asn Lys Ile Ser Lys Ile Ser Pro Glu Ala Phe Lys
                110                 115                 120

cct ctc gtg aag ttg gaa agg ctt tac ctg tct aag aac caa cta aag 617
Pro Leu Val Lys Leu Glu Arg Leu Tyr Leu Ser Lys Asn Gln Leu Lys
            125                 130                 135

gaa ctg cct gaa aaa atg ccc aga act ctc cag gaa ctt cgt gtc cat 665
Glu Leu Pro Glu Lys Met Pro Arg Thr Leu Gln Glu Leu Arg Val His
        140                 145                 150

gag aat gag atc acc aag ctg cgg aaa tcc gac ttc aat gga ctg aac 713
Glu Asn Glu Ile Thr Lys Leu Arg Lys Ser Asp Phe Asn Gly Leu Asn
    155                 160                 165

aat gtg ctt gtc ata gaa ctg ggc ggc aac cca ctg aaa aac tct ggg 761
Asn Val Leu Val Ile Glu Leu Gly Gly Asn Pro Leu Lys Asn Ser Gly
170                 175                 180                 185

att gaa aac gga gcc ttc cag gga ctg aag agt ctc tca tac att cgc 809
Ile Glu Asn Gly Ala Phe Gln Gly Leu Lys Ser Leu Ser Tyr Ile Arg
                190                 195                 200

atc tca gac acc aac ata act gcg atc cct caa ggt ctg cct act tct 857
Ile Ser Asp Thr Asn Ile Thr Ala Ile Pro Gln Gly Leu Pro Thr Ser
            205                 210                 215

ctc act gaa gtg cat cta gat ggc aac aag atc acc aag gtt gat gca 905
Leu Thr Glu Val His Leu Asp Gly Asn Lys Ile Thr Lys Val Asp Ala
        220                 225                 230

ccc agc ctg aaa gga ctg att aat ttg tct aaa ctg gga ttg agc ttc 953
Pro Ser Leu Lys Gly Leu Ile Asn Leu Ser Lys Leu Gly Leu Ser Phe

235                    240                    245

aac agc atc acc gtt atg gag aat ggc agt ctg gcc aat gtt cct cat      1001
Asn Ser Ile Thr Val Met Glu Asn Gly Ser Leu Ala Asn Val Pro His
250                 255                 260                 265

ctg agg gaa ctc cac ttg gac aac aac aaa ctc ctc agg gtg cct gct      1049
Leu Arg Glu Leu His Leu Asp Asn Asn Lys Leu Leu Arg Val Pro Ala
                270                 275                 280

ggg ctg gca cag cat aag tat atc cag gtc gtc tac ctt cac aac aac      1097
Gly Leu Ala Gln His Lys Tyr Ile Gln Val Val Tyr Leu His Asn Asn
            285                 290                 295

aac atc tcc gca gtt ggg caa aat gac ttc tgc cga gct gga cac ccc      1145
Asn Ile Ser Ala Val Gly Gln Asn Asp Phe Cys Arg Ala Gly His Pro
        300                 305                 310

tct cga aag gct tcc tac tcg gct gtg agt ctt tac ggc aac cct gtc      1193
Ser Arg Lys Ala Ser Tyr Ser Ala Val Ser Leu Tyr Gly Asn Pro Val
    315                 320                 325

cgg tat tgg gaa atc ttt cca aac acc ttc aga tgt gtc tat gtg cgt      1241
Arg Tyr Trp Glu Ile Phe Pro Asn Thr Phe Arg Cys Val Tyr Val Arg
330                 335                 340                 345

tct gcc att caa ctt gga aac tac aag taa ccctcagacg gcctaattct      1291
Ser Ala Ile Gln Leu Gly Asn Tyr Lys
                350

tataatctgg aaaaacaccc caatatgtca atatcattgc taaaaagaaa aaatactaaa      1351

aaagaaagaa agaatgctag attctgaaaa ttcaagcaca ctgtgcatgc cttttccaca      1411

tgacttatta tgcaagctga atatacagat tgaataattg cctacaataa aaatatttta      1471

cttgtagtat tcagaatcac ttttaaagtt gtctgtagtt gtgaactgag ttatcaaagt      1531

ctgatgtaat cataaatgtc aaccacttag taaagcgtta aaaggaacag aatacaaagt      1591

ctctgtaatt catagagcta aattaactct tttgacataa agtcaaatgc cgccgaactc      1651

tagcaatgta ttaatctcct ttattattgg tgaagcctta tttgagtatg taagttcaat      1711

atgggctatg taaaattttc actgtcatat tgaaaaaaat aaaattttaa aaatgc      1767

<210>  12
<211>  354
<212>  PRT
<213>  Mus musculus

<400>  12

Met Lys Ala Thr Leu Ile Phe Phe Leu Leu Ala Gln Val Ser Trp Ala
1               5                   10                  15

Gly Pro Phe Glu Gln Arg Gly Leu Phe Asp Phe Met Leu Glu Asp Glu
            20                  25                  30

Ala Ser Gly Ile Ile Pro Tyr Asp Pro Asp Asn Pro Leu Ile Ser Met
        35                  40                  45

Cys Pro Tyr Arg Cys Gln Cys His Leu Arg Val Val Gln Cys Ser Asp
    50                  55                  60

Leu Gly Leu Asp Lys Val Pro Trp Asp Phe Pro Pro Asp Thr Thr Leu
65                  70                  75                  80

Leu Asp Leu Gln Asn Asn Lys Ile Thr Glu Ile Lys Glu Gly Ala Phe
                85                  90                  95

Lys Asn Leu Lys Asp Leu His Thr Leu Ile Leu Val Asn Asn Lys Ile
            100                 105                 110

Ser Lys Ile Ser Pro Glu Ala Phe Lys Pro Leu Val Lys Leu Glu Arg
        115                 120                 125

Leu Tyr Leu Ser Lys Asn Gln Leu Lys Glu Leu Pro Glu Lys Met Pro
    130                 135                 140

Arg Thr Leu Gln Glu Leu Arg Val His Glu Asn Glu Ile Thr Lys Leu
145                 150                 155                 160

Arg Lys Ser Asp Phe Asn Gly Leu Asn Asn Val Leu Val Ile Glu Leu
                165                 170                 175

Gly Gly Asn Pro Leu Lys Asn Ser Gly Ile Glu Asn Gly Ala Phe Gln
            180                 185                 190

Gly Leu Lys Ser Leu Ser Tyr Ile Arg Ile Ser Asp Thr Asn Ile Thr
        195                 200                 205

Ala Ile Pro Gln Gly Leu Pro Thr Ser Leu Thr Glu Val His Leu Asp
    210                 215                 220

Gly Asn Lys Ile Thr Lys Val Asp Ala Pro Ser Leu Lys Gly Leu Ile
225                 230                 235                 240

Asn Leu Ser Lys Leu Gly Leu Ser Phe Asn Ser Ile Thr Val Met Glu
                245                 250                 255

Asn Gly Ser Leu Ala Asn Val Pro His Leu Arg Glu Leu His Leu Asp
            260                 265                 270

Asn Asn Lys Leu Leu Arg Val Pro Ala Gly Leu Ala Gln His Lys Tyr
        275                 280                 285

Ile Gln Val Val Tyr Leu His Asn Asn Asn Ile Ser Ala Val Gly Gln
    290                 295                 300

Asn Asp Phe Cys Arg Ala Gly His Pro Ser Arg Lys Ala Ser Tyr Ser
305                 310                 315                 320

Ala Val Ser Leu Tyr Gly Asn Pro Val Arg Tyr Trp Glu Ile Phe Pro
                325                 330                 335

Asn Thr Phe Arg Cys Val Tyr Val Arg Ser Ala Ile Gln Leu Gly Asn
            340                 345                 350

Tyr Lys

<210>  13
<211>  1474
<212>  DNA
<213>  Mus musculus

<220>
<221>  CDS
<222>  (152)..(556)

<400>  13
gcctctgtct ccctctctcc acgaactgcc caggagcgag cagctgctcc cggttggccc      60

tgacggacag acaaaccgac agcctgacaa cctagtccac caactaagca gcctgcacct     120

ggctgcttgt ccctccccag gaacattgac c atg tgt ccc ctg tgg cta ctc        172
                                   Met Cys Pro Leu Trp Leu Leu
                                   1               5

acc ttg ctg ctg gcc ctg agc cag gcc ttg ccc ttt gag cag aag ggt       220
Thr Leu Leu Leu Ala Leu Ser Gln Ala Leu Pro Phe Glu Gln Lys Gly
        10                  15                  20

ttc tgg gac ttc acc ttg gat gat ggg ctg ctc atg atg aat gat gag       268
Phe Trp Asp Phe Thr Leu Asp Asp Gly Leu Leu Met Met Asn Asp Glu
    25                  30                  35

gag gct tca ggt tca gac acc act tca ggt gtc ccc gac ctg gac tct       316
Glu Ala Ser Gly Ser Asp Thr Thr Ser Gly Val Pro Asp Leu Asp Ser
40                  45                  50                  55

gtc aca cct acc ttc agt gcc atg tgt cct ttc ggt tgc cac tgc cac       364
Val Thr Pro Thr Phe Ser Ala Met Cys Pro Phe Gly Cys His Cys His
                60                  65                  70

ctg cgg gtt gtt cag tgc tct gac ttg ggt ctg aag act gtg ccc aag       412
Leu Arg Val Val Gln Cys Ser Asp Leu Gly Leu Lys Thr Val Pro Lys
            75                  80                  85

gag atc tca cct gac acc aca ctg cta gac ctg cag aac aat gac att       460
Glu Ile Ser Pro Asp Thr Thr Leu Leu Asp Leu Gln Asn Asn Asp Ile
        90                  95                  100

tct gag ctt cgc aag gat gac ttc aaa ggc ctc cag cac ctc tac gta       508
Ser Glu Leu Arg Lys Asp Asp Phe Lys Gly Leu Gln His Leu Tyr Val
    105                 110                 115

agc ctc ctc ctg cct cct gcc ttt tct gag gag cca gga gga gtt tga       556
Ser Leu Leu Leu Pro Pro Ala Phe Ser Glu Glu Pro Gly Gly Val
120                 125                 130

tgttcctatg acttggaaga agggtactca caaatgaggt tgggaagagg ggtgggaacc     616

gatggagtcc tgagataagt ctggagctgg ctgtaatgag aacgcccaaa accatgcatt     676

atatgtatgg gtacaagacg aaatctgtca ggtcccattt ttttttgaca gaaatcttta      736

cgttttgttg actcatctga acaacacaga aagtgtattc catcatagct cttagagaaa     796

ctgtctagca aagtatgcta ggcagtgatg gcattaccag ggcagcttgg agctaacctc     856

agctccttgc cctacactct actcaccaac acatactgat ctccatgaga ccattaaagg 916

ttcagatgtt tcccaagtgc catcagtagc aagttcagga ggcagaggag aggggaggtg 976

gagtgggggg ctggggtgct caccctggtt gcctgccccg ttttaggccc tggtcttggt 1036

aaacaataag atctccaaga tccatgagaa ggcctttagc cctctgcgga agctgcaaaa 1096

actctacatc tccaagaacc acctggtgga gattcctccc aacctgccca gctccctggt 1156

agaactacga atccatgaca accgtatccg caaagtgccc aagggcgtgt tcagcgggct 1216

ccggaacatg aactgcattg gtgagactag tctgtacctt agcatcaggc ataaaggaca 1276

caggtagagg atatctcttt cagccagggc agctgggtcc atgtagtcat ggaaaagccc 1336

tgtgtgcctc agaatgttcc agatgtttgt tttgggacat gaagaaaaag actgtggtgg 1396

ctataaaggc tcggttgaag aaagagtctc aaatatagtc taaaaaagac cagtatgttt 1456

ctgaaaaaaa aaaaaaaa 1474

<210> 14
<211> 134
<212> PRT
<213> Mus musculus

<400> 14

Met Cys Pro Leu Trp Leu Leu Thr Leu Leu Leu Ala Leu Ser Gln Ala
1               5                   10                  15

Leu Pro Phe Glu Gln Lys Gly Phe Trp Asp Phe Thr Leu Asp Asp Gly
            20                  25                  30

Leu Leu Met Met Asn Asp Glu Glu Ala Ser Gly Ser Asp Thr Thr Ser
        35                  40                  45

Gly Val Pro Asp Leu Asp Ser Val Thr Pro Thr Phe Ser Ala Met Cys
    50                  55                  60

Pro Phe Gly Cys His Cys His Leu Arg Val Val Gln Cys Ser Asp Leu
65                  70                  75                  80

Gly Leu Lys Thr Val Pro Lys Glu Ile Ser Pro Asp Thr Thr Leu Leu
                85                  90                  95

Asp Leu Gln Asn Asn Asp Ile Ser Glu Leu Arg Lys Asp Asp Phe Lys
            100                 105                 110

Gly Leu Gln His Leu Tyr Val Ser Leu Leu Leu Pro Pro Ala Phe Ser
        115                 120                 125

Glu Glu Pro Gly Gly Val
    130

<210> 15
<211> 2860
<212> DNA

<213>  Mus musculus

<220>
<221>  CDS
<222>  (78)..(1208)

<400>  15
gctgccacat tctccaaccc aaggagacca gacagaagga cgtggtcact ctgaacagtt      60

caacccaaga gacaaaa atg cag tgg gcc tcc gtc ctg ctg ctg gct ggg      110
                   Met Gln Trp Ala Ser Val Leu Leu Leu Ala Gly
                   1               5                   10

ctc tgc tca ctt tcc cag ggc cag tat gat gaa gac tct cac tgg tgg      158
Leu Cys Ser Leu Ser Gln Gly Gln Tyr Asp Glu Asp Ser His Trp Trp
            15                  20                  25

atc caa tac ctc cga aac cag cag tcc acc tac tac gac ccc tat gac      206
Ile Gln Tyr Leu Arg Asn Gln Gln Ser Thr Tyr Tyr Asp Pro Tyr Asp
        30                  35                  40

cct tac ccc tat gag ccc tct gag cct tac ccc tat gga gtg gaa gaa      254
Pro Tyr Pro Tyr Glu Pro Ser Glu Pro Tyr Pro Tyr Gly Val Glu Glu
    45                  50                  55

ggc cca gcc tat gcc tat ggt gca cca cct cca cca gag ccc cgt gat      302
Gly Pro Ala Tyr Ala Tyr Gly Ala Pro Pro Pro Pro Glu Pro Arg Asp
60                  65                  70                  75

tgt ccc caa gaa tgc gac tgt ccc ccc aac ttc ccc aca gcc atg tac      350
Cys Pro Gln Glu Cys Asp Cys Pro Pro Asn Phe Pro Thr Ala Met Tyr
                80                  85                  90

tgt gac aac cgc aac ctc aag tac ctg cct ttt gtg ccc tcc cgc atg      398
Cys Asp Asn Arg Asn Leu Lys Tyr Leu Pro Phe Val Pro Ser Arg Met
            95                  100                 105

aag tac gtc tac ttc cag aac aac cag atc tct gcc atc cag gaa ggt      446
Lys Tyr Val Tyr Phe Gln Asn Asn Gln Ile Ser Ala Ile Gln Glu Gly
        110                 115                 120

gtc ttt gac aat gcc act ggg ctc ctc tgg gtc gct cta cat ggc aac      494
Val Phe Asp Asn Ala Thr Gly Leu Leu Trp Val Ala Leu His Gly Asn
    125                 130                 135

cag att acc agt gac aag gta ggc agg aaa gtc ttc tcc aag ctg agg      542
Gln Ile Thr Ser Asp Lys Val Gly Arg Lys Val Phe Ser Lys Leu Arg
140                 145                 150                 155

cac ttg gag agg ctg tac ttg gac cac aac aac ctg acc cgg atg ccc      590
His Leu Glu Arg Leu Tyr Leu Asp His Asn Asn Leu Thr Arg Met Pro
                160                 165                 170

ggc ccg ctg ccc cga tcc ctg aga gag ctc cac ctg gac cac aac cag      638
Gly Pro Leu Pro Arg Ser Leu Arg Glu Leu His Leu Asp His Asn Gln
            175                 180                 185

atc tca cgg gtc ccc aac aat gct ttg gag ggc ctg gag aac ctc acg      686
Ile Ser Arg Val Pro Asn Asn Ala Leu Glu Gly Leu Glu Asn Leu Thr
        190                 195                 200

gcc tta tat ctc cac cac aat gag atc cag gaa gtg ggg agt tcc atg      734
Ala Leu Tyr Leu His His Asn Glu Ile Gln Glu Val Gly Ser Ser Met
    205                 210                 215

aga ggc ctc cgg tcc ctg atc cta cta gac ctg agt tat aac cac ctt      782
Arg Gly Leu Arg Ser Leu Ile Leu Leu Asp Leu Ser Tyr Asn His Leu
220                 225                 230                 235

cgg agg gta ccc gac ggt ctg ccc tca gcc ctg gag cag ctg tac cta     830
Arg Arg Val Pro Asp Gly Leu Pro Ser Ala Leu Glu Gln Leu Tyr Leu
                240                 245                 250

gaa cac aac aat gtc tac acc gtc cct gac agc tac ttc cgg ggg tca     878
Glu His Asn Asn Val Tyr Thr Val Pro Asp Ser Tyr Phe Arg Gly Ser
            255                 260                 265

ccc aag ctg ctg tac gtc cgg ttg tct cac aac agt ctc act aac aac     926
Pro Lys Leu Leu Tyr Val Arg Leu Ser His Asn Ser Leu Thr Asn Asn
        270                 275                 280

ggc ctt gct acc aac acc ttc aac tcc agc agc ctt ctt gag cta gac     974
Gly Leu Ala Thr Asn Thr Phe Asn Ser Ser Ser Leu Leu Glu Leu Asp
    285                 290                 295

ttg tct tac aat cag ctg cag aag atc cct cct gtc aac acc aac ctg     1022
Leu Ser Tyr Asn Gln Leu Gln Lys Ile Pro Pro Val Asn Thr Asn Leu
300                 305                 310                 315

gag aat ctt tac ctc cag ggc aac agg atc aat gag ttc tcc atc agc     1070
Glu Asn Leu Tyr Leu Gln Gly Asn Arg Ile Asn Glu Phe Ser Ile Ser
                320                 325                 330

agc ttc tgc acg gtg gtg gac gtc atg aac ttc tcc aag ctg cag gtg     1118
Ser Phe Cys Thr Val Val Asp Val Met Asn Phe Ser Lys Leu Gln Val
            335                 340                 345

cta cgc ctg gat ggg aac gag atc aag cgc agc gca atg ccg gtg gac     1166
Leu Arg Leu Asp Gly Asn Glu Ile Lys Arg Ser Ala Met Pro Val Asp
        350                 355                 360

gcc cca ctc tgc ctg cgc ctc gcc aac ctc atc gag atc tga             1208
Ala Pro Leu Cys Leu Arg Leu Ala Asn Leu Ile Glu Ile
    365                 370                 375

ggggcctcgc cttgggctcc tggctccggg ctccgagtac cgtgcaccgt gcaccgggct     1268

cggggtacat gccctgccac cccgctgcat gtttggcttt tgctggatgg tctgggacac     1328

gcatgtgaca gaagtccaca ggatcttatt caatctcctt ccaacaggca gagttaggtg     1388

ggatcagggg ccaggccagt ttctgcaggg ggatgaattt ggtggtaaaa gaaatatagc     1448

tatagagtct agccccaaaa tcttcttgct tggacagtag catataacca gttccaattt     1508

gacctttttt gagctgtgct caacagcatg gccagctgct tctgcagttg ctctgggctc     1568

ctgctctttg ctcctcaaaa catcacaaca cacctcttgc ccagtcacct cctcccagcc     1628

ccagctcacc ctctctgccc tctttcactg gggcctcttc taatgtctta ggcagtcagg     1688

agacacccac accctaaggg cacactctta tctgaggcta aacggatggc taaaatcaca     1748

cacttacccc cacctcacct gtttaaagtc accatattga acaccataat gatgagctgt     1808

taacataggg gataaccgac tctataatgg aggatctatc agaggaggga agtgtccagt     1868

ggtcattatc agtatccata gtaagatggg gaagagacac acctcaggat agcagaactg     1928

aaggggcagc cccctttcca gtttcatctg acacaacatg atctgcaccc cttcttgggc     1988

ttttagtatc gaaggggcaa gcgtggtttt caaaacatga gaaagagcct ctgctcatcc     2048

tttggtctag tatgaagggt tgttacgcaa atggcagagg cagactccac ccgccgagga     2108

cctacccact aacccagact agggggtgat ctcaatagct cgtttgtcca taatgctaga     2168

gcatcaggaa ccagccctgt ggagatgctc actggggtgc aagaagccct gccaggccca 2228

agtcctgccg acactagacc tggcttcctc tgcattcaca ctatgcagtc cgccttatgc 2288

ccagtggtgc ctgtgatttg tagtttgtct aagaacagcc ccatcttctc ctttttaaag 2348

gtgatattgc ctaggtacaa ccaacagctt cttctagacc ccactgggat agagccacca 2408

gggtagcgag tggcaagaag cagttgtaag atgctagcct gaggtgggtc cctgccttag 2468

acccctgact ctgtgtgcac ttgggagcaa gcttctctgg aaaggacagg gtggggcggg 2528

gtgcttagac tgtgctacgg gttccagaac tgcaatccac aaaagccaaa ccagcttgtt 2588

tcaaccgggg agtgccacct gcggagcaag gctgccctgg ccagggttct tgggaagcac 2648

ctgcatgtat caccatcctg cctctgaaga gccctcagca tgaagcaagc atgaatggcg 2708

gccaacctaa ccaataaagt tattttataa gtgcatctgc aaggatggaa tggttggtgg 2768

cagccctcgg catcgcctcg gggtacctag acacacaggc cttgaaccgt gtcagctctt 2828

tataaagagt agcctatata aaaactcaag tt 2860

<210> 16
<211> 376
<212> PRT
<213> Mus musculus

<400> 16

Met Gln Trp Ala Ser Val Leu Leu Leu Ala Gly Leu Cys Ser Leu Ser
1               5                   10                  15

Gln Gly Gln Tyr Asp Glu Asp Ser His Trp Trp Ile Gln Tyr Leu Arg
            20                  25                  30

Asn Gln Gln Ser Thr Tyr Tyr Asp Pro Tyr Asp Pro Tyr Pro Tyr Glu
        35                  40                  45

Pro Ser Glu Pro Tyr Pro Tyr Gly Val Glu Glu Gly Pro Ala Tyr Ala
    50                  55                  60

Tyr Gly Ala Pro Pro Pro Pro Glu Pro Arg Asp Cys Pro Gln Glu Cys
65                  70                  75                  80

Asp Cys Pro Pro Asn Phe Pro Thr Ala Met Tyr Cys Asp Asn Arg Asn
                85                  90                  95

Leu Lys Tyr Leu Pro Phe Val Pro Ser Arg Met Lys Tyr Val Tyr Phe
            100                 105                 110

Gln Asn Asn Gln Ile Ser Ala Ile Gln Glu Gly Val Phe Asp Asn Ala
        115                 120                 125

Thr Gly Leu Leu Trp Val Ala Leu His Gly Asn Gln Ile Thr Ser Asp
    130                 135                 140

Lys Val Gly Arg Lys Val Phe Ser Lys Leu Arg His Leu Glu Arg Leu

145                 150                 155                 160

Tyr Leu Asp His Asn Asn Leu Thr Arg Met Pro Gly Pro Leu Pro Arg
                165                 170                 175

Ser Leu Arg Glu Leu His Leu Asp His Asn Gln Ile Ser Arg Val Pro
            180                 185                 190

Asn Asn Ala Leu Glu Gly Leu Glu Asn Leu Thr Ala Leu Tyr Leu His
        195                 200                 205

His Asn Glu Ile Gln Glu Val Gly Ser Ser Met Arg Gly Leu Arg Ser
    210                 215                 220

Leu Ile Leu Leu Asp Leu Ser Tyr Asn His Leu Arg Arg Val Pro Asp
225                 230                 235                 240

Gly Leu Pro Ser Ala Leu Glu Gln Leu Tyr Leu Glu His Asn Asn Val
                245                 250                 255

Tyr Thr Val Pro Asp Ser Tyr Phe Arg Gly Ser Pro Lys Leu Leu Tyr
            260                 265                 270

Val Arg Leu Ser His Asn Ser Leu Thr Asn Asn Gly Leu Ala Thr Asn
        275                 280                 285

Thr Phe Asn Ser Ser Ser Leu Leu Glu Leu Asp Leu Ser Tyr Asn Gln
    290                 295                 300

Leu Gln Lys Ile Pro Pro Val Asn Thr Asn Leu Glu Asn Leu Tyr Leu
305                 310                 315                 320

Gln Gly Asn Arg Ile Asn Glu Phe Ser Ile Ser Ser Phe Cys Thr Val
                325                 330                 335

Val Asp Val Met Asn Phe Ser Lys Leu Gln Val Leu Arg Leu Asp Gly
            340                 345                 350

Asn Glu Ile Lys Arg Ser Ala Met Pro Val Asp Ala Pro Leu Cys Leu
        355                 360                 365

Arg Leu Ala Asn Leu Ile Glu Ile
    370                 375

<210>  17
<211>  1723
<212>  DNA
<213>  Mus musculus

<220>
<221>  CDS
<222>  (125)..(1093)

<400> 17
gacagttgga agatgacagt tgaccagaat tcctatccat tggtcagggg caaataccaa 60

ggactctgta tgctcagtca ctctgtgcga cttcatcaag tcggagccaa agaaatcttg 120

aaag atg gga atg cta gca aga gtt gcc ctg gga ctt atc atc att gat 169
Met Gly Met Leu Ala Arg Val Ala Leu Gly Leu Ile Ile Ile Asp
1               5                   10                  15

gct gta ttg gct gca cca act aca gag ctc ttt aac tat gac tca gaa 217
Ala Val Leu Ala Ala Pro Thr Thr Glu Leu Phe Asn Tyr Asp Ser Glu
                20                  25                  30

gtg tat gat gcc atc ttg gag gac aca ggg acc ttt tat aac tat gaa 265
Val Tyr Asp Ala Ile Leu Glu Asp Thr Gly Thr Phe Tyr Asn Tyr Glu
            35                  40                  45

cac att cct gac aac cat gta gag aac gag aaa gtg tct gag agg ctt 313
His Ile Pro Asp Asn His Val Glu Asn Glu Lys Val Ser Glu Arg Leu
        50                  55                  60

tct ggg aac aga gag ctc ctg acc cca ggc cca cag cta ggg gac aac 361
Ser Gly Asn Arg Glu Leu Leu Thr Pro Gly Pro Gln Leu Gly Asp Asn
    65                  70                  75

cag gat gaa gac aag gat gaa gaa tct act ccc agg ctg atc gat ggc 409
Gln Asp Glu Asp Lys Asp Glu Glu Ser Thr Pro Arg Leu Ile Asp Gly
80                  85                  90                  95

tct tcc cca cag gag cca gaa ttc ccg ggg ctt ctg ggt cca cac acc 457
Ser Ser Pro Gln Glu Pro Glu Phe Pro Gly Leu Leu Gly Pro His Thr
                100                 105                 110

aac gaa gac ttt cca act tgt ctc ctg tgt act tgt ata agt acc act 505
Asn Glu Asp Phe Pro Thr Cys Leu Leu Cys Thr Cys Ile Ser Thr Thr
            115                 120                 125

gta tac tgt gat gac cat gaa ctg gac gct att cct cca ctg ccc aag 553
Val Tyr Cys Asp Asp His Glu Leu Asp Ala Ile Pro Pro Leu Pro Lys
        130                 135                 140

aag act aca tat ttc tat tca cgt ttt aac aga att aaa aag atc aac 601
Lys Thr Thr Tyr Phe Tyr Ser Arg Phe Asn Arg Ile Lys Lys Ile Asn
    145                 150                 155

aaa aat gac ttt gca agc tta aat gat tta aaa agg atc gat ctg aca 649
Lys Asn Asp Phe Ala Ser Leu Asn Asp Leu Lys Arg Ile Asp Leu Thr
160                 165                 170                 175

tca aat tta ata tcg gag att gat gaa gat gca ttc cga aag ctg cct 697
Ser Asn Leu Ile Ser Glu Ile Asp Glu Asp Ala Phe Arg Lys Leu Pro
                180                 185                 190

cac ctc caa gag ctg gtt ctg cgt gac aat aag ata aag cag ctt cca 745
His Leu Gln Glu Leu Val Leu Arg Asp Asn Lys Ile Lys Gln Leu Pro
            195                 200                 205

gaa ttg cca aac act ttg aca ttt att gat atc agc aac aat aga ctg 793
Glu Leu Pro Asn Thr Leu Thr Phe Ile Asp Ile Ser Asn Asn Arg Leu
        210                 215                 220

gga aga aaa ggc att aaa caa gaa gct ttt aaa gat atg tat gat ctc 841
Gly Arg Lys Gly Ile Lys Gln Glu Ala Phe Lys Asp Met Tyr Asp Leu
    225                 230                 235

cac cat ctc tat atc acc gat aac agc ttg gac cac atc cct cta cca 889
His His Leu Tyr Ile Thr Asp Asn Ser Leu Asp His Ile Pro Leu Pro
240                 245                 250                 255

ctc cca gaa agt ctg cgg gct ctt cac ctc cag aat aat gac att ctg 937
Leu Pro Glu Ser Leu Arg Ala Leu His Leu Gln Asn Asn Asp Ile Leu
                260                 265                 270

gag atg cat gaa gat act ttc tgc aat gtt aaa aat tta act tat gtt 985
Glu Met His Glu Asp Thr Phe Cys Asn Val Lys Asn Leu Thr Tyr Val
            275                 280                 285

cgt aag gca tta gag gat att cga ctg gat gga aac cct atc aac ctc 1033
Arg Lys Ala Leu Glu Asp Ile Arg Leu Asp Gly Asn Pro Ile Asn Leu
        290                 295                 300

agc aga act cct caa gcc tac atg tgt cta cct cgt ttg ccc att ggg 1081
Ser Arg Thr Pro Gln Ala Tyr Met Cys Leu Pro Arg Leu Pro Ile Gly
    305                 310                 315

agt ttt atc taa tgttaggatg ctaggcaaat atcagaattg tgatgctttc 1133
Ser Phe Ile
320

tgtagccaag agaagcatat atctcttcag aagcaaactc agtatatttg agatgcgttt 1193

aaaacatgtg gcacaatgat ataaacaata agctaaaggt gttgagataa aataagagtt 1253

caataattta attaaacatg attaggtata aattaacaaa atatatagac agtaagttaa 1313

ataaaatgta tgccaactag tcatcaaatc tcatcaagaa ttgtaggtgt tcatgtcaag 1373

ttaaatatgt aagaaataaa atattggtaa tgaatacttt agatacgttg ctaagatgta 1433

gacattttaa ttaaacttct ctagtcttta ttttcactca tgtatgtttc ccacctgatt 1493

cagttaagag cagaaaaaaa tattaaatgt gtttgaaaga tatatggtct tgtgaaaaag 1553

tatttcttat aattgtagtg tgcaaataac tatgtaatag cagtatacat tccttcagtt 1613

tctaatgaca tctatcatct ataagaacat caactcattt tgcaaacttg gttagttgac 1673

agctacctgt attcctctac atacaaaaaa taaaatatga gatatagagc 1723

<210> 18
<211> 322
<212> PRT
<213> Mus musculus

<400> 18

Met Gly Met Leu Ala Arg Val Ala Leu Gly Leu Ile Ile Ile Asp Ala
1               5                   10                  15

Val Leu Ala Ala Pro Thr Thr Glu Leu Phe Asn Tyr Asp Ser Glu Val
            20                  25                  30

Tyr Asp Ala Ile Leu Glu Asp Thr Gly Thr Phe Tyr Asn Tyr Glu His
        35                  40                  45

Ile Pro Asp Asn His Val Glu Asn Glu Lys Val Ser Glu Arg Leu Ser
    50                  55                  60

Gly Asn Arg Glu Leu Leu Thr Pro Gly Pro Gln Leu Gly Asp Asn Gln
65                  70                  75                  80

Asp Glu Asp Lys Asp Glu Glu Ser Thr Pro Arg Leu Ile Asp Gly Ser

85                  90                  95

Ser Pro Gln Glu Pro Glu Phe Pro Gly Leu Leu Gly Pro His Thr Asn
            100                 105                 110

Glu Asp Phe Pro Thr Cys Leu Leu Cys Thr Cys Ile Ser Thr Thr Val
        115                 120                 125

Tyr Cys Asp Asp His Glu Leu Asp Ala Ile Pro Pro Leu Pro Lys Lys
    130                 135                 140

Thr Thr Tyr Phe Tyr Ser Arg Phe Asn Arg Ile Lys Lys Ile Asn Lys
145                 150                 155                 160

Asn Asp Phe Ala Ser Leu Asn Asp Leu Lys Arg Ile Asp Leu Thr Ser
                165                 170                 175

Asn Leu Ile Ser Glu Ile Asp Glu Asp Ala Phe Arg Lys Leu Pro His
            180                 185                 190

Leu Gln Glu Leu Val Leu Arg Asp Asn Lys Ile Lys Gln Leu Pro Glu
        195                 200                 205

Leu Pro Asn Thr Leu Thr Phe Ile Asp Ile Ser Asn Asn Arg Leu Gly
    210                 215                 220

Arg Lys Gly Ile Lys Gln Glu Ala Phe Lys Asp Met Tyr Asp Leu His
225                 230                 235                 240

His Leu Tyr Ile Thr Asp Asn Ser Leu Asp His Ile Pro Leu Pro Leu
                245                 250                 255

Pro Glu Ser Leu Arg Ala Leu His Leu Gln Asn Asn Asp Ile Leu Glu
            260                 265                 270

Met His Glu Asp Thr Phe Cys Asn Val Lys Asn Leu Thr Tyr Val Arg
        275                 280                 285

Lys Ala Leu Glu Asp Ile Arg Leu Asp Gly Asn Pro Ile Asn Leu Ser
    290                 295                 300

Arg Thr Pro Gln Ala Tyr Met Cys Leu Pro Arg Leu Pro Ile Gly Ser
305                 310                 315                 320

Phe Ile

<210>  19
<211>  4909
<212>  DNA
<213>  Mus musculus

<220>
<221> CDS
<222> (390)..(3296)

<400> 19
gcagctccga gctaggtgct atcgcaaggc cagagcgcac agcccggcgg agagagcaga 60

tccttgctca gatcgagtca aatcggggcc aaggcggagg acgaagagtc caggctccta 120

ttctggactt gttccccagc tccgggggcg cttctaggtc ctgcagcagc caggagtgcg 180

gagccaccaa ctcggtgctg gaatgaaaaa attcccgcgc gccagtgcag aatctttcta 240

agtgacccgg agcttcgggt gctagctctg cacgaacttt cccatcaaag tgatcgtgaa 300

ttttaagcat caggagcagg ccagcgaagc tctacgcgtc taaacgtcta tccagaccaa 360

gagttctctg cggtgcaggg tgcggtgcc atg cag cca aaa gtc cct ttg ggg 413
Met Gln Pro Lys Val Pro Leu Gly
1               5

tca cgc aag cag aag ccc tgc tcc gac atg ggg gac gtc cag cgg gca 461
Ser Arg Lys Gln Lys Pro Cys Ser Asp Met Gly Asp Val Gln Arg Ala
    10                  15                  20

gcg aga tct cgg ggc tct ctg tcc gca cac atg ctg ttg ctg ctc ctc 509
Ala Arg Ser Arg Gly Ser Leu Ser Ala His Met Leu Leu Leu Leu Leu
25                  30                  35                  40

gct tcc ata aca atg ctg cta tgt gcg cgg ggc gca cac ggg cgc ccc 557
Ala Ser Ile Thr Met Leu Leu Cys Ala Arg Gly Ala His Gly Arg Pro
                45                  50                  55

acg gag gaa gat gag gag ctg gtc ctg ccc tcg ctg gag cgc gcc ccg 605
Thr Glu Glu Asp Glu Glu Leu Val Leu Pro Ser Leu Glu Arg Ala Pro
            60                  65                  70

ggc cac gat tcc acc acc aca cgc ctt cgt ctg gac gcc ttt ggc cag 653
Gly His Asp Ser Thr Thr Thr Arg Leu Arg Leu Asp Ala Phe Gly Gln
        75                  80                  85

cag cta cat ctg aag ttg cag ccg gac agc ggt ttc ttg gcg cct ggc 701
Gln Leu His Leu Lys Leu Gln Pro Asp Ser Gly Phe Leu Ala Pro Gly
    90                  95                  100

ttc acc ctg cag act gtg ggg cgc agt ccc ggg tcc gag gca caa cat 749
Phe Thr Leu Gln Thr Val Gly Arg Ser Pro Gly Ser Glu Ala Gln His
105                 110                 115                 120

ctg gac ccc acc ggg gac ctg gct cac tgc ttc tac tct ggc acg gtg 797
Leu Asp Pro Thr Gly Asp Leu Ala His Cys Phe Tyr Ser Gly Thr Val
                125                 130                 135

aac ggt gat ccc ggc tct gcc gca gcc ctc agc ctc tgt gaa ggt gtg 845
Asn Gly Asp Pro Gly Ser Ala Ala Ala Leu Ser Leu Cys Glu Gly Val
            140                 145                 150

cgt ggt gcc ttc tac cta caa gga gag gag ttc ttc att cag cca gcg 893
Arg Gly Ala Phe Tyr Leu Gln Gly Glu Glu Phe Phe Ile Gln Pro Ala
        155                 160                 165

cct gga gtg gcc acc gag cgc ctg gcc cct gcc gtg ccc gag gag gag 941
Pro Gly Val Ala Thr Glu Arg Leu Ala Pro Ala Val Pro Glu Glu Glu
    170                 175                 180

tca tcc gca cgg ccg cag ttc cac atc ctg agg cga agg cgg cgg ggc 989
Ser Ser Ala Arg Pro Gln Phe His Ile Leu Arg Arg Arg Arg Arg Gly
185                 190                 195                 200

agt ggc ggc gcc aag tgc ggc gtc atg gac gac gag acc ctg cca acc 1037
Ser Gly Gly Ala Lys Cys Gly Val Met Asp Asp Glu Thr Leu Pro Thr
                205                 210                 215

agc gac tcg cga ccc gag agc cag aac acc cgg aac cag tgg cct gtg 1085
Ser Asp Ser Arg Pro Glu Ser Gln Asn Thr Arg Asn Gln Trp Pro Val
            220                 225                 230

cgg gac ccc acg cct cag gac gcg gga aag cca tca gga cca gga agc 1133
Arg Asp Pro Thr Pro Gln Asp Ala Gly Lys Pro Ser Gly Pro Gly Ser
        235                 240                 245

ata agg aag aag cga ttt gtg tcc agc ccc cgt tat gtg gaa acc atg 1181
Ile Arg Lys Lys Arg Phe Val Ser Ser Pro Arg Tyr Val Glu Thr Met
    250                 255                 260

ctc gtg gct gac cag tcc atg gcc gac ttc cac ggc agc ggt cta aag 1229
Leu Val Ala Asp Gln Ser Met Ala Asp Phe His Gly Ser Gly Leu Lys
265                 270                 275                 280

cat tac ctt cta acc ctg ttc tcg gtg gca gcc agg ttt tac aag cat 1277
His Tyr Leu Leu Thr Leu Phe Ser Val Ala Ala Arg Phe Tyr Lys His
                285                 290                 295

ccc agc att agg aat tca att agc ctg gtg gtg gtg aag atc ttg gtc 1325
Pro Ser Ile Arg Asn Ser Ile Ser Leu Val Val Val Lys Ile Leu Val
            300                 305                 310

ata tat gag gag cag aag gga cca gaa gtt acc tcc aat gca gct ctc 1373
Ile Tyr Glu Glu Gln Lys Gly Pro Glu Val Thr Ser Asn Ala Ala Leu
        315                 320                 325

acc ctt cgg aat ttc tgc aac tgg cag aaa caa cac aac agc ccc agt 1421
Thr Leu Arg Asn Phe Cys Asn Trp Gln Lys Gln His Asn Ser Pro Ser
    330                 335                 340

gac cgg gat cca gag cac tat gac act gca att ctg ttc acc aga cag 1469
Asp Arg Asp Pro Glu His Tyr Asp Thr Ala Ile Leu Phe Thr Arg Gln
345                 350                 355                 360

gat tta tgt ggc tcc cac acg tgt gac act ctc ggg atg gca gat gtt 1517
Asp Leu Cys Gly Ser His Thr Cys Asp Thr Leu Gly Met Ala Asp Val
                365                 370                 375

gga act gta tgt gac ccc agc agg agc tgc tca gtc ata gaa gat gat 1565
Gly Thr Val Cys Asp Pro Ser Arg Ser Cys Ser Val Ile Glu Asp Asp
            380                 385                 390

ggt ttg caa gcc gcc ttc acc aca gcc cac gaa ttg ggc cat gtg ttt 1613
Gly Leu Gln Ala Ala Phe Thr Thr Ala His Glu Leu Gly His Val Phe
        395                 400                 405

aac atg ccg cac gat gat gct aag cac tgt gcc agc ttg aat ggt gtg 1661
Asn Met Pro His Asp Asp Ala Lys His Cys Ala Ser Leu Asn Gly Val
    410                 415                 420

act ggc gat tct cat ctg atg gcc tcg atg ctc tcc agc tta gac cat 1709
Thr Gly Asp Ser His Leu Met Ala Ser Met Leu Ser Ser Leu Asp His
425                 430                 435                 440

agc cag ccc tgg tca cct tgc agt gcc tac atg gtc acg tcc ttc cta 1757
Ser Gln Pro Trp Ser Pro Cys Ser Ala Tyr Met Val Thr Ser Phe Leu
                445                 450                 455

gat aat gga cac ggg gaa tgt ttg atg gac aag ccc cag aat cca atc 1805
Asp Asn Gly His Gly Glu Cys Leu Met Asp Lys Pro Gln Asn Pro Ile
            460                 465                 470

aag ctc cct tct gat ctt ccc ggt acc ttg tac gat gcc aac cgc cag 1853

Lys Leu Pro Ser Asp Leu Pro Gly Thr Leu Tyr Asp Ala Asn Arg Gln
        475                 480                 485

tgt cag ttt aca ttc gga gag gaa tcc aag cac tgc cct gat gca gcc      1901
Cys Gln Phe Thr Phe Gly Glu Glu Ser Lys His Cys Pro Asp Ala Ala
    490                 495                 500

agc aca tgt act acc ctg tgg tgc act ggc acc tcc ggt ggc tta ctg      1949
Ser Thr Cys Thr Thr Leu Trp Cys Thr Gly Thr Ser Gly Gly Leu Leu
505                 510                 515                 520

gtg tgc caa aca aaa cac ttc cct tgg gca gat ggc acc agc tgt gga      1997
Val Cys Gln Thr Lys His Phe Pro Trp Ala Asp Gly Thr Ser Cys Gly
                525                 530                 535

gaa ggg aag tgg tgt gtc agt ggc aag tgc gtg aac aag aca gac atg      2045
Glu Gly Lys Trp Cys Val Ser Gly Lys Cys Val Asn Lys Thr Asp Met
            540                 545                 550

aag cat ttt gct act cct gtt cat gga agc tgg gga cca tgg gga ccg      2093
Lys His Phe Ala Thr Pro Val His Gly Ser Trp Gly Pro Trp Gly Pro
        555                 560                 565

tgg gga gac tgc tca aga acc tgt ggt ggt gga gtt caa tac aca atg      2141
Trp Gly Asp Cys Ser Arg Thr Cys Gly Gly Gly Val Gln Tyr Thr Met
    570                 575                 580

aga gaa tgt gac aac cca gtc cca aag aac gga ggg aag tac tgt gaa      2189
Arg Glu Cys Asp Asn Pro Val Pro Lys Asn Gly Gly Lys Tyr Cys Glu
585                 590                 595                 600

ggc aaa cga gtc cgc tac agg tcc tgt aac atc gag gac tgt cca gac      2237
Gly Lys Arg Val Arg Tyr Arg Ser Cys Asn Ile Glu Asp Cys Pro Asp
                605                 610                 615

aat aac gga aaa acg ttc aga gag gag cag tgc gag gcg cac aat gag      2285
Asn Asn Gly Lys Thr Phe Arg Glu Glu Gln Cys Glu Ala His Asn Glu
            620                 625                 630

ttt tcc aaa gct tcc ttt ggg aat gag ccc act gta gag tgg aca ccc      2333
Phe Ser Lys Ala Ser Phe Gly Asn Glu Pro Thr Val Glu Trp Thr Pro
        635                 640                 645

aag tac gcc ggc gtc tcg cca aag gac agg tgc aag ctc acc tgt gaa      2381
Lys Tyr Ala Gly Val Ser Pro Lys Asp Arg Cys Lys Leu Thr Cys Glu
    650                 655                 660

gcc aaa ggc att ggc tac ttt ttc gtc tta cag ccc aag gtt gta gat      2429
Ala Lys Gly Ile Gly Tyr Phe Phe Val Leu Gln Pro Lys Val Val Asp
665                 670                 675                 680

ggc act ccc tgt agt cca gac tct acc tct gtc tgt gtg caa ggg cag      2477
Gly Thr Pro Cys Ser Pro Asp Ser Thr Ser Val Cys Val Gln Gly Gln
                685                 690                 695

tgt gtg aaa gct ggc tgt gat cgc atc ata gac tcc aaa aag aag ttt      2525
Cys Val Lys Ala Gly Cys Asp Arg Ile Ile Asp Ser Lys Lys Lys Phe
            700                 705                 710

gat aag tgt ggc gtt tgt gga gga aac ggt tcc aca tgc aag aag atg      2573
Asp Lys Cys Gly Val Cys Gly Gly Asn Gly Ser Thr Cys Lys Lys Met
        715                 720                 725

tca gga ata gtc act agt aca aga cct ggg tat cat gac att gtc aca      2621
Ser Gly Ile Val Thr Ser Thr Arg Pro Gly Tyr His Asp Ile Val Thr
    730                 735                 740

att cct gct gga gcc acc aac att gaa gtg aaa cat cgg aat caa agg      2669
Ile Pro Ala Gly Ala Thr Asn Ile Glu Val Lys His Arg Asn Gln Arg

745                 750                 755                 760

ggg tcc aga aac aat ggc agc ttt ctg gct att aga gcc gct gat ggt      2717
Gly Ser Arg Asn Asn Gly Ser Phe Leu Ala Ile Arg Ala Ala Asp Gly
                765                 770                 775

acc tat att ctg aat gga aac ttc act ctg tcc aca cta gag caa gac      2765
Thr Tyr Ile Leu Asn Gly Asn Phe Thr Leu Ser Thr Leu Glu Gln Asp
            780                 785                 790

ctc acc tac aaa ggt act gtc tta agg tac agt ggt tcc tcg gct gcg      2813
Leu Thr Tyr Lys Gly Thr Val Leu Arg Tyr Ser Gly Ser Ser Ala Ala
        795                 800                 805

ctg gag aga atc cgc agc ttt agt cca ctc aaa gaa ccc tta acc atc      2861
Leu Glu Arg Ile Arg Ser Phe Ser Pro Leu Lys Glu Pro Leu Thr Ile
    810                 815                 820

cag gtt ctt atg gta ggc cat gct ctc cga ccc aaa att aaa ttc acc      2909
Gln Val Leu Met Val Gly His Ala Leu Arg Pro Lys Ile Lys Phe Thr
825                 830                 835                 840

tac ttt atg aag aag aag aca gag tca ttc aac gcc att ccc aca ttt      2957
Tyr Phe Met Lys Lys Lys Thr Glu Ser Phe Asn Ala Ile Pro Thr Phe
                845                 850                 855

tct gag tgg gtg att gaa gag tgg ggg gag tgc tcc aag aca tgc ggc      3005
Ser Glu Trp Val Ile Glu Glu Trp Gly Glu Cys Ser Lys Thr Cys Gly
            860                 865                 870

tca ggt tgg cag aga aga gta gtg cag tgc aga gac att aat gga cac      3053
Ser Gly Trp Gln Arg Arg Val Val Gln Cys Arg Asp Ile Asn Gly His
        875                 880                 885

cct gct tcc gaa tgt gca aag gaa gtg aag cca gcc agt acc aga cct      3101
Pro Ala Ser Glu Cys Ala Lys Glu Val Lys Pro Ala Ser Thr Arg Pro
    890                 895                 900

tgt gca gac ctt cct tgc cca cac tgg cag gtg ggg gat tgg tca cca      3149
Cys Ala Asp Leu Pro Cys Pro His Trp Gln Val Gly Asp Trp Ser Pro
905                 910                 915                 920

tgt tcc aaa act tgc ggg aag ggt tac aag aag aga acc ttg aaa tgt      3197
Cys Ser Lys Thr Cys Gly Lys Gly Tyr Lys Lys Arg Thr Leu Lys Cys
                925                 930                 935

gtg tcc cac gat ggg ggc gtg tta tca aat gag agc tgt gat cct ttg      3245
Val Ser His Asp Gly Gly Val Leu Ser Asn Glu Ser Cys Asp Pro Leu
            940                 945                 950

aag aag cca aag cat tac att gac ttt tgc aca ctg aca cag tgc agt      3293
Lys Lys Pro Lys His Tyr Ile Asp Phe Cys Thr Leu Thr Gln Cys Ser
        955                 960                 965

taa gaggcgttag aggacaaggt agcgtgggga gggctgatac actgagtgct      3346

ggagggatcc agtgagtcaa accagtaagc agtgaggtgt ggcaaggagg tgtgtgtagg      3406

ggatacatag caaaggaggt agatcaggac actaccctgc cagttacatt ctgataaggt      3466

agttaatgag gcacagtagc atctgaaaga ccatacagag cactaaggag ccccaaagca      3526

ctattagtat ctcttttctt atatctatcg cccaaataat tttcagagtc tggcagaagc      3586

cctgttgcac tgtactgact agatacttct tatcacaaag attgggaaag gcaaagcaga      3646

aagatggtaa gactgggttt caaacaaggc ttggtttcta tcactggagg caaggaggag      3706

gggacaaaca agatcattat tcgaagtcgc tggttgctgt ggttttacgg aaggttgatg      3766

catcattcct atcaacagtg aaaagttcag cttgttcaac gtgacagaaa ggctcatctc 3826

cgtgaaagag ctcctgattt cttcttacac catctcagtt cttaactata attcatgttg 3886

aggtagaaac aattcatcta tttataaaat gtacattgga aaaaaaaaag tgaagtttat 3946

gaggtacaca taaaaactga aggaaacaat gagcaacatg cctcctgctt tgcttcctcc 4006

tgaggtaaac ctgcctggcg attgaggttg tttaagatta tccatggctc acaagaggca 4066

gtaaaataat acatgttgtg ccagagttag aatggggtat agagatcagg gtcccatgag 4126

atggggaaca tggtgatcac tcatctcaca tgggaggctg ctgcagggta gcaggtccac 4186

tcctggcagc tggtccaaca gtcgtatcct ggtgaatgtc tgttcagctc ttctactgag 4246

agagaatatg actgtttcca tatgtatatg tatatagtaa aatatgttac tatgaattgc 4306

atgtacttta taagtattgg tgtgtctgtt ccttctaaga aggactatag tttataataa 4366

atgcctataa taacatattt atttttatac atttatttct aatgataaaa cctttaagtt 4426

atatcgcttt tgtaaaagtg catataaaaa tagagtattt atacaatata tgttaactag 4486

aaataataaa agaacacttt tgaatgtgta tgcctatttt ctggagtggg attaacttct 4546

gggcaagaaa tctgatgaga cacaaacatt ggacttcaag acagttttaa attttgggta 4606

aatgaactgt atttcctgtt tatagacgta ctaataaaaa agaagttgat gatgtcttta 4666

gtggtaagat tgttactaat gtggttggca aattgctgta aagagccaga tagtaagcat 4726

ttatggcatt gtaggctatc tttcctgcca caaccatgtg acagtgagtg ctttgtagga 4786

ctgagagcag ccataaatga catgtaaatg ataaactgtg gctgtgcttt aataaaactt 4846

tatttacaaa aacaaaaaaa aaaaaaaaaa aaaaaaaaaa aaaaaaaaaa aaaaaaaaaa 4906

aaa 4909

<210> 20
<211> 968
<212> PRT
<213> Mus musculus

<400> 20

Met Gln Pro Lys Val Pro Leu Gly Ser Arg Lys Gln Lys Pro Cys Ser
1               5                   10                  15

Asp Met Gly Asp Val Gln Arg Ala Ala Arg Ser Arg Gly Ser Leu Ser
            20                  25                  30

Ala His Met Leu Leu Leu Leu Leu Ala Ser Ile Thr Met Leu Leu Cys
        35                  40                  45

Ala Arg Gly Ala His Gly Arg Pro Thr Glu Glu Asp Glu Glu Leu Val
    50                  55                  60

Leu Pro Ser Leu Glu Arg Ala Pro Gly His Asp Ser Thr Thr Thr Arg
65                  70                  75                  80

Leu Arg Leu Asp Ala Phe Gly Gln Gln Leu His Leu Lys Leu Gln Pro
                85                  90                  95

Asp Ser Gly Phe Leu Ala Pro Gly Phe Thr Leu Gln Thr Val Gly Arg
            100                 105                 110

Ser Pro Gly Ser Glu Ala Gln His Leu Asp Pro Thr Gly Asp Leu Ala
        115                 120                 125

His Cys Phe Tyr Ser Gly Thr Val Asn Gly Asp Pro Gly Ser Ala Ala
    130                 135                 140

Ala Leu Ser Leu Cys Glu Gly Val Arg Gly Ala Phe Tyr Leu Gln Gly
145                 150                 155                 160

Glu Glu Phe Phe Ile Gln Pro Ala Pro Gly Val Ala Thr Glu Arg Leu
                165                 170                 175

Ala Pro Ala Val Pro Glu Glu Glu Ser Ser Ala Arg Pro Gln Phe His
            180                 185                 190

Ile Leu Arg Arg Arg Arg Arg Gly Ser Gly Gly Ala Lys Cys Gly Val
        195                 200                 205

Met Asp Asp Glu Thr Leu Pro Thr Ser Asp Ser Arg Pro Glu Ser Gln
    210                 215                 220

Asn Thr Arg Asn Gln Trp Pro Val Arg Asp Pro Thr Pro Gln Asp Ala
225                 230                 235                 240

Gly Lys Pro Ser Gly Pro Gly Ser Ile Arg Lys Lys Arg Phe Val Ser
                245                 250                 255

Ser Pro Arg Tyr Val Glu Thr Met Leu Val Ala Asp Gln Ser Met Ala
            260                 265                 270

Asp Phe His Gly Ser Gly Leu Lys His Tyr Leu Leu Thr Leu Phe Ser
        275                 280                 285

Val Ala Ala Arg Phe Tyr Lys His Pro Ser Ile Arg Asn Ser Ile Ser
    290                 295                 300

Leu Val Val Val Lys Ile Leu Val Ile Tyr Glu Glu Gln Lys Gly Pro
305                 310                 315                 320

Glu Val Thr Ser Asn Ala Ala Leu Thr Leu Arg Asn Phe Cys Asn Trp
                325                 330                 335

Gln Lys Gln His Asn Ser Pro Ser Asp Arg Asp Pro Glu His Tyr Asp
            340                 345                 350

Thr Ala Ile Leu Phe Thr Arg Gln Asp Leu Cys Gly Ser His Thr Cys

        355                 360                 365

Asp Thr Leu Gly Met Ala Asp Val Gly Thr Val Cys Asp Pro Ser Arg
    370                 375                 380

Ser Cys Ser Val Ile Glu Asp Asp Gly Leu Gln Ala Ala Phe Thr Thr
385                 390                 395                 400

Ala His Glu Leu Gly His Val Phe Asn Met Pro His Asp Asp Ala Lys
                405                 410                 415

His Cys Ala Ser Leu Asn Gly Val Thr Gly Asp Ser His Leu Met Ala
            420                 425                 430

Ser Met Leu Ser Ser Leu Asp His Ser Gln Pro Trp Ser Pro Cys Ser
        435                 440                 445

Ala Tyr Met Val Thr Ser Phe Leu Asp Asn Gly His Gly Glu Cys Leu
    450                 455                 460

Met Asp Lys Pro Gln Asn Pro Ile Lys Leu Pro Ser Asp Leu Pro Gly
465                 470                 475                 480

Thr Leu Tyr Asp Ala Asn Arg Gln Cys Gln Phe Thr Phe Gly Glu Glu
                485                 490                 495

Ser Lys His Cys Pro Asp Ala Ala Ser Thr Cys Thr Thr Leu Trp Cys
            500                 505                 510

Thr Gly Thr Ser Gly Gly Leu Leu Val Cys Gln Thr Lys His Phe Pro
        515                 520                 525

Trp Ala Asp Gly Thr Ser Cys Gly Glu Gly Lys Trp Cys Val Ser Gly
    530                 535                 540

Lys Cys Val Asn Lys Thr Asp Met Lys His Phe Ala Thr Pro Val His
545                 550                 555                 560

Gly Ser Trp Gly Pro Trp Gly Pro Trp Gly Asp Cys Ser Arg Thr Cys
                565                 570                 575

Gly Gly Gly Val Gln Tyr Thr Met Arg Glu Cys Asp Asn Pro Val Pro
            580                 585                 590

Lys Asn Gly Gly Lys Tyr Cys Glu Gly Lys Arg Val Arg Tyr Arg Ser
        595                 600                 605

Cys Asn Ile Glu Asp Cys Pro Asp Asn Asn Gly Lys Thr Phe Arg Glu
    610                 615                 620

Glu Gln Cys Glu Ala His Asn Glu Phe Ser Lys Ala Ser Phe Gly Asn
625                 630                 635                 640

Glu Pro Thr Val Glu Trp Thr Pro Lys Tyr Ala Gly Val Ser Pro Lys
                645                 650                 655

Asp Arg Cys Lys Leu Thr Cys Glu Ala Lys Gly Ile Gly Tyr Phe Phe
            660                 665                 670

Val Leu Gln Pro Lys Val Val Asp Gly Thr Pro Cys Ser Pro Asp Ser
        675                 680                 685

Thr Ser Val Cys Val Gln Gly Gln Cys Val Lys Ala Gly Cys Asp Arg
    690                 695                 700

Ile Ile Asp Ser Lys Lys Lys Phe Asp Lys Cys Gly Val Cys Gly Gly
705                 710                 715                 720

Asn Gly Ser Thr Cys Lys Lys Met Ser Gly Ile Val Thr Ser Thr Arg
                725                 730                 735

Pro Gly Tyr His Asp Ile Val Thr Ile Pro Ala Gly Ala Thr Asn Ile
            740                 745                 750

Glu Val Lys His Arg Asn Gln Arg Gly Ser Arg Asn Asn Gly Ser Phe
        755                 760                 765

Leu Ala Ile Arg Ala Ala Asp Gly Thr Tyr Ile Leu Asn Gly Asn Phe
    770                 775                 780

Thr Leu Ser Thr Leu Glu Gln Asp Leu Thr Tyr Lys Gly Thr Val Leu
785                 790                 795                 800

Arg Tyr Ser Gly Ser Ser Ala Ala Leu Glu Arg Ile Arg Ser Phe Ser
                805                 810                 815

Pro Leu Lys Glu Pro Leu Thr Ile Gln Val Leu Met Val Gly His Ala
            820                 825                 830

Leu Arg Pro Lys Ile Lys Phe Thr Tyr Phe Met Lys Lys Lys Thr Glu
        835                 840                 845

Ser Phe Asn Ala Ile Pro Thr Phe Ser Glu Trp Val Ile Glu Glu Trp
    850                 855                 860

Gly Glu Cys Ser Lys Thr Cys Gly Ser Gly Trp Gln Arg Arg Val Val
865                 870                 875                 880

Gln Cys Arg Asp Ile Asn Gly His Pro Ala Ser Glu Cys Ala Lys Glu
                885                 890                 895

Val Lys Pro Ala Ser Thr Arg Pro Cys Ala Asp Leu Pro Cys Pro His
            900                 905                 910

Trp Gln Val Gly Asp Trp Ser Pro Cys Ser Lys Thr Cys Gly Lys Gly
        915                 920                 925

Tyr Lys Lys Arg Thr Leu Lys Cys Val Ser His Asp Gly Gly Val Leu
    930                 935                 940

Ser Asn Glu Ser Cys Asp Pro Leu Lys Lys Pro Lys His Tyr Ile Asp
945                 950                 955                 960

Phe Cys Thr Leu Thr Gln Cys Ser
                965

<210>  21
<211>  3673
<212>  DNA
<213>  Mus musculus

<220>
<221>  CDS
<222>  (392)..(2929)

<400>  21
ggggagaacc cggggaagac ccacagatac acagaaacga gagagacaga agaggagaga      60

gacagagaca aagagacagc agcaggagga ggaggaggag gaggcaaaga cagggcgagc     120

actgaggcaa agccagacag ctgagcagag ggagaagcca aggagtcaca gacaaagacc     180

tgggacagaa aggcaaagga ggaagttttg gagcagaaag aacccgtggg cacttttcct     240

gagcccaggg gctgttttct tttcatcttc ctctgaaagt ccctagccca tttaaaaact     300

ttgcctcaga ttttggcaca agccccaaag cccttctggc tttaacgagg agccttccac     360

agttagggtg tggagcattt tggtgccgca g atg gcc tca atc cat ccc agc        412
                                   Met Ala Ser Ile His Pro Ser
                                   1               5

tgc agc ccg ggt acc atg tcc cag atg ggc ttg cat ccc agg agg ggc       460
Cys Ser Pro Gly Thr Met Ser Gln Met Gly Leu His Pro Arg Arg Gly
        10                  15                  20

ttg act ggg cac tgg ctg caa aga ttc caa ccc tgc ttg ccg ctt cac       508
Leu Thr Gly His Trp Leu Gln Arg Phe Gln Pro Cys Leu Pro Leu His
    25                  30                  35

act gtg cag tgg cgg agg ctg ctg ctg ctg gcc ttc ctc ctg tcc tta       556
Thr Val Gln Trp Arg Arg Leu Leu Leu Leu Ala Phe Leu Leu Ser Leu
40                  45                  50                  55

gcg tgg ccc gcc agc ccc ctc ccc cgg gag gag gag atc gtg ttt cca       604
Ala Trp Pro Ala Ser Pro Leu Pro Arg Glu Glu Glu Ile Val Phe Pro
                60                  65                  70

gag aag ctc aat ggc agt agc atc cta cct gga tca ggc gtt cct gcc       652
Glu Lys Leu Asn Gly Ser Ser Ile Leu Pro Gly Ser Gly Val Pro Ala
            75                  80                  85

agg ctg ctg tac cga ttg cca gcc ttt ggg gag atg ttg cta cta gaa       700
Arg Leu Leu Tyr Arg Leu Pro Ala Phe Gly Glu Met Leu Leu Leu Glu
        90                  95                  100

cta gaa cag gac cct ggg gtg cag gta gag ggt ttg act gta cag tac       748

Leu Glu Gln Asp Pro Gly Val Gln Val Glu Gly Leu Thr Val Gln Tyr
    105                 110                 115

ctg ggc cag gca cct gag atg ctg ggt ggg gca gag cca ggt acc tac      796
Leu Gly Gln Ala Pro Glu Met Leu Gly Gly Ala Glu Pro Gly Thr Tyr
120                 125                 130                 135

ctg act ggc acc atc aat gga gat ccg gag tcg gtg gca tct ctg cac      844
Leu Thr Gly Thr Ile Asn Gly Asp Pro Glu Ser Val Ala Ser Leu His
                140                 145                 150

tgg gac ggg gga gcc cta tta ggg gta ctg cag tac cgt ggg gcc gaa      892
Trp Asp Gly Gly Ala Leu Leu Gly Val Leu Gln Tyr Arg Gly Ala Glu
            155                 160                 165

ctc cac ctc cag cct ctg gaa gga ggc gcc ctt aac tct gct ggg gga      940
Leu His Leu Gln Pro Leu Glu Gly Gly Ala Leu Asn Ser Ala Gly Gly
    170                 175                 180

ccg ggg gct cac atc cta cgc cgg aag agt cct gcc agc agc caa ggt      988
Pro Gly Ala His Ile Leu Arg Arg Lys Ser Pro Ala Ser Ser Gln Gly
    185                 190                 195

ccc atg tgc acc gtc aag gct cct tct ggg agc ccg agt ccc att tcc      1036
Pro Met Cys Thr Val Lys Ala Pro Ser Gly Ser Pro Ser Pro Ile Ser
200                 205                 210                 215

cgc aga acc aag cgc ttc gct tct ctg agt aga ttc gtg gag aca ctg      1084
Arg Arg Thr Lys Arg Phe Ala Ser Leu Ser Arg Phe Val Glu Thr Leu
                220                 225                 230

gtg gta gca gat gac aag atg gca gca ttc cat ggt aca ggg tta aag      1132
Val Val Ala Asp Asp Lys Met Ala Ala Phe His Gly Thr Gly Leu Lys
            235                 240                 245

cgc tac ctg ctg acg gtt atg gca gct gcc gct aaa gcc ttt aaa cac      1180
Arg Tyr Leu Leu Thr Val Met Ala Ala Ala Ala Lys Ala Phe Lys His
    250                 255                 260

cca agc atc cga aac cct gtc aac ttg gtg gtg acg cgc ctg gtg atc      1228
Pro Ser Ile Arg Asn Pro Val Asn Leu Val Val Thr Arg Leu Val Ile
    265                 270                 275

ctg ggg tcc ggc cag gaa ggg ccc caa gtg ggg cca agt gcc gcc cag      1276
Leu Gly Ser Gly Gln Glu Gly Pro Gln Val Gly Pro Ser Ala Ala Gln
280                 285                 290                 295

acc cta cgc agc ttc tgc acc tgg cag cgg ggc ctc aac acc cct aac      1324
Thr Leu Arg Ser Phe Cys Thr Trp Gln Arg Gly Leu Asn Thr Pro Asn
                300                 305                 310

gac tca gat cct gac cac ttt gac aca gcc att ctg ttc acc cgg cag      1372
Asp Ser Asp Pro Asp His Phe Asp Thr Ala Ile Leu Phe Thr Arg Gln
            315                 320                 325

gac ctg tgt ggg gtc tcc act tgt gac acc ctg ggt atg gct gat gtg      1420
Asp Leu Cys Gly Val Ser Thr Cys Asp Thr Leu Gly Met Ala Asp Val
    330                 335                 340

ggc aca gtg tgt gat cca gct agg agc tgt gct att gtg gaa gat gat      1468
Gly Thr Val Cys Asp Pro Ala Arg Ser Cys Ala Ile Val Glu Asp Asp
    345                 350                 355

ggg ctc cag tca gcc ttc act gct gct cat gaa ctg ggc cat gtc ttc      1516
Gly Leu Gln Ser Ala Phe Thr Ala Ala His Glu Leu Gly His Val Phe
360                 365                 370                 375

aac atg ctc cat gat aac tcc aag cca tgc act aac ttg aat ggg cag      1564
Asn Met Leu His Asp Asn Ser Lys Pro Cys Thr Asn Leu Asn Gly Gln

                380                 385                 390

ggg ggt tcc tct cgc cat gtc atg gct cct gtc atg gcc cat gtg gac 1612
Gly Gly Ser Ser Arg His Val Met Ala Pro Val Met Ala His Val Asp
            395                 400                 405

cct gaa gag ccc tgg tcg ccc tgc agt gcc cga ttc atc act gac ttc 1660
Pro Glu Glu Pro Trp Ser Pro Cys Ser Ala Arg Phe Ile Thr Asp Phe
        410                 415                 420

ctg gac aat ggt tat ggg cac tgc ctc tta gac aaa ccg gag gct ccc 1708
Leu Asp Asn Gly Tyr Gly His Cys Leu Leu Asp Lys Pro Glu Ala Pro
    425                 430                 435

ctc cat cta cca gcg act ttt cct ggc aag gac tat gac gct gac cgc 1756
Leu His Leu Pro Ala Thr Phe Pro Gly Lys Asp Tyr Asp Ala Asp Arg
440                 445                 450                 455

caa tgc caa ctg acc ttc ggt cct gac tca agc cat tgt cca cag ctg 1804
Gln Cys Gln Leu Thr Phe Gly Pro Asp Ser Ser His Cys Pro Gln Leu
                460                 465                 470

cca ccg ccc tgt gct gcc ctc tgg tgc tct ggc cac ctc aat ggc cat 1852
Pro Pro Pro Cys Ala Ala Leu Trp Cys Ser Gly His Leu Asn Gly His
            475                 480                 485

gcc atg tgc cag acg aag cac tca cct tgg gct gat ggc act ccc tgc 1900
Ala Met Cys Gln Thr Lys His Ser Pro Trp Ala Asp Gly Thr Pro Cys
        490                 495                 500

ggg tct tca cag gcc tgc atg ggt ggc cgc tgt ctg cac gtg gac cag 1948
Gly Ser Ser Gln Ala Cys Met Gly Gly Arg Cys Leu His Val Asp Gln
    505                 510                 515

ctc aag gac ttc aat gtt cct cag gct gga ggc tgg ggc ccc tgg gga 1996
Leu Lys Asp Phe Asn Val Pro Gln Ala Gly Gly Trp Gly Pro Trp Gly
520                 525                 530                 535

cca tgg ggt gac tgc tcc agg act tgt ggg ggt ggt gtc cag ttc tcc 2044
Pro Trp Gly Asp Cys Ser Arg Thr Cys Gly Gly Gly Val Gln Phe Ser
                540                 545                 550

tcc cgg gat tgc acg agg ccc gtc ccc cgg aac ggt ggc aag tat tgt 2092
Ser Arg Asp Cys Thr Arg Pro Val Pro Arg Asn Gly Gly Lys Tyr Cys
            555                 560                 565

gag ggc cgc cgg act cgc ttc cgc tcc tgc aac acg gag aac tgc cca 2140
Glu Gly Arg Arg Thr Arg Phe Arg Ser Cys Asn Thr Glu Asn Cys Pro
        570                 575                 580

cac ggc tca gca ttg acc ttc cgt gaa gag cag tgt gct gcc tac aac 2188
His Gly Ser Ala Leu Thr Phe Arg Glu Glu Gln Cys Ala Ala Tyr Asn
    585                 590                 595

cac cga acc gac ctc ttc aag agc ttt cca ggg ccc atg gac tgg gtt 2236
His Arg Thr Asp Leu Phe Lys Ser Phe Pro Gly Pro Met Asp Trp Val
600                 605                 610                 615

ccg cgc tac aca ggt gtg gcc cct cga gac caa tgc aaa ctc acc tgc 2284
Pro Arg Tyr Thr Gly Val Ala Pro Arg Asp Gln Cys Lys Leu Thr Cys
                620                 625                 630

cag gcc cgg gca ctg ggc tac tac tac gta ttg gag ccc cgg gtg gca 2332
Gln Ala Arg Ala Leu Gly Tyr Tyr Tyr Val Leu Glu Pro Arg Val Ala
            635                 640                 645

gat ggg act ccc tgc tcc cca gac acc tcc tct gtc tgt gtc cag ggc 2380
Asp Gly Thr Pro Cys Ser Pro Asp Thr Ser Ser Val Cys Val Gln Gly
        650                 655                 660

cgc tgt atc cat gct ggc tgt gac cgg atc att ggc tcc aaa aag aaa 2428
Arg Cys Ile His Ala Gly Cys Asp Arg Ile Ile Gly Ser Lys Lys Lys
    665                 670                 675

ttt gac aag tgc atg gtg tgc ggc ggg gat ggc tct cgc tgc agc aag 2476
Phe Asp Lys Cys Met Val Cys Gly Gly Asp Gly Ser Arg Cys Ser Lys
680                 685                 690                 695

cag tcg ggc tcc ttc aaa aaa ttc agg tat gga tac agc gat gtg gtc 2524
Gln Ser Gly Ser Phe Lys Lys Phe Arg Tyr Gly Tyr Ser Asp Val Val
                700                 705                 710

acg atc cct gcg ggg gcc acc cat atc ctt gta cgg cag cag ggg ggg 2572
Thr Ile Pro Ala Gly Ala Thr His Ile Leu Val Arg Gln Gln Gly Gly
            715                 720                 725

tct ggt ctc aag agc atc tac ctg gcc ctg aag ctt tct gac ggt tct 2620
Ser Gly Leu Lys Ser Ile Tyr Leu Ala Leu Lys Leu Ser Asp Gly Ser
        730                 735                 740

tac gcc ctc aat ggt gaa tac acg ctg atg ccc tcc cca aca gat gtg 2668
Tyr Ala Leu Asn Gly Glu Tyr Thr Leu Met Pro Ser Pro Thr Asp Val
    745                 750                 755

gtt ctt cct ggg gca gtc agc ttg cgc tac agc gga gcc aca gca gcc 2716
Val Leu Pro Gly Ala Val Ser Leu Arg Tyr Ser Gly Ala Thr Ala Ala
760                 765                 770                 775

tca gag aca ctg tct gga cat ggg ccg ctg gcc cag ccc ttg acg ctg 2764
Ser Glu Thr Leu Ser Gly His Gly Pro Leu Ala Gln Pro Leu Thr Leu
                780                 785                 790

caa gtc ctg gtg gct ggc aac cca cag aat gca cgt ctg cgg tac agt 2812
Gln Val Leu Val Ala Gly Asn Pro Gln Asn Ala Arg Leu Arg Tyr Ser
            795                 800                 805

ttc ttt gtc ccg cgg cca gtc cct tca aca cca cgc cct cct ccc caa 2860
Phe Phe Val Pro Arg Pro Val Pro Ser Thr Pro Arg Pro Pro Pro Gln
        810                 815                 820

gac tgg ctg caa cgc agg gca gag ata ctg aag atc ctt cgg aag cgt 2908
Asp Trp Leu Gln Arg Arg Ala Glu Ile Leu Lys Ile Leu Arg Lys Arg
    825                 830                 835

ccc tgg gca ggc cgg aaa taa cctcactgtc ccggctgccc tttttgggcg 2959
Pro Trp Ala Gly Arg Lys
840                 845

ccggggcctc ggactcatct gggagaatga gcaggcttct gcaactgcct cctgctaaaa 3019

cacagtaggg aggtgtagag ggtgagatct gcctgcctca ctgccccaaa ccgcaggctg 3079

gccctgccct ggcttcctgc cctgggaggc agtgatgtct tggtgaatgg aaaggggcta 3139

ggtgacagta ccctatctac taaactgccc cctctaccct gcaggtcaca ggaggaatgg 3199

ggggaagaca gggtgggtcc tgggccctag ttgtatttat ttggtattta ttcattttta 3259

tttagcacca ggaaagggga ctagggtctt ggggaaactc acctattata gccctaacct 3319

agctatgaaa tccagggtgt tggtgacaaa tatgagtggt gtgtgtgtgt gtgtgtgtgt 3379

gtgtgtgtgt gtttatgtat gaggtacaac ctgccctgct ttcctctccc taattttttt 3439

tttttctgg gaaaaggaaa gtcaaaggta ggactgcctt cagggagtaa gggatgattg 3499

tgtttttaaa ttgaagtttg ctatttatat gctcttttg gagtcagaca aatgtgggtt 3559

atattctggc cccgcatctt tgagcattag ttttctcatg tgccaataat aatcccttag 3619

aaattggttg taaggattaa atgatgttaa taaagaacta gcatagagcc tctc 3673

<210> 22
<211> 845
<212> PRT
<213> Mus musculus

<400> 22

Met Ala Ser Ile His Pro Ser Cys Ser Pro Gly Thr Met Ser Gln Met
1               5                   10                  15

Gly Leu His Pro Arg Arg Gly Leu Thr Gly His Trp Leu Gln Arg Phe
            20                  25                  30

Gln Pro Cys Leu Pro Leu His Thr Val Gln Trp Arg Arg Leu Leu Leu
        35                  40                  45

Leu Ala Phe Leu Leu Ser Leu Ala Trp Pro Ala Ser Pro Leu Pro Arg
    50                  55                  60

Glu Glu Glu Ile Val Phe Pro Glu Lys Leu Asn Gly Ser Ser Ile Leu
65                  70                  75                  80

Pro Gly Ser Gly Val Pro Ala Arg Leu Leu Tyr Arg Leu Pro Ala Phe
                85                  90                  95

Gly Glu Met Leu Leu Leu Glu Leu Glu Gln Asp Pro Gly Val Gln Val
            100                 105                 110

Glu Gly Leu Thr Val Gln Tyr Leu Gly Gln Ala Pro Glu Met Leu Gly
        115                 120                 125

Gly Ala Glu Pro Gly Thr Tyr Leu Thr Gly Thr Ile Asn Gly Asp Pro
    130                 135                 140

Glu Ser Val Ala Ser Leu His Trp Asp Gly Gly Ala Leu Leu Gly Val
145                 150                 155                 160

Leu Gln Tyr Arg Gly Ala Glu Leu His Leu Gln Pro Leu Glu Gly Gly
                165                 170                 175

Ala Leu Asn Ser Ala Gly Gly Pro Gly Ala His Ile Leu Arg Arg Lys
            180                 185                 190

Ser Pro Ala Ser Ser Gln Gly Pro Met Cys Thr Val Lys Ala Pro Ser
        195                 200                 205

Gly Ser Pro Ser Pro Ile Ser Arg Arg Thr Lys Arg Phe Ala Ser Leu
    210                 215                 220

Ser Arg Phe Val Glu Thr Leu Val Val Ala Asp Asp Lys Met Ala Ala

225                 230                 235                 240

Phe His Gly Thr Gly Leu Lys Arg Tyr Leu Leu Thr Val Met Ala Ala
                245                 250                 255

Ala Ala Lys Ala Phe Lys His Pro Ser Ile Arg Asn Pro Val Asn Leu
            260                 265                 270

Val Val Thr Arg Leu Val Ile Leu Gly Ser Gly Gln Glu Gly Pro Gln
        275                 280                 285

Val Gly Pro Ser Ala Ala Gln Thr Leu Arg Ser Phe Cys Thr Trp Gln
    290                 295                 300

Arg Gly Leu Asn Thr Pro Asn Asp Ser Asp Pro Asp His Phe Asp Thr
305                 310                 315                 320

Ala Ile Leu Phe Thr Arg Gln Asp Leu Cys Gly Val Ser Thr Cys Asp
                325                 330                 335

Thr Leu Gly Met Ala Asp Val Gly Thr Val Cys Asp Pro Ala Arg Ser
            340                 345                 350

Cys Ala Ile Val Glu Asp Asp Gly Leu Gln Ser Ala Phe Thr Ala Ala
        355                 360                 365

His Glu Leu Gly His Val Phe Asn Met Leu His Asp Asn Ser Lys Pro
    370                 375                 380

Cys Thr Asn Leu Asn Gly Gln Gly Gly Ser Ser Arg His Val Met Ala
385                 390                 395                 400

Pro Val Met Ala His Val Asp Pro Glu Glu Pro Trp Ser Pro Cys Ser
                405                 410                 415

Ala Arg Phe Ile Thr Asp Phe Leu Asp Asn Gly Tyr Gly His Cys Leu
            420                 425                 430

Leu Asp Lys Pro Glu Ala Pro Leu His Leu Pro Ala Thr Phe Pro Gly
        435                 440                 445

Lys Asp Tyr Asp Ala Asp Arg Gln Cys Gln Leu Thr Phe Gly Pro Asp
    450                 455                 460

Ser Ser His Cys Pro Gln Leu Pro Pro Pro Cys Ala Ala Leu Trp Cys
465                 470                 475                 480

Ser Gly His Leu Asn Gly His Ala Met Cys Gln Thr Lys His Ser Pro
                485                 490                 495

Trp Ala Asp Gly Thr Pro Cys Gly Ser Ser Gln Ala Cys Met Gly Gly
            500                 505                 510

Arg Cys Leu His Val Asp Gln Leu Lys Asp Phe Asn Val Pro Gln Ala
        515                 520                 525

Gly Gly Trp Gly Pro Trp Gly Pro Trp Gly Asp Cys Ser Arg Thr Cys
    530                 535                 540

Gly Gly Gly Val Gln Phe Ser Ser Arg Asp Cys Thr Arg Pro Val Pro
545                 550                 555                 560

Arg Asn Gly Gly Lys Tyr Cys Glu Gly Arg Arg Thr Arg Phe Arg Ser
                565                 570                 575

Cys Asn Thr Glu Asn Cys Pro His Gly Ser Ala Leu Thr Phe Arg Glu
            580                 585                 590

Glu Gln Cys Ala Ala Tyr Asn His Arg Thr Asp Leu Phe Lys Ser Phe
        595                 600                 605

Pro Gly Pro Met Asp Trp Val Pro Arg Tyr Thr Gly Val Ala Pro Arg
    610                 615                 620

Asp Gln Cys Lys Leu Thr Cys Gln Ala Arg Ala Leu Gly Tyr Tyr Tyr
625                 630                 635                 640

Val Leu Glu Pro Arg Val Ala Asp Gly Thr Pro Cys Ser Pro Asp Thr
                645                 650                 655

Ser Ser Val Cys Val Gln Gly Arg Cys Ile His Ala Gly Cys Asp Arg
            660                 665                 670

Ile Ile Gly Ser Lys Lys Lys Phe Asp Lys Cys Met Val Cys Gly Gly
        675                 680                 685

Asp Gly Ser Arg Cys Ser Lys Gln Ser Gly Ser Phe Lys Lys Phe Arg
    690                 695                 700

Tyr Gly Tyr Ser Asp Val Val Thr Ile Pro Ala Gly Ala Thr His Ile
705                 710                 715                 720

Leu Val Arg Gln Gln Gly Gly Ser Gly Leu Lys Ser Ile Tyr Leu Ala
                725                 730                 735

Leu Lys Leu Ser Asp Gly Ser Tyr Ala Leu Asn Gly Glu Tyr Thr Leu
            740                 745                 750

Met Pro Ser Pro Thr Asp Val Val Leu Pro Gly Ala Val Ser Leu Arg
        755                 760                 765

Tyr Ser Gly Ala Thr Ala Ala Ser Glu Thr Leu Ser Gly His Gly Pro
    770                 775                 780

Leu Ala Gln Pro Leu Thr Leu Gln Val Leu Val Ala Gly Asn Pro Gln
785                 790                 795                 800

Asn Ala Arg Leu Arg Tyr Ser Phe Phe Val Pro Arg Pro Val Pro Ser
                805                 810                 815

Thr Pro Arg Pro Pro Pro Gln Asp Trp Leu Gln Arg Arg Ala Glu Ile
            820                 825                 830

Leu Lys Ile Leu Arg Lys Arg Pro Trp Ala Gly Arg Lys
        835                 840                 845

<210>  23
<211>  3002
<212>  DNA
<213>  Mus musculus

<220>
<221>  CDS
<222>  (18)..(2810)

<400>  23
ccggcgggca gcgcact atg cgg ctc gag tgg gcg tcc ttg ttg ctg cta      50
                   Met Arg Leu Glu Trp Ala Ser Leu Leu Leu Leu
                   1               5                   10

ctg ctg ctg ctg agc gcg tcc tgc ctg tcc ctg gcc gct gac agc ccc      98
Leu Leu Leu Leu Ser Ala Ser Cys Leu Ser Leu Ala Ala Asp Ser Pro
            15                  20                  25

gcc gcg gca cct gcc cag gat aaa acc agg cag cct cag gct gca gca      146
Ala Ala Ala Pro Ala Gln Asp Lys Thr Arg Gln Pro Gln Ala Ala Ala
        30                  35                  40

gcg gcc gcc gag ccg gac cag ccg cag ggg gag gaa aca cgg gag cga      194
Ala Ala Ala Glu Pro Asp Gln Pro Gln Gly Glu Glu Thr Arg Glu Arg
    45                  50                  55

ggc cat tta caa ccc ttg gcc ggg cag cgc agg agc ggc ggg ctg gtc      242
Gly His Leu Gln Pro Leu Ala Gly Gln Arg Arg Ser Gly Gly Leu Val
60                  65                  70                  75

cat aat ata gac caa ctc tac tct ggc ggt ggc aaa gtg ggc tac ctt      290
His Asn Ile Asp Gln Leu Tyr Ser Gly Gly Gly Lys Val Gly Tyr Leu
                80                  85                  90

gtc tac gcg ggc ggc cgg agg ttc ctg ctg gac ctg gag aga gat gac      338
Val Tyr Ala Gly Gly Arg Arg Phe Leu Leu Asp Leu Glu Arg Asp Asp
            95                  100                 105

aca gtg ggt gct gct ggt agc atc gtt act gca gga gga ggg ctg agc      386
Thr Val Gly Ala Ala Gly Ser Ile Val Thr Ala Gly Gly Gly Leu Ser
        110                 115                 120

gca tcc tct ggc cac cgg ggt cac tgt ttc tac aga ggc acc gtg gac      434
Ala Ser Ser Gly His Arg Gly His Cys Phe Tyr Arg Gly Thr Val Asp
    125                 130                 135

ggc agc cct cga tcc cta gct gtc ttt gac ctc tgc ggg ggt ctc gat      482
Gly Ser Pro Arg Ser Leu Ala Val Phe Asp Leu Cys Gly Gly Leu Asp
140                 145                 150                 155

ggc ttc ttt gca gtc aag cat gcg cgc tac act cta aag cca ctc ctg      530

Gly Phe Phe Ala Val Lys His Ala Arg Tyr Thr Leu Lys Pro Leu Leu
                160                 165                 170

cgt ggg tcc tgg gca gag tat gaa cga att tat ggg gat gga tct tcc 578
Arg Gly Ser Trp Ala Glu Tyr Glu Arg Ile Tyr Gly Asp Gly Ser Ser
            175                 180                 185

cgc atc ctg cat gtc tac aac cgc gag ggc ttt agc ttc gag gcc ctg 626
Arg Ile Leu His Val Tyr Asn Arg Glu Gly Phe Ser Phe Glu Ala Leu
        190                 195                 200

ccg cca cgc gcc agt tgc gag act cct gca tcc cca tct ggg ccc caa 674
Pro Pro Arg Ala Ser Cys Glu Thr Pro Ala Ser Pro Ser Gly Pro Gln
    205                 210                 215

gag agc ccc tcg gtg cac agt aga tct agg aga cgc tca gcg ctg gcc 722
Glu Ser Pro Ser Val His Ser Arg Ser Arg Arg Arg Ser Ala Leu Ala
220                 225                 230                 235

ccg cag ctg ctg gac cac tca gct ttc tcg cca tct ggg aac gcg gga 770
Pro Gln Leu Leu Asp His Ser Ala Phe Ser Pro Ser Gly Asn Ala Gly
                240                 245                 250

cct cag act tgg tgg agg cgt agg cgc cgt tcc atc tcc agg gcc cgc 818
Pro Gln Thr Trp Trp Arg Arg Arg Arg Arg Ser Ile Ser Arg Ala Arg
            255                 260                 265

cag gtg gag ctc ctc ttg gtg gct gac tcg tcc atg gcc agg atg tat 866
Gln Val Glu Leu Leu Leu Val Ala Asp Ser Ser Met Ala Arg Met Tyr
        270                 275                 280

ggg cgg ggc ctg cag cat tac ctg ctg acc ctg gcc tcc atc gcc aac 914
Gly Arg Gly Leu Gln His Tyr Leu Leu Thr Leu Ala Ser Ile Ala Asn
    285                 290                 295

agg ctg tac agt cat gca agc att gag aac cac atc cgc ctg gcg gtg 962
Arg Leu Tyr Ser His Ala Ser Ile Glu Asn His Ile Arg Leu Ala Val
300                 305                 310                 315

gtg aag gtg gtg gtg ctg acg gac aag gac acg agt ctg gag gtg agc 1010
Val Lys Val Val Val Leu Thr Asp Lys Asp Thr Ser Leu Glu Val Ser
                320                 325                 330

aag aat gcg gcc acg acc ctc aag aac ttt tgc aaa tgg cag cac caa 1058
Lys Asn Ala Ala Thr Thr Leu Lys Asn Phe Cys Lys Trp Gln His Gln
            335                 340                 345

cat aac cag cta ggg gat gat cac gaa gag cac tac gat gca gcc atc 1106
His Asn Gln Leu Gly Asp Asp His Glu Glu His Tyr Asp Ala Ala Ile
        350                 355                 360

ctg ttc acc cga gag gat tta tgt ggg cat cat tca tgt gac acc ctg 1154
Leu Phe Thr Arg Glu Asp Leu Cys Gly His His Ser Cys Asp Thr Leu
    365                 370                 375

gga atg gca gac gtt ggg acc ata tgt tct ccg gag cgc agc tgt gca 1202
Gly Met Ala Asp Val Gly Thr Ile Cys Ser Pro Glu Arg Ser Cys Ala
380                 385                 390                 395

gtg att gaa gat gat ggc ctc cat gca gcc ttc act gtg gct cat gaa 1250
Val Ile Glu Asp Asp Gly Leu His Ala Ala Phe Thr Val Ala His Glu
                400                 405                 410

att ggg cat cta ctt ggc ctt tct cat gac gat tcc aaa ttc tgt gaa 1298
Ile Gly His Leu Leu Gly Leu Ser His Asp Asp Ser Lys Phe Cys Glu
            415                 420                 425

gag aac ttc ggt act aca gaa gac aag cgt tta atg tct tca atc ctt 1346
Glu Asn Phe Gly Thr Thr Glu Asp Lys Arg Leu Met Ser Ser Ile Leu

        430                 435                 440

acc agc atc gat gca tcc aag ccc tgg tcc aaa tgc acg tca gcc acc 1394
Thr Ser Ile Asp Ala Ser Lys Pro Trp Ser Lys Cys Thr Ser Ala Thr
    445                 450                 455

atc aca gaa ttc ctg gat gat ggt cat ggt aat tgt ttg cta gac cta 1442
Ile Thr Glu Phe Leu Asp Asp Gly His Gly Asn Cys Leu Leu Asp Leu
460                 465                 470                 475

cca cgg aag cag att ttg ggt ccc gag gaa ctc cca gga cag acc tac 1490
Pro Arg Lys Gln Ile Leu Gly Pro Glu Glu Leu Pro Gly Gln Thr Tyr
                480                 485                 490

gat gcc acc cag cag tgc aac ttg aca ttt ggg cct gag tac tcg gtg 1538
Asp Ala Thr Gln Gln Cys Asn Leu Thr Phe Gly Pro Glu Tyr Ser Val
            495                 500                 505

tgc cct ggc atg gat gtc tgt gcg cgg ctg tgg tgt gct gtg gtg cgc 1586
Cys Pro Gly Met Asp Val Cys Ala Arg Leu Trp Cys Ala Val Val Arg
        510                 515                 520

caa ggc caa atg gtg tgt ctg acc aag aag ctg ccg gct gtg gag ggc 1634
Gln Gly Gln Met Val Cys Leu Thr Lys Lys Leu Pro Ala Val Glu Gly
    525                 530                 535

act ccc tgt ggg aag gga aga gtc tgc ctt caa ggc aaa tgt gtg gac 1682
Thr Pro Cys Gly Lys Gly Arg Val Cys Leu Gln Gly Lys Cys Val Asp
540                 545                 550                 555

aaa act aag aaa aaa tat tac tcg aca tca agc cat gga aat tgg ggg 1730
Lys Thr Lys Lys Lys Tyr Tyr Ser Thr Ser Ser His Gly Asn Trp Gly
                560                 565                 570

tcc tgg ggc ccc tgg ggt cag tgt tct cgc tca tgc ggg gga gga gtg 1778
Ser Trp Gly Pro Trp Gly Gln Cys Ser Arg Ser Cys Gly Gly Gly Val
            575                 580                 585

cag ttt gcc tac cgc cat tgt aat aac cct gca cct cga aac agt ggc 1826
Gln Phe Ala Tyr Arg His Cys Asn Asn Pro Ala Pro Arg Asn Ser Gly
        590                 595                 600

cgc tac tgc aca ggg aag agg gcc ata tac cgt tcc tgc agt gtt aca 1874
Arg Tyr Cys Thr Gly Lys Arg Ala Ile Tyr Arg Ser Cys Ser Val Thr
    605                 610                 615

ccc tgc cca ccc aat ggt aaa tct ttt cgc cat gag cag tgt gaa gcc 1922
Pro Cys Pro Pro Asn Gly Lys Ser Phe Arg His Glu Gln Cys Glu Ala
620                 625                 630                 635

aaa aat ggc tat cag tct gat gca aaa gga gtc aaa aca ttt gta gaa 1970
Lys Asn Gly Tyr Gln Ser Asp Ala Lys Gly Val Lys Thr Phe Val Glu
                640                 645                 650

tgg gtt ccc aaa tat gca ggt gtc ctg ccg gca gat gtg tgc aag ctt 2018
Trp Val Pro Lys Tyr Ala Gly Val Leu Pro Ala Asp Val Cys Lys Leu
            655                 660                 665

acc tgc aga gct aag ggc aca ggc tac tat gtg gtc ttt tct cca aag 2066
Thr Cys Arg Ala Lys Gly Thr Gly Tyr Tyr Val Val Phe Ser Pro Lys
        670                 675                 680

gtt acg gat ggg act gaa tgc agg ccg tac agc aac tct gtg tgt gtc 2114
Val Thr Asp Gly Thr Glu Cys Arg Pro Tyr Ser Asn Ser Val Cys Val
    685                 690                 695

cga gga cgg tgt gtg aga act gga tgt gac ggc att att ggc tca aag 2162
Arg Gly Arg Cys Val Arg Thr Gly Cys Asp Gly Ile Ile Gly Ser Lys
700                 705                 710                 715

cta caa tat gac aag tgt gga gtg tgc gga ggg gat aac tcc agt tgt 2210
Leu Gln Tyr Asp Lys Cys Gly Val Cys Gly Gly Asp Asn Ser Ser Cys
                720                 725                 730

aca aag att atc gga acc ttc aat aaa aaa agc aag ggt tat act gac 2258
Thr Lys Ile Ile Gly Thr Phe Asn Lys Lys Ser Lys Gly Tyr Thr Asp
            735                 740                 745

gtt gtg agg atc cct gaa gga gca acc cac ata aaa gtc cga cag ttc 2306
Val Val Arg Ile Pro Glu Gly Ala Thr His Ile Lys Val Arg Gln Phe
        750                 755                 760

aaa gcc aaa gac cag act aga ttc cct gcc tac tta gcc ctg aag aag 2354
Lys Ala Lys Asp Gln Thr Arg Phe Pro Ala Tyr Leu Ala Leu Lys Lys
    765                 770                 775

aaa act ggc gag tac ctt atc aat ggc aag tac atg att tcc act tca 2402
Lys Thr Gly Glu Tyr Leu Ile Asn Gly Lys Tyr Met Ile Ser Thr Ser
780                 785                 790                 795

gag acc atc atc gac atc aat ggt acc gtc atg aac tac agt gga tgg 2450
Glu Thr Ile Ile Asp Ile Asn Gly Thr Val Met Asn Tyr Ser Gly Trp
                800                 805                 810

agc cac aga gat gat ttt tta cat ggg atg ggc tat tca gcc aca aaa 2498
Ser His Arg Asp Asp Phe Leu His Gly Met Gly Tyr Ser Ala Thr Lys
            815                 820                 825

gaa atc ctg atc gtg cag atc ctt gcc aca gac cca act aaa gcg cta 2546
Glu Ile Leu Ile Val Gln Ile Leu Ala Thr Asp Pro Thr Lys Ala Leu
        830                 835                 840

ggc gtc cgt tac agc ttt ttt gtt ccc aag aag acc act caa aaa gta 2594
Gly Val Arg Tyr Ser Phe Phe Val Pro Lys Lys Thr Thr Gln Lys Val
    845                 850                 855

aac tct gtc atc agc cat ggc agc aac aag gtg gga cca cac tct aca 2642
Asn Ser Val Ile Ser His Gly Ser Asn Lys Val Gly Pro His Ser Thr
860                 865                 870                 875

cag ctg cag tgg gtg aca ggt cca tgg ctg gcc tgc tcc agg acc tgt 2690
Gln Leu Gln Trp Val Thr Gly Pro Trp Leu Ala Cys Ser Arg Thr Cys
                880                 885                 890

gac aca ggc tgg cac act agg acc gtg cag tgc cag gat gga aac agg 2738
Asp Thr Gly Trp His Thr Arg Thr Val Gln Cys Gln Asp Gly Asn Arg
            895                 900                 905

aaa tta gct aaa gga tgc ctt ctc tct cag agg cct tct gca ttt aag 2786
Lys Leu Ala Lys Gly Cys Leu Leu Ser Gln Arg Pro Ser Ala Phe Lys
        910                 915                 920

caa tgt ctg ctg aag aaa tgt tag cctgtggttt actctaatgc acaaaaaaac 2840
Gln Cys Leu Leu Lys Lys Cys
    925                 930

aacaggagga tcatcgcaga tacagctgtg gtgaagacaa ggcctaccca aagcacagaa 2900

agtcatgcct tcatgtcatt gtcaccacga gtcgaattat gggcagaatc tgctctctgc 2960

gaccaaaagg tttactctac ttggtgaatg atggtaccgt ga 3002

<210> 24
<211> 930
<212> PRT
<213> Mus musculus

<400> 24

Met Arg Leu Glu Trp Ala Ser Leu Leu Leu Leu Leu Leu Leu Leu Ser
1               5                   10                  15

Ala Ser Cys Leu Ser Leu Ala Ala Asp Ser Pro Ala Ala Ala Pro Ala
            20                  25                  30

Gln Asp Lys Thr Arg Gln Pro Gln Ala Ala Ala Ala Ala Ala Glu Pro
        35                  40                  45

Asp Gln Pro Gln Gly Glu Glu Thr Arg Glu Arg Gly His Leu Gln Pro
    50                  55                  60

Leu Ala Gly Gln Arg Arg Ser Gly Gly Leu Val His Asn Ile Asp Gln
65                  70                  75                  80

Leu Tyr Ser Gly Gly Gly Lys Val Gly Tyr Leu Val Tyr Ala Gly Gly
                85                  90                  95

Arg Arg Phe Leu Leu Asp Leu Glu Arg Asp Asp Thr Val Gly Ala Ala
            100                 105                 110

Gly Ser Ile Val Thr Ala Gly Gly Gly Leu Ser Ala Ser Ser Gly His
        115                 120                 125

Arg Gly His Cys Phe Tyr Arg Gly Thr Val Asp Gly Ser Pro Arg Ser
    130                 135                 140

Leu Ala Val Phe Asp Leu Cys Gly Gly Leu Asp Gly Phe Phe Ala Val
145                 150                 155                 160

Lys His Ala Arg Tyr Thr Leu Lys Pro Leu Leu Arg Gly Ser Trp Ala
                165                 170                 175

Glu Tyr Glu Arg Ile Tyr Gly Asp Gly Ser Ser Arg Ile Leu His Val
            180                 185                 190

Tyr Asn Arg Glu Gly Phe Ser Phe Glu Ala Leu Pro Pro Arg Ala Ser
        195                 200                 205

Cys Glu Thr Pro Ala Ser Pro Ser Gly Pro Gln Glu Ser Pro Ser Val
    210                 215                 220

His Ser Arg Ser Arg Arg Arg Ser Ala Leu Ala Pro Gln Leu Leu Asp
225                 230                 235                 240

His Ser Ala Phe Ser Pro Ser Gly Asn Ala Gly Pro Gln Thr Trp Trp
                245                 250                 255

Arg Arg Arg Arg Arg Ser Ile Ser Arg Ala Arg Gln Val Glu Leu Leu
            260                 265                 270

Leu Val Ala Asp Ser Ser Met Ala Arg Met Tyr Gly Arg Gly Leu Gln
        275                 280                 285

His Tyr Leu Leu Thr Leu Ala Ser Ile Ala Asn Arg Leu Tyr Ser His
    290                 295                 300

Ala Ser Ile Glu Asn His Ile Arg Leu Ala Val Val Lys Val Val Val
305                 310                 315                 320

Leu Thr Asp Lys Asp Thr Ser Leu Glu Val Ser Lys Asn Ala Ala Thr
                325                 330                 335

Thr Leu Lys Asn Phe Cys Lys Trp Gln His Gln His Asn Gln Leu Gly
            340                 345                 350

Asp Asp His Glu Glu His Tyr Asp Ala Ala Ile Leu Phe Thr Arg Glu
        355                 360                 365

Asp Leu Cys Gly His His Ser Cys Asp Thr Leu Gly Met Ala Asp Val
    370                 375                 380

Gly Thr Ile Cys Ser Pro Glu Arg Ser Cys Ala Val Ile Glu Asp Asp
385                 390                 395                 400

Gly Leu His Ala Ala Phe Thr Val Ala His Glu Ile Gly His Leu Leu
                405                 410                 415

Gly Leu Ser His Asp Asp Ser Lys Phe Cys Glu Glu Asn Phe Gly Thr
            420                 425                 430

Thr Glu Asp Lys Arg Leu Met Ser Ser Ile Leu Thr Ser Ile Asp Ala
        435                 440                 445

Ser Lys Pro Trp Ser Lys Cys Thr Ser Ala Thr Ile Thr Glu Phe Leu
    450                 455                 460

Asp Asp Gly His Gly Asn Cys Leu Leu Asp Leu Pro Arg Lys Gln Ile
465                 470                 475                 480

Leu Gly Pro Glu Glu Leu Pro Gly Gln Thr Tyr Asp Ala Thr Gln Gln
                485                 490                 495

Cys Asn Leu Thr Phe Gly Pro Glu Tyr Ser Val Cys Pro Gly Met Asp
            500                 505                 510

Val Cys Ala Arg Leu Trp Cys Ala Val Val Arg Gln Gly Gln Met Val
        515                 520                 525

Cys Leu Thr Lys Lys Leu Pro Ala Val Glu Gly Thr Pro Cys Gly Lys
    530                 535                 540

Gly Arg Val Cys Leu Gln Gly Lys Cys Val Asp Lys Thr Lys Lys Lys
545                 550                 555                 560

Tyr Tyr Ser Thr Ser Ser His Gly Asn Trp Gly Ser Trp Gly Pro Trp
                565                 570                 575

Gly Gln Cys Ser Arg Ser Cys Gly Gly Gly Val Gln Phe Ala Tyr Arg
            580                 585                 590

His Cys Asn Asn Pro Ala Pro Arg Asn Ser Gly Arg Tyr Cys Thr Gly
        595                 600                 605

Lys Arg Ala Ile Tyr Arg Ser Cys Ser Val Thr Pro Cys Pro Pro Asn
    610                 615                 620

Gly Lys Ser Phe Arg His Glu Gln Cys Glu Ala Lys Asn Gly Tyr Gln
625                 630                 635                 640

Ser Asp Ala Lys Gly Val Lys Thr Phe Val Glu Trp Val Pro Lys Tyr
                645                 650                 655

Ala Gly Val Leu Pro Ala Asp Val Cys Lys Leu Thr Cys Arg Ala Lys
            660                 665                 670

Gly Thr Gly Tyr Tyr Val Val Phe Ser Pro Lys Val Thr Asp Gly Thr
        675                 680                 685

Glu Cys Arg Pro Tyr Ser Asn Ser Val Cys Val Arg Gly Arg Cys Val
    690                 695                 700

Arg Thr Gly Cys Asp Gly Ile Ile Gly Ser Lys Leu Gln Tyr Asp Lys
705                 710                 715                 720

Cys Gly Val Cys Gly Gly Asp Asn Ser Ser Cys Thr Lys Ile Ile Gly
                725                 730                 735

Thr Phe Asn Lys Lys Ser Lys Gly Tyr Thr Asp Val Val Arg Ile Pro
            740                 745                 750

Glu Gly Ala Thr His Ile Lys Val Arg Gln Phe Lys Ala Lys Asp Gln
        755                 760                 765

Thr Arg Phe Pro Ala Tyr Leu Ala Leu Lys Lys Lys Thr Gly Glu Tyr
    770                 775                 780

Leu Ile Asn Gly Lys Tyr Met Ile Ser Thr Ser Glu Thr Ile Ile Asp
785                 790                 795                 800

Ile Asn Gly Thr Val Met Asn Tyr Ser Gly Trp Ser His Arg Asp Asp
                805                 810                 815

Phe Leu His Gly Met Gly Tyr Ser Ala Thr Lys Glu Ile Leu Ile Val

820                 825                 830

Gln Ile Leu Ala Thr Asp Pro Thr Lys Ala Leu Gly Val Arg Tyr Ser
        835                 840                 845

Phe Phe Val Pro Lys Lys Thr Thr Gln Lys Val Asn Ser Val Ile Ser
    850                 855                 860

His Gly Ser Asn Lys Val Gly Pro His Ser Thr Gln Leu Gln Trp Val
865                 870                 875                 880

Thr Gly Pro Trp Leu Ala Cys Ser Arg Thr Cys Asp Thr Gly Trp His
                885                 890                 895

Thr Arg Thr Val Gln Cys Gln Asp Gly Asn Arg Lys Leu Ala Lys Gly
            900                 905                 910

Cys Leu Leu Ser Gln Arg Pro Ser Ala Phe Lys Gln Cys Leu Leu Lys
        915                 920                 925

Lys Cys
    930

<210>  25
<211>  3077
<212>  DNA
<213>  Mus musculus

<220>
<221>  CDS
<222>  (59)..(2200)

<400>  25
ccacgcgtcc ggccagcccc agggaccgca gcaatctccc aaactcccct tccccagg      58

atg aca gag gag tta atc acc cct gtg tac tgc acc ggg gtg tca gcc      106
Met Thr Glu Glu Leu Ile Thr Pro Val Tyr Cys Thr Gly Val Ser Ala
1               5                   10                  15

caa gta cag aag aag cgg gac aag gag ctg ggc ttg ggc cgc cat gaa      154
Gln Val Gln Lys Lys Arg Asp Lys Glu Leu Gly Leu Gly Arg His Glu
            20                  25                  30

aac gcc atc aag tac ctg ggc cag gat tat gaa acg ctt cgg gca aga      202
Asn Ala Ile Lys Tyr Leu Gly Gln Asp Tyr Glu Thr Leu Arg Ala Arg
        35                  40                  45

tgc ctg cag agt ggg gtc ctc ttc caa gac gag gcc ttc cct ccg gtt      250
Cys Leu Gln Ser Gly Val Leu Phe Gln Asp Glu Ala Phe Pro Pro Val
    50                  55                  60

tct cat agc ctg ggc ttc aag gaa ctg ggt cct cat tcc tct aaa acc      298
Ser His Ser Leu Gly Phe Lys Glu Leu Gly Pro His Ser Ser Lys Thr
65                  70                  75                  80

tat ggc atc aaa tgg aag cgg cct acg gaa ctg atg tca aac ccc cag      346
Tyr Gly Ile Lys Trp Lys Arg Pro Thr Glu Leu Met Ser Asn Pro Gln
                85                  90                  95

ttc atc gtg gat gga gcc acc cgg aca gac atc tgc cag gga gca ctg      394

Phe Ile Val Asp Gly Ala Thr Arg Thr Asp Ile Cys Gln Gly Ala Leu
            100                 105                 110

ggg gac tgt tgg ctc ctg gct gcc att gcc tcc ctc acc ctc aac gag 442
Gly Asp Cys Trp Leu Leu Ala Ala Ile Ala Ser Leu Thr Leu Asn Glu
        115                 120                 125

act att ctg cac cga gtg gtt ccc tac ggc cag agc ttt cag gat ggc 490
Thr Ile Leu His Arg Val Val Pro Tyr Gly Gln Ser Phe Gln Asp Gly
    130                 135                 140

tac gct ggc atc ttt cat ttc cag ctg tgg cag ttc ggg gag tgg gtc 538
Tyr Ala Gly Ile Phe His Phe Gln Leu Trp Gln Phe Gly Glu Trp Val
145                 150                 155                 160

gac gtg gtc ata gat gac ttg ctg ccc acc aag gac ggg aag ctg gtg 586
Asp Val Val Ile Asp Asp Leu Leu Pro Thr Lys Asp Gly Lys Leu Val
                165                 170                 175

ttc gtg cac tct gcc cag ggc aac gag ttc tgg agc gca ctg ctg gag 634
Phe Val His Ser Ala Gln Gly Asn Glu Phe Trp Ser Ala Leu Leu Glu
            180                 185                 190

aaa gcc tat gct aaa gtg aat ggc agc tat gag gct ctt tcg gga ggc 682
Lys Ala Tyr Ala Lys Val Asn Gly Ser Tyr Glu Ala Leu Ser Gly Gly
        195                 200                 205

tgc acc tca gag gcc ttc gag gac ttt acc ggt ggg gtc act gag tgg 730
Cys Thr Ser Glu Ala Phe Glu Asp Phe Thr Gly Gly Val Thr Glu Trp
    210                 215                 220

tac gac ctg cag aag gcc ccc agc gac ctc tac cag atc att cta aag 778
Tyr Asp Leu Gln Lys Ala Pro Ser Asp Leu Tyr Gln Ile Ile Leu Lys
225                 230                 235                 240

gcc ctg gaa cga ggc tcc ttg ctg ggc tgc tcc att aat atc tcc gat 826
Ala Leu Glu Arg Gly Ser Leu Leu Gly Cys Ser Ile Asn Ile Ser Asp
                245                 250                 255

atc cgt gat tta gag gct att act ttt aag aac ctg gtg agg ggc cat 874
Ile Arg Asp Leu Glu Ala Ile Thr Phe Lys Asn Leu Val Arg Gly His
            260                 265                 270

gcg tac tct gtg acg ggc gcc aag cag gta act tac cag ggc cag cgg 922
Ala Tyr Ser Val Thr Gly Ala Lys Gln Val Thr Tyr Gln Gly Gln Arg
        275                 280                 285

gtg aac cta atc cgg atg cgg aac ccc tgg ggt gaa gtg gag tgg aaa 970
Val Asn Leu Ile Arg Met Arg Asn Pro Trp Gly Glu Val Glu Trp Lys
    290                 295                 300

gga ccc tgg agt gac agc tcc tat gag tgg aac aaa gtg gac ccc tat 1018
Gly Pro Trp Ser Asp Ser Ser Tyr Glu Trp Asn Lys Val Asp Pro Tyr
305                 310                 315                 320

gaa cga gag cag ctg agg gtc aag atg gag gat ggg gag ttc tgg atg 1066
Glu Arg Glu Gln Leu Arg Val Lys Met Glu Asp Gly Glu Phe Trp Met
                325                 330                 335

tcg ttc cga gac ttc atc cgt gaa ttc acc aaa ctg gaa atc tgc aac 1114
Ser Phe Arg Asp Phe Ile Arg Glu Phe Thr Lys Leu Glu Ile Cys Asn
            340                 345                 350

ctt aca ccg gac gcc ctt aag agc agg acc ctc cgg aat tgg aat acc 1162
Leu Thr Pro Asp Ala Leu Lys Ser Arg Thr Leu Arg Asn Trp Asn Thr
        355                 360                 365

aca ttt tac gag ggc acc tgg cgt cgg gga agc acc gct gga ggc tgc 1210
Thr Phe Tyr Glu Gly Thr Trp Arg Arg Gly Ser Thr Ala Gly Gly Cys

370                 375                 380

agg aac tac cca gct acc ttc tgg gta aac ccc cag ttc aag atc cgg      1258
Arg Asn Tyr Pro Ala Thr Phe Trp Val Asn Pro Gln Phe Lys Ile Arg
385                 390                 395                 400

ttg gag gag gtg gat gac gca gac gac tat gac aac cgg gag tcg ggc      1306
Leu Glu Glu Val Asp Asp Ala Asp Asp Tyr Asp Asn Arg Glu Ser Gly
            405                 410                 415

tgc agc ttc ttg ctg gcc ctc atg cag aaa cac cgc cgc agg gag cgt      1354
Cys Ser Phe Leu Leu Ala Leu Met Gln Lys His Arg Arg Arg Glu Arg
        420                 425                 430

cgc ttt ggc cgg gac atg gag acc atc ggt ttt gca gtg tac cag gtc      1402
Arg Phe Gly Arg Asp Met Glu Thr Ile Gly Phe Ala Val Tyr Gln Val
    435                 440                 445

cct cgg gag ctg gcg ggt cag cct gtg cac ttg aag cgt gat ttc ttc      1450
Pro Arg Glu Leu Ala Gly Gln Pro Val His Leu Lys Arg Asp Phe Phe
    450                 455                 460

ctg gcc aac gct tct cgg gcg cag tca gag cac ttc atc aac ctt cgg      1498
Leu Ala Asn Ala Ser Arg Ala Gln Ser Glu His Phe Ile Asn Leu Arg
465                 470                 475                 480

gaa gtc agc aac cgt atc cgc ctg ccg ccc ggg gag tac ata gtg gtg      1546
Glu Val Ser Asn Arg Ile Arg Leu Pro Pro Gly Glu Tyr Ile Val Val
            485                 490                 495

ccc tcc acc ttc gag ccc aac aaa gaa ggc gac ttc ctg ctg cgc ttc      1594
Pro Ser Thr Phe Glu Pro Asn Lys Glu Gly Asp Phe Leu Leu Arg Phe
            500                 505                 510

ttc tca gag aag aag gct ggg acc cag gaa cta gat gac cag atc cag      1642
Phe Ser Glu Lys Lys Ala Gly Thr Gln Glu Leu Asp Asp Gln Ile Gln
        515                 520                 525

gcc aac ctc cct gat gag aaa gtt ctc tct gaa gag gag att gat gac      1690
Ala Asn Leu Pro Asp Glu Lys Val Leu Ser Glu Glu Glu Ile Asp Asp
    530                 535                 540

aac ttc aag acc ttg ttc agc aag ttg gca ggg gat gac atg gag atc      1738
Asn Phe Lys Thr Leu Phe Ser Lys Leu Ala Gly Asp Asp Met Glu Ile
545                 550                 555                 560

agc gtc aag gag cta cag acc atc ttg aac agg atc att agc aaa cac      1786
Ser Val Lys Glu Leu Gln Thr Ile Leu Asn Arg Ile Ile Ser Lys His
            565                 570                 575

aaa gac ctg cgc act aat ggc ttc agc ctg gag tcg tgc cgc agc atg      1834
Lys Asp Leu Arg Thr Asn Gly Phe Ser Leu Glu Ser Cys Arg Ser Met
        580                 585                 590

gtg aac ctc atg gat cga gat ggc aac ggg aag ctg ggt ctg gtg gag      1882
Val Asn Leu Met Asp Arg Asp Gly Asn Gly Lys Leu Gly Leu Val Glu
    595                 600                 605

ttc aac atc ctg tgg aac cgc atc cga aat tac ctg acc atc ttc cgg      1930
Phe Asn Ile Leu Trp Asn Arg Ile Arg Asn Tyr Leu Thr Ile Phe Arg
    610                 615                 620

aag ttt gac ctg gac aag tct ggc agc atg agt gcc tat gag atg agg      1978
Lys Phe Asp Leu Asp Lys Ser Gly Ser Met Ser Ala Tyr Glu Met Arg
625                 630                 635                 640

atg gcc atc gag gct gca ggc ttc aag ctt aac aag aag ctg cat gaa      2026
Met Ala Ile Glu Ala Ala Gly Phe Lys Leu Asn Lys Lys Leu His Glu
            645                 650                 655

ctc ata atc acc cgc tac tcg gag ccc gac ctg gcc gtg gac ttt gac     2074
Leu Ile Ile Thr Arg Tyr Ser Glu Pro Asp Leu Ala Val Asp Phe Asp
        660                 665                 670

aac ttt gtg tgc tgt ctt gtg cgg ctg gag acc atg ttc cgg ttt ttc     2122
Asn Phe Val Cys Cys Leu Val Arg Leu Glu Thr Met Phe Arg Phe Phe
        675                 680                 685

aaa ctt ctg gac aca gac ctg gat ggt gtt gtg acc ttt gat cta ttt     2170
Lys Leu Leu Asp Thr Asp Leu Asp Gly Val Val Thr Phe Asp Leu Phe
    690                 695                 700

aag tgg ctc cag ctg act atg ttt gcc tga agtggggaat cgctccgctc     2220
Lys Trp Leu Gln Leu Thr Met Phe Ala
705                 710

cagttgcctt tgttcttcca ttcttcctct gccaagccac gcctccccgc caagccacgc     2280

ctccccgccg tgccactcca gactacacca gcttctggtg ccttcctcag aacaggaagg     2340

aaggcaccct tgtcctcctg ttccccttgt cctcctgtcc tttcacctcc ccactacctc     2400

tgttcgactg ctctgggcag tcacggggcc ctccctgccc ctgtagtcag actgggcacc     2460

ctgacccctg ctcagggcta agaccaggaa gacagctccc tgctcatccc tgcctcgggg     2520

ctggggcagg ggcaggggca ggggcagggg caggcacccc tgcccctccc cctcgctgct     2580

actgtccgga tgtgagtctt gcctaatctg tggaggctct agcccctgtc atcccctaga     2640

gctgcagact tcataaccac tagctagctc cctgtctgca cccgggtgtt gaaggcctca     2700

cctcactgtg ggagccccac agccagactc accttcctgt gccttctgtg cagcagccaa     2760

gatccctctg caaaccaaac tacacctctc ctagcctgtg tgcccaccag gaacttcctg     2820

gccccgaggg tgctcaggcc ctccccatcc ccgacccctc tccctctctt tttttgtata     2880

tatactggtc attttcaagg gaatgcctga gggatgagtg tactggttat ggaggaactg     2940

ggggtggggt agggaggtcc cgtgtttcag tgcagtgaga aaataaaatg aaaggctgta     3000

aaaaaaaaaa aaaaaaaaaa aaaaaaaaaa aaaaaaaaaa aaaaaaaaaa aaaaaaaaaa     3060

aaaaaaaaaa aaaaaaa                                                    3077

<210>  26
<211>  713
<212>  PRT
<213>  Mus musculus

<400>  26

Met Thr Glu Glu Leu Ile Thr Pro Val Tyr Cys Thr Gly Val Ser Ala
1               5                   10                  15

Gln Val Gln Lys Lys Arg Asp Lys Glu Leu Gly Leu Gly Arg His Glu
            20                  25                  30

Asn Ala Ile Lys Tyr Leu Gly Gln Asp Tyr Glu Thr Leu Arg Ala Arg
        35                  40                  45

Cys Leu Gln Ser Gly Val Leu Phe Gln Asp Glu Ala Phe Pro Pro Val
    50                  55                  60

Ser His Ser Leu Gly Phe Lys Glu Leu Gly Pro His Ser Ser Lys Thr
65                  70                  75                  80

Tyr Gly Ile Lys Trp Lys Arg Pro Thr Glu Leu Met Ser Asn Pro Gln
                85                  90                  95

Phe Ile Val Asp Gly Ala Thr Arg Thr Asp Ile Cys Gln Gly Ala Leu
            100                 105                 110

Gly Asp Cys Trp Leu Leu Ala Ala Ile Ala Ser Leu Thr Leu Asn Glu
        115                 120                 125

Thr Ile Leu His Arg Val Val Pro Tyr Gly Gln Ser Phe Gln Asp Gly
    130                 135                 140

Tyr Ala Gly Ile Phe His Phe Gln Leu Trp Gln Phe Gly Glu Trp Val
145                 150                 155                 160

Asp Val Val Ile Asp Asp Leu Leu Pro Thr Lys Asp Gly Lys Leu Val
                165                 170                 175

Phe Val His Ser Ala Gln Gly Asn Glu Phe Trp Ser Ala Leu Leu Glu
            180                 185                 190

Lys Ala Tyr Ala Lys Val Asn Gly Ser Tyr Glu Ala Leu Ser Gly Gly
        195                 200                 205

Cys Thr Ser Glu Ala Phe Glu Asp Phe Thr Gly Gly Val Thr Glu Trp
    210                 215                 220

Tyr Asp Leu Gln Lys Ala Pro Ser Asp Leu Tyr Gln Ile Ile Leu Lys
225                 230                 235                 240

Ala Leu Glu Arg Gly Ser Leu Leu Gly Cys Ser Ile Asn Ile Ser Asp
                245                 250                 255

Ile Arg Asp Leu Glu Ala Ile Thr Phe Lys Asn Leu Val Arg Gly His
            260                 265                 270

Ala Tyr Ser Val Thr Gly Ala Lys Gln Val Thr Tyr Gln Gly Gln Arg
        275                 280                 285

Val Asn Leu Ile Arg Met Arg Asn Pro Trp Gly Glu Val Glu Trp Lys
    290                 295                 300

Gly Pro Trp Ser Asp Ser Ser Tyr Glu Trp Asn Lys Val Asp Pro Tyr
305                 310                 315                 320

Glu Arg Glu Gln Leu Arg Val Lys Met Glu Asp Gly Glu Phe Trp Met
                325                 330                 335

Ser Phe Arg Asp Phe Ile Arg Glu Phe Thr Lys Leu Glu Ile Cys Asn
            340                 345                 350

Leu Thr Pro Asp Ala Leu Lys Ser Arg Thr Leu Arg Asn Trp Asn Thr
        355                 360                 365

Thr Phe Tyr Glu Gly Thr Trp Arg Arg Gly Ser Thr Ala Gly Gly Cys
    370                 375                 380

Arg Asn Tyr Pro Ala Thr Phe Trp Val Asn Pro Gln Phe Lys Ile Arg
385                 390                 395                 400

Leu Glu Glu Val Asp Asp Ala Asp Asp Tyr Asp Asn Arg Glu Ser Gly
                405                 410                 415

Cys Ser Phe Leu Leu Ala Leu Met Gln Lys His Arg Arg Arg Glu Arg
            420                 425                 430

Arg Phe Gly Arg Asp Met Glu Thr Ile Gly Phe Ala Val Tyr Gln Val
        435                 440                 445

Pro Arg Glu Leu Ala Gly Gln Pro Val His Leu Lys Arg Asp Phe Phe
    450                 455                 460

Leu Ala Asn Ala Ser Arg Ala Gln Ser Glu His Phe Ile Asn Leu Arg
465                 470                 475                 480

Glu Val Ser Asn Arg Ile Arg Leu Pro Pro Gly Glu Tyr Ile Val Val
                485                 490                 495

Pro Ser Thr Phe Glu Pro Asn Lys Glu Gly Asp Phe Leu Leu Arg Phe
            500                 505                 510

Phe Ser Glu Lys Lys Ala Gly Thr Gln Glu Leu Asp Asp Gln Ile Gln
        515                 520                 525

Ala Asn Leu Pro Asp Glu Lys Val Leu Ser Glu Glu Glu Ile Asp Asp
    530                 535                 540

Asn Phe Lys Thr Leu Phe Ser Lys Leu Ala Gly Asp Asp Met Glu Ile
545                 550                 555                 560

Ser Val Lys Glu Leu Gln Thr Ile Leu Asn Arg Ile Ile Ser Lys His
                565                 570                 575

Lys Asp Leu Arg Thr Asn Gly Phe Ser Leu Glu Ser Cys Arg Ser Met
            580                 585                 590

Val Asn Leu Met Asp Arg Asp Gly Asn Gly Lys Leu Gly Leu Val Glu
        595                 600                 605

Phe Asn Ile Leu Trp Asn Arg Ile Arg Asn Tyr Leu Thr Ile Phe Arg
    610                 615                 620

Lys Phe Asp Leu Asp Lys Ser Gly Ser Met Ser Ala Tyr Glu Met Arg
625                 630                 635                 640

Met Ala Ile Glu Ala Ala Gly Phe Lys Leu Asn Lys Lys Leu His Glu
                645                 650                 655

Leu Ile Ile Thr Arg Tyr Ser Glu Pro Asp Leu Ala Val Asp Phe Asp
            660                 665                 670

Asn Phe Val Cys Cys Leu Val Arg Leu Glu Thr Met Phe Arg Phe Phe
        675                 680                 685

Lys Leu Leu Asp Thr Asp Leu Asp Gly Val Val Thr Phe Asp Leu Phe
    690                 695                 700

Lys Trp Leu Gln Leu Thr Met Phe Ala
705                 710

<210>  27
<211>  2105
<212>  DNA
<213>  Mus musculus

<220>
<221>  CDS
<222>  (418)..(1671)

<400>  27
gagccttccc ggcgcgtgag ccggatccgg tggcaccgcg gggaagagac aggaccgggc      60

ggtggcggca gagacagggg gacgcacccg gtgcagaaga tccaatagga gcacgccgcc     120

gcaacctctc ccgcgcgctc cggtcgccga ctctacgccg atcgcccact ccccgcacct     180

tggactacac cgggaaaaag acgcagcctg gcccgaactg gggcggagat ccttcttgct     240

tcccaagagc tcgggaggga agattctccc gccgccgaga accccgccgg actggaggaa     300

cccgtggcct ggagagcgct ggctgtgcca gaccccagcc tgatggatgt ctggtgtgga     360

taatgaggga agaacgtgcc ttttacaccc aagaggtgac cccggagcgt gccccgg        417

atg acc cca caa ctc gga acg atg cgg cta gcc tgc atg ttc tcg tcc       465
Met Thr Pro Gln Leu Gly Thr Met Arg Leu Ala Cys Met Phe Ser Ser
1               5                   10                  15

atc ctg ctg ttt gga gct gcg ggc ctg ctc ctc ttc atc agc ctc cag       513
Ile Leu Leu Phe Gly Ala Ala Gly Leu Leu Leu Phe Ile Ser Leu Gln
            20                  25                  30

gac cct ata gag ctc agc ccc cag caa gtt cca ggt ata aag ttc agc       561
Asp Pro Ile Glu Leu Ser Pro Gln Gln Val Pro Gly Ile Lys Phe Ser
        35                  40                  45

atc agg ccc cag caa ccc cag cat gat agc cac ttg agg ata tcc aca       609
Ile Arg Pro Gln Gln Pro Gln His Asp Ser His Leu Arg Ile Ser Thr
    50                  55                  60

gaa aag ggc aca cga gat tca ccc agc ggg tcg cca aga ggc ctc cag     657
Glu Lys Gly Thr Arg Asp Ser Pro Ser Gly Ser Pro Arg Gly Leu Gln
65                  70                  75                  80

ctg caa gcg cct gac caa cct cga cct cac ccg aag gca gcg gga tct     705
Leu Gln Ala Pro Asp Gln Pro Arg Pro His Pro Lys Ala Ala Gly Ser
                85                  90                  95

cct ttg cgc ctc cgg cag cgc agg cgg aga ctg ctc atc aaa aag atg     753
Pro Leu Arg Leu Arg Gln Arg Arg Arg Arg Leu Leu Ile Lys Lys Met
            100                 105                 110

cca gcc gca ggg act aac caa ggc aac aac tcg tcc gaa acc ttt atc     801
Pro Ala Ala Gly Thr Asn Gln Gly Asn Asn Ser Ser Glu Thr Phe Ile
        115                 120                 125

cag ccg aga ccc cgc acc atg gac agt cgt tgg gtc agc ctg cac cag     849
Gln Pro Arg Pro Arg Thr Met Asp Ser Arg Trp Val Ser Leu His Gln
    130                 135                 140

acc caa cag gag cgc aag cgt gtg atg cga gaa gcc tgc gct aaa tac     897
Thr Gln Gln Glu Arg Lys Arg Val Met Arg Glu Ala Cys Ala Lys Tyr
145                 150                 155                 160

agg gcc agc agc agc cgc aga gct gtc act ccc cgc cac gtc tcc cgc     945
Arg Ala Ser Ser Ser Arg Arg Ala Val Thr Pro Arg His Val Ser Arg
                165                 170                 175

atc ttc gtg gag gac cgc cac cgt gta ctg tac tgt gaa gta ccc aag     993
Ile Phe Val Glu Asp Arg His Arg Val Leu Tyr Cys Glu Val Pro Lys
            180                 185                 190

gca ggc tgc tcc aac tgg aag agg gtg ctc atg gtg ctg gca ggg tta     1041
Ala Gly Cys Ser Asn Trp Lys Arg Val Leu Met Val Leu Ala Gly Leu
        195                 200                 205

gcc tca tcc acg gca gat atc caa cac aac acc gtc cac tat ggc agc     1089
Ala Ser Ser Thr Ala Asp Ile Gln His Asn Thr Val His Tyr Gly Ser
    210                 215                 220

gcc ctt aag cgc ctg gat act ttt gac cgg cag ggc ata gtg cac cgc     1137
Ala Leu Lys Arg Leu Asp Thr Phe Asp Arg Gln Gly Ile Val His Arg
225                 230                 235                 240

ctc agt acc tac acc aag atg ctc ttt gtc cgg gaa ccc ttt gag cgg     1185
Leu Ser Thr Tyr Thr Lys Met Leu Phe Val Arg Glu Pro Phe Glu Arg
                245                 250                 255

ctg gtc tct gct ttc cga gac aag ttt gag cat cct aac agc tac tat     1233
Leu Val Ser Ala Phe Arg Asp Lys Phe Glu His Pro Asn Ser Tyr Tyr
            260                 265                 270

cat cct gtc ttt ggc aag gct atc ctg gcc cgg tac cgc gcc aac gcc     1281
His Pro Val Phe Gly Lys Ala Ile Leu Ala Arg Tyr Arg Ala Asn Ala
        275                 280                 285

tcg cgg gag gca ctg cgg act ggc tcc ggt gtg cag ttc ccc gag ttc     1329
Ser Arg Glu Ala Leu Arg Thr Gly Ser Gly Val Gln Phe Pro Glu Phe
    290                 295                 300

gtc cag tac ctg ttg gat gtc cac cgg ccc gtg ggc atg gac atc cac     1377
Val Gln Tyr Leu Leu Asp Val His Arg Pro Val Gly Met Asp Ile His
305                 310                 315                 320

tgg gac cat gtt agc cgg ctg tgc agc ccc tgc ctc atc gac tat gac     1425
Trp Asp His Val Ser Arg Leu Cys Ser Pro Cys Leu Ile Asp Tyr Asp
                325                 330                 335

ttt gtg ggc aag ttc gag agc atg gaa gac gat gcc aac ttc ttc ctg     1473

Phe Val Gly Lys Phe Glu Ser Met Glu Asp Asp Ala Asn Phe Phe Leu
            340                 345                 350

cgt ctc atc cat gcg ccc ggg aac ctg act ttc ccg agg ttc aag gac      1521
Arg Leu Ile His Ala Pro Gly Asn Leu Thr Phe Pro Arg Phe Lys Asp
        355                 360                 365

agg cac tcc gag gag gcg cgg acc aca tcg aga atc acc cat cag tac      1569
Arg His Ser Glu Glu Ala Arg Thr Thr Ser Arg Ile Thr His Gln Tyr
    370                 375                 380

ttc gct cag ctc tcc tcg ctg cag cga cag cga acc tac gac ttc tac      1617
Phe Ala Gln Leu Ser Ser Leu Gln Arg Gln Arg Thr Tyr Asp Phe Tyr
385                 390                 395                 400

tac atg gat tac ctg atg ttc aac tac tcc aaa cct ttc tcg gac ctg      1665
Tyr Met Asp Tyr Leu Met Phe Asn Tyr Ser Lys Pro Phe Ser Asp Leu
                405                 410                 415

tac tga gggcggggcc tgctggtcag gggcggggtc tgccggtcat gcccactcac      1721
Tyr

ctgcgcatag gcggcctccg gggactgagc tctgaggatg tgaggccttg tggctgtggc    1781

cctagggtgg gccacagagg cccagacaat ggaccttgac ccttgtccca cacccatttc    1841

ctcattgggt tggctgagtt tgagacggag cacgactcgg atggatgctt taagaactca    1901

gctgagctat gctgagctat gctgtccagg gaagcctgag acccagaaga gggccccagc    1961

gtcgagggat gtcctacatc cccttatcct ttgccttgta ccaaaccacg tggtttgctg    2021

cttttctatg acccagggtc atctgaataa agcacatggt tttcagagca aaaaaaaaaa    2081

aaaaaaaaaa aaaaaaaaaa aaaa                                           2105

<210>  28
<211>  417
<212>  PRT
<213>  Mus musculus

<400>  28

Met Thr Pro Gln Leu Gly Thr Met Arg Leu Ala Cys Met Phe Ser Ser
1               5                   10                  15

Ile Leu Leu Phe Gly Ala Ala Gly Leu Leu Leu Phe Ile Ser Leu Gln
            20                  25                  30

Asp Pro Ile Glu Leu Ser Pro Gln Gln Val Pro Gly Ile Lys Phe Ser
        35                  40                  45

Ile Arg Pro Gln Gln Pro Gln His Asp Ser His Leu Arg Ile Ser Thr
    50                  55                  60

Glu Lys Gly Thr Arg Asp Ser Pro Ser Gly Ser Pro Arg Gly Leu Gln
65                  70                  75                  80

Leu Gln Ala Pro Asp Gln Pro Arg Pro His Pro Lys Ala Ala Gly Ser
                85                  90                  95

Pro Leu Arg Leu Arg Gln Arg Arg Arg Arg Leu Leu Ile Lys Lys Met
            100                 105                 110

Pro Ala Ala Gly Thr Asn Gln Gly Asn Asn Ser Ser Glu Thr Phe Ile
        115                 120                 125

Gln Pro Arg Pro Arg Thr Met Asp Ser Arg Trp Val Ser Leu His Gln
    130                 135                 140

Thr Gln Gln Glu Arg Lys Arg Val Met Arg Glu Ala Cys Ala Lys Tyr
145                 150                 155                 160

Arg Ala Ser Ser Ser Arg Arg Ala Val Thr Pro Arg His Val Ser Arg
                165                 170                 175

Ile Phe Val Glu Asp Arg His Arg Val Leu Tyr Cys Glu Val Pro Lys
            180                 185                 190

Ala Gly Cys Ser Asn Trp Lys Arg Val Leu Met Val Leu Ala Gly Leu
        195                 200                 205

Ala Ser Ser Thr Ala Asp Ile Gln His Asn Thr Val His Tyr Gly Ser
    210                 215                 220

Ala Leu Lys Arg Leu Asp Thr Phe Asp Arg Gln Gly Ile Val His Arg
225                 230                 235                 240

Leu Ser Thr Tyr Thr Lys Met Leu Phe Val Arg Glu Pro Phe Glu Arg
                245                 250                 255

Leu Val Ser Ala Phe Arg Asp Lys Phe Glu His Pro Asn Ser Tyr Tyr
            260                 265                 270

His Pro Val Phe Gly Lys Ala Ile Leu Ala Arg Tyr Arg Ala Asn Ala
        275                 280                 285

Ser Arg Glu Ala Leu Arg Thr Gly Ser Gly Val Gln Phe Pro Glu Phe
    290                 295                 300

Val Gln Tyr Leu Leu Asp Val His Arg Pro Val Gly Met Asp Ile His
305                 310                 315                 320

Trp Asp His Val Ser Arg Leu Cys Ser Pro Cys Leu Ile Asp Tyr Asp
                325                 330                 335

Phe Val Gly Lys Phe Glu Ser Met Glu Asp Asp Ala Asn Phe Phe Leu
            340                 345                 350

Arg Leu Ile His Ala Pro Gly Asn Leu Thr Phe Pro Arg Phe Lys Asp
        355                 360                 365

Arg His Ser Glu Glu Ala Arg Thr Thr Ser Arg Ile Thr His Gln Tyr

370                 375                 380

Phe Ala Gln Leu Ser Ser Leu Gln Arg Gln Arg Thr Tyr Asp Phe Tyr
385                 390                 395                 400

Tyr Met Asp Tyr Leu Met Phe Asn Tyr Ser Lys Pro Phe Ser Asp Leu
                405                 410                 415

Tyr

<210>  29
<211>  1369
<212>  DNA
<213>  Mus musculus

<220>
<221>  CDS
<222>  (40)..(1281)

<400>  29
attcagacat gaagagagac acagtggtct gaagtggtc atg aaa gcc aaa caa      54
                                           Met Lys Ala Lys Gln
                                           1               5

gtc ttc ttt tct gtc ctg ctg ttt ggg aca gca ggg ctt ctg ctc ttc     102
Val Phe Phe Ser Val Leu Leu Phe Gly Thr Ala Gly Leu Leu Leu Phe
                10                  15                  20

atg tac ttg caa gca tgg att gaa gaa cat cat aca ggg aaa ata gag     150
Met Tyr Leu Gln Ala Trp Ile Glu Glu His His Thr Gly Lys Ile Glu
            25                  30                  35

aag aaa agg gat cag aaa gga gta tcg gtg act acg gga aaa atc cag     198
Lys Lys Arg Asp Gln Lys Gly Val Ser Val Thr Thr Gly Lys Ile Gln
        40                  45                  50

aaa cag atc acg aat cag aac tct gag gtt cac atg cct gaa gat ctg     246
Lys Gln Ile Thr Asn Gln Asn Ser Glu Val His Met Pro Glu Asp Leu
    55                  60                  65

aag aag aaa ggg gga gat ctg ctc aac cta ggg agt cca aca agg gtt     294
Lys Lys Lys Gly Gly Asp Leu Leu Asn Leu Gly Ser Pro Thr Arg Val
70                  75                  80                  85

tta agg aag atc agc cat tca caa agg gag aac gga gct tac aga tca     342
Leu Arg Lys Ile Ser His Ser Gln Arg Glu Asn Gly Ala Tyr Arg Ser
                90                  95                  100

act gaa gca cat caa gga gct aaa att gaa gtt ttt cag aaa ccc atc     390
Thr Glu Ala His Gln Gly Ala Lys Ile Glu Val Phe Gln Lys Pro Ile
            105                 110                 115

cag atg gac tgg cca ctg gtc act cag ccc tta aac aaa agt ttg gtc     438
Gln Met Asp Trp Pro Leu Val Thr Gln Pro Leu Asn Lys Ser Leu Val
        120                 125                 130

caa ggc aac aaa tgg aag aaa gca gat gca acc caa gag aag cgt cgg     486
Gln Gly Asn Lys Trp Lys Lys Ala Asp Ala Thr Gln Glu Lys Arg Arg
    135                 140                 145

tca ttc ctt cat gag ttt tgc aag aaa tat ggt aga gta aat gat ccc     534
Ser Phe Leu His Glu Phe Cys Lys Lys Tyr Gly Arg Val Asn Asp Pro
150                 155                 160                 165

aag ttc aac ctt ttt cat ata gta tct agg ata tat gta gaa gac aaa 582
Lys Phe Asn Leu Phe His Ile Val Ser Arg Ile Tyr Val Glu Asp Lys
                170                 175                 180

cac aaa atc ctg tac tgt gaa gta cca aaa gct ggc tgc tct aat tgg 630
His Lys Ile Leu Tyr Cys Glu Val Pro Lys Ala Gly Cys Ser Asn Trp
            185                 190                 195

aaa aga att ctg atg gtc cta aat gga ttg gct tcc tct gca tac aat 678
Lys Arg Ile Leu Met Val Leu Asn Gly Leu Ala Ser Ser Ala Tyr Asn
        200                 205                 210

atc tcc cat gat act gtg cac tat ggg aag cat ctg aaa aca ctg gat 726
Ile Ser His Asp Thr Val His Tyr Gly Lys His Leu Lys Thr Leu Asp
    215                 220                 225

agt ttt gac tta aaa gga gta cac atg cgt ttg aat aca tat acc aaa 774
Ser Phe Asp Leu Lys Gly Val His Met Arg Leu Asn Thr Tyr Thr Lys
230                 235                 240                 245

gct gtg ttt gtt aga gat ccc atg gaa aga tta gtc tcc gca ttt agg 822
Ala Val Phe Val Arg Asp Pro Met Glu Arg Leu Val Ser Ala Phe Arg
                250                 255                 260

gat aaa ttt gag cat ccc aat agt tac tac cat ccg gtg ttt gga aag 870
Asp Lys Phe Glu His Pro Asn Ser Tyr Tyr His Pro Val Phe Gly Lys
            265                 270                 275

gca att atc aag aaa tat cga cca aat gcc tct gca gaa gca tta aat 918
Ala Ile Ile Lys Lys Tyr Arg Pro Asn Ala Ser Ala Glu Ala Leu Asn
        280                 285                 290

aat gga tct gga gtc aaa ttc aaa gaa ttc gcc tac tat ttg ctg gat 966
Asn Gly Ser Gly Val Lys Phe Lys Glu Phe Ala Tyr Tyr Leu Leu Asp
    295                 300                 305

gct cac cgt cca gta gga atg gat att cac tgg gaa aga gtc agc aaa 1014
Ala His Arg Pro Val Gly Met Asp Ile His Trp Glu Arg Val Ser Lys
310                 315                 320                 325

ctg tgt tat ccg tgt ttg atc aac tat gac ttt gta ggg aag ttt gag 1062
Leu Cys Tyr Pro Cys Leu Ile Asn Tyr Asp Phe Val Gly Lys Phe Glu
                330                 335                 340

acc tta gga gag gat gcc aat tac ttt cta cag ttg att ggt gct cca 1110
Thr Leu Gly Glu Asp Ala Asn Tyr Phe Leu Gln Leu Ile Gly Ala Pro
            345                 350                 355

aaa gag ttg aca ttt cca aac ttt aag gat agg cac tcc tct gat gaa 1158
Lys Glu Leu Thr Phe Pro Asn Phe Lys Asp Arg His Ser Ser Asp Glu
        360                 365                 370

aga acc aat gcc cac gtg gta agg cag tat tta aag gac ctg agc aca 1206
Arg Thr Asn Ala His Val Val Arg Gln Tyr Leu Lys Asp Leu Ser Thr
    375                 380                 385

gcc gaa aga cag ctc atc tat gac ttc tat cac ttg gac tat ttg atg 1254
Ala Glu Arg Gln Leu Ile Tyr Asp Phe Tyr His Leu Asp Tyr Leu Met
390                 395                 400                 405

ttt aat tac aca act cca cat ttg taa tttgcattca tttttctaaa 1301
Phe Asn Tyr Thr Thr Pro His Leu
                410

gccctacata gatttaatga tgatggcctc aaataagcta ctgtaattgt cctacaattc 1361

tctgtatg 1369

<210>  30
<211>  413
<212>  PRT
<213>  Mus musculus

<400>  30

Met Lys Ala Lys Gln Val Phe Phe Ser Val Leu Leu Phe Gly Thr Ala
1               5                   10                  15

Gly Leu Leu Leu Phe Met Tyr Leu Gln Ala Trp Ile Glu Glu His His
            20                  25                  30

Thr Gly Lys Ile Glu Lys Lys Arg Asp Gln Lys Gly Val Ser Val Thr
        35                  40                  45

Thr Gly Lys Ile Gln Lys Gln Ile Thr Asn Gln Asn Ser Glu Val His
    50                  55                  60

Met Pro Glu Asp Leu Lys Lys Lys Gly Gly Asp Leu Leu Asn Leu Gly
65                  70                  75                  80

Ser Pro Thr Arg Val Leu Arg Lys Ile Ser His Ser Gln Arg Glu Asn
                85                  90                  95

Gly Ala Tyr Arg Ser Thr Glu Ala His Gln Gly Ala Lys Ile Glu Val
            100                 105                 110

Phe Gln Lys Pro Ile Gln Met Asp Trp Pro Leu Val Thr Gln Pro Leu
        115                 120                 125

Asn Lys Ser Leu Val Gln Gly Asn Lys Trp Lys Lys Ala Asp Ala Thr
    130                 135                 140

Gln Glu Lys Arg Arg Ser Phe Leu His Glu Phe Cys Lys Lys Tyr Gly
145                 150                 155                 160

Arg Val Asn Asp Pro Lys Phe Asn Leu Phe His Ile Val Ser Arg Ile
                165                 170                 175

Tyr Val Glu Asp Lys His Lys Ile Leu Tyr Cys Glu Val Pro Lys Ala
            180                 185                 190

Gly Cys Ser Asn Trp Lys Arg Ile Leu Met Val Leu Asn Gly Leu Ala
        195                 200                 205

Ser Ser Ala Tyr Asn Ile Ser His Asp Thr Val His Tyr Gly Lys His
    210                 215                 220

Leu Lys Thr Leu Asp Ser Phe Asp Leu Lys Gly Val His Met Arg Leu
225                 230                 235                 240

Asn Thr Tyr Thr Lys Ala Val Phe Val Arg Asp Pro Met Glu Arg Leu

                245                 250                 255

Val Ser Ala Phe Arg Asp Lys Phe Glu His Pro Asn Ser Tyr Tyr His
            260                 265                 270

Pro Val Phe Gly Lys Ala Ile Ile Lys Lys Tyr Arg Pro Asn Ala Ser
        275                 280                 285

Ala Glu Ala Leu Asn Asn Gly Ser Gly Val Lys Phe Lys Glu Phe Ala
    290                 295                 300

Tyr Tyr Leu Leu Asp Ala His Arg Pro Val Gly Met Asp Ile His Trp
305                 310                 315                 320

Glu Arg Val Ser Lys Leu Cys Tyr Pro Cys Leu Ile Asn Tyr Asp Phe
                325                 330                 335

Val Gly Lys Phe Glu Thr Leu Gly Glu Asp Ala Asn Tyr Phe Leu Gln
            340                 345                 350

Leu Ile Gly Ala Pro Lys Glu Leu Thr Phe Pro Asn Phe Lys Asp Arg
        355                 360                 365

His Ser Ser Asp Glu Arg Thr Asn Ala His Val Val Arg Gln Tyr Leu
    370                 375                 380

Lys Asp Leu Ser Thr Ala Glu Arg Gln Leu Ile Tyr Asp Phe Tyr His
385                 390                 395                 400

Leu Asp Tyr Leu Met Phe Asn Tyr Thr Thr Pro His Leu
                405                 410

<210> 31
<211> 4264
<212> DNA
<213> Mus musculus

<220>
<221> CDS
<222> (561)..(2246)

<400> 31
ggagtcaggt tgacgcgccc tgggtgcggg gtgctaggcg cggggctggg cgcgcgctcc 60

cgcggagtcg gagctgcaga ggcttccgag atcgtcttgg gcttttcagg tcacctagca 120

ttggtatgaa tctgctcctc aagaagagaa aatattctga aggcatgtcc gtttacctgt 180

caacgaggaa cccataatgt cttctcatct ccagtgtggc tccatgctct ctcctgatgc 240

tactccaact ttctttggtg ccatgggaaa aacctgaagc acgagcagag tcacctccaa 300

atctgtgctc acttcatttt gttgcatggc tggtgcggaa cagactattg gaatttgctc 360

ttgtgtgttg cccttcgtac ctgaaccaaa gactgtgcct cattctctag tgcattgctg 420

cctgctgtct tcctcgttca tttctgttgc ttgtgccttc ctactgtctt ctgtcatcac 480

aggacaccct taaagtttct cactggactc ctcgagggct ggcccatggc tctctaagcc 540

agctgtccaa ggtctctacc atg agg cat tgc att aat tgc tgc gtc cag ctg 593
Met Arg His Cys Ile Asn Cys Cys Val Gln Leu
1               5                   10

ttc ccc gaa gac aca cac aaa cag caa gtt gcc tgc caa gga ggc ccc 641
Phe Pro Glu Asp Thr His Lys Gln Gln Val Ala Cys Gln Gly Gly Pro
            15                  20                  25

cat cac agt cat cag gcg tgc ccc act tgc aaa gga gaa aac aaa att 689
His His Ser His Gln Ala Cys Pro Thr Cys Lys Gly Glu Asn Lys Ile
        30                  35                  40

ctg ttt cgt gtg gac agt aag cag atg aac ttg ctt gct gtt ctc gaa 737
Leu Phe Arg Val Asp Ser Lys Gln Met Asn Leu Leu Ala Val Leu Glu
    45                  50                  55

gtg agg act gag ggc aat gaa aac tgg ggt ggg ttt ttg cgc ttc agg 785
Val Arg Thr Glu Gly Asn Glu Asn Trp Gly Gly Phe Leu Arg Phe Arg
60                  65                  70                  75

aag ggg aag cga tgt agc ctg gtc ttt gga ttg ata ata atg acc ttg 833
Lys Gly Lys Arg Cys Ser Leu Val Phe Gly Leu Ile Ile Met Thr Leu
                80                  85                  90

gtg atg gct tct tac atc ctt tcc ggg gct cat cag gaa ctt ctg att 881
Val Met Ala Ser Tyr Ile Leu Ser Gly Ala His Gln Glu Leu Leu Ile
            95                  100                 105

tcc tcc cct ttc cat tat ggg ggc ttt ccc agc aac ccc agc gtg atg 929
Ser Ser Pro Phe His Tyr Gly Gly Phe Pro Ser Asn Pro Ser Val Met
        110                 115                 120

gat ggt gag aac ccc agt gat gtg aaa gag cat cac tac cag cct tct 977
Asp Gly Glu Asn Pro Ser Asp Val Lys Glu His His Tyr Gln Pro Ser
    125                 130                 135

gta aat aac atc tcc tat gta aag gac tat ccg agc att aaa ctg att 1025
Val Asn Asn Ile Ser Tyr Val Lys Asp Tyr Pro Ser Ile Lys Leu Ile
140                 145                 150                 155

atc gac agc att gct gcc agg att gag ttc aca acc agg cag ctc ccg 1073
Ile Asp Ser Ile Ala Ala Arg Ile Glu Phe Thr Thr Arg Gln Leu Pro
                160                 165                 170

gac tta caa gat ctc aag aga caa gag ttg cat atg ttt tct gta atc 1121
Asp Leu Gln Asp Leu Lys Arg Gln Glu Leu His Met Phe Ser Val Ile
            175                 180                 185

ccc agc aaa ttc ctc ccg act agc aag agc cct tgt tgg tat gag gag 1169
Pro Ser Lys Phe Leu Pro Thr Ser Lys Ser Pro Cys Trp Tyr Glu Glu
        190                 195                 200

ttc tcg ggc agg aac acc act gac ccc tac ctg acc aat tcc tac gtg 1217
Phe Ser Gly Arg Asn Thr Thr Asp Pro Tyr Leu Thr Asn Ser Tyr Val
    205                 210                 215

ctc tac tcc aag cgg ttc cgc tct acc ttt gac gct ctg cgc aag gtt 1265
Leu Tyr Ser Lys Arg Phe Arg Ser Thr Phe Asp Ala Leu Arg Lys Val
220                 225                 230                 235

ttc tgg ggc cac ctg tcc cac gtg cag ggc aag cac ttc cgc ctg cgc 1313
Phe Trp Gly His Leu Ser His Val Gln Gly Lys His Phe Arg Leu Arg
                240                 245                 250

tgc ctg ccc cac ttc tac atc att ggg cag ccc aag tgt gga acc act 1361
Cys Leu Pro His Phe Tyr Ile Ile Gly Gln Pro Lys Cys Gly Thr Thr

            255                 260                 265

gac ctg tac gac cgc ctc cgg ctg cat cca gaa gtg aaa ttc tca gcc 1409
Asp Leu Tyr Asp Arg Leu Arg Leu His Pro Glu Val Lys Phe Ser Ala
        270                 275                 280

atc aag gag ccg cac tgg tgg acc cgg aag cgc ttt gga att gtc cgc 1457
Ile Lys Glu Pro His Trp Trp Thr Arg Lys Arg Phe Gly Ile Val Arg
    285                 290                 295

ctg cgg gac gga cta cga gac cgc tac cct gtg gaa gat tac ctg gac 1505
Leu Arg Asp Gly Leu Arg Asp Arg Tyr Pro Val Glu Asp Tyr Leu Asp
300                 305                 310                 315

ctc ttt gac ttg gct gca cac cag atc cat caa gga ctg cag gct gcc 1553
Leu Phe Asp Leu Ala Ala His Gln Ile His Gln Gly Leu Gln Ala Ala
                320                 325                 330

tct gca gag cag ccc agc aag atg aat aag atc att att gga gag gcc 1601
Ser Ala Glu Gln Pro Ser Lys Met Asn Lys Ile Ile Ile Gly Glu Ala
            335                 340                 345

agc gcc tct aca atg tgg gat aac aat gcc tgg acc ttc ttc tat gac 1649
Ser Ala Ser Thr Met Trp Asp Asn Asn Ala Trp Thr Phe Phe Tyr Asp
        350                 355                 360

aac agc aca gac ggc gag cca cca ttc ctg acc caa gac ttc atc cat 1697
Asn Ser Thr Asp Gly Glu Pro Pro Phe Leu Thr Gln Asp Phe Ile His
    365                 370                 375

gcc ttt caa cca gaa gcc aag ctc att gtt atg ctc agg gat cct gtg 1745
Ala Phe Gln Pro Glu Ala Lys Leu Ile Val Met Leu Arg Asp Pro Val
380                 385                 390                 395

gag agg ttg tac tca gac tat ctc tac ttc gca agt tcc aat aaa tct 1793
Glu Arg Leu Tyr Ser Asp Tyr Leu Tyr Phe Ala Ser Ser Asn Lys Ser
                400                 405                 410

gca gat gac ttc cac gag aaa gtg acg gag gct ctg cag ctg ttt gaa 1841
Ala Asp Asp Phe His Glu Lys Val Thr Glu Ala Leu Gln Leu Phe Glu
            415                 420                 425

aat tgc atg ctg gat tat tca ctg cgc gcc tgc gtc tac aac aac acc 1889
Asn Cys Met Leu Asp Tyr Ser Leu Arg Ala Cys Val Tyr Asn Asn Thr
        430                 435                 440

ctg aac aat gcc atg ccc gtg agg ctc cag gtt ggc ctg tat gct gtg 1937
Leu Asn Asn Ala Met Pro Val Arg Leu Gln Val Gly Leu Tyr Ala Val
    445                 450                 455

tac ctc ctg gat tgg ctg act gtc ttc agt aag gag cag ttc ctc att 1985
Tyr Leu Leu Asp Trp Leu Thr Val Phe Ser Lys Glu Gln Phe Leu Ile
460                 465                 470                 475

ctc cgt ctg gaa gac cac gca tcc aat gtc aag tat acc atg cac aag 2033
Leu Arg Leu Glu Asp His Ala Ser Asn Val Lys Tyr Thr Met His Lys
                480                 485                 490

gtc ttc cag ttt cta aac ctg ggg cct ctg agt gag aag cag gaa gct 2081
Val Phe Gln Phe Leu Asn Leu Gly Pro Leu Ser Glu Lys Gln Glu Ala
            495                 500                 505

ctg atg acc aag agc cct gca tcc aac aca cgg cgt cct gag gat cgt 2129
Leu Met Thr Lys Ser Pro Ala Ser Asn Thr Arg Arg Pro Glu Asp Arg
        510                 515                 520

agt ctg gga ccc atg tgg ccc atc acc cag aag att ctg cgt gaa ttc 2177
Ser Leu Gly Pro Met Trp Pro Ile Thr Gln Lys Ile Leu Arg Glu Phe
    525                 530                 535

tat ggg cct ttc aac aca agg ctg gca cag gtc ttg gat gac gaa gca 2225
Tyr Gly Pro Phe Asn Thr Arg Leu Ala Gln Val Leu Asp Asp Glu Ala
540                 545                 550                 555

ttt gcc tgg aag aca acg tga gagctgagtc ccttctgcag aagctgggcc 2276
Phe Ala Trp Lys Thr Thr
                560

cactgactct gtcattgcca tgactttaaa agtctccagg cggagaactg tcacccacag 2336

cgtggagaaa gccgctggag ccccacccaa tcctcttctc atgtccaagg cacctggtgt 2396

gggaactatt ttaaagcttt ccttgccatg ttcagcagct ctgctcttga ttgcaaggct 2456

cgtccacctg aatccttgga aacgacactg ggggtaggct tctgtctgtt cccagagtgc 2516

cttggatctc aggagggcag tggatctctg gcacccagtg ggtaggaaga ggaagctacg 2576

ggaggccata gaccagcctt gccttccttc ctccaggaga gtttgtctgg gtgtagggtt 2636

ggacagctct gggatccgat taaggccatg ttctggggct gcccctcacg tgttatatcc 2696

cagcaggcct tgcataaatc gacaacccca tgtggacaga gaacagagct gcttggagaa 2756

tggaggctct ttcgtttttg ttcgtattcc caagtcagcc cttcggtgac atcagaaatg 2816

agccctgttc cctttgctgt gctcagaaga aactcagaag tcagcttttg gcagtaggct 2876

cgtctctctg acctattctg cccatacact ttgtttatgc ccagcgtaga gaaggagcgt 2936

tttaaaacaa actgcttctc gcatttgact ggccctgcct ctcttttctg ctcccttgga 2996

tgttttcctt aaggacttca gtttatattt ggaaagggaa gttttgctgt taatgccaga 3056

acagaacacc tcaaggcatc gaacagtgga aaaggaagga agtgaagccc tccccatttg 3116

tgggggtcac tggagagtgt ggggatgttt ttggttctgg ctggttacag gctcccccct 3176

gtcttcctgt tggaggaatc tttatccaca ttctctgtca tgtgcagcca aagttctgta 3236

actcactaag ggtgagtctc aagacaccct tagaatgtcc tcagaaacac atctcacaaa 3296

ctaagctcgg gccacatgtg gtgtcactag gagatagctt tgatttttat gaacctttcc 3356

aggcctgcta gcagcttggt tcgcaaagga tatgctgaaa gtcttgtcat tttaaaaaaa 3416

aaaaaagacc ccagggaata ccagagagaa caacttaaac ccacttggca cacaggacaa 3476

ggtggggatg tcacaaagct tttcattgga gcgttgccaa gggatgctgc ctgtgaagct 3536

gtgttctcag gtcaggatcc tgttcaccag cgtcagaaag tgtacacacc acatgcccga 3596

ctgtcacttg gacgtgtgag aaaagctggg ctttggcagc aggaggctgt gcagttgctc 3656

gactgtgtga gttctgctgc ggtccaccct tgttggagat gggactctca gctcattgga 3716

aggttccggg agagctctgg gcatcagcat ggactgggct cgttctgcta ctgaaggggc 3776

tgtgtgtcat gtggcctgtg ttccaggaca attctccctt ctctcacggt ggccacttgg 3836

ctcggcagcc agtgggaaaa tctagcctga gtacaaggat gctttgggaa gaaatgacga 3896

gagagagaga gagagagaga gagagagaga gagagagaga gagagcgaga gagagcgcaa 3956

gctcaagcgc aagcgtgtga gaaagagcac gagagagtat ttatttggaa taccgtgtgt 4016

gtgtgtgtgt gtgtgtgtgt gtgtgtgtgt gtgtgtgtgt gtgcgtgcgc gcgcgagtgt 4076

gcgcgcgaac actcgtgctt atactgagca gggccctctc agctgtcttg ttgatggggc 4136

ttctcctcca ttagagacat gatgccttta tgaattaatt tctccaaccc ttcgcctgtc 4196

atttccagga catatcttac tgccccacac caaccacgac tctaagagat tttttctgac 4256

gcactccc 4264

<210> 32
<211> 561
<212> PRT
<213> Mus musculus

<400> 32

Met Arg His Cys Ile Asn Cys Cys Val Gln Leu Phe Pro Glu Asp Thr
1               5                   10                  15

His Lys Gln Gln Val Ala Cys Gln Gly Gly Pro His His Ser His Gln
            20                  25                  30

Ala Cys Pro Thr Cys Lys Gly Glu Asn Lys Ile Leu Phe Arg Val Asp
        35                  40                  45

Ser Lys Gln Met Asn Leu Leu Ala Val Leu Glu Val Arg Thr Glu Gly
    50                  55                  60

Asn Glu Asn Trp Gly Gly Phe Leu Arg Phe Arg Lys Gly Lys Arg Cys
65                  70                  75                  80

Ser Leu Val Phe Gly Leu Ile Ile Met Thr Leu Val Met Ala Ser Tyr
                85                  90                  95

Ile Leu Ser Gly Ala His Gln Glu Leu Leu Ile Ser Ser Pro Phe His
            100                 105                 110

Tyr Gly Gly Phe Pro Ser Asn Pro Ser Val Met Asp Gly Glu Asn Pro
        115                 120                 125

Ser Asp Val Lys Glu His His Tyr Gln Pro Ser Val Asn Asn Ile Ser
    130                 135                 140

Tyr Val Lys Asp Tyr Pro Ser Ile Lys Leu Ile Ile Asp Ser Ile Ala
145                 150                 155                 160

Ala Arg Ile Glu Phe Thr Thr Arg Gln Leu Pro Asp Leu Gln Asp Leu
                165                 170                 175

Lys Arg Gln Glu Leu His Met Phe Ser Val Ile Pro Ser Lys Phe Leu
            180                 185                 190

Pro Thr Ser Lys Ser Pro Cys Trp Tyr Glu Glu Phe Ser Gly Arg Asn
        195                 200                 205

Thr Thr Asp Pro Tyr Leu Thr Asn Ser Tyr Val Leu Tyr Ser Lys Arg

210                 215                 220

Phe Arg Ser Thr Phe Asp Ala Leu Arg Lys Val Phe Trp Gly His Leu
225                 230                 235                 240

Ser His Val Gln Gly Lys His Phe Arg Leu Arg Cys Leu Pro His Phe
                245                 250                 255

Tyr Ile Ile Gly Gln Pro Lys Cys Gly Thr Thr Asp Leu Tyr Asp Arg
            260                 265                 270

Leu Arg Leu His Pro Glu Val Lys Phe Ser Ala Ile Lys Glu Pro His
        275                 280                 285

Trp Trp Thr Arg Lys Arg Phe Gly Ile Val Arg Leu Arg Asp Gly Leu
    290                 295                 300

Arg Asp Arg Tyr Pro Val Glu Asp Tyr Leu Asp Leu Phe Asp Leu Ala
305                 310                 315                 320

Ala His Gln Ile His Gln Gly Leu Gln Ala Ala Ser Ala Glu Gln Pro
                325                 330                 335

Ser Lys Met Asn Lys Ile Ile Ile Gly Glu Ala Ser Ala Ser Thr Met
            340                 345                 350

Trp Asp Asn Asn Ala Trp Thr Phe Phe Tyr Asp Asn Ser Thr Asp Gly
        355                 360                 365

Glu Pro Pro Phe Leu Thr Gln Asp Phe Ile His Ala Phe Gln Pro Glu
    370                 375                 380

Ala Lys Leu Ile Val Met Leu Arg Asp Pro Val Glu Arg Leu Tyr Ser
385                 390                 395                 400

Asp Tyr Leu Tyr Phe Ala Ser Ser Asn Lys Ser Ala Asp Asp Phe His
                405                 410                 415

Glu Lys Val Thr Glu Ala Leu Gln Leu Phe Glu Asn Cys Met Leu Asp
            420                 425                 430

Tyr Ser Leu Arg Ala Cys Val Tyr Asn Asn Thr Leu Asn Asn Ala Met
        435                 440                 445

Pro Val Arg Leu Gln Val Gly Leu Tyr Ala Val Tyr Leu Leu Asp Trp
    450                 455                 460

Leu Thr Val Phe Ser Lys Glu Gln Phe Leu Ile Leu Arg Leu Glu Asp
465                 470                 475                 480

His Ala Ser Asn Val Lys Tyr Thr Met His Lys Val Phe Gln Phe Leu
                485                 490                 495

Asn Leu Gly Pro Leu Ser Glu Lys Gln Glu Ala Leu Met Thr Lys Ser
            500                 505                 510

Pro Ala Ser Asn Thr Arg Arg Pro Glu Asp Arg Ser Leu Gly Pro Met
        515                 520                 525

Trp Pro Ile Thr Gln Lys Ile Leu Arg Glu Phe Tyr Gly Pro Phe Asn
    530                 535                 540

Thr Arg Leu Ala Gln Val Leu Asp Asp Glu Ala Phe Ala Trp Lys Thr
545                 550                 555                 560

Thr

<210>  33
<211>  1718
<212>  DNA
<213>  Mus musculus

<220>
<221>  CDS
<222>  (374)..(1564)

<400>  33
acaaagcccg gggggtccac gctggcgctg gaggcgagcc gggagcgagc ccggggcgcg      60

gcgggacttg gggcgcaggg ctgctacggg acgcggggct gctagcagga cgcgcgacca      120

ggcgtgggtt gcgatcgcca cccccacggc ggggcccggg acattttggg ccctttgggg      180

ccgcagcgag cggtggggac caagacgagc agagtaccct gagcccggca acttcggccc      240

ctccccgccc ccacccggct gccctcagcg cgtccctctc catgtgcaga cagccggcta      300

ggcgctccgg ctggcggcgg atgggtgacc tcttcctggc cggggcgggc tgttcgaggc      360

ggcggagccg gcg atg aag aag aag cag cag cag cat ccc ggc ggc ggc      409
               Met Lys Lys Lys Gln Gln Gln His Pro Gly Gly Gly
               1               5                   10

acg gat ccc tgg ccc cat ggg gcc cct gtg ggg ggc gcc cct ccg tgc      457
Thr Asp Pro Trp Pro His Gly Ala Pro Val Gly Gly Ala Pro Pro Cys
        15                  20                  25

ctg ggc agc tgc aag cgc cgg atc ccg ctg ctg cct ttc ctg cgc ttc      505
Leu Gly Ser Cys Lys Arg Arg Ile Pro Leu Leu Pro Phe Leu Arg Phe
    30                  35                  40

tcc ctc cgg gac tac ggt ttc tgc atg gct acc ctg gtg gtc ttc tgc      553
Ser Leu Arg Asp Tyr Gly Phe Cys Met Ala Thr Leu Val Val Phe Cys
45                  50                  55                  60

ctg ggc tcc ctc ttc tac cag ctc agc ggg gga ccc cct cgc ttc ctg      601
Leu Gly Ser Leu Phe Tyr Gln Leu Ser Gly Gly Pro Pro Arg Phe Leu
                65                  70                  75

ctc gac ctg cgg cag tat ttg gga aat tcc act tac ttg gat gac cat      649
Leu Asp Leu Arg Gln Tyr Leu Gly Asn Ser Thr Tyr Leu Asp Asp His
            80                  85                  90

gga cca cct cct agt aag gtc ctt ccc ttc ccc agc cag gtg gtg tac          697
Gly Pro Pro Pro Ser Lys Val Leu Pro Phe Pro Ser Gln Val Val Tyr
        95                  100                 105

aac aga gtt ggc aag tgt ggc agt cgt acc gtg gtc ttg ctt ctg aga          745
Asn Arg Val Gly Lys Cys Gly Ser Arg Thr Val Val Leu Leu Leu Arg
    110                 115                 120

atc ttg tca gag aag cac ggc ttc aat ttg gtc aca tca gac att cac          793
Ile Leu Ser Glu Lys His Gly Phe Asn Leu Val Thr Ser Asp Ile His
125                 130                 135                 140

aac aag acc cgg ctt act aaa aat gaa caa atg gaa ctg att aag aat          841
Asn Lys Thr Arg Leu Thr Lys Asn Glu Gln Met Glu Leu Ile Lys Asn
                145                 150                 155

ata agt acc gcc gaa cag ccc tat tta ttc acg aga cat gtc cac ttc          889
Ile Ser Thr Ala Glu Gln Pro Tyr Leu Phe Thr Arg His Val His Phe
            160                 165                 170

ctc aac ttc tcc agg ttt gga gga gac cag cct gtc tac atc aac atc          937
Leu Asn Phe Ser Arg Phe Gly Gly Asp Gln Pro Val Tyr Ile Asn Ile
        175                 180                 185

atc aga gac ccc gtc agt aga ttt ctg tct aac tat ttc ttc cgt cgc          985
Ile Arg Asp Pro Val Ser Arg Phe Leu Ser Asn Tyr Phe Phe Arg Arg
    190                 195                 200

ttt gga gat tgg aga ggg gaa cag aat cac atg atc cgc acc ccc agc          1033
Phe Gly Asp Trp Arg Gly Glu Gln Asn His Met Ile Arg Thr Pro Ser
205                 210                 215                 220

atg agg cag gag gag cgc tac ctg gac atc aat gag tgc att ctg gaa          1081
Met Arg Gln Glu Glu Arg Tyr Leu Asp Ile Asn Glu Cys Ile Leu Glu
                225                 230                 235

aac tac cct gag tgt tcc aac ccc agg ctg ttt tac atc atc cca tat          1129
Asn Tyr Pro Glu Cys Ser Asn Pro Arg Leu Phe Tyr Ile Ile Pro Tyr
            240                 245                 250

ttc tgt gga cag cac ccc aga tgt agg gag ccc ggc gag tgg gcg ctg          1177
Phe Cys Gly Gln His Pro Arg Cys Arg Glu Pro Gly Glu Trp Ala Leu
        255                 260                 265

gag cgg gcc aag ctg aac gtg aac gaa aac ttc ctg ctc gtg ggg atc          1225
Glu Arg Ala Lys Leu Asn Val Asn Glu Asn Phe Leu Leu Val Gly Ile
    270                 275                 280

ctg gag gag ctg gaa gat gtg ctg ctc tta ctg gaa aga ttt tta cct          1273
Leu Glu Glu Leu Glu Asp Val Leu Leu Leu Leu Glu Arg Phe Leu Pro
285                 290                 295                 300

cat tac ttc aag ggt gtg ctg agc atc tac aag gac cca gag cac agg          1321
His Tyr Phe Lys Gly Val Leu Ser Ile Tyr Lys Asp Pro Glu His Arg
                305                 310                 315

aaa gct tgg caa cat gac tgt gac cgt gag gaa gac tgt ccc ctc ccc          1369
Lys Ala Trp Gln His Asp Cys Asp Arg Glu Glu Asp Cys Pro Leu Pro
            320                 325                 330

cga ggc ggt gca gat tct cta cca gcg aat gag ata cga gta cga gtt          1417
Arg Gly Gly Ala Asp Ser Leu Pro Ala Asn Glu Ile Arg Val Arg Val
        335                 340                 345

cta cca cta cgt cag aga aca att cca cct gct caa gcg caa gct tgg          1465
Leu Pro Leu Arg Gln Arg Thr Ile Pro Pro Ala Gln Ala Gln Ala Trp
    350                 355                 360

act gaa gtc tcg cgt gag cgg acc tcc cgt gag acc aca gtt ctt cat          1513

Thr Glu Val Ser Arg Glu Arg Thr Ser Arg Glu Thr Thr Val Leu His
365                 370                 375                 380

ccc cac gcc cct gga gac gga gga gcc aat cga cga cga gga gca aga      1561
Pro His Ala Pro Gly Asp Gly Gly Ala Asn Arg Arg Arg Gly Ala Arg
                385                 390                 395

tga cgaaaaatgg ctagaagata tttataagag gtgatacctg cgccagattg      1614

tggcttctgt ggcctcatcc ccccttttac aaagttcttt ggggaagaga aattctgaag      1674

ggactttcat taatgctcga gtgcattaaa aagaacaaaa catt      1718

<210>  34
<211>  396
<212>  PRT
<213>  Mus musculus

<400>  34

Met Lys Lys Lys Gln Gln Gln His Pro Gly Gly Gly Thr Asp Pro Trp
1               5                   10                  15

Pro His Gly Ala Pro Val Gly Gly Ala Pro Pro Cys Leu Gly Ser Cys
            20                  25                  30

Lys Arg Arg Ile Pro Leu Leu Pro Phe Leu Arg Phe Ser Leu Arg Asp
        35                  40                  45

Tyr Gly Phe Cys Met Ala Thr Leu Val Val Phe Cys Leu Gly Ser Leu
    50                  55                  60

Phe Tyr Gln Leu Ser Gly Gly Pro Pro Arg Phe Leu Leu Asp Leu Arg
65                  70                  75                  80

Gln Tyr Leu Gly Asn Ser Thr Tyr Leu Asp Asp His Gly Pro Pro Pro
                85                  90                  95

Ser Lys Val Leu Pro Phe Pro Ser Gln Val Val Tyr Asn Arg Val Gly
            100                 105                 110

Lys Cys Gly Ser Arg Thr Val Val Leu Leu Leu Arg Ile Leu Ser Glu
        115                 120                 125

Lys His Gly Phe Asn Leu Val Thr Ser Asp Ile His Asn Lys Thr Arg
    130                 135                 140

Leu Thr Lys Asn Glu Gln Met Glu Leu Ile Lys Asn Ile Ser Thr Ala
145                 150                 155                 160

Glu Gln Pro Tyr Leu Phe Thr Arg His Val His Phe Leu Asn Phe Ser
                165                 170                 175

Arg Phe Gly Gly Asp Gln Pro Val Tyr Ile Asn Ile Ile Arg Asp Pro
            180                 185                 190

Val Ser Arg Phe Leu Ser Asn Tyr Phe Phe Arg Arg Phe Gly Asp Trp
        195                 200                 205

Arg Gly Glu Gln Asn His Met Ile Arg Thr Pro Ser Met Arg Gln Glu
    210                 215                 220

Glu Arg Tyr Leu Asp Ile Asn Glu Cys Ile Leu Glu Asn Tyr Pro Glu
225                 230                 235                 240

Cys Ser Asn Pro Arg Leu Phe Tyr Ile Ile Pro Tyr Phe Cys Gly Gln
                245                 250                 255

His Pro Arg Cys Arg Glu Pro Gly Glu Trp Ala Leu Glu Arg Ala Lys
            260                 265                 270

Leu Asn Val Asn Glu Asn Phe Leu Leu Val Gly Ile Leu Glu Glu Leu
        275                 280                 285

Glu Asp Val Leu Leu Leu Leu Glu Arg Phe Leu Pro His Tyr Phe Lys
    290                 295                 300

Gly Val Leu Ser Ile Tyr Lys Asp Pro Glu His Arg Lys Ala Trp Gln
305                 310                 315                 320

His Asp Cys Asp Arg Glu Glu Asp Cys Pro Leu Pro Arg Gly Gly Ala
                325                 330                 335

Asp Ser Leu Pro Ala Asn Glu Ile Arg Val Arg Val Leu Pro Leu Arg
            340                 345                 350

Gln Arg Thr Ile Pro Pro Ala Gln Ala Gln Ala Trp Thr Glu Val Ser
        355                 360                 365

Arg Glu Arg Thr Ser Arg Glu Thr Thr Val Leu His Pro His Ala Pro
    370                 375                 380

Gly Asp Gly Gly Ala Asn Arg Arg Arg Gly Ala Arg
385                 390                 395

<210>  35
<211>  5091
<212>  DNA
<213>  Mus musculus


<220>
<221>  CDS
<222>  (318)..(1595)

<400>  35
ggcggcaccc aaacagctac cccgtgcgga gacccaaact ttctactgcc acttggagtc      60

tcgcggccgc cgcctccgcc ccgcgcgtcc ggggcctgcc cgtcagtccg tcggtccgcg     120

tggagcagct cgggcgccgc cgtgctcccg atccccgcag ctgcagcgcc gcagtcctgg     180

cccggacgcc cgggcaagtt ctccagagtt aaaagcgaag ttcgggcgag gcgcgggccg 240

agctgcctct gagcgccccc ggcgtcccca gtgcgcccag ccccgccggg ggcgccggtg 300

acccttcggt gccagcc atg tcg tcc atc ctg ccc ttc acc ccc ccg atc 350
Met Ser Ser Ile Leu Pro Phe Thr Pro Pro Ile
1               5                   10

gtg aag cgc ctg ctg ggt tgg aag aag ggc gag cag aac ggg cag gag 398
Val Lys Arg Leu Leu Gly Trp Lys Lys Gly Glu Gln Asn Gly Gln Glu
            15                  20                  25

gag aag tgg tgc gag aag gcg gtc aag agc ttg gtg aag aag ctc aag 446
Glu Lys Trp Cys Glu Lys Ala Val Lys Ser Leu Val Lys Lys Leu Lys
        30                  35                  40

aag acg ggg cag ttg gac gag ctg gag aag gcc atc acc acg cag aac 494
Lys Thr Gly Gln Leu Asp Glu Leu Glu Lys Ala Ile Thr Thr Gln Asn
    45                  50                  55

gtg aac acc aag tgc att acc atc ccc agg tca ctg gat ggt cgg ctg 542
Val Asn Thr Lys Cys Ile Thr Ile Pro Arg Ser Leu Asp Gly Arg Leu
60                  65                  70                  75

cag gtg tcc cat cgg aag ggg ctc cct cac gtt atc tac tgc cgc ctg 590
Gln Val Ser His Arg Lys Gly Leu Pro His Val Ile Tyr Cys Arg Leu
                80                  85                  90

tgg cga tgg ccc gac ctg cac agc cac cat gaa tta cgg gcc atg gag 638
Trp Arg Trp Pro Asp Leu His Ser His His Glu Leu Arg Ala Met Glu
            95                  100                 105

ctc tgt gag ttt gcc ttc aac atg aag aag gat gaa gtg tgt gta aat 686
Leu Cys Glu Phe Ala Phe Asn Met Lys Lys Asp Glu Val Cys Val Asn
        110                 115                 120

cct tac cac tat cag aga gta gag acg cca gtt cta cct cca gtg ttg 734
Pro Tyr His Tyr Gln Arg Val Glu Thr Pro Val Leu Pro Pro Val Leu
    125                 130                 135

gtg cca cgc cac acc gag atc ccg gcc gag ttc ccc cca ctg gat gac 782
Val Pro Arg His Thr Glu Ile Pro Ala Glu Phe Pro Pro Leu Asp Asp
140                 145                 150                 155

tac agc cat tcc att ccc gag aac act aac ttc cct gct ggc att gag 830
Tyr Ser His Ser Ile Pro Glu Asn Thr Asn Phe Pro Ala Gly Ile Glu
                160                 165                 170

ccc cag agc aat att cca gaa acc cca cct cct ggc tac ctg agt gaa 878
Pro Gln Ser Asn Ile Pro Glu Thr Pro Pro Pro Gly Tyr Leu Ser Glu
            175                 180                 185

gat gga gaa acc agt gac cac cag atg aac cac agc atg gac gca ggt 926
Asp Gly Glu Thr Ser Asp His Gln Met Asn His Ser Met Asp Ala Gly
        190                 195                 200

tct cca aac ctc tcc ccg aat ccg atg tcc cca gca cac aat aac ttg 974
Ser Pro Asn Leu Ser Pro Asn Pro Met Ser Pro Ala His Asn Asn Leu
    205                 210                 215

gac cta cag cca gtc acc tac tgt gag ccg gcc ttc tgg tgc tcc atc 1022
Asp Leu Gln Pro Val Thr Tyr Cys Glu Pro Ala Phe Trp Cys Ser Ile
220                 225                 230                 235

tcc tac tac gag ctg aac cag cga gtt ggg gag aca ttc cac gcc tca 1070
Ser Tyr Tyr Glu Leu Asn Gln Arg Val Gly Glu Thr Phe His Ala Ser
                240                 245                 250

cag cca tcc atg aca gta gat ggc ttc act gac ccc tcc aac tcg gag 1118

Gln Pro Ser Met Thr Val Asp Gly Phe Thr Asp Pro Ser Asn Ser Glu
            255                 260                 265

cgc ttc tgc ctg ggc cta ctg tcc aat gtc aac cgg aat gca gcc gtg      1166
Arg Phe Cys Leu Gly Leu Leu Ser Asn Val Asn Arg Asn Ala Ala Val
    270                 275                 280

gaa ctt aca agg cga cac att ggg aga ggt gtg cgg ctc tac tac atc      1214
Glu Leu Thr Arg Arg His Ile Gly Arg Gly Val Arg Leu Tyr Tyr Ile
    285                 290                 295

gga ggg gag gtc ttt gcg gag tgc ctc agt gac agt gct att ttc gtc      1262
Gly Gly Glu Val Phe Ala Glu Cys Leu Ser Asp Ser Ala Ile Phe Val
300                 305                 310                 315

cag tct ccc aac tgc aac cag cgc tat ggc tgg cac ccg gcc act gtc      1310
Gln Ser Pro Asn Cys Asn Gln Arg Tyr Gly Trp His Pro Ala Thr Val
                320                 325                 330

tgc aag atc cca cca ggc tgc aac ctg aag atc ttc aac aac cag gaa      1358
Cys Lys Ile Pro Pro Gly Cys Asn Leu Lys Ile Phe Asn Asn Gln Glu
            335                 340                 345

ttt gct gcc ctc cta gct cag tct gtc aac cag ggc ttt gag gct gtc      1406
Phe Ala Ala Leu Leu Ala Gln Ser Val Asn Gln Gly Phe Glu Ala Val
        350                 355                 360

tac cag ctg acg cgc atg tgc acc atc cgt atg agc ttc gtc aaa ggc      1454
Tyr Gln Leu Thr Arg Met Cys Thr Ile Arg Met Ser Phe Val Lys Gly
    365                 370                 375

tgg gga gca gag tac agg aga cag aca gtg acc agc acc ccc tgc tgg      1502
Trp Gly Ala Glu Tyr Arg Arg Gln Thr Val Thr Ser Thr Pro Cys Trp
380                 385                 390                 395

att gag cta cac ctg aat gga ccc ttg cag tgg ctt gtc aag gtc ctc      1550
Ile Glu Leu His Leu Asn Gly Pro Leu Gln Trp Leu Val Lys Val Leu
                400                 405                 410

acc cag atg ggt tcc ccg agc atc cgc tgt tcc agt gtg tct tag          1595
Thr Gln Met Gly Ser Pro Ser Ile Arg Cys Ser Ser Val Ser
            415                 420                 425

agacactagg agtaaaggga gcgggttggg gagggcgggc ttggggaaaa tgaccttgga      1655

agagaactcc atccaacttg gtcttgtcaa agaacaccga ttccactcaa ctaaggcacc      1715

agcctgtttc tgagaccaca gaagaaaacc ccagggatgg atttatgaac agctgtgtct      1775

gctacataca cgtgcccctg tctgaaggcc aagtgatggc ttctgttctg gtggcttgaa      1835

ctaacaggtg gtgtatcgcc acctgactcc ttgtttaatg acagaggtct gggatgtcac      1895

agtccaaaag gaaagtgcct ttctccatgg ctggagtatg gagtttacct ttggagaagt      1955

tgtaatggag catgccctgt cccaccactc tcagagaggg tgtacctgtc aaactggatg      2015

gcctacatag gtactccccc ctacccctag gatgcagaga gacgggaaca cgccggaggg      2075

tagagctggg gagaacccat tcttccttgg aaggatccgc tgaaggtcag cgtataggtg      2135

atgtacagtt cctaatatca catcagtctc agagtgttca caggaagcag caagggcact      2195

cgtggagtat gtgtcctggg tgaggtggca ccacaccgaa tgaatgcatc tctgggagct      2255

ggcaccacaa ccctgatgta taggctgtgt tggtttatgg agacaagttg catcaatgaa      2315

ttcacctagc ataggctctt tgaaatgtcc tctgtttgat aaaaaacaat cctgggtacg      2375

tatgttggct ggaaaaccac aatggaccct gccactgctt cttgccctga ggtttggaag 2435
ctgagagtta tagaagccaa ttcaccagga ggtaagacat cccaggctga catgggcaaa 2495
tgaaaagggc tattaaaatt tttttacacc ttggaaaatt gcaggcttgg tgcagagcgc 2555
tctgtcatct tcaccctggg atgtaggatt acctagctat ggtaaaggat tgccacagca 2615
aactgtgaca ctgtgtaatg agcactgttc ccagcggcaa ttacagagaa aacgagtgta 2675
aattatttca actggaaaaa agtccctttc ttggctgttt tagaacaggg tacacaggat 2735
cgccacctgc aactggtact cgcttcttgg ctgctgcttc tgttgtgaaa agacgagccc 2795
atgtttctgc atggatttcc catggaagtc ctgtcctgct acagagggga agaaagtgta 2855
ccctccaatg tgataaatct tctgatgccc ccagactctt ggagcacatc ctggtgcccc 2915
tcctgcagga gcctgtggca tatttccagc tgggcatgct gatcctcctt gagacacaga 2975
tgcctgtgtg agtctccgtt gatacaattc tgaacccctc aggttctctg aaagggcaca 3035
gaccatgggc gtgaacattg tgccgtacct gagttggtct gtggactgct gcttcagaca 3095
cacgagtcct cggaactgcc tggctgcctg tcacgcatgc tctgagtcag aacacaccaa 3155
cgctctgctg tggctcctag ggaagcattc atggtcctct gttatcagca ggggtttatg 3215
tcacttgctg tccggtttcc taggggcttc ctgtgcccct tccccagcta tcctccaggt 3275
ggctagggac agtctattct gctgcaactg gaaagtagag ggaaccggca ctgctcagag 3335
cagatggcgg cttctggagg cacacagtgg gagtacaccc cttcatgtta ttggccagtt 3395
gctggagaat gctgtaggag aaaattctag gcaggtctac tcttggcatc cctgagagtc 3455
aaaggcttgg agtctaggaa aggtcacacc atgatggaga acacaggtca tttgggtacg 3515
tgtaatcaaa gtgccctccc aaatcagttc tccttttcgt atgaacagca tctctacttt 3575
taaagaggag ttgaggatcg agaagatgac agtgcagcag tgggtgtggc ctgactacat 3635
gtgctgttcc agccctgggt gcccactgac accgaccccc aggcagaggc ctttgtcttc 3695
agcactcctg agaagttggc tctttacccc ttctcctctg ctgcccctcc ttcctgctgg 3755
ttcaggtagc cccagccacg tgggttagag tcctgtgctg gcctgccatg gcagctggct 3815
accttccaga ccaactgtag agatacctgg cattttgaag aaagcctaga ctggagagca 3875
gggcctctct tgggaaggac acaaggcggg caaggctgct ggcagacttc tccactgacc 3935
tgagtgtgct ttttttttcc cctaaatgta ttgcatcaag cctcagtgct tatggagtgc 3995
agtggtcttc atcttcccca acttgcttct cagagctggg atgtattcca gagcctgatg 4055
tttttattca aaccacagaa aagttttctt taagtagcct gtgcagtcat gcatgtgcct 4115
gagttgtctg gagcagaggc aaacatctga cttcattctt agccccaagc tgccatttct 4175
gagtccttga gaggctgaga aggctctagc tttgtactgt attcttactg tgtgactaga 4235
tgcgtgagcg ctttacatta gaaggaacct ggttagagct cgctcctcct gtctttgtgt 4295
ggcatttgtg ttccattacc ggccccttta agtaacggcc ttcacagcac cttcccagtg 4355
ggtagaaagc cacacaccag gatgtgggtc aaccatgaag atgtggcatt gcagacgggg 4415
gaacatgtgg atgcatggct atcgccctga acagtccctg cagctacttg tgttaacaca 4475

gaactgatgt ttagcattct gccgctttcg tatttatgta acaattcctt aaagccattc 4535
aaatggctaa ctatttaatt tctttaggac agttgtaaag gtctctctcc tgaggacaat 4595
gacttggaag aactggggca cagccagtcc cagacactgg tggaggctgc agtgactttt 4655
tttggctcaa gatccacaag cattagagta gactgggcca acaagtcaac aagtggtggc 4715
gtgtgcaaac gggctgccct agtcaagccc agtcccttca acagtatgtc tgatgcacca 4775
caggccctcc ctactggaag tgggaacttc aaatggaaat tggagccatc ttttatccca 4835
gaagcctttg ctgctgccag ggcaagtggg ctggtgtgga ctcttgttta ggaggctgag 4895
gttcttgtca ctccttagcc agccaggcct ttagtgtctt tgtaaaaagc atgtatttat 4955
agtgttttag atttttctaa cttttgtatc ttacagcatt gtaccccatt gttaaagagc 5015
cgtgtcccct cttcttataa acgccctttt aataaacttt tcaccgtaaa gctcctgaga 5075
caggagcaca gtctgt 5091

<210> 36
<211> 425
<212> PRT
<213> Mus musculus

<400> 36

Met Ser Ser Ile Leu Pro Phe Thr Pro Pro Ile Val Lys Arg Leu Leu
1               5                   10                  15

Gly Trp Lys Lys Gly Glu Gln Asn Gly Gln Glu Glu Lys Trp Cys Glu
            20                  25                  30

Lys Ala Val Lys Ser Leu Val Lys Lys Leu Lys Lys Thr Gly Gln Leu
        35                  40                  45

Asp Glu Leu Glu Lys Ala Ile Thr Thr Gln Asn Val Asn Thr Lys Cys
    50                  55                  60

Ile Thr Ile Pro Arg Ser Leu Asp Gly Arg Leu Gln Val Ser His Arg
65                  70                  75                  80

Lys Gly Leu Pro His Val Ile Tyr Cys Arg Leu Trp Arg Trp Pro Asp
                85                  90                  95

Leu His Ser His His Glu Leu Arg Ala Met Glu Leu Cys Glu Phe Ala
            100                 105                 110

Phe Asn Met Lys Lys Asp Glu Val Cys Val Asn Pro Tyr His Tyr Gln
        115                 120                 125

Arg Val Glu Thr Pro Val Leu Pro Pro Val Leu Val Pro Arg His Thr
    130                 135                 140

Glu Ile Pro Ala Glu Phe Pro Pro Leu Asp Asp Tyr Ser His Ser Ile
145                 150                 155                 160

Pro Glu Asn Thr Asn Phe Pro Ala Gly Ile Glu Pro Gln Ser Asn Ile
                165                 170                 175

Pro Glu Thr Pro Pro Pro Gly Tyr Leu Ser Glu Asp Gly Glu Thr Ser
            180                 185                 190

Asp His Gln Met Asn His Ser Met Asp Ala Gly Ser Pro Asn Leu Ser
        195                 200                 205

Pro Asn Pro Met Ser Pro Ala His Asn Asn Leu Asp Leu Gln Pro Val
    210                 215                 220

Thr Tyr Cys Glu Pro Ala Phe Trp Cys Ser Ile Ser Tyr Tyr Glu Leu
225                 230                 235                 240

Asn Gln Arg Val Gly Glu Thr Phe His Ala Ser Gln Pro Ser Met Thr
                245                 250                 255

Val Asp Gly Phe Thr Asp Pro Ser Asn Ser Glu Arg Phe Cys Leu Gly
            260                 265                 270

Leu Leu Ser Asn Val Asn Arg Asn Ala Ala Val Glu Leu Thr Arg Arg
        275                 280                 285

His Ile Gly Arg Gly Val Arg Leu Tyr Tyr Ile Gly Gly Glu Val Phe
    290                 295                 300

Ala Glu Cys Leu Ser Asp Ser Ala Ile Phe Val Gln Ser Pro Asn Cys
305                 310                 315                 320

Asn Gln Arg Tyr Gly Trp His Pro Ala Thr Val Cys Lys Ile Pro Pro
                325                 330                 335

Gly Cys Asn Leu Lys Ile Phe Asn Asn Gln Glu Phe Ala Ala Leu Leu
            340                 345                 350

Ala Gln Ser Val Asn Gln Gly Phe Glu Ala Val Tyr Gln Leu Thr Arg
        355                 360                 365

Met Cys Thr Ile Arg Met Ser Phe Val Lys Gly Trp Gly Ala Glu Tyr
    370                 375                 380

Arg Arg Gln Thr Val Thr Ser Thr Pro Cys Trp Ile Glu Leu His Leu
385                 390                 395                 400

Asn Gly Pro Leu Gln Trp Leu Val Lys Val Leu Thr Gln Met Gly Ser
                405                 410                 415

Pro Ser Ile Arg Cys Ser Ser Val Ser
            420                 425

<210>  37
<211>  1983
<212>  DNA
<213>  Mus musculus


<220>
<221>  CDS
<222>  (80)..(1171)

<400>  37
tggagctggt cggttatggt tggaactttc cctgcccttc gttgactgag tgaaccagcc      60

agaaatacat tcccaggaa atg gcc tct aaa tct tgg ctg aat ttt tta gtc      112
                     Met Ala Ser Lys Ser Trp Leu Asn Phe Leu Val
                     1               5                   10

ttc ctc tgt gga tca gca ata ggg ttt ttt tta tgt tct caa ctc ttg      160
Phe Leu Cys Gly Ser Ala Ile Gly Phe Phe Leu Cys Ser Gln Leu Leu
            15                  20                  25

agt att ttg ttg cga gaa gag gct gcc att cag cct aac atg ctt cac      208
Ser Ile Leu Leu Arg Glu Glu Ala Ala Ile Gln Pro Asn Met Leu His
        30                  35                  40

aat gac cct cat gca agg cat tca gat gac aat gga cac agt cac ctc      256
Asn Asp Pro His Ala Arg His Ser Asp Asp Asn Gly His Ser His Leu
    45                  50                  55

aaa gga cag atg aac ttc aat gca gat tcc agc caa cat aaa gat gag      304
Lys Gly Gln Met Asn Phe Asn Ala Asp Ser Ser Gln His Lys Asp Glu
60                  65                  70                  75

aac ata gac gtt gct gag aac ctc tat cag aaa gtt aaa att ctt tgt      352
Asn Ile Asp Val Ala Glu Asn Leu Tyr Gln Lys Val Lys Ile Leu Cys
                80                  85                  90

tgg gtt atg aca agt cct caa aat cta gag aaa aag gcc aaa cat gtc      400
Trp Val Met Thr Ser Pro Gln Asn Leu Glu Lys Lys Ala Lys His Val
            95                  100                 105

aaa gct acg tgg gcc cag cgt tgt aat aaa gtg tta ttt atg agt tcg      448
Lys Ala Thr Trp Ala Gln Arg Cys Asn Lys Val Leu Phe Met Ser Ser
        110                 115                 120

gaa gaa aat cag gac ttc cct act gtg gga ttg aaa acc aaa gaa ggc      496
Glu Glu Asn Gln Asp Phe Pro Thr Val Gly Leu Lys Thr Lys Glu Gly
    125                 130                 135

aga gag caa cta tat tgg aaa aca att aaa gct ttc cag tat gta cat      544
Arg Glu Gln Leu Tyr Trp Lys Thr Ile Lys Ala Phe Gln Tyr Val His
140                 145                 150                 155

gac cat tat tta gaa gat gct gac tgg ttt atg aaa gca gat gac gac      592
Asp His Tyr Leu Glu Asp Ala Asp Trp Phe Met Lys Ala Asp Asp Asp
                160                 165                 170

aca tac gtc att gtg gac aac ctg aga tgg ctt cta tca aag tat gac      640
Thr Tyr Val Ile Val Asp Asn Leu Arg Trp Leu Leu Ser Lys Tyr Asp
            175                 180                 185

cct gaa caa ccc att tac ttt ggg cga aga ttt aag ccc tat gtg aag      688
Pro Glu Gln Pro Ile Tyr Phe Gly Arg Arg Phe Lys Pro Tyr Val Lys
        190                 195                 200

cag gga tac atg agc gga gga gcg ggc tat gtc cta agc aag gaa gcc      736
Gln Gly Tyr Met Ser Gly Gly Ala Gly Tyr Val Leu Ser Lys Glu Ala
    205                 210                 215

ttg aga aga ttt gtt aat gca ttt aaa aca gaa aag tgt aca cat agt     784
Leu Arg Arg Phe Val Asn Ala Phe Lys Thr Glu Lys Cys Thr His Ser
220                 225                 230                 235

tcc tcc atc gaa gac tta gct ctg gga agg tgc atg gaa att ata aat     832
Ser Ser Ile Glu Asp Leu Ala Leu Gly Arg Cys Met Glu Ile Ile Asn
                240                 245                 250

gta gaa gct gga gat tcc aga gat acc att ggg aaa gaa acc ttc cat     880
Val Glu Ala Gly Asp Ser Arg Asp Thr Ile Gly Lys Glu Thr Phe His
            255                 260                 265

cca ttt gta cca gaa cac cac tta atc aaa ggt tat cta cca aaa aca     928
Pro Phe Val Pro Glu His His Leu Ile Lys Gly Tyr Leu Pro Lys Thr
        270                 275                 280

ttt tgg tac tgg aat tac aac tat tat cct ccc ata gag ggt cct gga     976
Phe Trp Tyr Trp Asn Tyr Asn Tyr Tyr Pro Pro Ile Glu Gly Pro Gly
    285                 290                 295

tgc tgt tct gat att gca gtt tct ttt cac tat gtt gat ggg aca act     1024
Cys Cys Ser Asp Ile Ala Val Ser Phe His Tyr Val Asp Gly Thr Thr
300                 305                 310                 315

atg tat gaa tta gaa tac ctc gtt tat cgt ctt cgt cca tat ggt tgt     1072
Met Tyr Glu Leu Glu Tyr Leu Val Tyr Arg Leu Arg Pro Tyr Gly Cys
                320                 325                 330

tta tat aga tat caa cct gcc tta cct gag aat ata ctg aaa gaa att     1120
Leu Tyr Arg Tyr Gln Pro Ala Leu Pro Glu Asn Ile Leu Lys Glu Ile
            335                 340                 345

aat caa gta aac aga aag gaa gat aca aaa ata aaa tta ggc aac ccc     1168
Asn Gln Val Asn Arg Lys Glu Asp Thr Lys Ile Lys Leu Gly Asn Pro
        350                 355                 360

tga aagcagaaca taagtggtct acattgcact gaaggactct tgcctttcta     1221

cggaaccctg aaatcccagt gaggaactca cctgaagtga acattccata tagaaatctt     1281

tcaaatggat gactataaac tgaagcattt aaagagctgt gaagtttgct aaaacgtgtc     1341

ttgatacagt aatatataaa tataaataaa tatatatgaa gaatttgtgt ttttaaatgg     1401

tgacagaaga gctagaaaga ctctgtttct gactgttccc aacagtgtat gttgttatca     1461

ctacagttgg agtggttagt ttataatcac cttctgcacc acaccagtta gaacacaatg     1521

ctgaacagat ctgccttaaa gaaagaaaga aagaactgtt gctcagtgtt gtttctcagc     1581

tgatatgtgt atgctgcaaa ccagccttcc agttgccgaa tgaaaacttt cctgtgatta     1641

tattatttat aattggcagt gcttcctcaa gaagaataag aagggcactt accccaaagt     1701

gcagtaatta accacatttc caattattcc gagccttgac tccattagat acttagttct     1761

aaagataatc aacccgatac caagtcccca ttgtcccctt tccaaatgtt tgaccctgaa     1821

taccattttt cactattaat gctaatttac tcaaagcatt ggcagcattc tgtaaatcat     1881

gcattttcaa atatgttgtt ccccgattca cccattgtta attgtaggta acaattaaat     1941

ttaatgctgt ttggacaagc aaaaaaaaaa aaaaaaaaaa aa     1983

<210>  38
<211>  363
<212>  PRT

<213>  Mus musculus

<400>  38

Met Ala Ser Lys Ser Trp Leu Asn Phe Leu Val Phe Leu Cys Gly Ser
1               5                   10                  15

Ala Ile Gly Phe Phe Leu Cys Ser Gln Leu Leu Ser Ile Leu Leu Arg
            20                  25                  30

Glu Glu Ala Ala Ile Gln Pro Asn Met Leu His Asn Asp Pro His Ala
        35                  40                  45

Arg His Ser Asp Asp Asn Gly His Ser His Leu Lys Gly Gln Met Asn
    50                  55                  60

Phe Asn Ala Asp Ser Ser Gln His Lys Asp Glu Asn Ile Asp Val Ala
65                  70                  75                  80

Glu Asn Leu Tyr Gln Lys Val Lys Ile Leu Cys Trp Val Met Thr Ser
                85                  90                  95

Pro Gln Asn Leu Glu Lys Lys Ala Lys His Val Lys Ala Thr Trp Ala
            100                 105                 110

Gln Arg Cys Asn Lys Val Leu Phe Met Ser Ser Glu Glu Asn Gln Asp
        115                 120                 125

Phe Pro Thr Val Gly Leu Lys Thr Lys Glu Gly Arg Glu Gln Leu Tyr
    130                 135                 140

Trp Lys Thr Ile Lys Ala Phe Gln Tyr Val His Asp His Tyr Leu Glu
145                 150                 155                 160

Asp Ala Asp Trp Phe Met Lys Ala Asp Asp Asp Thr Tyr Val Ile Val
                165                 170                 175

Asp Asn Leu Arg Trp Leu Leu Ser Lys Tyr Asp Pro Glu Gln Pro Ile
            180                 185                 190

Tyr Phe Gly Arg Arg Phe Lys Pro Tyr Val Lys Gln Gly Tyr Met Ser
        195                 200                 205

Gly Gly Ala Gly Tyr Val Leu Ser Lys Glu Ala Leu Arg Arg Phe Val
    210                 215                 220

Asn Ala Phe Lys Thr Glu Lys Cys Thr His Ser Ser Ser Ile Glu Asp
225                 230                 235                 240

Leu Ala Leu Gly Arg Cys Met Glu Ile Ile Asn Val Glu Ala Gly Asp
                245                 250                 255

Ser Arg Asp Thr Ile Gly Lys Glu Thr Phe His Pro Phe Val Pro Glu

260                 265                 270

His His Leu Ile Lys Gly Tyr Leu Pro Lys Thr Phe Trp Tyr Trp Asn
        275                 280                 285

Tyr Asn Tyr Tyr Pro Pro Ile Glu Gly Pro Gly Cys Cys Ser Asp Ile
    290                 295                 300

Ala Val Ser Phe His Tyr Val Asp Gly Thr Thr Met Tyr Glu Leu Glu
305                 310                 315                 320

Tyr Leu Val Tyr Arg Leu Arg Pro Tyr Gly Cys Leu Tyr Arg Tyr Gln
                325                 330                 335

Pro Ala Leu Pro Glu Asn Ile Leu Lys Glu Ile Asn Gln Val Asn Arg
            340                 345                 350

Lys Glu Asp Thr Lys Ile Lys Leu Gly Asn Pro
        355                 360

<210>  39
<211>  4263
<212>  DNA
<213>  Mus musculus

<220>
<221>  CDS
<222>  (602)..(1576)

<400>  39
ccgagaagga gagagcggca caggtttctg ccgcggcgct cgaacgggag cttagggacg      60

cagcatcctc ggtgtccacc cgggatcgca gctgcaccgg ccgacatgtg acggaggaaa     120

gccctcagca ttcccggcaa ccgccttcaa aggggtcccg gaacgctcgc ccaggccagc     180

agctggatcc tcttagccct cagcgacgcg ggacaggagc ggcaaggtac aacgcctggt     240

acgttccagg ctcgcactcg ggaaaagagc gcagcgggga ccgccgcctg gcttgcactc     300

ggcacaactc cgggacctgc ggagtgcggc tggaccgtgt ccagattccc gccgacttca     360

tccagagtga cccaagaccc tagccggtcc tcctgcaccc cctggagttc tagcttgccc     420

tgcatctgcg ccgaccccac aacgcccagg tccccgcaac gccccagtcc ggaaggggag     480

cgcgcttgcg gcttctccct gattgcagac gctgccgctg tcctaactgc acacagaggt     540

cccctacccg gagcctagtc cttgcagggg gtggtgtccg agacgctggg gagcgcgcac     600

c atg aag tcc gcg ctg tgc agc cgc ttc ttc atc ctc ctg ccc tgg atc      649
  Met Lys Ser Ala Leu Cys Ser Arg Phe Phe Ile Leu Leu Pro Trp Ile
  1               5                   10                  15

ctg att gtc atc atc atg ttg gac gtg gac ccc cgc agg ccc gcg ccc      697
Leu Ile Val Ile Ile Met Leu Asp Val Asp Pro Arg Arg Pro Ala Pro
            20                  25                  30

caa ctc act tcc cgt ccc tac ttc tct ccc cat gca gtg ggc tgc ggg      745
Gln Leu Thr Ser Arg Pro Tyr Phe Ser Pro His Ala Val Gly Cys Gly
        35                  40                  45

ggc tcc cga gtc ccg ctc cga cgg agt agc ccc ggg cgt gac gct gcc 793
Gly Ser Arg Val Pro Leu Arg Arg Ser Ser Pro Gly Arg Asp Ala Ala
    50                  55                  60

gag aag cgg aac gag tct cgg cct cag ctg cag ccg gag ccg cgc ttg 841
Glu Lys Arg Asn Glu Ser Arg Pro Gln Leu Gln Pro Glu Pro Arg Leu
65                  70                  75                  80

ccc acc atc tat gcc atc aca ccc acc tac agt cgc ccg gtg cag aaa 889
Pro Thr Ile Tyr Ala Ile Thr Pro Thr Tyr Ser Arg Pro Val Gln Lys
                85                  90                  95

gcc gag ctc act cgc ctg gcc aac acc ttc cgc cag gtg gcg cag ttg 937
Ala Glu Leu Thr Arg Leu Ala Asn Thr Phe Arg Gln Val Ala Gln Leu
            100                 105                 110

cac tgg atc ctg gtg gag gac cgg gcg acg cgc agc gag ctg gtg agc 985
His Trp Ile Leu Val Glu Asp Arg Ala Thr Arg Ser Glu Leu Val Ser
        115                 120                 125

agc ttc cta gct cgg gcc ggc ctg ccc aac acg cac ctg cat gtg ccc 1033
Ser Phe Leu Ala Arg Ala Gly Leu Pro Asn Thr His Leu His Val Pro
    130                 135                 140

acg ccg cgg cgc tac aag cga ccg tgg ctg ccg cgc gcc acc gaa cag 1081
Thr Pro Arg Arg Tyr Lys Arg Pro Trp Leu Pro Arg Ala Thr Glu Gln
145                 150                 155                 160

cgc aac gcg ggc ctc gcc tgg ctg cgc cag agg cat cag cat cag agc 1129
Arg Asn Ala Gly Leu Ala Trp Leu Arg Gln Arg His Gln His Gln Ser
                165                 170                 175

gcg caa ccc ggc gtg ctc ttc ttc gcc gac gat gac aac acg tac agt 1177
Ala Gln Pro Gly Val Leu Phe Phe Ala Asp Asp Asp Asn Thr Tyr Ser
            180                 185                 190

ctg gag ctc ttc cag gag atg agg acc act cgc aag gtt tct gtc tgg 1225
Leu Glu Leu Phe Gln Glu Met Arg Thr Thr Arg Lys Val Ser Val Trp
        195                 200                 205

cct gtg gga cta gtt ggt ggg cga cgc tat gaa cgt ccg ttg gta aaa 1273
Pro Val Gly Leu Val Gly Gly Arg Arg Tyr Glu Arg Pro Leu Val Lys
    210                 215                 220

aat ggc aaa gtt gtt ggc tgg tac acc gga tgg aga gaa gac agg cct 1321
Asn Gly Lys Val Val Gly Trp Tyr Thr Gly Trp Arg Glu Asp Arg Pro
225                 230                 235                 240

ttt gcc att gac atg gct ggg ttt gct gtg agt ctg caa gtc atc ttg 1369
Phe Ala Ile Asp Met Ala Gly Phe Ala Val Ser Leu Gln Val Ile Leu
                245                 250                 255

tcc aat ccg aaa gct gta ttt aag cgc cgt gga tcc cag cca ggg atg 1417
Ser Asn Pro Lys Ala Val Phe Lys Arg Arg Gly Ser Gln Pro Gly Met
            260                 265                 270

caa gaa tct gac ttt cta aag cag ata aca aca gtt gaa gaa ctg gaa 1465
Gln Glu Ser Asp Phe Leu Lys Gln Ile Thr Thr Val Glu Glu Leu Glu
        275                 280                 285

cca aaa gct agt aac tgc acc aag gtt ctc gtg tgg cac act cgg aca 1513
Pro Lys Ala Ser Asn Cys Thr Lys Val Leu Val Trp His Thr Arg Thr
    290                 295                 300

gag aag gtc aat cta gcc aat gag cca aag tat cac ctg gac aca gtg 1561
Glu Lys Val Asn Leu Ala Asn Glu Pro Lys Tyr His Leu Asp Thr Val
305                 310                 315                 320

aac att gag gtg tag ccgccaggac cagcaggtga ggacaggaag tctggagcgt 1616
Asn Ile Glu Val

gggctgctgc ccattcaggt actgttcctt tgccttcagc ctttccttac cgccgatgga 1676
cggccgtttc gacagctacc tggatgctac aaaatgtgtt gtcttcattt gtctgcatgt 1736
gttttctgaa ctggaagagt tttgtagagt gaagccacat gtcactgaaa aggacaacga 1796
agtatctcac actgctccct ccctccctcc ctccctccct ccatccctcc ctcccacaag 1856
ccatctcaat actgctagaa tgacaggttt tagaagctga tgaggagtac tctgtcaatt 1916
aaatattccc agttaaattc ttggcttcac aacatgaact atttggaaag ctgatggcag 1976
tgtacactgt aggaaataaa acccacaaat aaaaaggtgt gtggattcat ctgtttaata 2036
tagggaaata acattttgtc aatactaggc accataaaat gtaaacacaa ttactgtcat 2096
aaacctggat ataccttcaa ggattaaaag tggctttgtt ttagttaccc tgtgtgcata 2156
tagtgcagaa aaaggtcttc atattagcac tatgtacatg agaagaggcc cacattacaa 2216
gaggcaatgg aaatttgaat tctgaaacct tcattgagtt ttacagtagc atccaggttt 2276
atctccgttc actaaccacg ttccctgtta agcacatcat aacaacagca cagtggagtg 2336
agtgatgaat aaaagaattt gatacactag aaaacagtgc tcagtgatac atttacattc 2396
tatttatatg attaaacatt tgatcataca gtacctttct acaggattac tggctaattt 2456
gggggtgggg tttatactgt tagaggtatt actaacatga taactacttc ccttatatgc 2516
aaacgttaga gctataattt tgcaaataaa actgattatg caagttaact gagcagcttc 2576
ccaatgtggc ttaggacatc atggggagta tgagcaaagc tgccctccac acagatggtt 2636
tctcaacctg ctttcacatc caatttaatc agcacagacc aacacggtca atcaggtcat 2696
tcttaatgtg agcaaatggg ggaaagaaaa aatacgttat gtacatgaat aaatgggaac 2756
tagatgcctt tgcagcgagg aatgttgggg tcggttctgg atggaactct gtgacagctt 2816
cttacttcca cagctgtgtg ctcagcgtct gccccgatga gcttccagac caccctgctg 2876
tcgtgctggg aggctggcca aaacctgcac tagcgttagc actactgagg ttcatggcag 2936
ccatctgctg attcatctgc gaaacacaaa aggcttggtt tacaaggagg accctctttt 2996
cactcctgac tccttagcta cagccagaca cttcatcaga tgtgaaaatt agtccagaca 3056
ctggatgtgg actacactga agtctttcta ctgaaatcag ttactttttt cttacttttt 3116
tctgagttag gtgttactta taaagtaatc cataattagt tcactttcta gattaaccac 3176
aactgaaaac acatcaaagt aatctttgag tttatatctg agaccatcac tcttctgtga 3236
ctccaagact gagacatttt agaaagtaaa acatctaaaa ttaccctaac atttacaaaa 3296
tactcatacc tctctattaa catcctaaat gaccttaata tacttcctaa acattaaaaa 3356
ggtcatatag catatgtttt aggtttatgt tataataaca taaacagcta aaattaaatt 3416
tcattatatg ctatacaata aacaactaca tgtacttttc tttcaggcta aaatccagta 3476
tatttatcag gctcttagca cagtagttct cccactaccc agtttggtga cggaacaaga 3536
gatgcaggat ttacctaaaa tagtttcaaa cctatgttca acattatctg tgttctttgt 3596

gcaaattttt tttaaaaccc aagacataat aagcacacat gtgtctacag acaggtagct 3656
tccatcttcc cttctgctca ctcttgtggc cttgctgttc ttcactggct ctcctgtact 3716
ctaacctgag tcatgtgagc ctgagcatcc tggtcacaga aaagctgttc ctagtatctg 3776
acatcattcc cgaggtttcc aatgcataag cagtgtgtac gtatatacct ataaaccgca 3836
gctgtcacag cacacgagca tttgtaagat acacggtgat tctgtgaatg ttagttagca 3896
gacattgttc tgaaggtaaa ctaggggtct tagtggtcct ggttcctgtg agatgctcgg 3956
tggaccatca tgcaggaatg ggaatgggac catgagcact aagtcaaagc ctctgatgtg 4016
gggtaacagc agaaatgtta ctctaaccta agccagacag acatggaatt tatcagtgaa 4076
tacctgaggg aagcagggtt tacattctca ccatggggat tcctgagcca atggatgttc 4136
ctcatacatc aacaggcagt aaccacatga cagcggcagc cttgatccct cataaaactg 4196
tgctttgaaa atatgaagct ggatttctat ctcactgata agaattaata aaaaaaaaaa 4256
aaaaaaa 4263

<210> 40
<211> 324
<212> PRT
<213> Mus musculus

<400> 40

Met Lys Ser Ala Leu Cys Ser Arg Phe Phe Ile Leu Leu Pro Trp Ile
1               5                   10                  15

Leu Ile Val Ile Ile Met Leu Asp Val Asp Pro Arg Arg Pro Ala Pro
            20                  25                  30

Gln Leu Thr Ser Arg Pro Tyr Phe Ser Pro His Ala Val Gly Cys Gly
        35                  40                  45

Gly Ser Arg Val Pro Leu Arg Arg Ser Ser Pro Gly Arg Asp Ala Ala
    50                  55                  60

Glu Lys Arg Asn Glu Ser Arg Pro Gln Leu Gln Pro Glu Pro Arg Leu
65                  70                  75                  80

Pro Thr Ile Tyr Ala Ile Thr Pro Thr Tyr Ser Arg Pro Val Gln Lys
                85                  90                  95

Ala Glu Leu Thr Arg Leu Ala Asn Thr Phe Arg Gln Val Ala Gln Leu
            100                 105                 110

His Trp Ile Leu Val Glu Asp Arg Ala Thr Arg Ser Glu Leu Val Ser
        115                 120                 125

Ser Phe Leu Ala Arg Ala Gly Leu Pro Asn Thr His Leu His Val Pro
    130                 135                 140

Thr Pro Arg Arg Tyr Lys Arg Pro Trp Leu Pro Arg Ala Thr Glu Gln
145                 150                 155                 160

Arg Asn Ala Gly Leu Ala Trp Leu Arg Gln Arg His Gln His Gln Ser
                165                 170                 175

Ala Gln Pro Gly Val Leu Phe Phe Ala Asp Asp Asp Asn Thr Tyr Ser
            180                 185                 190

Leu Glu Leu Phe Gln Glu Met Arg Thr Thr Arg Lys Val Ser Val Trp
        195                 200                 205

Pro Val Gly Leu Val Gly Gly Arg Arg Tyr Glu Arg Pro Leu Val Lys
    210                 215                 220

Asn Gly Lys Val Val Gly Trp Tyr Thr Gly Trp Arg Glu Asp Arg Pro
225                 230                 235                 240

Phe Ala Ile Asp Met Ala Gly Phe Ala Val Ser Leu Gln Val Ile Leu
                245                 250                 255

Ser Asn Pro Lys Ala Val Phe Lys Arg Arg Gly Ser Gln Pro Gly Met
            260                 265                 270

Gln Glu Ser Asp Phe Leu Lys Gln Ile Thr Thr Val Glu Glu Leu Glu
        275                 280                 285

Pro Lys Ala Ser Asn Cys Thr Lys Val Leu Val Trp His Thr Arg Thr
    290                 295                 300

Glu Lys Val Asn Leu Ala Asn Glu Pro Lys Tyr His Leu Asp Thr Val
305                 310                 315                 320

Asn Ile Glu Val


<210>  41
<211>  1599
<212>  DNA
<213>  Mus musculus


<220>
<221>  CDS
<222>  (175)..(1182)

<400>  41
gaagtggtcc caggtgtcgc tcagagtccc gcgttcccgg gaactagtaa gggagcctgg      60

ggaaggcggg gcgcggcccg ggcggccggg gcggggccgg cgggagggcg gagggccggg     120

cggacctgtc cctgccggtc ccggtggacc ccgcctgccc cggtggcacc ggcc atg       177
                                                            Met
                                                            1

aag ctg aag ctg aag aac gtg ttt ctt gcc tac ttc ctg gtg tcg atc       225
Lys Leu Lys Leu Lys Asn Val Phe Leu Ala Tyr Phe Leu Val Ser Ile

5                   10                  15

gcc ggc ctc ctc tac gct ctg gtg cag ctc ggc cag cct tgc gac tgc        273
Ala Gly Leu Leu Tyr Ala Leu Val Gln Leu Gly Gln Pro Cys Asp Cys
    20                  25                  30

ctc cct ccg ctt cga gct gca gct gag cag ctt cgg cag aag gac ctg        321
Leu Pro Pro Leu Arg Ala Ala Ala Glu Gln Leu Arg Gln Lys Asp Leu
    35                  40                  45

agg ata tcc cag ttg caa gct gat ctc cgt cgc cca ccc cct gtc cca        369
Arg Ile Ser Gln Leu Gln Ala Asp Leu Arg Arg Pro Pro Pro Val Pro
50                  55                  60                  65

gcc cag ccc cct gaa cct gag gcc ctg cct act atc tat gtc att acc        417
Ala Gln Pro Pro Glu Pro Glu Ala Leu Pro Thr Ile Tyr Val Ile Thr
                70                  75                  80

ccc acc tac gcc agg ctg gta caa aag gca gag ctg gtt cgg ctg tcc        465
Pro Thr Tyr Ala Arg Leu Val Gln Lys Ala Glu Leu Val Arg Leu Ser
            85                  90                  95

cag acc ctg agc ttg gtg ccc cgt cta cac tgg ctg cta gtg gag gac        513
Gln Thr Leu Ser Leu Val Pro Arg Leu His Trp Leu Leu Val Glu Asp
        100                 105                 110

gct gag agc cct acc ccg ctg gtc tcg ggg ctg ttg gcc gcc tct ggt        561
Ala Glu Ser Pro Thr Pro Leu Val Ser Gly Leu Leu Ala Ala Ser Gly
    115                 120                 125

ctc ctc ttt aca cac ctg gct gtc ctt acc ccc aag gct caa cgg ctt        609
Leu Leu Phe Thr His Leu Ala Val Leu Thr Pro Lys Ala Gln Arg Leu
130                 135                 140                 145

agg gaa ggt gag cca ggc tgg gtc cgg ccc cga gga gtg gaa cag cgc        657
Arg Glu Gly Glu Pro Gly Trp Val Arg Pro Arg Gly Val Glu Gln Arg
                150                 155                 160

aat aag gcc ctc gac tgg ctc cga gga aaa ggg ggt gct gtt ggg ggg        705
Asn Lys Ala Leu Asp Trp Leu Arg Gly Lys Gly Gly Ala Val Gly Gly
            165                 170                 175

gag aag gat cca ccg cca cca ggg acc caa gga gtc gtg tat ttt gct        753
Glu Lys Asp Pro Pro Pro Pro Gly Thr Gln Gly Val Val Tyr Phe Ala
        180                 185                 190

gac gat gac aac acc tac agc cgg gag ctc ttt aag gag atg cgt tgg        801
Asp Asp Asp Asn Thr Tyr Ser Arg Glu Leu Phe Lys Glu Met Arg Trp
    195                 200                 205

act cgc ggt gtc tca gtg tgg cct gtg ggg ctg gtg ggt ggc ctg cga        849
Thr Arg Gly Val Ser Val Trp Pro Val Gly Leu Val Gly Gly Leu Arg
210                 215                 220                 225

ttt gaa ggt cct cag gta cag gat ggc cgc gtt gtg ggt ttc cac aca        897
Phe Glu Gly Pro Gln Val Gln Asp Gly Arg Val Val Gly Phe His Thr
                230                 235                 240

gca tgg gaa ccc aac agg ccc ttt ccc ttg gac atg gcg gga ttt gcg        945
Ala Trp Glu Pro Asn Arg Pro Phe Pro Leu Asp Met Ala Gly Phe Ala
            245                 250                 255

gtt gcc ctg ccc ttg cta ttg gct aag ccc aat gcc cag ttt gat gct        993
Val Ala Leu Pro Leu Leu Leu Ala Lys Pro Asn Ala Gln Phe Asp Ala
        260                 265                 270

act gca ccc cgg ggc cac ctg gaa agt agt ctc ctg agc cac ctt gta       1041
Thr Ala Pro Arg Gly His Leu Glu Ser Ser Leu Leu Ser His Leu Val
    275                 280                 285

gat ccc aag gac ctg gag cca cgg gct gcc aat tgt act cag gta ctg 1089
Asp Pro Lys Asp Leu Glu Pro Arg Ala Ala Asn Cys Thr Gln Val Leu
290                 295                 300                 305

gta tgg cac acc cgg aca gag aaa cct aag atg aag cag gag gag cag 1137
Val Trp His Thr Arg Thr Glu Lys Pro Lys Met Lys Gln Glu Glu Gln
                310                 315                 320

cta caa cgg cag ggc cag ggc tca gac cca gcc att gag gtg tga 1182
Leu Gln Arg Gln Gly Gln Gly Ser Asp Pro Ala Ile Glu Val
            325                 330                 335

tggcaacctc accctgactt ctaccttatt ttaggcacat accttgtggg actgggctcc 1242

aggccagccc aggatgtggt ttttttttta aagacctgac ccctgagaac cagaggcagc 1302

ccctccaacc acagggtgtg tcccagttgc ttccctccct tcccccagcc tgccatgtgg 1362

cactgcccac atgttgggga caagcagctt ctctagtgag ccagatcagc cccatctggg 1422

gcggagcagg acagatggac tcagaaagga gggtagctgt gggaaaaggg taacttattg 1482

gggacaagca tgcagtgggg ggctagagga gctgggctgg accctcccca cctgagcatg 1542

cgatcccctt cctacctcta gaataaagaa tctcaacccg gaaaaaaaaa aaaaaaa 1599

<210> 42
<211> 335
<212> PRT
<213> Mus musculus

<400> 42

Met Lys Leu Lys Leu Lys Asn Val Phe Leu Ala Tyr Phe Leu Val Ser
1               5                   10                  15

Ile Ala Gly Leu Leu Tyr Ala Leu Val Gln Leu Gly Gln Pro Cys Asp
            20                  25                  30

Cys Leu Pro Pro Leu Arg Ala Ala Ala Glu Gln Leu Arg Gln Lys Asp
        35                  40                  45

Leu Arg Ile Ser Gln Leu Gln Ala Asp Leu Arg Arg Pro Pro Pro Val
    50                  55                  60

Pro Ala Gln Pro Pro Glu Pro Glu Ala Leu Pro Thr Ile Tyr Val Ile
65                  70                  75                  80

Thr Pro Thr Tyr Ala Arg Leu Val Gln Lys Ala Glu Leu Val Arg Leu
                85                  90                  95

Ser Gln Thr Leu Ser Leu Val Pro Arg Leu His Trp Leu Leu Val Glu
            100                 105                 110

Asp Ala Glu Ser Pro Thr Pro Leu Val Ser Gly Leu Leu Ala Ala Ser
        115                 120                 125

Gly Leu Leu Phe Thr His Leu Ala Val Leu Thr Pro Lys Ala Gln Arg
    130                 135                 140

Leu Arg Glu Gly Glu Pro Gly Trp Val Arg Pro Arg Gly Val Glu Gln
145                 150                 155                 160

Arg Asn Lys Ala Leu Asp Trp Leu Arg Gly Lys Gly Gly Ala Val Gly
                165                 170                 175

Gly Glu Lys Asp Pro Pro Pro Pro Gly Thr Gln Gly Val Val Tyr Phe
            180                 185                 190

Ala Asp Asp Asp Asn Thr Tyr Ser Arg Glu Leu Phe Lys Glu Met Arg
        195                 200                 205

Trp Thr Arg Gly Val Ser Val Trp Pro Val Gly Leu Val Gly Gly Leu
    210                 215                 220

Arg Phe Glu Gly Pro Gln Val Gln Asp Gly Arg Val Val Gly Phe His
225                 230                 235                 240

Thr Ala Trp Glu Pro Asn Arg Pro Phe Pro Leu Asp Met Ala Gly Phe
                245                 250                 255

Ala Val Ala Leu Pro Leu Leu Leu Ala Lys Pro Asn Ala Gln Phe Asp
            260                 265                 270

Ala Thr Ala Pro Arg Gly His Leu Glu Ser Ser Leu Leu Ser His Leu
        275                 280                 285

Val Asp Pro Lys Asp Leu Glu Pro Arg Ala Ala Asn Cys Thr Gln Val
    290                 295                 300

Leu Val Trp His Thr Arg Thr Glu Lys Pro Lys Met Lys Gln Glu Glu
305                 310                 315                 320

Gln Leu Gln Arg Gln Gly Gln Gly Ser Asp Pro Ala Ile Glu Val
                325                 330                 335

<210>  43
<211>  2974
<212>  DNA
<213>  Mus musculus

<220>
<221>  CDS
<222>  (197)..(2203)

<400>  43
gataggggcc ccaggcttcc cacagcatgg agacacaggg agtgtggagg gtgcccccca      60

gcccacggac aacaccttca ctccgaagtg tgagattgtg ggcaaggacg cactgtcagc     120

actggcccgg gccagcacca agcagtgtca acaggagatc gctaatgtag tgtgcctgca     180

ccaagctggg aaccta atg ccc aag tct gtg ccc cgg cac tgc cag ctg gct     232
                  Met Pro Lys Ser Val Pro Arg His Cys Gln Leu Ala

                  1               5                   10

ggc aag atg agc cct ggc gtc caa tgg gaa gag atc cgg gcc cag cag 280
Gly Lys Met Ser Pro Gly Val Gln Trp Glu Glu Ile Arg Ala Gln Gln
        15                  20                  25

cct gtt ggt ggc cct cca gta cgc atc gcc tac atg ctg gtg gtt cac 328
Pro Val Gly Gly Pro Pro Val Arg Ile Ala Tyr Met Leu Val Val His
    30                  35                  40

ggc cgt gct atc cgc cag ctg aag cgt ctt ctg aag gcc gtc tac cat 376
Gly Arg Ala Ile Arg Gln Leu Lys Arg Leu Leu Lys Ala Val Tyr His
45                  50                  55                  60

gag caa cac ttc ttt tat ata cat gtg gac aag cgt tcc aac tac ctg 424
Glu Gln His Phe Phe Tyr Ile His Val Asp Lys Arg Ser Asn Tyr Leu
                65                  70                  75

tac cgg gag gta gtg gaa ctg gcc cag cac tac gag aat gtg cgg gtg 472
Tyr Arg Glu Val Val Glu Leu Ala Gln His Tyr Glu Asn Val Arg Val
            80                  85                  90

aca cct tgg cgc atg gtc acc atc tgg ggc ggg gcc agc ctt ctg agg 520
Thr Pro Trp Arg Met Val Thr Ile Trp Gly Gly Ala Ser Leu Leu Arg
        95                  100                 105

atg tat ctg agg agc atg aag gac ctt ctg gaa att cct ggc tgg acc 568
Met Tyr Leu Arg Ser Met Lys Asp Leu Leu Glu Ile Pro Gly Trp Thr
    110                 115                 120

tgg gac ttc ttc atc aac ctg agt gcc act gac tat cca acg agg acg 616
Trp Asp Phe Phe Ile Asn Leu Ser Ala Thr Asp Tyr Pro Thr Arg Thr
125                 130                 135                 140

aat gag gag ctg gta gca ttc tta tcc aag aac cgg gac aag aat ttc 664
Asn Glu Glu Leu Val Ala Phe Leu Ser Lys Asn Arg Asp Lys Asn Phe
                145                 150                 155

ctc aag tca cat ggg cga gac aat tcc agg ttc att aag aag caa ggc 712
Leu Lys Ser His Gly Arg Asp Asn Ser Arg Phe Ile Lys Lys Gln Gly
            160                 165                 170

ctg gac cgg ctt ttc cat gag tgt gat tcc cac atg tgg cgc ctg ggt 760
Leu Asp Arg Leu Phe His Glu Cys Asp Ser His Met Trp Arg Leu Gly
        175                 180                 185

gaa cgg cag atc ccg gca ggc att gtg gtg gat ggt ggc tct gac tgg 808
Glu Arg Gln Ile Pro Ala Gly Ile Val Val Asp Gly Gly Ser Asp Trp
    190                 195                 200

ttt gtg ctg aca cgc agc ttt gtg gaa tat gtg gtg tat aca gat gac 856
Phe Val Leu Thr Arg Ser Phe Val Glu Tyr Val Val Tyr Thr Asp Asp
205                 210                 215                 220

ccc ctc gtg gcc cag ctt cgc cag ttc tat aca tac aca ttg ctt ccg 904
Pro Leu Val Ala Gln Leu Arg Gln Phe Tyr Thr Tyr Thr Leu Leu Pro
                225                 230                 235

gct gag tca ttc ttc cac aca gtg ctg gag aac agc ccg gcc tgt gcg 952
Ala Glu Ser Phe Phe His Thr Val Leu Glu Asn Ser Pro Ala Cys Ala
            240                 245                 250

agc ctg gtg gac aac aac ctg cgg gtc acc aac tgg aac cgc aag ctg 1000
Ser Leu Val Asp Asn Asn Leu Arg Val Thr Asn Trp Asn Arg Lys Leu
        255                 260                 265

ggc tgc aaa tgc cag tac aag cac atc gtg gac tgg tgc ggc tgc tcc 1048
Gly Cys Lys Cys Gln Tyr Lys His Ile Val Asp Trp Cys Gly Cys Ser
    270                 275                 280

ccc aac gac ttc aag cca cag gac ttc ctg agg ctt cag caa gtc tcc 1096
Pro Asn Asp Phe Lys Pro Gln Asp Phe Leu Arg Leu Gln Gln Val Ser
285                 290                 295                 300

aga ccc acc ttc ttt gcc cgg aag ttc gag tcg act gtg aac cag gaa 1144
Arg Pro Thr Phe Phe Ala Arg Lys Phe Glu Ser Thr Val Asn Gln Glu
                305                 310                 315

gtc ctg gaa att ttg gac ttc cat cta tat ggc agc tac cca ccc agc 1192
Val Leu Glu Ile Leu Asp Phe His Leu Tyr Gly Ser Tyr Pro Pro Ser
            320                 325                 330

aca cca gcc ctc aaa gcc tac tgg gag aac atc tac gac gtg gcc gat 1240
Thr Pro Ala Leu Lys Ala Tyr Trp Glu Asn Ile Tyr Asp Val Ala Asp
        335                 340                 345

ggc cct ggc gga ctc agc gac gtc cta ctc aca gcc tac aca gcc ttt 1288
Gly Pro Gly Gly Leu Ser Asp Val Leu Leu Thr Ala Tyr Thr Ala Phe
    350                 355                 360

gcc cgt ctc agc ctg cgt cat gct gcc act gct gta tcc cca ctg gcc 1336
Ala Arg Leu Ser Leu Arg His Ala Ala Thr Ala Val Ser Pro Leu Ala
365                 370                 375                 380

act gca gtc tgc agg ttt gag ccc agg ggg ttg ccg tcc agc gtg cac 1384
Thr Ala Val Cys Arg Phe Glu Pro Arg Gly Leu Pro Ser Ser Val His
                385                 390                 395

ctg tat ttc tat gac gac cat ttc cag ggc tac ctg gtg acg cag gcg 1432
Leu Tyr Phe Tyr Asp Asp His Phe Gln Gly Tyr Leu Val Thr Gln Ala
            400                 405                 410

gtg cag ccc tca gcc cag ggg cca gca gag aca ctt gag atg tgg ctg 1480
Val Gln Pro Ser Ala Gln Gly Pro Ala Glu Thr Leu Glu Met Trp Leu
        415                 420                 425

atg ccc cag agg tcg ctg aag ctg ttg ggg cac agt gac cag gcc agc 1528
Met Pro Gln Arg Ser Leu Lys Leu Leu Gly His Ser Asp Gln Ala Ser
    430                 435                 440

cgg ctc cag agt ctg gag gtt ggt act gag tgg gac ccc aaa gaa cgt 1576
Arg Leu Gln Ser Leu Glu Val Gly Thr Glu Trp Asp Pro Lys Glu Arg
445                 450                 455                 460

ctc ttc cgg aac ttt ggg ggc ctg ctg gga cca ctg gat gag cct gta 1624
Leu Phe Arg Asn Phe Gly Gly Leu Leu Gly Pro Leu Asp Glu Pro Val
                465                 470                 475

gcc atg cag cgc tgg gcc cgg ggc ccc aac ctc aca gcc act gtg gtc 1672
Ala Met Gln Arg Trp Ala Arg Gly Pro Asn Leu Thr Ala Thr Val Val
            480                 485                 490

tgg att gac ccc acc tat gtt gtg gcc aca tcg tat gac atc acg gta 1720
Trp Ile Asp Pro Thr Tyr Val Val Ala Thr Ser Tyr Asp Ile Thr Val
        495                 500                 505

gat gcg gat acc gaa gtc acg cag tac aag cct cca ctg agc ctg cca 1768
Asp Ala Asp Thr Glu Val Thr Gln Tyr Lys Pro Pro Leu Ser Leu Pro
    510                 515                 520

ctg cgg ccg gga gcc tgg act gtc cgc ttg ctt cag ttc tgg gaa ccc 1816
Leu Arg Pro Gly Ala Trp Thr Val Arg Leu Leu Gln Phe Trp Glu Pro
525                 530                 535                 540

ctg ggt gag acc cgc ttc ctc gtg ctg ccg ttg acg ttc aac cgc aaa 1864
Leu Gly Glu Thr Arg Phe Leu Val Leu Pro Leu Thr Phe Asn Arg Lys
                545                 550                 555

cta cct ctc agg aaa gat gat gcc agc tgg ctg cat gcg gga cca ccc     1912
Leu Pro Leu Arg Lys Asp Asp Ala Ser Trp Leu His Ala Gly Pro Pro
            560                 565                 570

cac aac gaa tac atg gag cag agt ttc cag ggc cta agt ggc atc ctg     1960
His Asn Glu Tyr Met Glu Gln Ser Phe Gln Gly Leu Ser Gly Ile Leu
        575                 580                 585

aat ctg cct cag gca gag gcc ctg gag gag gct gcc cgg agg cac aca     2008
Asn Leu Pro Gln Ala Glu Ala Leu Glu Glu Ala Ala Arg Arg His Thr
    590                 595                 600

gag ctc acg ggt tct gca ctt gag gcc tgg aca gat ggg gaa ctg agc     2056
Glu Leu Thr Gly Ser Ala Leu Glu Ala Trp Thr Asp Gly Glu Leu Ser
605                 610                 615                 620

aat ttc tgg tct gtg gca gga ttg tgt gcc ata gga cct tct gct tgt     2104
Asn Phe Trp Ser Val Ala Gly Leu Cys Ala Ile Gly Pro Ser Ala Cys
                625                 630                 635

ccc tcc ctg gag ctc tgc aga ctg acc agc tgg agc tct ctg tct cct     2152
Pro Ser Leu Glu Leu Cys Arg Leu Thr Ser Trp Ser Ser Leu Ser Pro
            640                 645                 650

gac ccc aag tca gag ctg ggg cct gtc aaa gct gac ggg cga ctc agg     2200
Asp Pro Lys Ser Glu Leu Gly Pro Val Lys Ala Asp Gly Arg Leu Arg
        655                 660                 665

tag caggacccca gccagcacaa cccggaggag ccggggaatt gcaccttaca     2253

gacaacggag ggacatctct cccacagaca agaaccagag gcccaggcag tttgcccatt     2313

tgagccaaca ggaacctaca ctgagggcag ggaaggtggg tgcagtactc cctactgcta     2373

acggccctgc aagagaccag cggtccactg gagccatgag gaggatcttc ctctgctcct     2433

cgttagttta tactttttta ataattaaaa tgagtggcag agggaggacg atgtgcaata     2493

gggctcagag cagccccagg atatgggcca tggccatggg gagcaccaca gagcatatcg     2553

gtttctgctc tggggaggca tgggctgcct ccctctccag agcctccatc cccagcctgc     2613

atctgaactg cgcactgctg aggctctgag gcccggagga caggtgatct gtgggggcta     2673

ctgtggagtt ggactttagt gagcaggcct ctcctctggg agccagggag ccctagagca     2733

gagctggggc cggctctcgg gcttccctag gaaatgcgcg ccatcagggc ctgctgagat     2793

ccatgaagtt tccctactgg aggaggaagc ccactggcgg cacaggaagg ctgtctgtca     2853

gagctctcac aagagtcgag ttgtgcctga agctcagtgc gaagtctgaa atatgcaaca     2913

gaggaaatat atctctatct ctccaaaaaa aaaaaaaaaa aaaaaaaaaa aaaaaaaaaa     2973

a                                                                     2974

<210>  44
<211>  668
<212>  PRT
<213>  Mus musculus

<400>  44

Met Pro Lys Ser Val Pro Arg His Cys Gln Leu Ala Gly Lys Met Ser
1               5                   10                  15

Pro Gly Val Gln Trp Glu Glu Ile Arg Ala Gln Gln Pro Val Gly Gly

            20                  25                  30

Pro Pro Val Arg Ile Ala Tyr Met Leu Val Val His Gly Arg Ala Ile
        35                  40                  45

Arg Gln Leu Lys Arg Leu Leu Lys Ala Val Tyr His Glu Gln His Phe
    50                  55                  60

Phe Tyr Ile His Val Asp Lys Arg Ser Asn Tyr Leu Tyr Arg Glu Val
65                  70                  75                  80

Val Glu Leu Ala Gln His Tyr Glu Asn Val Arg Val Thr Pro Trp Arg
                85                  90                  95

Met Val Thr Ile Trp Gly Gly Ala Ser Leu Leu Arg Met Tyr Leu Arg
            100                 105                 110

Ser Met Lys Asp Leu Leu Glu Ile Pro Gly Trp Thr Trp Asp Phe Phe
        115                 120                 125

Ile Asn Leu Ser Ala Thr Asp Tyr Pro Thr Arg Thr Asn Glu Glu Leu
    130                 135                 140

Val Ala Phe Leu Ser Lys Asn Arg Asp Lys Asn Phe Leu Lys Ser His
145                 150                 155                 160

Gly Arg Asp Asn Ser Arg Phe Ile Lys Lys Gln Gly Leu Asp Arg Leu
                165                 170                 175

Phe His Glu Cys Asp Ser His Met Trp Arg Leu Gly Glu Arg Gln Ile
            180                 185                 190

Pro Ala Gly Ile Val Val Asp Gly Gly Ser Asp Trp Phe Val Leu Thr
        195                 200                 205

Arg Ser Phe Val Glu Tyr Val Val Tyr Thr Asp Asp Pro Leu Val Ala
    210                 215                 220

Gln Leu Arg Gln Phe Tyr Thr Tyr Thr Leu Leu Pro Ala Glu Ser Phe
225                 230                 235                 240

Phe His Thr Val Leu Glu Asn Ser Pro Ala Cys Ala Ser Leu Val Asp
                245                 250                 255

Asn Asn Leu Arg Val Thr Asn Trp Asn Arg Lys Leu Gly Cys Lys Cys
            260                 265                 270

Gln Tyr Lys His Ile Val Asp Trp Cys Gly Cys Ser Pro Asn Asp Phe
        275                 280                 285

Lys Pro Gln Asp Phe Leu Arg Leu Gln Gln Val Ser Arg Pro Thr Phe
    290                 295                 300

Phe Ala Arg Lys Phe Glu Ser Thr Val Asn Gln Glu Val Leu Glu Ile
305                 310                 315                 320

Leu Asp Phe His Leu Tyr Gly Ser Tyr Pro Pro Ser Thr Pro Ala Leu
                325                 330                 335

Lys Ala Tyr Trp Glu Asn Ile Tyr Asp Val Ala Asp Gly Pro Gly Gly
            340                 345                 350

Leu Ser Asp Val Leu Leu Thr Ala Tyr Thr Ala Phe Ala Arg Leu Ser
        355                 360                 365

Leu Arg His Ala Ala Thr Ala Val Ser Pro Leu Ala Thr Ala Val Cys
    370                 375                 380

Arg Phe Glu Pro Arg Gly Leu Pro Ser Ser Val His Leu Tyr Phe Tyr
385                 390                 395                 400

Asp Asp His Phe Gln Gly Tyr Leu Val Thr Gln Ala Val Gln Pro Ser
                405                 410                 415

Ala Gln Gly Pro Ala Glu Thr Leu Glu Met Trp Leu Met Pro Gln Arg
            420                 425                 430

Ser Leu Lys Leu Leu Gly His Ser Asp Gln Ala Ser Arg Leu Gln Ser
        435                 440                 445

Leu Glu Val Gly Thr Glu Trp Asp Pro Lys Glu Arg Leu Phe Arg Asn
    450                 455                 460

Phe Gly Gly Leu Leu Gly Pro Leu Asp Glu Pro Val Ala Met Gln Arg
465                 470                 475                 480

Trp Ala Arg Gly Pro Asn Leu Thr Ala Thr Val Val Trp Ile Asp Pro
                485                 490                 495

Thr Tyr Val Val Ala Thr Ser Tyr Asp Ile Thr Val Asp Ala Asp Thr
            500                 505                 510

Glu Val Thr Gln Tyr Lys Pro Pro Leu Ser Leu Pro Leu Arg Pro Gly
        515                 520                 525

Ala Trp Thr Val Arg Leu Leu Gln Phe Trp Glu Pro Leu Gly Glu Thr
    530                 535                 540

Arg Phe Leu Val Leu Pro Leu Thr Phe Asn Arg Lys Leu Pro Leu Arg
545                 550                 555                 560

Lys Asp Asp Ala Ser Trp Leu His Ala Gly Pro Pro His Asn Glu Tyr
                565                 570                 575

Met Glu Gln Ser Phe Gln Gly Leu Ser Gly Ile Leu Asn Leu Pro Gln
            580                 585                 590

Ala Glu Ala Leu Glu Glu Ala Ala Arg Arg His Thr Glu Leu Thr Gly
        595                 600                 605

Ser Ala Leu Glu Ala Trp Thr Asp Gly Glu Leu Ser Asn Phe Trp Ser
    610                 615                 620

Val Ala Gly Leu Cys Ala Ile Gly Pro Ser Ala Cys Pro Ser Leu Glu
625                 630                 635                 640

Leu Cys Arg Leu Thr Ser Trp Ser Ser Leu Ser Pro Asp Pro Lys Ser
                645                 650                 655

Glu Leu Gly Pro Val Lys Ala Asp Gly Arg Leu Arg
            660                 665

<210>  45
<211>  5532
<212>  DNA
<213>  Mus musculus

<220>
<221>  CDS
<222>  (369)..(1427)

<400>  45
   ccagtgcacg gcggcggcgg caccttcctt ctcgcatccc ggtcgcccgg cccgtgagga      60
   ggcagcggcg gccgcaggcg ccagcagagg aggcggcgga gcatcgagca gaggcgaggc     120
   ggaccggcag gagcgcgcgg ccggagccac gcatcctcac acttgcacac caactcctgc     180
   gcctttcacc ctctagacca gagtcggggc ctcggagagc ctcggtgaag ctagcgggag     240
   ccagctccgg agcccagcca gctctgttct gagcctcccc ggtgctggta cccccgcggc     300
   ggggtccccg cttctgcgct cgggacgcgc ctcccggaca gccgggtccc cgcggccagg     360
   acaaagcc atg aag ccg gcg ctg ctg gaa gtg atg agg atg aac aga att      410
            Met Lys Pro Ala Leu Leu Glu Val Met Arg Met Asn Arg Ile
            1               5                   10
   tgc cgg atg gtg ctg gcc act tgc ttc gga tcc ttt atc ttg gtc atc      458
   Cys Arg Met Val Leu Ala Thr Cys Phe Gly Ser Phe Ile Leu Val Ile
   15                  20                  25                  30
   ttc tat ttc caa agt atg ttg cac cca gtc atg cgg agg aac ccc ttc      506
   Phe Tyr Phe Gln Ser Met Leu His Pro Val Met Arg Arg Asn Pro Phe
                   35                  40                  45
   ggt gtg gac atc tgc tgc cgg aag gga tcg aga agt ccc ctg cag gag      554
   Gly Val Asp Ile Cys Cys Arg Lys Gly Ser Arg Ser Pro Leu Gln Glu
               50                  55                  60
   ctc tac aat ccc atc cag ctg gag cta tcc aac act gcc atc ctg cac      602
   Leu Tyr Asn Pro Ile Gln Leu Glu Leu Ser Asn Thr Ala Ile Leu His
           65                  70                  75
   cag atg aga cgg gac cag gtg aca gac acc tgc cgg gcc aac agt gcc      650
   Gln Met Arg Arg Asp Gln Val Thr Asp Thr Cys Arg Ala Asn Ser Ala
       80                  85                  90
   atg agc cgc aag cgc agg gtg ctg acc ccc aac gac ctg aag cac ctg      698
   Met Ser Arg Lys Arg Arg Val Leu Thr Pro Asn Asp Leu Lys His Leu
   95                  100                 105                 110
   gtg gtg gat gag gac cac gaa ctc atc tac tgc tat gtg ccc aag gta      746
   Val Val Asp Glu Asp His Glu Leu Ile Tyr Cys Tyr Val Pro Lys Val
                   115                 120                 125
   gcg tgc acc aac tgg aag agg ctc atg atg gtc ctg agt ggc cgg ggc      794
   Ala Cys Thr Asn Trp Lys Arg Leu Met Met Val Leu Ser Gly Arg Gly
               130                 135                 140
   aag tac agc gat ccc atg gag atc cca gcc aac gaa gcc cac gtg tcg      842
   Lys Tyr Ser Asp Pro Met Glu Ile Pro Ala Asn Glu Ala His Val Ser
           145                 150                 155
   gcc aac ctg aag acc ctt aac cag tac agc atc cca gag atc aac cac      890
   Ala Asn Leu Lys Thr Leu Asn Gln Tyr Ser Ile Pro Glu Ile Asn His
       160                 165                 170
   cgc ttg aaa agc tac atg aag ttc ctg ttc gtg cgg gaa ccc ttc gag      938
   Arg Leu Lys Ser Tyr Met Lys Phe Leu Phe Val Arg Glu Pro Phe Glu
   175                 180                 185                 190
   agg ctg gtg tct gcc tac cgc aac aag ttc acg cag aag tac aac acc      986
   Arg Leu Val Ser Ala Tyr Arg Asn Lys Phe Thr Gln Lys Tyr Asn Thr
                   195                 200                 205
   tcc ttc cac aag cgc tac ggc acc aag atc atc cga cgc cag cgg aag      1034
   Ser Phe His Lys Arg Tyr Gly Thr Lys Ile Ile Arg Arg Gln Arg Lys
               210                 215                 220
   aac gcc acg cag gag gcc ctg cgc aag ggg gac gat gtc aag ttc gag      1082
   Asn Ala Thr Gln Glu Ala Leu Arg Lys Gly Asp Asp Val Lys Phe Glu
           225                 230                 235
   gag ttc gtg gcc tac ctc atc gac ccc cac acc cag cgg gag gag ccc      1130
   Glu Phe Val Ala Tyr Leu Ile Asp Pro His Thr Gln Arg Glu Glu Pro
       240                 245                 250
   ttc aac gag cac tgg cag acg gtc tac tct ctc tgc cac ccg tgc cac      1178
   Phe Asn Glu His Trp Gln Thr Val Tyr Ser Leu Cys His Pro Cys His
   255                 260                 265                 270
   atc cac tac gac ctc gtg ggc aag tat gag aca ctg gag gag gac tcc      1226
   Ile His Tyr Asp Leu Val Gly Lys Tyr Glu Thr Leu Glu Glu Asp Ser
                   275                 280                 285
   aat tac gta ctg cag ctg gcc gga gtg agc ggc tac ctg aag ttc ccc      1274
   Asn Tyr Val Leu Gln Leu Ala Gly Val Ser Gly Tyr Leu Lys Phe Pro
               290                 295                 300
   acc tat gca aag tcc acc cga act acc gac gag atg acc acg gag ttc      1322
   Thr Tyr Ala Lys Ser Thr Arg Thr Thr Asp Glu Met Thr Thr Glu Phe
           305                 310                 315
   ttc cag aac atc agc gcc gag cac cag aca cag ctg tac gaa gtc tac      1370
   Phe Gln Asn Ile Ser Ala Glu His Gln Thr Gln Leu Tyr Glu Val Tyr
       320                 325                 330
   aaa ctg gac ttt tta atg ttc aac tac tca gtg cca aac tac ctg aag      1418
   Lys Leu Asp Phe Leu Met Phe Asn Tyr Ser Val Pro Asn Tyr Leu Lys
   335                 340                 345                 350
   ttg gat tag agggctgcgg aggggagggg gaggggtggt tgggggagag             1467
   Leu Asp
   ggagagaatc ctgcttttta atttaagatt tttatttgtc aaaagaattc tatggagact 1527
   gggttatttt gtaagttaat atgtcttcgg gggagatgct gcgagcggca tggttaagaa 1587
   ttatttaaaa attctccacg gggaaggaca gctgtctttg caggggagcg gggtggaata 1647
   tccctgtttt tagaagtgga tactgcaaca ctgtctccaa ggtgtccttg tgttctggtg 1707
   aagtccacaa actgcattcc ataaagtcta atgaatctta tttatagtta tttaaacgtg 1767
   gtctgtggca gcagcctgct cctgtctgtg cagaggagag tacagtctgt gcttcgctgg 1827
   ctatggctca ttgggggagg ggagggggtt atctccccca ctgaccgtgt gtagaatccc 1887
   atggtgacag ctgcgggatg tgtgcctgtg gccattcaag aaaccctgct agaaagaagt 1947
   caattgtcct tgcactaaga aacagggatg agactggttc aaaccgattg ctagcaaccc 2007
   caaagtccct tgtagtaaag aaatgcaaat ttttttaacc caagaagaga gcagatgata 2067
   gcgatttctc tttaccgaaa ttagcaggct ataagaatag gctttctgtg gaaatcttta 2127
   agtggcactt tccagtgcta gcgaaggcag aataatggtt gttttctagt caataagtat 2187
   tgagcatcta ctattctcca ggcttgactg cagatagcac attgagtgaa accaggagcc 2247
   ttggccttca gcaagcttta tgtgaacaca gggcatccag ctcaacagtg aaccaacacc 2307
   cccatccctt tggtcctcac acagacttta gactattctc aagtggtcca gagggtccag 2367
   gaccctagca aatttgcgta tttctacagt tcaaatttga aagccacaca tttggatcag 2427
   gccacttaac tagctaagta gttcattgcc caacacaaga aatgacagaa acccttttga 2487
   tcatgtatat ctagtgcagt gtcttcatag tagaagccta taagccacaa tattctccaa 2547
   gcgcaaggat ggaggactgc tgtgattgat tagtgatgtc tgccttgact aaggaaaggg 2607
   aggaacattt ctcaagggcc tcctactgtg atctccagtc cacatcttgt cctgcctgcc 2667
   acttaaaaca gtgcactgca tacatttgga gactggcgga gttcaagtgt gaaggtcaaa 2727
   ggtggagtgt tggtacccac aggcacatgc ccaccctcca tgtcctgcat tggcagagca 2787
   tccaggacct ccctctcctc cagaaggttg gaaatttgct ctttcctgtt ccttgctgat 2847
   caatgcccaa acctattcct catttctagt tgtttctgat actttgcggt tgacatttga 2907
   gccacattcc catgacagcc agtgtccggg tacgtagcag atgctgtaca tagttctgat 2967
   gataagggag ctggtaggct gtaaattttg ctttttgtgt ttttgtgggg ttttgtttgt 3027
   tttgatgcag tatagagtga agggggtaag aatattctga aaaacataaa attgagtaat 3087
   ttattcacag aaactattaa aatggtattg ttaggctcac aaggaggctg ggggcagcgg 3147
   taactgctac actgtctcgc agaggaaatt attttattta atgtgagaga aatgtgagcc 3207
   agagtggctg cctgaggcta tttataaaac gcaggtacct tccccaattt ctctcccctc 3267
   gcctttaata aatagattta tgtcatgtgt gtgaccaaga acagtgacag atcttggctg 3327
   tccctggaat agagaggcag tcatggtgct caggccctgg gcgtggtgta gctgatgcat 3387
   gtcccaccga gcccaggctc tccaaggcat cagtttgtgg tcacatatcc ttagaaaaca 3447
   aaattaagtc aggttcctgt ctgcaggagt tttcagagcc ttgaatacat agtctgcctt 3507
   gtgaaactgg gagaatgtag catttctggg ctgcatgacc taggtatcct gtatggcaga 3567
   ccatgtctta gctggttttt cacacagtag ccttcagtga gggtgatcta gcctgtctga 3627
   ggtctgcaaa gattgacagc tgcttcctag atccttccac aaatcagcag aaacagtgtc 3687
   catctgggaa ctttatgaga gtgttgcttt acctagttgg ggtgtcttca tctggatact 3747
   gccccagaga gtgatcccgt ccctccaagc acagcggagg gaagcctagg gtgttatgga 3807
   gtggggcagg tggcttaagg acaccttaaa ggccacttgg tggggataat ggcctcgtgg 3867
   ggatactcct cacttccacc cacagtttgg aactgttcac tgtgtgcttc aggagtttta 3927
   aatggagatt cgtgttgatt tccatggtcc gcatctgttt gtcttccgtc tgtggtccag 3987
   tgctgtgtgc agatggtact tactcccttt ctctctctta cagggagaaa gactgtcctt 4047
   ctggggagag tgtatcaata cgcttccaga atgagggagt ctttctcctg tcttgtgttc 4107
   acacttccta gtgacaattt tcaaaatatg tatcggcttg actgtttatt gtagagtgta 4167
   gaaaggtttc tgtgttcctg gctaaagaca tccattggtg aaatgtgctt ggaaatccaa 4227
   gtgttttcta ctgaggaaaa aaaaatgttg gaaaagcttt ctcctgcatg cctcttgtgc 4287
   ttagctagaa aggggagctg ccagcgtccc agctctgagc cactgttcaa aggtgcagct 4347
   gtgttttagg actaggtaca ggcagagtga taaggacatg cctgcttagt gtactcattt 4407
   atcgagaaag tttagactat gagccacagg ggtccctgcc tgcagtccct atagcctccc 4467
   cctgccccag tgcctttctc agctggtctg ggaatcaggg cagcccattg aggggggctc 4527
   agacaaccct ttcttcctgg ttctgggagt aaggctttct ctgcagccac aaggagaaca 4587
   tgggaaccag aaacttctct ttggtcagcc cttctgagca cacaggttta gcccatgctc 4647
   caagaatgct cctgagcatt gtataagccc ctaggactct tagctcatgt gataatgatt 4707
   gcattactat cctgctttaa ctgacaaaac cacagagaat tttgcttgct ttcctactct 4767
   cttcctctgc ccaggcctcc tccatacgta tacatacaca catgggcatg tatacacaca 4827
   catgcacaat agacatgcac acacacatgc acacatgggt atacatatac acacatgcac 4887
   aatagacatg catatataca tatacatgca cacattgata ggcatacaca catgcacagt 4947
   agacatgcac acatgggtat acatagacac acatgcacag acatgcatac acacacatgc 5007
   acacataaat gtgcacacac acatacacaa attgcccaag cctgtgcact cctggtaccc 5067
   atgtcccatc ctggcccacc tctttgtcag caggactgac tccatcactc cctcaggttt 5127
   tgcccaccag caccccccag ctttccccca gcagagctaa acagcccctc tgaatagaca 5187
   acaggacctt ctagaaacag ctgaaccctt ggacagcagc aggcagattt tgatctgctc 5247
   tcatcaggta caaaaggtgg tatccgtatt ctcttctgag catgctcagt aagcatatgg 5307
   acatagaatt taacatctct gtggagtgtg tgttttttac atatttgtat gcagtcgagg 5367
   agggcctgtt gtagaattct ctccctgtat cttactatac tgttaaagaa gctgaattct 5427
   atgttgccaa cagatgcgtg aaatgttcct ccaggaaaag ccattcaagc ctgattattt 5487
   ttctaagtaa cttcaattaa attgaagaag aaaaaaaaaa aaaaa 5532

<210>  46
<211>  352
<212>  PRT
<213>  Mus musculus

<400>  46

Met Lys Pro Ala Leu Leu Glu Val Met Arg Met Asn Arg Ile Cys Arg
1               5                   10                  15

Met Val Leu Ala Thr Cys Phe Gly Ser Phe Ile Leu Val Ile Phe Tyr
            20                  25                  30

Phe Gln Ser Met Leu His Pro Val Met Arg Arg Asn Pro Phe Gly Val
        35                  40                  45

Asp Ile Cys Cys Arg Lys Gly Ser Arg Ser Pro Leu Gln Glu Leu Tyr
    50                  55                  60

Asn Pro Ile Gln Leu Glu Leu Ser Asn Thr Ala Ile Leu His Gln Met
65                  70                  75                  80

Arg Arg Asp Gln Val Thr Asp Thr Cys Arg Ala Asn Ser Ala Met Ser
                85                  90                  95

Arg Lys Arg Arg Val Leu Thr Pro Asn Asp Leu Lys His Leu Val Val
            100                 105                 110

Asp Glu Asp His Glu Leu Ile Tyr Cys Tyr Val Pro Lys Val Ala Cys
        115                 120                 125

Thr Asn Trp Lys Arg Leu Met Met Val Leu Ser Gly Arg Gly Lys Tyr
    130                 135                 140

Ser Asp Pro Met Glu Ile Pro Ala Asn Glu Ala His Val Ser Ala Asn
145                 150                 155                 160

Leu Lys Thr Leu Asn Gln Tyr Ser Ile Pro Glu Ile Asn His Arg Leu
                165                 170                 175

Lys Ser Tyr Met Lys Phe Leu Phe Val Arg Glu Pro Phe Glu Arg Leu
            180                 185                 190

Val Ser Ala Tyr Arg Asn Lys Phe Thr Gln Lys Tyr Asn Thr Ser Phe
        195                 200                 205

His Lys Arg Tyr Gly Thr Lys Ile Ile Arg Arg Gln Arg Lys Asn Ala
    210                 215                 220

Thr Gln Glu Ala Leu Arg Lys Gly Asp Asp Val Lys Phe Glu Glu Phe
225                 230                 235                 240

Val Ala Tyr Leu Ile Asp Pro His Thr Gln Arg Glu Glu Pro Phe Asn

245                 250                 255

Glu His Trp Gln Thr Val Tyr Ser Leu Cys His Pro Cys His Ile His
            260                 265                 270

Tyr Asp Leu Val Gly Lys Tyr Glu Thr Leu Glu Glu Asp Ser Asn Tyr
        275                 280                 285

Val Leu Gln Leu Ala Gly Val Ser Gly Tyr Leu Lys Phe Pro Thr Tyr
    290                 295                 300

Ala Lys Ser Thr Arg Thr Thr Asp Glu Met Thr Thr Glu Phe Phe Gln
305                 310                 315                 320

Asn Ile Ser Ala Glu His Gln Thr Gln Leu Tyr Glu Val Tyr Lys Leu
                325                 330                 335

Asp Phe Leu Met Phe Asn Tyr Ser Val Pro Asn Tyr Leu Lys Leu Asp
            340                 345                 350

<210>  47
<211>  1785
<212>  DNA
<213>  Mus musculus

<220>
<221>  CDS
<222>  (149)..(1408)

<400>  47
ggcgatttcg gctgcagaat cagcatcacc agcaacagca gcagcggcgg tgactgtggc      60

gggcgctagg tccgtctcct agggaccatg tcccagctgt gcacaaggct gaagtgaagg     120

gccaggagtg ggcccagccc agggcagc atg acc aag ccg cgg ctc ttc cgg        172
                               Met Thr Lys Pro Arg Leu Phe Arg
                               1               5

ctg tgg ctg gta cta ggg tcg gct ctc atg atc ctt ttg atc att gta       220
Leu Trp Leu Val Leu Gly Ser Ala Leu Met Ile Leu Leu Ile Ile Val
    10                  15                  20

tat tgg gac aac gtg gga acc gcc cac ttc tat ctg cac acg tct ctc       268
Tyr Trp Asp Asn Val Gly Thr Ala His Phe Tyr Leu His Thr Ser Leu
25                  30                  35                  40

tcc agg cca cac atc cta gaa ccc ctt ccc acc cag gga ttg gtg gag       316
Ser Arg Pro His Ile Leu Glu Pro Leu Pro Thr Gln Gly Leu Val Glu
                45                  50                  55

gag aac gtg ttc aca tct gac gtg gat gag ttt ttg gat act ctc ctt       364
Glu Asn Val Phe Thr Ser Asp Val Asp Glu Phe Leu Asp Thr Leu Leu
            60                  65                  70

agt tct gac gcg aag cac aac gac ctt tcc agg aga aaa act gag cag       412
Ser Ser Asp Ala Lys His Asn Asp Leu Ser Arg Arg Lys Thr Glu Gln
        75                  80                  85

ccc ccg gcg ccc gcc ccc agc aag cca gtc ttg agc cac atg gag gag       460
Pro Pro Ala Pro Ala Pro Ser Lys Pro Val Leu Ser His Met Glu Glu
    90                  95                  100

aac gtg aga ggc tac gac tgg tcc act cat gat gcc cat cag aac cct     508
Asn Val Arg Gly Tyr Asp Trp Ser Thr His Asp Ala His Gln Asn Pro
105                 110                 115                 120

gac cgg gac agg cag cag gcc gag agg agg agc ctg ctg aga gac ttc     556
Asp Arg Asp Arg Gln Gln Ala Glu Arg Arg Ser Leu Leu Arg Asp Phe
                125                 130                 135

tgt gcc aac gcc agc ctg gca ttc ccc acc aag gac cgc tct ttt gac     604
Cys Ala Asn Ala Ser Leu Ala Phe Pro Thr Lys Asp Arg Ser Phe Asp
            140                 145                 150

gac atc ccc aac tac gaa ctg aac cac ctg atc gtg gac gac cgc cac     652
Asp Ile Pro Asn Tyr Glu Leu Asn His Leu Ile Val Asp Asp Arg His
        155                 160                 165

ggg gtc atc tac tgc tac gtg ccc aag gtg gcc tgc acc aac tgg aag     700
Gly Val Ile Tyr Cys Tyr Val Pro Lys Val Ala Cys Thr Asn Trp Lys
    170                 175                 180

cga gtg atg atc gtg ctg agc gag agc ctg ctg gac cgg ggc agc ccc     748
Arg Val Met Ile Val Leu Ser Glu Ser Leu Leu Asp Arg Gly Ser Pro
185                 190                 195                 200

tac cga gac ccc ctg gac atc ccc cgg gaa cac gtg cac aac acc agc     796
Tyr Arg Asp Pro Leu Asp Ile Pro Arg Glu His Val His Asn Thr Ser
                205                 210                 215

acg cac ctc acc ttc aac aag ttc tgg cgc cgc tac gga aag ttc tcc     844
Thr His Leu Thr Phe Asn Lys Phe Trp Arg Arg Tyr Gly Lys Phe Ser
            220                 225                 230

cgt cac ctc atg aag gtg aag ctg aag aag tac acc aag ttc ctg ttc     892
Arg His Leu Met Lys Val Lys Leu Lys Lys Tyr Thr Lys Phe Leu Phe
        235                 240                 245

gtg cgc gac ccc ttt gtg cgc ctc atc tca gcc ttc cgc agc aag ttc     940
Val Arg Asp Pro Phe Val Arg Leu Ile Ser Ala Phe Arg Ser Lys Phe
    250                 255                 260

gag ctg gag aac gaa gag ttt tac cgc aag ttc gcg gtg ccc atg ctc     988
Glu Leu Glu Asn Glu Glu Phe Tyr Arg Lys Phe Ala Val Pro Met Leu
265                 270                 275                 280

cga ctg tac gcc aac cac acc agc ctg ccc gcc tcg gtg agt gag gct     1036
Arg Leu Tyr Ala Asn His Thr Ser Leu Pro Ala Ser Val Ser Glu Ala
                285                 290                 295

ttc agc gcc ggg ctc aag gtc tcc ttc gcc aac ttc atc cag tac ctc     1084
Phe Ser Ala Gly Leu Lys Val Ser Phe Ala Asn Phe Ile Gln Tyr Leu
            300                 305                 310

cta gac cca cac acg gag aag ctg gcg cct ttc aac gag cac tgg cga     1132
Leu Asp Pro His Thr Glu Lys Leu Ala Pro Phe Asn Glu His Trp Arg
        315                 320                 325

cag gtg tac cgc ctc tgc cac ccg tgc cag ata gac tat gac ttc gtg     1180
Gln Val Tyr Arg Leu Cys His Pro Cys Gln Ile Asp Tyr Asp Phe Val
    330                 335                 340

ggg aag ctg gag acg ctc gat gag gac gct gcc cag ctc ctg agg ttc     1228
Gly Lys Leu Glu Thr Leu Asp Glu Asp Ala Ala Gln Leu Leu Arg Phe
345                 350                 355                 360

ctc aag gta gac tcc cag ctc cac ttc ccc ccc agt tat cgg aac agg     1276
Leu Lys Val Asp Ser Gln Leu His Phe Pro Pro Ser Tyr Arg Asn Arg
                365                 370                 375

acg gcc agc agc tgg gag gaa gac tgg ttt gcc aac atc ccc ctg gca 1324
Thr Ala Ser Ser Trp Glu Glu Asp Trp Phe Ala Asn Ile Pro Leu Ala
            380                 385                 390

tgg agg caa cag ctc tat aaa ctc tac gag gcc gac ttt gtt ctc ttt 1372
Trp Arg Gln Gln Leu Tyr Lys Leu Tyr Glu Ala Asp Phe Val Leu Phe
        395                 400                 405

ggc tac ccc aag cca gaa aac ctg ctc agg gac tga gcccccagaa 1418
Gly Tyr Pro Lys Pro Glu Asn Leu Leu Arg Asp
    410                 415

gccctcacgc tgcccccaac aaattgaatg gctgtcccat gaggccgtcc tttgaggatg 1478

ggaccctgtg gcctcctggg ttctctcctg gcttcctttg cttcctggtg tgacaggcag 1538

aggattccac gcccccccct cgcatctgga gaccgtggta cagccaagac cgaagcacct 1598

cactctccag agttttgcgc tccccacccc cgcccttttg caatctggat ttgtttactc 1658

cacagcctgt attcatggaa cactgtgtta aatactgttt tctaagatta atatatttca 1718

gatatattta atacgaaagt gggaggaagc tggagtaaag tgtggcgccc gcaaaaaaaa 1778

aaaaaaa 1785

<210> 48
<211> 419
<212> PRT
<213> Mus musculus

<400> 48

Met Thr Lys Pro Arg Leu Phe Arg Leu Trp Leu Val Leu Gly Ser Ala
1               5                   10                  15

Leu Met Ile Leu Leu Ile Ile Val Tyr Trp Asp Asn Val Gly Thr Ala
            20                  25                  30

His Phe Tyr Leu His Thr Ser Leu Ser Arg Pro His Ile Leu Glu Pro
        35                  40                  45

Leu Pro Thr Gln Gly Leu Val Glu Glu Asn Val Phe Thr Ser Asp Val
    50                  55                  60

Asp Glu Phe Leu Asp Thr Leu Leu Ser Ser Asp Ala Lys His Asn Asp
65                  70                  75                  80

Leu Ser Arg Arg Lys Thr Glu Gln Pro Pro Ala Pro Ala Pro Ser Lys
                85                  90                  95

Pro Val Leu Ser His Met Glu Glu Asn Val Arg Gly Tyr Asp Trp Ser
            100                 105                 110

Thr His Asp Ala His Gln Asn Pro Asp Arg Asp Arg Gln Gln Ala Glu
        115                 120                 125

Arg Arg Ser Leu Leu Arg Asp Phe Cys Ala Asn Ala Ser Leu Ala Phe
    130                 135                 140

Pro Thr Lys Asp Arg Ser Phe Asp Asp Ile Pro Asn Tyr Glu Leu Asn
145                 150                 155                 160

His Leu Ile Val Asp Asp Arg His Gly Val Ile Tyr Cys Tyr Val Pro
                165                 170                 175

Lys Val Ala Cys Thr Asn Trp Lys Arg Val Met Ile Val Leu Ser Glu
            180                 185                 190

Ser Leu Leu Asp Arg Gly Ser Pro Tyr Arg Asp Pro Leu Asp Ile Pro
        195                 200                 205

Arg Glu His Val His Asn Thr Ser Thr His Leu Thr Phe Asn Lys Phe
    210                 215                 220

Trp Arg Arg Tyr Gly Lys Phe Ser Arg His Leu Met Lys Val Lys Leu
225                 230                 235                 240

Lys Lys Tyr Thr Lys Phe Leu Phe Val Arg Asp Pro Phe Val Arg Leu
                245                 250                 255

Ile Ser Ala Phe Arg Ser Lys Phe Glu Leu Glu Asn Glu Glu Phe Tyr
            260                 265                 270

Arg Lys Phe Ala Val Pro Met Leu Arg Leu Tyr Ala Asn His Thr Ser
        275                 280                 285

Leu Pro Ala Ser Val Ser Glu Ala Phe Ser Ala Gly Leu Lys Val Ser
    290                 295                 300

Phe Ala Asn Phe Ile Gln Tyr Leu Leu Asp Pro His Thr Glu Lys Leu
305                 310                 315                 320

Ala Pro Phe Asn Glu His Trp Arg Gln Val Tyr Arg Leu Cys His Pro
                325                 330                 335

Cys Gln Ile Asp Tyr Asp Phe Val Gly Lys Leu Glu Thr Leu Asp Glu
            340                 345                 350

Asp Ala Ala Gln Leu Leu Arg Phe Leu Lys Val Asp Ser Gln Leu His
        355                 360                 365

Phe Pro Pro Ser Tyr Arg Asn Arg Thr Ala Ser Ser Trp Glu Glu Asp
    370                 375                 380

Trp Phe Ala Asn Ile Pro Leu Ala Trp Arg Gln Gln Leu Tyr Lys Leu
385                 390                 395                 400

Tyr Glu Ala Asp Phe Val Leu Phe Gly Tyr Pro Lys Pro Glu Asn Leu
                405                 410                 415

Leu Arg Asp

<210> 49
<211> 1728
<212> DNA
<213> Mus musculus

<220>
<221> CDS
<222> (1)..(1116)

<400> 49
atg act gtc gcc tgc cac gcg tgc cag gca cag cat ggg aag acg ctc 48
Met Thr Val Ala Cys His Ala Cys Gln Ala Gln His Gly Lys Thr Leu
1               5                   10                  15

ctg ttg cag gcg gcc ctt gcc ggt ggt ggc aag tct ggg tgc tgc act 96
Leu Leu Gln Ala Ala Leu Ala Gly Gly Gly Lys Ser Gly Cys Cys Thr
            20                  25                  30

cct gct cct gtg cgc cct gcg tcc cgg gta acc aca gga aag gat gcc 144
Pro Ala Pro Val Arg Pro Ala Ser Arg Val Thr Thr Gly Lys Asp Ala
        35                  40                  45

cag gac act gaa tgg cag ggc tcc cca aaa gcc ctt ttg ggg gtt ccg 192
Gln Asp Thr Glu Trp Gln Gly Ser Pro Lys Ala Leu Leu Gly Val Pro
    50                  55                  60

aca ttt gaa aat aaa gct ctg ggc tcc agc tgg ttc ggt gga gtg agg 240
Thr Phe Glu Asn Lys Ala Leu Gly Ser Ser Trp Phe Gly Gly Val Arg
65                  70                  75                  80

aag agt ccc cta cag ctg ttg cgt gac ctg gac cag ggt cca cgc tcc 288
Lys Ser Pro Leu Gln Leu Leu Arg Asp Leu Asp Gln Gly Pro Arg Ser
                85                  90                  95

gcg atg gcc gag gtg cac cag cag cgg cgt gag ctg ctg cgc cgg gcc 336
Ala Met Ala Glu Val His Gln Gln Arg Arg Glu Leu Leu Arg Arg Ala
            100                 105                 110

tgc agc cgc cac acg cga cgc caa cgc ctg ctg cag ccg gag gac ctg 384
Cys Ser Arg His Thr Arg Arg Gln Arg Leu Leu Gln Pro Glu Asp Leu
        115                 120                 125

cgt cac gtg ctg gtg gac gac gcg cac cgg ctg ctg tac tgc tac gtg 432
Arg His Val Leu Val Asp Asp Ala His Arg Leu Leu Tyr Cys Tyr Val
    130                 135                 140

cct aag gtg gcc tgc acc aac tgg aag cgt gtg atg ctg gcg ttg cgc 480
Pro Lys Val Ala Cys Thr Asn Trp Lys Arg Val Met Leu Ala Leu Arg
145                 150                 155                 160

ggc cgt ggg gat cca agc gca atc cct gcg cac gag gcg cat gcg cct 528
Gly Arg Gly Asp Pro Ser Ala Ile Pro Ala His Glu Ala His Ala Pro
                165                 170                 175

ggc ctg ctg ccc tcg ctg gcc gac ttt gcg ccg gct gag gtc aac tgg 576
Gly Leu Leu Pro Ser Leu Ala Asp Phe Ala Pro Ala Glu Val Asn Trp
            180                 185                 190

cgg ctg cgc gac tac ctg acc ttt ctc ttc gtg cgg gag ccc ttc gag 624
Arg Leu Arg Asp Tyr Leu Thr Phe Leu Phe Val Arg Glu Pro Phe Glu
        195                 200                 205

cgc ctg gcg tca gcc tac cgc aac aag ctg gcg cgg cca cac agc gcg 672
Arg Leu Ala Ser Ala Tyr Arg Asn Lys Leu Ala Arg Pro His Ser Ala

210                 215                 220

gcc ttc cag cgg cgc tat ggc aca cgc atc gtg cgt cgc cta cga cca     720
Ala Phe Gln Arg Arg Tyr Gly Thr Arg Ile Val Arg Arg Leu Arg Pro
225                 230                 235                 240

cac gcg cag ccc gat gcg ctg gcc cgc ggc cac gac gtg cgc ttc gcc     768
His Ala Gln Pro Asp Ala Leu Ala Arg Gly His Asp Val Arg Phe Ala
                245                 250                 255

gag ttc ctg gcc tac ctg ctc gac ccg cgc acg cgc cgt cat gag ccc     816
Glu Phe Leu Ala Tyr Leu Leu Asp Pro Arg Thr Arg Arg His Glu Pro
            260                 265                 270

ttc aac gaa cac tgg gag cgc gca cac gcg ctg tgc cat ccg tgc cta     864
Phe Asn Glu His Trp Glu Arg Ala His Ala Leu Cys His Pro Cys Leu
        275                 280                 285

gtg cgc tat gat gta gtg ggc aag ttt gag acg ata gca gat gat gct     912
Val Arg Tyr Asp Val Val Gly Lys Phe Glu Thr Ile Ala Asp Asp Ala
    290                 295                 300

gcc ttc gtg ctg gac ctg gtg ggt gag cct ggg cta cgt ttc cct gct     960
Ala Phe Val Leu Asp Leu Val Gly Glu Pro Gly Leu Arg Phe Pro Ala
305                 310                 315                 320

cca ccg ctg agg cca gag aag gac ctt acg cgt gag cag gcc cgg cgc     1008
Pro Pro Leu Arg Pro Glu Lys Asp Leu Thr Arg Glu Gln Ala Arg Arg
                325                 330                 335

ctt ttc cag gac atc agc ccc ttc tac cag cgt cgc ctc ttt aac ctc     1056
Leu Phe Gln Asp Ile Ser Pro Phe Tyr Gln Arg Arg Leu Phe Asn Leu
            340                 345                 350

tat aag atg gac ttt ctg ctc ttc aat tac tct gcc cct tcc tac ctg     1104
Tyr Lys Met Asp Phe Leu Leu Phe Asn Tyr Ser Ala Pro Ser Tyr Leu
        355                 360                 365

cga ctg caa taa gggtgttggg tgcaatagag ccagtggctg ctgtgaccag         1156
Arg Leu Gln
    370

gaggccacca ggaggtctgg aacgaacctg gttgtgtgga ttggagacct tatccagtgg   1216

gcctgaccaa gagtctggcc actggtcaca ctcattccga ctgggtaggg tacaggttgc   1276

tttaggtgac cataaccttg tcaggccgtt tctgctgtta gtttgatgtg tgtctcttcc   1336

tcccactctg cagatgtcag gcttcttcct aggactccag gtttgtagtt ctttggtttg   1396

gtttgagagg ccatttctca gtcttgtctg tgtagaacct gtccgtggca tggtgctaca   1456

agacagaatg tcatggcttg gttcagtgtg gcctaaggtc ttgtcagcat ttactgctta   1516

ggagttaaca tgagctgcct gccaccccag cagtcacagg atggtgagca ctaccactcc   1576

acatctacct ggctgatcta cctttgatct cagccttgca agaggctgga tcttcccctg   1636

tgtcagcaaa ggccaagatg caatactgtg gcagcttttc cagctcactt ttattttttt   1696

tgttgttttt ttaaataaat atgttttgtt ac                                 1728

<210>  50
<211>  371
<212>  PRT
<213>  Mus musculus

<400>  50

Met Thr Val Ala Cys His Ala Cys Gln Ala Gln His Gly Lys Thr Leu
1               5                   10                  15

Leu Leu Gln Ala Ala Leu Ala Gly Gly Gly Lys Ser Gly Cys Cys Thr
            20                  25                  30

Pro Ala Pro Val Arg Pro Ala Ser Arg Val Thr Thr Gly Lys Asp Ala
        35                  40                  45

Gln Asp Thr Glu Trp Gln Gly Ser Pro Lys Ala Leu Leu Gly Val Pro
    50                  55                  60

Thr Phe Glu Asn Lys Ala Leu Gly Ser Ser Trp Phe Gly Gly Val Arg
65                  70                  75                  80

Lys Ser Pro Leu Gln Leu Leu Arg Asp Leu Asp Gln Gly Pro Arg Ser
                85                  90                  95

Ala Met Ala Glu Val His Gln Gln Arg Arg Glu Leu Leu Arg Arg Ala
            100                 105                 110

Cys Ser Arg His Thr Arg Arg Gln Arg Leu Leu Gln Pro Glu Asp Leu
        115                 120                 125

Arg His Val Leu Val Asp Asp Ala His Arg Leu Leu Tyr Cys Tyr Val
    130                 135                 140

Pro Lys Val Ala Cys Thr Asn Trp Lys Arg Val Met Leu Ala Leu Arg
145                 150                 155                 160

Gly Arg Gly Asp Pro Ser Ala Ile Pro Ala His Glu Ala His Ala Pro
                165                 170                 175

Gly Leu Leu Pro Ser Leu Ala Asp Phe Ala Pro Ala Glu Val Asn Trp
            180                 185                 190

Arg Leu Arg Asp Tyr Leu Thr Phe Leu Phe Val Arg Glu Pro Phe Glu
        195                 200                 205

Arg Leu Ala Ser Ala Tyr Arg Asn Lys Leu Ala Arg Pro His Ser Ala
    210                 215                 220

Ala Phe Gln Arg Arg Tyr Gly Thr Arg Ile Val Arg Arg Leu Arg Pro
225                 230                 235                 240

His Ala Gln Pro Asp Ala Leu Ala Arg Gly His Asp Val Arg Phe Ala
                245                 250                 255

Glu Phe Leu Ala Tyr Leu Leu Asp Pro Arg Thr Arg Arg His Glu Pro
            260                 265                 270

Phe Asn Glu His Trp Glu Arg Ala His Ala Leu Cys His Pro Cys Leu
        275                 280                 285

Val Arg Tyr Asp Val Val Gly Lys Phe Glu Thr Ile Ala Asp Asp Ala
    290                 295                 300

Ala Phe Val Leu Asp Leu Val Gly Glu Pro Gly Leu Arg Phe Pro Ala
305                 310                 315                 320

Pro Pro Leu Arg Pro Glu Lys Asp Leu Thr Arg Glu Gln Ala Arg Arg
                325                 330                 335

Leu Phe Gln Asp Ile Ser Pro Phe Tyr Gln Arg Arg Leu Phe Asn Leu
            340                 345                 350

Tyr Lys Met Asp Phe Leu Leu Phe Asn Tyr Ser Ala Pro Ser Tyr Leu
        355                 360                 365

Arg Leu Gln
    370

<210>  51
<211>  2073
<212>  DNA
<213>  Mus musculus

<220>
<221>  CDS
<222>  (129)..(1259)

<400>  51
ccggccctgg tccctgcctg caccccggga gctggccacc cactatccct cccctcccga      60

gacctccagc ccctgctgca gtcacctccc ctgcagcctc gaggtcggcg aggtctggcc     120

gcagcacc atg ttt ccc cgc cct ctg acc cca ctg gct gcc ccg aaa agc      170
         Met Phe Pro Arg Pro Leu Thr Pro Leu Ala Ala Pro Lys Ser
         1               5                   10

gcg gag acc ctg ggc cgc acg cca agg cgg gcc cca ttg ggc cgg gcc      218
Ala Glu Thr Leu Gly Arg Thr Pro Arg Arg Ala Pro Leu Gly Arg Ala
15                  20                  25                  30

cgg gct ggg ctc ggg ggg ccg ccc ctg ctg ctg ccg tcc atg ctg atg      266
Arg Ala Gly Leu Gly Gly Pro Pro Leu Leu Leu Pro Ser Met Leu Met
                35                  40                  45

ttc gct gta atc gtg gcc tcc agc gga ctg ctg ctc atg atc gag cga      314
Phe Ala Val Ile Val Ala Ser Ser Gly Leu Leu Leu Met Ile Glu Arg
            50                  55                  60

ggc atc cta tcg gag atg aaa ccc ctt ccc ctg cac cct ccc agc cac      362
Gly Ile Leu Ser Glu Met Lys Pro Leu Pro Leu His Pro Pro Ser His
        65                  70                  75

aaa ggc gcg gcc tgg agc ggg aca gat cct aag cct aga ggc cta tcc      410
Lys Gly Ala Ala Trp Ser Gly Thr Asp Pro Lys Pro Arg Gly Leu Ser
    80                  85                  90

ttg gat gct ggg gac tcg gac ttg caa gtg agg gag gac atc cga aac      458
Leu Asp Ala Gly Asp Ser Asp Leu Gln Val Arg Glu Asp Ile Arg Asn

95                      100                     105                     110

cgg acc ttg agg gcc gtg tgc gga caa cca ggc atg ccc cgg gac ccc      506
Arg Thr Leu Arg Ala Val Cys Gly Gln Pro Gly Met Pro Arg Asp Pro
                115                 120                 125

tgg gac ttg ccg gtg gga cag cgg cgc acc ctg ctg cgc cac att ctc      554
Trp Asp Leu Pro Val Gly Gln Arg Arg Thr Leu Leu Arg His Ile Leu
            130                 135                 140

gta agt gac cgc tac cgc ttc ctc tac tgc tat gtc ccc aaa gtg gcc      602
Val Ser Asp Arg Tyr Arg Phe Leu Tyr Cys Tyr Val Pro Lys Val Ala
        145                 150                 155

tgc tct aac tgg aaa cgt gtg ctg aag gtg ctg gct ggc gtc ctg aac      650
Cys Ser Asn Trp Lys Arg Val Leu Lys Val Leu Ala Gly Val Leu Asn
    160                 165                 170

aac gtg gat gtc cgc ctc aag atg gac cac ccc agt gac ttg gtg ttt      698
Asn Val Asp Val Arg Leu Lys Met Asp His Pro Ser Asp Leu Val Phe
175                 180                 185                 190

ctg gca gac ctg cgg cct gag gag att cgc tac cgt ctg cag cac tac      746
Leu Ala Asp Leu Arg Pro Glu Glu Ile Arg Tyr Arg Leu Gln His Tyr
                195                 200                 205

ttc aag ttc ctg ttt gtg cga gac ccc ttg gaa cgc ctc ctg tct gct      794
Phe Lys Phe Leu Phe Val Arg Asp Pro Leu Glu Arg Leu Leu Ser Ala
            210                 215                 220

tac cgt aac aag ttt gga gag atc cga gag tac cag cag cga tat ggg      842
Tyr Arg Asn Lys Phe Gly Glu Ile Arg Glu Tyr Gln Gln Arg Tyr Gly
        225                 230                 235

gcc gaa att gtc agg cgc tac agg gct gga gct ggt ccc agc cct gca      890
Ala Glu Ile Val Arg Arg Tyr Arg Ala Gly Ala Gly Pro Ser Pro Ala
    240                 245                 250

ggg gac gat gtc acc ttc cca gag ttc ctg aga tac ctg gtg gat gag      938
Gly Asp Asp Val Thr Phe Pro Glu Phe Leu Arg Tyr Leu Val Asp Glu
255                 260                 265                 270

gat cct gaa cat atg aat gag cat tgg atg cct gtg tac cac ctg tgc      986
Asp Pro Glu His Met Asn Glu His Trp Met Pro Val Tyr His Leu Cys
                275                 280                 285

caa cca tgt gct gtg cac tac gac ttc gtg ggt tcc tat gag agg ctg      1034
Gln Pro Cys Ala Val His Tyr Asp Phe Val Gly Ser Tyr Glu Arg Leu
            290                 295                 300

gag gct gat gcc aac cag gtg ctg gag tgg gtg cgg gcc cca ccc cat      1082
Glu Ala Asp Ala Asn Gln Val Leu Glu Trp Val Arg Ala Pro Pro His
        305                 310                 315

gtc cgg ttc cca gct cgc cag gcc tgg tac cgg cca gcc agc cca gaa      1130
Val Arg Phe Pro Ala Arg Gln Ala Trp Tyr Arg Pro Ala Ser Pro Glu
    320                 325                 330

agt ctg cat tac cac ttg tgc aat gtt cca cgg gcc ctg ctt caa gat      1178
Ser Leu His Tyr His Leu Cys Asn Val Pro Arg Ala Leu Leu Gln Asp
335                 340                 345                 350

gtg cta cct aag tat atc ctg gac ttc tcc ctc ttt gct tac cca ctg      1226
Val Leu Pro Lys Tyr Ile Leu Asp Phe Ser Leu Phe Ala Tyr Pro Leu
                355                 360                 365

ccc aat gtc acc aag gaa gcc tgt cac caa tga cagtaggcca gcaccttttg    1279
Pro Asn Val Thr Lys Glu Ala Cys His Gln
            370                 375

gagtttgggt ttaatgatat cagctttggg atgtctttca gagaaactcc tggctctggg 1339

tggcttcctg gtttctctag gtgtctccat atctcagtgg taaggactgt ccttggaggt 1399

ccttgtccac agtggctcag aggacagagc tagaaaggag gcctgctgct ttcactggtg 1459

aactgcctct cttaggggcc tgtggtatcc gtgtctgcag ggcaccagtg gttattaaag 1519

ccatatgttt gatcgaaaga ctgacttcag ccccctggct gctgggtcta tgcagtccac 1579

ctggtctgtt gtaatttaac ctgtggccaa atcccaaata tgacactagc caagcacatg 1639

atcatgccta ggaccaatgg ctgtgacccc ctattcaccc atcccatgga cctcaggact 1699

ggagtgagct gtggtgcctt agaaatgaaa tgtgtgcaat tctactccag acttttacat 1759

ttcctcctct tgctaggtct gaatcatttt tctaaggaaa gagaaacgga agtggggccc 1819

ttacctcgaa gctctaaagc ccagcccctc aagcatccaa agacgcctgt gcctgacctc 1879

ttcctagggc tcctggagca tcttcaataa gcctcccttc cctacaaacc tttggagact 1939

atgtgagact gtatggccca tatatctggc tgtcacttgt ctaatgcatt tatttaaaat 1999

gtgtatattt taataggatc cttgtaaggg ctgactttta ataaagcttt ttcatataca 2059

aaaaaaaaaa aaaa 2073

<210> 52
<211> 376
<212> PRT
<213> Mus musculus

<400> 52

Met Phe Pro Arg Pro Leu Thr Pro Leu Ala Ala Pro Lys Ser Ala Glu
1               5                   10                  15

Thr Leu Gly Arg Thr Pro Arg Arg Ala Pro Leu Gly Arg Ala Arg Ala
            20                  25                  30

Gly Leu Gly Gly Pro Pro Leu Leu Leu Pro Ser Met Leu Met Phe Ala
        35                  40                  45

Val Ile Val Ala Ser Ser Gly Leu Leu Leu Met Ile Glu Arg Gly Ile
    50                  55                  60

Leu Ser Glu Met Lys Pro Leu Pro Leu His Pro Pro Ser His Lys Gly
65                  70                  75                  80

Ala Ala Trp Ser Gly Thr Asp Pro Lys Pro Arg Gly Leu Ser Leu Asp
                85                  90                  95

Ala Gly Asp Ser Asp Leu Gln Val Arg Glu Asp Ile Arg Asn Arg Thr
            100                 105                 110

Leu Arg Ala Val Cys Gly Gln Pro Gly Met Pro Arg Asp Pro Trp Asp
        115                 120                 125

Leu Pro Val Gly Gln Arg Arg Thr Leu Leu Arg His Ile Leu Val Ser
    130                 135                 140

Asp Arg Tyr Arg Phe Leu Tyr Cys Tyr Val Pro Lys Val Ala Cys Ser
145                 150                 155                 160

Asn Trp Lys Arg Val Leu Lys Val Leu Ala Gly Val Leu Asn Asn Val
                165                 170                 175

Asp Val Arg Leu Lys Met Asp His Pro Ser Asp Leu Val Phe Leu Ala
            180                 185                 190

Asp Leu Arg Pro Glu Glu Ile Arg Tyr Arg Leu Gln His Tyr Phe Lys
        195                 200                 205

Phe Leu Phe Val Arg Asp Pro Leu Glu Arg Leu Leu Ser Ala Tyr Arg
    210                 215                 220

Asn Lys Phe Gly Glu Ile Arg Glu Tyr Gln Gln Arg Tyr Gly Ala Glu
225                 230                 235                 240

Ile Val Arg Arg Tyr Arg Ala Gly Ala Gly Pro Ser Pro Ala Gly Asp
                245                 250                 255

Asp Val Thr Phe Pro Glu Phe Leu Arg Tyr Leu Val Asp Glu Asp Pro
            260                 265                 270

Glu His Met Asn Glu His Trp Met Pro Val Tyr His Leu Cys Gln Pro
        275                 280                 285

Cys Ala Val His Tyr Asp Phe Val Gly Ser Tyr Glu Arg Leu Glu Ala
    290                 295                 300

Asp Ala Asn Gln Val Leu Glu Trp Val Arg Ala Pro Pro His Val Arg
305                 310                 315                 320

Phe Pro Ala Arg Gln Ala Trp Tyr Arg Pro Ala Ser Pro Glu Ser Leu
                325                 330                 335

His Tyr His Leu Cys Asn Val Pro Arg Ala Leu Leu Gln Asp Val Leu
            340                 345                 350

Pro Lys Tyr Ile Leu Asp Phe Ser Leu Phe Ala Tyr Pro Leu Pro Asn
        355                 360                 365

Val Thr Lys Glu Ala Cys His Gln
    370                 375

<210>  53
<211>  6000
<212>  DNA
<213>  Mus musculus

<220>
<221> CDS
<222> (496)..(1914)

<400> 53
cttagccact gaaccatctc tccagcctct gttttggggt gtttgtttga gacagaatca 60

aaaggctggt ctaaagtaca tattgtagct aaagatggct tccaactcct gccttgcctc 120

cagagtgtta gggtgtgacc tttaaatgac ctattaaaga aaccccaaca ggtgtagagt 180

acacctgtga tcccagctct taggaggttg gacagatgga taaagaggtc caggtgtccc 240

ggactacata gagagaccct atctcaaaag agaggcgaag acttaggttt tgagagtggg 300

cctgaggtct ctcttcaaaa gttttataga aagatgtctc tgttccctgt ctatgaacac 360

ggatggcctg agcacctgtc tcttcacagg atcagagtgt cccccacctg aagagggctg 420

attgggtccc aagctatgct cctgagctga gtgcctgcag ccagtctgag gaactccatg 480

gcgcccctc tcccc atg gag aaa gga ctc gct ttg cct cag gat ttc cgg 531
Met Glu Lys Gly Leu Ala Leu Pro Gln Asp Phe Arg
1               5                   10

gac ctt gta cac agc cta aag att cga ggc aga tac gtc ttg ttc ctg 579
Asp Leu Val His Ser Leu Lys Ile Arg Gly Arg Tyr Val Leu Phe Leu
        15                  20                  25

gca ttt gtg gtc ata gtt ttt atc ttc att gaa aag gaa aat aaa atc 627
Ala Phe Val Val Ile Val Phe Ile Phe Ile Glu Lys Glu Asn Lys Ile
    30                  35                  40

ata tcc agg gtc tcc gac aag ctg aag cag atc cct cat ttt gtg gca 675
Ile Ser Arg Val Ser Asp Lys Leu Lys Gln Ile Pro His Phe Val Ala
45                  50                  55                  60

gat gcc aac agc act gac cca gcc ctg ctc tta tcg gag aat gca tct 723
Asp Ala Asn Ser Thr Asp Pro Ala Leu Leu Leu Ser Glu Asn Ala Ser
                65                  70                  75

ctc ttg tcc ctg agc gag ttg gat tcc acc ttt tcc cat ctg cgg agc 771
Leu Leu Ser Leu Ser Glu Leu Asp Ser Thr Phe Ser His Leu Arg Ser
            80                  85                  90

cgc ctg cac aac ctg agc ctg cag ctg ggc gtg gag cca gca atg gag 819
Arg Leu His Asn Leu Ser Leu Gln Leu Gly Val Glu Pro Ala Met Glu
        95                  100                 105

agc cag gag gct ggg gca gag aag cca tcc cag cag gct gga gca ggg 867
Ser Gln Glu Ala Gly Ala Glu Lys Pro Ser Gln Gln Ala Gly Ala Gly
    110                 115                 120

acc cgg cgc cac gtg ctt ctc atg gcc acc acc cgc acg ggt tcc tcg 915
Thr Arg Arg His Val Leu Leu Met Ala Thr Thr Arg Thr Gly Ser Ser
125                 130                 135                 140

ttc gtg ggc gag ttc ttc aac cag cag ggc aat atc ttc tac ctc ttc 963
Phe Val Gly Glu Phe Phe Asn Gln Gln Gly Asn Ile Phe Tyr Leu Phe
                145                 150                 155

gag cca ctg tgg cac atc gag cgc acc gtg ttc ttc cag cag cga ggc 1011
Glu Pro Leu Trp His Ile Glu Arg Thr Val Phe Phe Gln Gln Arg Gly
            160                 165                 170

gcc agc gcg gct ggt tca gcc ttg gtc tac cgt gat gtc ctc aag cag 1059
Ala Ser Ala Ala Gly Ser Ala Leu Val Tyr Arg Asp Val Leu Lys Gln
        175                 180                 185

ttg ttg cta tgc gac ctg tat gtg ctg gag ccc ttc atc agc cct ccg 1107
Leu Leu Leu Cys Asp Leu Tyr Val Leu Glu Pro Phe Ile Ser Pro Pro
    190                 195                 200

ccc gag gac cac ttg act cag ttc ctg ttc cgc cgg gga tcc agc cgt 1155
Pro Glu Asp His Leu Thr Gln Phe Leu Phe Arg Arg Gly Ser Ser Arg
205                 210                 215                 220

tca ctc tgc gag gat ccg gtg tgc aca ccc ttc gtc aag aag gtc ttt 1203
Ser Leu Cys Glu Asp Pro Val Cys Thr Pro Phe Val Lys Lys Val Phe
                225                 230                 235

gag aag tac cac tgc agg aac cgt cgc tgc ggg cca ctc aac gtg acc 1251
Glu Lys Tyr His Cys Arg Asn Arg Arg Cys Gly Pro Leu Asn Val Thr
            240                 245                 250

ttg gcg ggc gag gcc tgc cgc cgc aag gac cac gtg gcc ctc aag gct 1299
Leu Ala Gly Glu Ala Cys Arg Arg Lys Asp His Val Ala Leu Lys Ala
        255                 260                 265

gtg cgc atc cgt cag ctg gag ttc ctg cag ccg cta gtt gag gac ccg 1347
Val Arg Ile Arg Gln Leu Glu Phe Leu Gln Pro Leu Val Glu Asp Pro
    270                 275                 280

agg ttg gat cta cga gtc att cag ctg gtg cgc gac ccc cgg gcc gtg 1395
Arg Leu Asp Leu Arg Val Ile Gln Leu Val Arg Asp Pro Arg Ala Val
285                 290                 295                 300

ctg gct tca cgc ata gtg gcc ttt gcg ggc aag tat gag aac tgg aag 1443
Leu Ala Ser Arg Ile Val Ala Phe Ala Gly Lys Tyr Glu Asn Trp Lys
                305                 310                 315

aag tgg ctg tcc gag ggg cag gac cag ctg agc gag gat gag gtg cag 1491
Lys Trp Leu Ser Glu Gly Gln Asp Gln Leu Ser Glu Asp Glu Val Gln
            320                 325                 330

cga ttg cgg ggc aac tgt gag agc atc cgc ctg tct gca gag ctg ggc 1539
Arg Leu Arg Gly Asn Cys Glu Ser Ile Arg Leu Ser Ala Glu Leu Gly
        335                 340                 345

ttg cgg cag cca gcc tgg ctg cgc ggt cgt tac atg ctg gtg cgc tat 1587
Leu Arg Gln Pro Ala Trp Leu Arg Gly Arg Tyr Met Leu Val Arg Tyr
    350                 355                 360

gag gat gtg gca cgc agg cca ctg cag aag gcc cga gag atg tac agc 1635
Glu Asp Val Ala Arg Arg Pro Leu Gln Lys Ala Arg Glu Met Tyr Ser
365                 370                 375                 380

ttt gcg ggc atc ccc ttg acc ccg cag gtg gag gac tgg atc cag aag 1683
Phe Ala Gly Ile Pro Leu Thr Pro Gln Val Glu Asp Trp Ile Gln Lys
                385                 390                 395

aac acg cag gcg aca cgc gac agc agc gat gtc tac tcc act cag aaa 1731
Asn Thr Gln Ala Thr Arg Asp Ser Ser Asp Val Tyr Ser Thr Gln Lys
            400                 405                 410

aac tct tct gag cag ttt gag aag tgg cgc ttc agc atg cct ttc aag 1779
Asn Ser Ser Glu Gln Phe Glu Lys Trp Arg Phe Ser Met Pro Phe Lys
        415                 420                 425

ctg gca cag gtg gta cag gct gcc tgt ggc ccg acc atg cac ctc ttt 1827
Leu Ala Gln Val Val Gln Ala Ala Cys Gly Pro Thr Met His Leu Phe
    430                 435                 440

ggc tac aag ttg gcc agg gat gcc gcc tca ctc acc aac cgc tcc atc 1875
Gly Tyr Lys Leu Ala Arg Asp Ala Ala Ser Leu Thr Asn Arg Ser Ile
445                 450                 455                 460

agc ctg ctg gag gag cgg ggc acc ttc tgg gtc acg tag tgggggatgt 1924
Ser Leu Leu Glu Glu Arg Gly Thr Phe Trp Val Thr
                465                 470

ctgggaccct tggaactcct tcttgtgaaa ggctggccct gcttccctca cacccagcct 1984

ggcagtgaga catagccctg gcagaaagtc aaaatgggga gcgatgatgg gaacatagcc 2044

cctggctgta gcggtagggc cccctgccag ctagactccc cagagcagct acagctaggg 2104

ccttgggctc ctctgaggac accctgtctc cttagtggta ctggtcataa ggtgtcctca 2164

ccctatgacc tgcagtgtcc cgagcaggtc aaggtaggtt cctgtgtgtg gacacacctc 2224

cctagctctt tctcacacag tctacacgaa gcctgtaaag gcctgtaggt tgtgtgggct 2284

ggagtccaag tatttaacaa ccagaagggg tgtagaccct ctgcagcctc tcagacctcc 2344

tactgtatta agtgttaacc tcttccgctc tgagtcagag aagctgggat ctggctgagt 2404

cctgggaagg aggaggacag cctagggtga ggaaggggac ctttgaagct cctcagggaa 2464

cagtggctgt gtaccagctc atagaaaatg gtttgatcag ctgttctagt cactcatgag 2524

tatcaatcag cctgtgtaga gcaagacaca caaagtgcat tgaaaacaga caaggccagg 2584

ggtgtggctc agcggtaaag cccttgcctc tctcatgccc agggccctag gtgtgattct 2644

tagcactata aatataaggg aaaataagcc atacatacgt gtgcagtaca gagagagaca 2704

gaaaagcgta ataaacattc ccccctctca taagcacacg ctctgcctct gtctctgtct 2764

ctgtctctct ctgtctctgt ctctctctgt ctctctctct cactctctct ctctctctct 2824

ctctctcaca cacacacaca cacacacaca cacaaacaca cacacacaca ctggtacaga 2884

aaagcttccc ttctccactc tttatgctca ccatgttttt aggatttttg tttgtttgtt 2944

ttgttttggg tttctatata tctaaacaag gagccttaaa ttacatcttt ttgggattac 3004

gtggcgaggg ggagcagcag ggagcatttg cttctggtaa aaggatagac cagaaaggac 3064

ttccccttgg atgatcggag tcatgcagag cccccagggt cccacatgtt ctgtgtgaac 3124

ttcatgtgga atgactcaca gaagtgacta ccttaggcgc tcgctctgta gctcaatgtg 3184

cgtagagttt gtctattgta taggaagccc tgggttctgt gccctgcccc acaccatata 3244

ggtatgatgg cgtgtaccca tatcccagca ttcaggaaat caaaggttgg cggtcaaaag 3304

ttcaaggtgc agctatgtat cttttgagat gtccgagtct ataagctccc tctactgaga 3364

gctgctagta ttccttctca agaacaggtt atttgaaaat ctcttcttgt aagccccaac 3424

atttggggga ggggaggatg ggggaactct tgactgagca gctaggtttc cccaagcaga 3484

agttcttctc tcccagtgca agctgaggtt tagtgccccg gctagcctcg gaactggctc 3544

agtgggggtt ggcacgctga cttcctggac aattgctgca tctgacactg tgagcgccca 3604

ggacaccgga tgtgtagcat ggatgtgggc ctagcacagg tgtggaagag gacagtcagg 3664

acactcactg ttggtgtttg ttatccacct gttcagccgc cgggtcccac acataggtca 3724

tctttcgtca ctcaagccgg cctctgtttt tgattttaac aatccaagag caagtgtgta 3784

ggggacaaag agacaccagc tcttcatact tgatggagac cgggacagga tgctgcaggc 3844

aggctcggga gtgcattatc gtttcttctg agctcttcat ccaggccata ttcccttttc 3904

tgcatcgcat ctggggtggg aggggctgag ctgggagttt ccgtccttct tccctgtggg 3964
ggtgggagat ggggctcaag gtctccatct ctcggctccc tgaggacaga acccccacag 4024
tggacctttg ggccttctca ggacattgac aatgttgtgt gcactgcaag ttgactttat 4084
ttattttgta ggaaaaagag atggtattct ttagcaggat ctgaaactgt accctagtca 4144
agaagtacaa ttaataacat tattattaat aacaattggg atgattcaaa ggtcacacac 4204
acaccagacc acccaagaac catgtaggag atgaggcgga gccaggtcat ggaagcccag 4264
aatgctacag agattctgtg ggtctattat tgatcagaaa atacaacatg ggggggcctt 4324
ttctatggca taactcaggt gtctgcaaag acagagcagc tcaggtgggt ggataggcag 4384
gcagatggag ccttgaatca catcccctga ggctcagcca gccagtgcga atcctcaagg 4444
ttcggaaagg gacagtgtag cggcaaacta ctgggcatct tctctgatgc ttagaggctc 4504
ttaccaagga gcttgatgga gaaagtggcc atgttggtgg catgggacaa ctgctcaccc 4564
aacttcaaag atcaaagggc acccaggtgg cctatgacag tgacactctt tcctaccatt 4624
aggggtgtct gcccccactg aaagccatga aatttctggc cctaaggggg taggaaggac 4684
ttaggagtag cagatggttt gatcccatcc cccccacaca cacacacaca tccctcagct 4744
gtcttccaca ttagagccac ttcagttgtc catggacttg tccctttgag acatcctgga 4804
ttttgaagga tagaaacatc cccaagatgg tctcgtgtta atcccacaac agaggcagaa 4864
gggtcatact gagagagaga gagagagaga gagagagaga gagaggagtg ggggccagac 4924
cctcaagaag ccaagtgggt ctggtcaacc tgtgcacatg agaaggaggg aacatcacta 4984
aaatcagggc ctgggctggt gtgttgttgg tgagatcccg tggagtggct ggctagatat 5044
ggatgagttt tctgagcatg ctcacacacc cccaacttca cattcttaga aatagcacaa 5104
ccataatgcc ttacctcaaa aggatgaggc aaagcttgca attaattccc agtgtctgaa 5164
gacctccggg atcctttgag ctgtgtgtcc ttgtcagtac atggggacag gctccttagg 5224
tattcctgac atagaggtaa ggtgctgccc ttgctgcctt gcataagctg tgacaagctt 5284
cttactgggc acatagaacg gctctgtcat ctgcttccac taaatttggg cttggacttt 5344
gcctcctgcc aaatctccat ctctgctgga tagtctagtc cctagggtct aaccaccacc 5404
ctccacttcc tggtgggtcc tacaagcgct gtctttgtgc cagtacccgg atggtgtcct 5464
gcctcatggc taaatggtac aggacatctt cccagactga gtggtcatgg catgtgcatg 5524
tatgttcatg agtacatgca tggtgtgtgg gggtggatgg gtgcattctg ttctgcttct 5584
ggtgcagcta cacagccaca acctctttct gtcattgacc ttcttggtct tcttgtagcc 5644
acagataatc ttccaatgcc cgattctgct gttctcatct gagagctgac aacccagcgc 5704
tcagagtaga gttcatgacc taggaaaccc ttctcctggt agcttactga acttatttaa 5764
ttaaaaacga acatataggg gttggagaga tggttcattg gttaagagca ccggctgctt 5824
ttgcaaagga cctgagttca aatcccagca accatatggt ggcttacaac catctgtaat 5884
gagttctggt gccctcttct ggcatgcagg tgtacatgca gatagagcac tcctatgcat 5944

aaaataaata aatcttttaa ataaataaat aaatgaacat gaaaaaaaaa aaaaaa 6000

<210> 54
<211> 472
<212> PRT
<213> Mus musculus

<400> 54

Met Glu Lys Gly Leu Ala Leu Pro Gln Asp Phe Arg Asp Leu Val His
1               5                   10                  15

Ser Leu Lys Ile Arg Gly Arg Tyr Val Leu Phe Leu Ala Phe Val Val
            20                  25                  30

Ile Val Phe Ile Phe Ile Glu Lys Glu Asn Lys Ile Ile Ser Arg Val
        35                  40                  45

Ser Asp Lys Leu Lys Gln Ile Pro His Phe Val Ala Asp Ala Asn Ser
    50                  55                  60

Thr Asp Pro Ala Leu Leu Leu Ser Glu Asn Ala Ser Leu Leu Ser Leu
65                  70                  75                  80

Ser Glu Leu Asp Ser Thr Phe Ser His Leu Arg Ser Arg Leu His Asn
                85                  90                  95

Leu Ser Leu Gln Leu Gly Val Glu Pro Ala Met Glu Ser Gln Glu Ala
            100                 105                 110

Gly Ala Glu Lys Pro Ser Gln Gln Ala Gly Ala Gly Thr Arg Arg His
        115                 120                 125

Val Leu Leu Met Ala Thr Thr Arg Thr Gly Ser Ser Phe Val Gly Glu
    130                 135                 140

Phe Phe Asn Gln Gln Gly Asn Ile Phe Tyr Leu Phe Glu Pro Leu Trp
145                 150                 155                 160

His Ile Glu Arg Thr Val Phe Phe Gln Gln Arg Gly Ala Ser Ala Ala
                165                 170                 175

Gly Ser Ala Leu Val Tyr Arg Asp Val Leu Lys Gln Leu Leu Leu Cys
            180                 185                 190

Asp Leu Tyr Val Leu Glu Pro Phe Ile Ser Pro Pro Pro Glu Asp His
        195                 200                 205

Leu Thr Gln Phe Leu Phe Arg Arg Gly Ser Ser Arg Ser Leu Cys Glu
    210                 215                 220

Asp Pro Val Cys Thr Pro Phe Val Lys Lys Val Phe Glu Lys Tyr His
225                 230                 235                 240

Cys Arg Asn Arg Arg Cys Gly Pro Leu Asn Val Thr Leu Ala Gly Glu
                245                 250                 255

Ala Cys Arg Arg Lys Asp His Val Ala Leu Lys Ala Val Arg Ile Arg
            260                 265                 270

Gln Leu Glu Phe Leu Gln Pro Leu Val Glu Asp Pro Arg Leu Asp Leu
        275                 280                 285

Arg Val Ile Gln Leu Val Arg Asp Pro Arg Ala Val Leu Ala Ser Arg
    290                 295                 300

Ile Val Ala Phe Ala Gly Lys Tyr Glu Asn Trp Lys Lys Trp Leu Ser
305                 310                 315                 320

Glu Gly Gln Asp Gln Leu Ser Glu Asp Glu Val Gln Arg Leu Arg Gly
                325                 330                 335

Asn Cys Glu Ser Ile Arg Leu Ser Ala Glu Leu Gly Leu Arg Gln Pro
            340                 345                 350

Ala Trp Leu Arg Gly Arg Tyr Met Leu Val Arg Tyr Glu Asp Val Ala
        355                 360                 365

Arg Arg Pro Leu Gln Lys Ala Arg Glu Met Tyr Ser Phe Ala Gly Ile
    370                 375                 380

Pro Leu Thr Pro Gln Val Glu Asp Trp Ile Gln Lys Asn Thr Gln Ala
385                 390                 395                 400

Thr Arg Asp Ser Ser Asp Val Tyr Ser Thr Gln Lys Asn Ser Ser Glu
                405                 410                 415

Gln Phe Glu Lys Trp Arg Phe Ser Met Pro Phe Lys Leu Ala Gln Val
            420                 425                 430

Val Gln Ala Ala Cys Gly Pro Thr Met His Leu Phe Gly Tyr Lys Leu
        435                 440                 445

Ala Arg Asp Ala Ala Ser Leu Thr Asn Arg Ser Ile Ser Leu Leu Glu
    450                 455                 460

Glu Arg Gly Thr Phe Trp Val Thr
465                 470

<210>  55
<211>  2080
<212>  DNA
<213>  Mus musculus

<220>
<221>  CDS

<222>  (147)..(1601)

<400>  55
caccgcccgg ctgctccact ccgctccacc caacattgag ggagacccga agaggccgga      60

gccgaggact ttgggctggg ttttctggac agaaccagca ggcgcctact ctgctctggg     120

tggggagagt gaggattcgg tgaact atg aag ggc cgg cgg cgg cgg cgc cga     173
                             Met Lys Gly Arg Arg Arg Arg Arg Arg
                             1               5

aag tat tgc aag ttc acg ctg ctc ttg gcg ctg tac acg ctt ttg cta     221
Lys Tyr Cys Lys Phe Thr Leu Leu Leu Ala Leu Tyr Thr Leu Leu Leu
10                  15                  20                  25

ctt ctt gtc ccc tct gta ctg gac agc cac agc gag cag gac aag ggc     269
Leu Leu Val Pro Ser Val Leu Asp Ser His Ser Glu Gln Asp Lys Gly
                30                  35                  40

agg aac tgc ccc ggc ctg cag cgc agc ttg ggt gtg tgg agc ctg gag     317
Arg Asn Cys Pro Gly Leu Gln Arg Ser Leu Gly Val Trp Ser Leu Glu
            45                  50                  55

gcg gcg gcg gcc ggg gaa cgt gag cag ggc gct gag gtg cgg tcc ctg     365
Ala Ala Ala Ala Gly Glu Arg Glu Gln Gly Ala Glu Val Arg Ser Leu
        60                  65                  70

gcc gaa gga aac ccg gat cga tcc ccc ggg tcc ccc ggc aac ctc agc     413
Ala Glu Gly Asn Pro Asp Arg Ser Pro Gly Ser Pro Gly Asn Leu Ser
    75                  80                  85

gcc gtc ggt gag gcg gtg acc cag gaa aag caa cac atc tat gtg cat     461
Ala Val Gly Glu Ala Val Thr Gln Glu Lys Gln His Ile Tyr Val His
90                  95                  100                 105

gcc acc tgg cgc acc ggc tcg tcc ttc ttg ggc gaa ctc ttc aac cag     509
Ala Thr Trp Arg Thr Gly Ser Ser Phe Leu Gly Glu Leu Phe Asn Gln
                110                 115                 120

cac ccg gac gtt ttc tac ttg tac gac ccc atg tgg cat ctg tgg cag     557
His Pro Asp Val Phe Tyr Leu Tyr Asp Pro Met Trp His Leu Trp Gln
            125                 130                 135

gca ctg tat ccg ggc gac gcg gag agc ctg cag ggc gca cta aga gac     605
Ala Leu Tyr Pro Gly Asp Ala Glu Ser Leu Gln Gly Ala Leu Arg Asp
        140                 145                 150

atg ctg cgc tcc ctc ttc cgc tgt gat ttc tct gtg ctg cgc ctg tac     653
Met Leu Arg Ser Leu Phe Arg Cys Asp Phe Ser Val Leu Arg Leu Tyr
    155                 160                 165

gcg cag cct ggg gac cct ggg gag cga gca ccg gac tcg gcc aac ctc     701
Ala Gln Pro Gly Asp Pro Gly Glu Arg Ala Pro Asp Ser Ala Asn Leu
170                 175                 180                 185

acc acg gcc atg ctt ttc cgc tgg cgg acc aac aag gtc atc tgc tcg     749
Thr Thr Ala Met Leu Phe Arg Trp Arg Thr Asn Lys Val Ile Cys Ser
                190                 195                 200

ccg cct ctg tgc ccc gcc gcg ccc cgg gca cgc gcg gac gtg gga ctc     797
Pro Pro Leu Cys Pro Ala Ala Pro Arg Ala Arg Ala Asp Val Gly Leu
            205                 210                 215

gtc gag gac aaa gcc tgc gaa agt acc tgc ccg ccc gtt tcg ctc cgc     845
Val Glu Asp Lys Ala Cys Glu Ser Thr Cys Pro Pro Val Ser Leu Arg
        220                 225                 230

gcc ctg gag gcc gag tgc cgc aag tac ccg gtg gtg gtc atc aaa gac     893
Ala Leu Glu Ala Glu Cys Arg Lys Tyr Pro Val Val Val Ile Lys Asp

235                     240                     245

gtg cgg cta ctg gac ctg gga gtg ctg gtc cct ctg ctg cgt gac cca     941
Val Arg Leu Leu Asp Leu Gly Val Leu Val Pro Leu Leu Arg Asp Pro
250                 255                 260                 265

ggc ctc aac cta aag gtg gtg caa ctc ttc cga gac cct cgg gcc gtg     989
Gly Leu Asn Leu Lys Val Val Gln Leu Phe Arg Asp Pro Arg Ala Val
                270                 275                 280

cac aac tcg cgc ctc aag tcg agg cag gga ctg ctg cgc gaa agc atc     1037
His Asn Ser Arg Leu Lys Ser Arg Gln Gly Leu Leu Arg Glu Ser Ile
            285                 290                 295

cag gtg ctg cgc acg cgc cag agg ggc gac cac ttc cac cgg gtg ctg     1085
Gln Val Leu Arg Thr Arg Gln Arg Gly Asp His Phe His Arg Val Leu
        300                 305                 310

ctg gcg cat gga gtg gat gcc cgt ccg gga ggc cag gcc cgg gct ctg     1133
Leu Ala His Gly Val Asp Ala Arg Pro Gly Gly Gln Ala Arg Ala Leu
    315                 320                 325

ccc tcg gcg cca cgc gct gat ttc ttc tta acc agc gcg ctt gag gtg     1181
Pro Ser Ala Pro Arg Ala Asp Phe Phe Leu Thr Ser Ala Leu Glu Val
330                 335                 340                 345

atc tgt gaa gcg tgg ctt cgc gac ctg cta ttc acc cgc ggc gcg ccc     1229
Ile Cys Glu Ala Trp Leu Arg Asp Leu Leu Phe Thr Arg Gly Ala Pro
                350                 355                 360

gcc tgg ctg agg cgt cgc tac ctg cgg ctg cgt tat gag gac ctg gtg     1277
Ala Trp Leu Arg Arg Arg Tyr Leu Arg Leu Arg Tyr Glu Asp Leu Val
            365                 370                 375

tgg cag ccc caa gcc cag ctg cgc cgc ctg ctg cgc ttc tct ggg ttg     1325
Trp Gln Pro Gln Ala Gln Leu Arg Arg Leu Leu Arg Phe Ser Gly Leu
        380                 385                 390

cgg aca ctc gcc gcg ctt gat gcc ttc gca ttc aat atg acg cgg ggc     1373
Arg Thr Leu Ala Ala Leu Asp Ala Phe Ala Phe Asn Met Thr Arg Gly
    395                 400                 405

tcg gcc tac ggc gcc gat cgt ccc ttc cac ttg tct gcg cgg gac gcc     1421
Ser Ala Tyr Gly Ala Asp Arg Pro Phe His Leu Ser Ala Arg Asp Ala
410                 415                 420                 425

cga gag gct gtg cac gcc tgg cgc gaa cgt ctg agc caa gag cag gtg     1469
Arg Glu Ala Val His Ala Trp Arg Glu Arg Leu Ser Gln Glu Gln Val
                430                 435                 440

cgc caa gtg gaa acc gcc tgc gcc cct gcc atg cgt ctg ctt gcc tac     1517
Arg Gln Val Glu Thr Ala Cys Ala Pro Ala Met Arg Leu Leu Ala Tyr
            445                 450                 455

cct cga agt ggg gac gaa cgc gac agg aag acc gtc agg gaa ggg gag     1565
Pro Arg Ser Gly Asp Glu Arg Asp Arg Lys Thr Val Arg Glu Gly Glu
        460                 465                 470

aca cca ctg gag acc aag gcc aat tgg gct gtg taa taccctgatc     1611
Thr Pro Leu Glu Thr Lys Ala Asn Trp Ala Val
    475                 480

cctgaaccct gccccggggc gtattcaggt cagtggccat aaaaaggtga actcagcatg     1671

ctgcccccgc actggagagg ctgcacggtg gaggcgatct atcacactgt gagacactgg     1731

gactgatttg gtatcaactg ctgtgccatt ctcctggtca ggagcatcac aagctgttaa     1791

gtaatgacag acaccttggc tgagatgaag tttccagaaa ggaagtaaca gtgcaatgtg     1851

gatatttgtg accacaacat aggaaaagct gtacttccca ggctgaactt ggctcagctt 1911
gagccatttc aacaaggcat cctcacaata atgaagagat gtgatctggt ttcctttcac 1971
atcagccaag atgtctggac aaaaccatca atgtgaataa gggccaagtg cagttgtgtc 2031
tctcttgatt aaattacttc atattaaata aaaaaaaaa aaaaaaaa 2080

<210> 56
<211> 484
<212> PRT
<213> Mus musculus

<400> 56

Met Lys Gly Arg Arg Arg Arg Arg Arg Lys Tyr Cys Lys Phe Thr Leu
1               5                   10                  15

Leu Leu Ala Leu Tyr Thr Leu Leu Leu Leu Leu Val Pro Ser Val Leu
            20                  25                  30

Asp Ser His Ser Glu Gln Asp Lys Gly Arg Asn Cys Pro Gly Leu Gln
        35                  40                  45

Arg Ser Leu Gly Val Trp Ser Leu Glu Ala Ala Ala Ala Gly Glu Arg
    50                  55                  60

Glu Gln Gly Ala Glu Val Arg Ser Leu Ala Glu Gly Asn Pro Asp Arg
65                  70                  75                  80

Ser Pro Gly Ser Pro Gly Asn Leu Ser Ala Val Gly Glu Ala Val Thr
                85                  90                  95

Gln Glu Lys Gln His Ile Tyr Val His Ala Thr Trp Arg Thr Gly Ser
            100                 105                 110

Ser Phe Leu Gly Glu Leu Phe Asn Gln His Pro Asp Val Phe Tyr Leu
        115                 120                 125

Tyr Asp Pro Met Trp His Leu Trp Gln Ala Leu Tyr Pro Gly Asp Ala
    130                 135                 140

Glu Ser Leu Gln Gly Ala Leu Arg Asp Met Leu Arg Ser Leu Phe Arg
145                 150                 155                 160

Cys Asp Phe Ser Val Leu Arg Leu Tyr Ala Gln Pro Gly Asp Pro Gly
                165                 170                 175

Glu Arg Ala Pro Asp Ser Ala Asn Leu Thr Thr Ala Met Leu Phe Arg
            180                 185                 190

Trp Arg Thr Asn Lys Val Ile Cys Ser Pro Pro Leu Cys Pro Ala Ala
        195                 200                 205

Pro Arg Ala Arg Ala Asp Val Gly Leu Val Glu Asp Lys Ala Cys Glu
    210                 215                 220

Ser Thr Cys Pro Pro Val Ser Leu Arg Ala Leu Glu Ala Glu Cys Arg
225                 230                 235                 240

Lys Tyr Pro Val Val Val Ile Lys Asp Val Arg Leu Leu Asp Leu Gly
                245                 250                 255

Val Leu Val Pro Leu Leu Arg Asp Pro Gly Leu Asn Leu Lys Val Val
            260                 265                 270

Gln Leu Phe Arg Asp Pro Arg Ala Val His Asn Ser Arg Leu Lys Ser
        275                 280                 285

Arg Gln Gly Leu Leu Arg Glu Ser Ile Gln Val Leu Arg Thr Arg Gln
    290                 295                 300

Arg Gly Asp His Phe His Arg Val Leu Leu Ala His Gly Val Asp Ala
305                 310                 315                 320

Arg Pro Gly Gly Gln Ala Arg Ala Leu Pro Ser Ala Pro Arg Ala Asp
                325                 330                 335

Phe Phe Leu Thr Ser Ala Leu Glu Val Ile Cys Glu Ala Trp Leu Arg
            340                 345                 350

Asp Leu Leu Phe Thr Arg Gly Ala Pro Ala Trp Leu Arg Arg Arg Tyr
        355                 360                 365

Leu Arg Leu Arg Tyr Glu Asp Leu Val Trp Gln Pro Gln Ala Gln Leu
    370                 375                 380

Arg Arg Leu Leu Arg Phe Ser Gly Leu Arg Thr Leu Ala Ala Leu Asp
385                 390                 395                 400

Ala Phe Ala Phe Asn Met Thr Arg Gly Ser Ala Tyr Gly Ala Asp Arg
                405                 410                 415

Pro Phe His Leu Ser Ala Arg Asp Ala Arg Glu Ala Val His Ala Trp
            420                 425                 430

Arg Glu Arg Leu Ser Gln Glu Gln Val Arg Gln Val Glu Thr Ala Cys
        435                 440                 445

Ala Pro Ala Met Arg Leu Leu Ala Tyr Pro Arg Ser Gly Asp Glu Arg
    450                 455                 460

Asp Arg Lys Thr Val Arg Glu Gly Glu Thr Pro Leu Glu Thr Lys Ala
465                 470                 475                 480

Asn Trp Ala Val

<210> 57
<211> 512
<212> DNA
<213> Mus musculus

<400> 57
atgaaaggca tcttatggat gtgctcaacc ttattactaa cccatgcact acatcaagcc 60

aaaatggaaa ccagcccacc tgttaaaggc tctctgtctg gaaaagtggt cctaccttgt 120

catctttcaa ccttacctac cttaccaccc aattacaaca cgagtgaatt tctcagaatc 180

aaatggtcta agatggaagt ggacaaaaat ggaaaagata taaaggagac gactgtcttg 240

gtggcccaga acggaaatat caagattggt caggactaca aggggcgagt gtccgtgcct 300

acacatcccg atgatgtagg tgatgcttcc ctcaccatgg tcaaactccg ggctagtgat 360

gcaggcgtct accgatgtga tgtcatgtat gggattgaag acactcagga caccatgtca 420

ctggctgtgg atggtgttgt gtttcactac agggcagcca ccagcaggta cactctgaac 480

tttgccgctg ctcaacaggc ttgtttggat at 512

<210> 58
<211> 536
<212> DNA
<213> Mus musculus

<400> 58
acaaagccat gaagccggcg ctgctggaag tgatgaggat gaacagaatt tgccggatgg 60

tgctggccac ttgcttcgga tcctttatct tggtcatctt ctatttccaa agtatgttgc 120

acccagtcat gcggaggaac cccttcggtg tggacatctg ctgccggaag ggatcgagaa 180

gtcccctgca ggagctctac aatcccatcc agctggagct atccaacact gccatcctgc 240

accagatgag acgggaccag gtgacagaca cctgccgggc caacagtgcc atgagccgca 300

agcgcagggt gctgaccccc aacgacctga agcacctggt ggtggatgag gaccacgaac 360

tcatctactg ctatgtgccc aaggtagcgt gcaccaactg gaagaggctc atgatggtcc 420

tgagtggccg gggcaagtac agcgatccca tggagatccc agccaacgaa gcccacgtgt 480

cggccaacct gaagaccctt aaccagtaca gcatcccaga gatcaaccac cgcttg 536

<210> 59
<211> 660
<212> DNA
<213> Mus musculus

<400> 59
gccaggagtg ggcccagccc agggcagcat gaccaagccg cggctcttcc ggctgtggct 60

ggtactaggg tcggctctca tgatcctttt gatcattgta tattgggaca acgtgggaac 120

cgcccacttc tatctgcaca cgtctctctc caggccacac atcctagaac cccttcccac 180

ccagggattg gtggaggaga acgtgttcac atctgacgtg gatgagtttt tggatactct 240

ccttagttct gacgcgaagc acaacgacct ttccaggaga aaaactgagc agcccccggc 300

gcccgccccc agcaagccag tcttgagcca catggaggag aacgtgagag gctacgactg 360

gtccactcat gatgcccatc agaaccctga ccgggacagg cagcaggccg agaggaggag 420

cctgctgaga gacttctgtg ccaacgccag cctggcattc cccaccaagg accgctcttt 480

tgacgacatc cccaactacg aactgaacca cctgatcgtg gacgaccgcc acggggtcat 540

ctactgctac gtgcccaagg tggcctgcac caactggaag cgagtgatga tcgtgctgag 600

cgagagcctg ctggaccggg gcagccccta ccgagacccc ctggacatcc cccgggaaca 660

<210> 60
<211> 600
<212> DNA
<213> Mus musculus

<400> 60
atgactgtcg cctgccacgc gtgccaggca cagcatggga agacgctcct gttgcaggcg 60

gcccttgccg gtggtggcaa gtctgggtgc tgcactcctg ctcctgtgcg ccctgcgtcc 120

cgggtaacca caggaaagga tgcccaggac actgaatggc agggctcccc aaaagccctt 180

ttgggggttc cgacatttga aaataaagct ctgggctcca gctggttcgg tggagtgagg 240

aagagtcccc tacagctgtt gcgtgacctg gaccagatgt ttggcagctg tgagctatgg 300

gtagtcagtg gggagcggca gaaagtgggt ccacgctccg cgatggccga ggtgcaccag 360

cagcggcgtg agctgctgcg ccgggcctgc agccgccaca cgcgacgcca acgcctgctg 420

cagccggagg acctgcgtca cgtgctggtg gacgacgcgc accggctgct gtactgctac 480

gtgcctaagg tggcctgcac caactggaag cgtgtgatgc tggcgttgcg cggccgtggg 540

gatccaagcg caatccctgc gcacgaggcg catgcgcctg gcctgctgcc ctcgctggcc 600

<210> 61
<211> 600
<212> DNA
<213> Mus musculus

<400> 61
gcgccccctc tccccatgga gaaaggactc gctttgcctc aggatttccg ggaccttgta 60

cacagcctaa agattcgagg cagatacgtc ttgttcctgg catttgtggt catagttttt 120

atcttcattg aaaaggaaaa taaaatcata tccagggtct ccgacaagct gaagcagatc 180

cctcattttg tggcagatgc caacagcact gacccagccc tgctcttatc ggagaatgca 240

tctctcttgt ccctgagcga gttggattcc accttttccc atctgcggag ccgcctgcac 300

aacctgagcc tgcagctggg cgtggagcca gcaatggaga gccaggaggc tggggcagag 360

aagccatccc agcaggctgg agcagggacc cggcgccacg tgcttctcat ggccaccacc 420

cgcacgggtt cctcgttcgt gggcgagttc ttcaaccagc agggcaatat cttctacctc 480

ttcgagccac tgtggcacat cgagcgcacc gtgttcttcc agcagcgagg cgccagcgcg 540

gctggttcag ccttggtcta ccgtgatgtc ctcaagcagt tgttgctatg cgacctgtat 600

<210> 62
<211> 720

<212>  DNA
<213>  Mus musculus

<400>  62
tggggagagt gaggattcgg tgaactatga agggccggcg gcggcggcgc cgagagtatt      60

gcaagttcac gctgctcttg gcgctgtaca cgcttttgct acttcttgtc ccctctgtac     120

tggacagcca cagcgagcag gacaagggca ggaactgccc cggcctgcag cgcagcttgg     180

gtgtgtggag cctggaggcg gcggcggccg gggaacgtga gcagggcgct gaggtgcggt     240

ccctggccga aggaaacccg gatcgatccc ccgggtcccc cggcaacctc agcgccgtcg     300

gtgaggcggt gacccaggaa aagcaacaca tctatgtgca tgccacctgg cgcaccggct     360

cgtccttctt gggcgaactc ttcaaccagc acccggacgt tttctacttg tacgagccca     420

tgtggcatct gtggcaggca ctgtatccgg gcgacgcgga gagcctgcag ggcgcactaa     480

gagacatgct gcgctccctc ttccgctgtg atttctctgt gctgcgcctg tacgcgcagc     540

ctggggaccc tggggagcga gcaccggact cggccaacct caccacggcc atgcttttcc     600

gctggcggac caacaaggtc atctgctcgc cgcctctgtg ccccgccgcg ccccgggcac     660

gcgcggacgt gggactcgtc gaggacaaag cctgcgaaag tacctgcccg cccgtttcgc     720

<210>  63
<211>  600
<212>  DNA
<213>  Mus musculus

<400>  63
accccacaac tcggaacgat gcggctagcc tgcatgttct cgtccatcct gctgtttgga      60

gctgcgggcc tgctcctctt catcagcctc caggacccta tagagctcag cccccagcaa     120

gttccaggta taaagttcag catcaggccc cagcaacccc agcatgatag ccacttgagg     180

atatccacag aaaagggcac acgagattca cccagcgggt cgccaagagg cctccagctg     240

caagcgcctg accaacctcg acctcacccg aaggcagcgg gatctccttt gcgcctccgg     300

cagcgcaggc ggagactgct catcaaaaag atgccagccg cagggactaa ccaaggcaac     360

aactcgtccg aaacctttat ccagccgaga ccccgcacca tggacagtcg ttgggtcagc     420

ctgcaccaga cccaacagga gcgcaagcgt gtgatgcgag aagcctgcgc taaatacagg     480

gccagcagca gccgcagagc tgtcactccc cgccacgtct cccgcatctt cgtggaggac     540

cgccaccgtg tactgtactg tgaagtaccc aaggcaggct gctccaactg gaagagggtg     600

<210>  64
<211>  735
<212>  DNA
<213>  Mus musculus

<400>  64
atatagtatc taggatatat gtagaagaca aacacaaaat cctgtactgt gaagtaccaa      60

aagctggctg ctctaattgg aaaagaattc tgatggtcct aaatggattg gcttcctctg     120

catacaatat ctcccatgat actgtgcact atgggaagca tctgaaaaca ctggatagtt     180

ttgacttaaa aggagtacac atgcgtttga atacatatac caaagctgtg tttgttagag     240

atcccatgga aagattagtc tccgcattta gggataaatt tgagcatccc aatagttact 300

accatccggt gtttggaaag gcaattatca agaaatatcg accaaatgcc tctgcagaag 360

cattaaataa tggatctgga gtcaaattca aagaattcgc ctactatttg ctggatgctc 420

accgtccagt aggaatggat attcactggg aaagagtcag caaactgtgt tatccgtgtt 480

tgatcaacta tgactttgta gggaagtttg agaccttagg agaggatgcc aattactttc 540

tacagttgat tggtgctcca aaagagttga catttccaaa ctttaaggat aggcactcct 600

ctgatgaaag aaccaatgcc cacgtggtaa ggcagtattt aaaggacctg agcacagccg 660

aaagacagct catctatgac ttctatcact tggactattt gatgtttaat tacacaactc 720

cacatttgta atttg 735

<210> 65
<211> 574
<212> DNA
<213> Mus musculus

<400> 65
ccagaagcca agctcattgt tatgctcagg gatcctgtgg agaggttgta ctcagactat 60

ctctacttcg caagttccaa taaatctgca gatgacttcc acgagaaagt gacggaggct 120

ctgcagctgt ttgaaaattg catgctggat tattcactgc gcgcctgcgt ctacaacaac 180

accctgaaca atgccatgcc cgtgaggctc caggttggcc tgtatgctgt gtacctcctg 240

gattggctga ctgtcttcag taaggagcag ttcctcattc tccgtctgga agaccacgca 300

tccaatgtca agtataccat gcacaaggtc ttccagtttc taaacctggg gcctctgagt 360

gagaagcagg aagctctgat gaccaagagc cctgcatcca acacacggcg tcctgaggat 420

cgtagtctgg gacccatgtg gcccatcacc cagaagattc tgcgtgaatt ctatgggcct 480

ttcaacacaa ggctggcaca ggtcttggat gacgaagcat ttgcctggaa gacaacgtga 540

gagctgagtc ccttctgcag aagctgggcc cact 574

<210> 66
<211> 1430
<212> DNA
<213> Mus sp.

<400> 66
tgcgacttca tcaagtcgga gccaaagaaa tcttgaaaga tgggaatgct agcaagagtt 60

gccctgggac ttatcatcat tgatgctgta ttggctgcac caactacaga gctctttaac 120

tatgactcag aagtgtatga tgccatcttg gaggacacag ggacctttta taactatgaa 180

cacattcctg acaaccatgt agagaacgag aaagtgtctg agaggctttc tgggaacaga 240

gagctcctga ccccaggccc acagctaggg gacaaccagg atgaagacaa ggatgaagaa 300

tctactccca ggctgatcga tggctcttcc ccacaggagc cagaattccc ggggcttctg 360

ggtccacaca ccaacgaaga ctttccaact tgtctcctgt gtacttgtat aagtaccact 420

gtatactgtg atgaccatga actggacgct attcctccac tgcccaagaa gactacatat 480

ttctattcac gttttaacag aattaaaaag atcaacaaaa atgactttgc aagcttaaat 540

gatttaaaaa ggatcgatct gacatcaaat ttaatatcgg agattgatga agatgcattc 600

cgaaagctgc ctcacctcca agagctggtt ctgcgtgaca ataagataaa gcagcttcca 660

gaattgccaa acactttgac atttattgat atcagcaaca atagactggg aagaaaaggc 720

attaaacaag aagcttttaa agatatgtat gatctccacc atctctatat caccgataac 780

agcttggacc acatccctct accactccca gaaagtctgc gggctcttca cctccagaat 840

aatgacattc tggagatgca tgaagatact ttctgcaatg ttaaaaattt aacttatgtt 900

cgtaaggcat tagaggatat tcgactggat ggaaacccta tcaacctcag cagaactcct 960

caagcctaca tgtgtctacc tcgtttgccc attgggagtt ttatctaatg ttaggatgct 1020

aggcaaatat cagaattgtg atgctttctg tagccaagag aagcatatat ctcttcagaa 1080

gcaaactcag tatatttgag atgcgtttaa aacatgtggc acaatgatat aaacaataag 1140

ctaaaggtgt tgagataaaa taagagttca ataatttaat taaacatgat taggtataaa 1200

ttaacaaaat atatagacag taagttaaat aaaatgtatg ccaactagtc atcaaatctc 1260

atcaagaatt gtaggtgttc atgtcaagtt aaatatgtaa gaaataaaat attggtaatg 1320

aatactttag atacgttgct aagatgtaga cattttaatt aaacttctct agtctttatt 1380

ttcactcatg tatgtttccc acctgattca gttaagagca gaaaaaaata 1430

<210> 67
<211> 20
<212> PRT
<213> Mus musculus

<400> 67

Asp Arg Ala Arg Ser Cys Ala Ile Val Glu Asp Asp Gly Leu Gln Ser
1               5                   10                  15

Ala Phe Thr Ala
            20

<210> 68
<211> 9120
<212> DNA
<213> Mus musculus

<400> 68
tgtatatctg agtatctgtg tatgtgcata tatgtgtgtc tatgtgtatg tgtgggtttg 60

tgcatgtgtg tctgtgtgta catgtctgtg tgtctctgtg tgtgtctgtg tatgtgtgtt 120

tacatgtgtg tgtgtgtttc tgtgtggacg catgtgtatg tgtctccgtg tgcctgcatg 180

tgtgtttgtg tatgtgtgta ggcatgagta catcatggtg tacatgtaga aattgagaga 240

gactcttggg tgtggttctc atctcccacc atatctgaag taaatgtctt attcacacca 300

ctgtgcacat cagaaagcta gccttgacct ccaaggaact ctgtctctgt ctcctgtctt 360

attgtaggta cctggggttt ataagcctat gctgctgcat atggcttcac ttgggttctg 420

gggatttgaa cttaggtcct cacagtttta gggcaagcat tttattcact gaacaatctc 480

ccatgatccc tagagtttat tgtaggcaac ttaaccttta gtcgtcattg agatttagca 540
ttgatttcag ttccagttgt attctgatgt tgagcgctac agcagaggta cccagggact 600
gctgattttc tgtggccatg aatcctctag tgattaataa atttcatcat aatgttctgt 660
ttatttctaa atgtccagac aactctaatt tcaaattaat ctcatcctaa acagcacagg 720
ataaaagtta cagattgtcc tatcttcatt catgtgatgc ctcaaatcag atgtttaatc 780
ctttagtcag ttatttgtga aacggaacct aattacacac actcgcatgc gcgtgcgcgc 840
acacacacac acacacacac acacacacac acacacacac acttgtagat ttgcccacac 900
agctgcttaa gcaatttgga gcaaatgtag aaatattatg aaatttcaca taaaaatggg 960
ttttcctctg tcttcttgag aaatgtgtga gtttcaatat gacagtaatc ttctggtgtt 1020
ggactataac cacttgaacc cctgaggtac taagtgtgta cctactacgt gcctggattc 1080
catcatttcc ctacttatat aagcttctcc atcctgtaga tcagcactgt gtaatggagt 1140
aacacaagtc attgaatcat gcaatcattt aatcaattta attggcactt ctgataagag 1200
aagtgggcac atatttagtt gtgtttgcag cctgtgctat gtcatttttc tctatatata 1260
ctttaagatt tttttctttt ttccatttac gggggggggg ttattccatt taatttacat 1320
gcttaatgac atataatttg catatcaggt gttcctttct ttagagttaa cttcttttag 1380
gaccattgtt tataatggat gtatatcatc cctaaattaa atcagccatt gtctacttaa 1440
cacaccagtt tctctgatgt ccctctggct cttcctcctc tgaggttcta gttataggaa 1500
agtctggcat tttgatgttt ttccatggct tcgtgggctc tgatcatcca cttacagtgt 1560
actttcatct cttgtctaca aactgcatat catctattga tctgcgttca aggctactac 1620
attttcatgc aattccactt tgctgttaaa ccttttctat gatccgtttt tatacttata 1680
aacaatccaa gtgttgtact ctttgggtat aaatgttctg tatttcacag gtcttaactt 1740
cttttcagac tatacaaaga aagattttct atttttttta tttgttgtat gcattttatg 1800
catttagcac tgggatttcc ataacaggca gttaagatca ttgaatacat actttgtgtg 1860
gtaaatataa tacggcttat ttgattctcc cccttaaaat gctcccatct tctcagtcat 1920
gtcaggttgc tatggagtcc accctgagca ccatgagtgt gatgctgtac agacactcct 1980
gatatgcctt ctgttatttg aactccacct gcaagttttc attcaagcat agctcctaat 2040
cccattcagt gtgcactttt cttaattgat ttgctggtag agagctatat gtaagcatgg 2100
cttagcatca aattgaactt tctcctctcc ctagcttctt tccagtatat tcttcctcac 2160
cttcatcaga ttttgaggtc ttgagatcta aaccttcatg ttacaatttg cagagatacc 2220
aggatctcta tcagacttga tgtgtctcca tgtaattcta gtatcagcct ttcataagat 2280
aattgcaaaa tatagaaccc cccctctctc cttttgacta ttacagcttt gtttctctcc 2340
caagttaata cagatgtatg caaatcaact aagaaaagca aacactgaat gggataagga 2400
gctatactca aaagaaagct tgcagttgga gttcaaataa cacaagtgga accaagagta 2460
tctgcacagc atcagactca ctgtgctcag gatggaatcc atagcaacct cccataactg 2520

agaggatgtg agcccctttt aaagggaaaa tcaaataagc agtattatat taaccacatg 2580
atgtgtatat tcaatgtaga ttgggctgca ccgaagttat gattttagta atgagtattt 2640
ctccttgggt tttcttctgg accaccctgg ttttcctttt aaaaatagta aaatagcagc 2700
aatgactgct tctcttaaaa tctgaacaac acagagcact tcagcatcct ttcaacctat 2760
ctaaatccat tgtagacctg cactgacttg ctgtgagcta ttgtgtcaca actattattg 2820
tagctaaaca ccttgaattt tcaaagctgc tctggactgt gtcttctttt agaattaaac 2880
ttgctaaaga tattggactc tgagatgctt attcaccact ttctataaaa gagtaaggat 2940
gacaatgctt tgagtagaag aagattgcat acgagggtac cagcatgtca ttatgtcccc 3000
agcagtcagt aaagtaagga tccagtgatg gtagagttat tatgtccatt atggaactaa 3060
tgtaaatgtt tatcaatgac tagaatggct gtaatcacca atccttagac aagtgtgagt 3120
aacatcatgc tcttttctta taataataga tagtcacaca caccatggtc ttaaacatta 3180
cattttgatt tttctctttg tgtctgtctt ttcgtacctc tactccactt acctgagcct 3240
tcagctaggg ctcctccaag acggcagtca cagtactagt gaggataatt ctcatatgga 3300
gacctgaagc agaataacct tcttccaagg aaaccaaaat cctagtcaac atcttctcca 3360
ctctttggtt atttattttt atttctattt ctattttgcc atttaagaat ttttaaactc 3420
ttttctgttt tcttatatat attctttttt ttacaacttt tgatattctt tatcaaatga 3480
tttatcccat tgcctttcct cctgcagcta tctgtgagtt ttagcatgga tatttagaat 3540
acaattggaa attatgaagg tcgaataaaa gttctctaag agccaggacc tcactagcct 3600
agttagttgc ttagtttcta atataaggca tggtttatct cctactgtat ggcccttaaa 3660
tccactagac atctgttggc ttccaacagc atacaattgc tgctattgta tgtttaggta 3720
tatattttta tgaaacacta tattctgatt ggtgttcatt cttcctatga aggggaagat 3780
tttattgcct gctcaaattc aagtcagtcc tttttgagta gacaaaggtt gtccaatctt 3840
gctaaaccct tttctttttt gcttggggaa tttctttagg gaaaattctc aaccatgaac 3900
caagcctaga gattgataat agctgaaggc agagtagcca gtcttagtaa gggatgagag 3960
aaagactgac caaagactga ataggaaatg gttgatagca ttcattggga ctagacaata 4020
aagttagcta ccccaacatg ggcaaatgga gaaaatgggg agagtcatat ggatatgtag 4080
ggaggagaag aaaggaaggg gtgagtttga agtatggaag agcctttaaa gcatttgtaa 4140
gatttgtaag aaaaagcttt tgagagacag agtattacct gccttcaaat cccttcaatt 4200
actgtcccta caagacatac ctgtgctcag ctgctcttgg accacaaggg ggcctaagag 4260
catgtttgtg tccctgtctt tatacaggct tctcatctgt atttctcaaa cgtaaataca 4320
aatgggggag ataacaaatg atatttctaa gtggtacaca ggtatgagaa ctgcacacaa 4380
tctatttata tgtataatgg ccagagctct cagcagggaa cagaggaagc ctaggtaact 4440
cctgctccag agcatgtctg tatggagacc tcagttcagc aactatgaaa tctgtgttca 4500
aagggtcatc actgggaaca gtttctggaa tagcagacat attcattctc tctctctctc 4560
tctctctctc tctctctctc tctctctctc tctctctctc tctctgtctg tctctgtctc 4620

tctctgtctg tctgtctgtc tctcccttct gtcaggggta tgcatacatg ggctgggaat 4680
tctatcttcc ctgcctgctg tctttggtgt ctccctaatg tggtgccatg tttacttaac 4740
ctgtctgaaa tggaggataa cctctggtct gataactgtc aagttactga aatgtcaggg 4800
aattgtgaat gaacccctct gaatgctggg cagatgtttt gagcacgtgg gtatccatag 4860
attttttttt cagcaggatt tccttcttta atatattgtg ggaacgaaac attgaagttt 4920
ccaacactaa gaaaagcaat gctctgggtt tgcaaagaag tctccaaagc tgtgggagag 4980
gcaatggctt tgcctcacaa aggtgatcct gatttggatt gggtgttcct cagtctccct 5040
catcctttgt ttgtggacca aacctctgat caaataatca gtatacagac aagtactgac 5100
tgactcctgt cactgtttcc catgtaaatt ctcaaccatg tgtgtcagaa ttgtttgagt 5160
catcaaagtt ccgtgatgct caggatggtg aataccagaa actgaagtct gaaatccaat 5220
ttggcctttc tcgggcactg taaactgagt gcaaatgtgc ccagttccct cgtggcccat 5280
ttatagcaca ataacacagt gcttgcttac aaaatctagt cccgattaaa gtatttattt 5340
aaagttgctt tagacaaata tatttctaaa tggaaggatg aaacagaaag aatattccaa 5400
ctgtacagtc tatattttgt ttctaaaaat ttttattaga attctgactt ttagaaaaca 5460
aatagatatt caaaaactac ttctttaatt aaataataac atcattatag acattctcca 5520
gccatttaga agtagcatga ttacaaagca ttcatgttcc ctttgttttt tgaaacagaa 5580
gtatttatat gacaaataaa aagcaaaaat ctcatatatg tgatagctat agatgcattg 5640
ataggaatta aatttaatca aaaagaactt ttaatggcaa taatgtggct actaacttca 5700
ttgtttgtaa taatgctgca aatgtttaat gaaaactgtt actaacaata caggagaaga 5760
ttacagatat gattaggtcg tgttgataga aatggaaaga gaaaggtcac tcactacctc 5820
agccacagaa cttgaggaat gtgtgagggt ttttgaggaa aaaagagaac aatgtaacat 5880
cacagcaaca tcttaatctg aaagtagacg acaaaggttt cagagtaggg gatcagaaat 5940
ttaagtatgc agagaagcaa ggcatatacg gcttagacaa tgtatgaaga acagcagcag 6000
acagcaagtg aaagaaatca cagtccacat ggaaatcaca cagaccacag tgtgcacaca 6060
gagacagaaa tcagcaaggg acacacggct tccagttatg aaagttagac agcaaaattt 6120
aagttgtccc acctaaaaca tgatgtatcg tctctcaaat ggggaagggg aaggagcagc 6180
aaacatctgg atatgtctaa aacctcacag agaacatctg ggatctttgc atttctccct 6240
ctgactttct caatatgtta gctacttcta gatagtttca ataatggtct ctaattgact 6300
atggagagac aggtctcatg tatgtgaaca tccttaacca ggagatggaa tgtgtttcct 6360
tgtcactcat tttctaagga agtgaattcc ttctgcattg cccaggattg gatgttacgt 6420
gaaactaaaa atcattgatt aaaaagaaat gtcattacca cagttgggtt gaatcaatca 6480
agacttatcc tagttacagg catcacctcc ccttaagcac aaatactgag aacagctaag 6540
ctcactttga aatctgggat gaggaggctt taggtcaagc atctgacaat gtgtgtgtgt 6600
atgtgtgtgt gtgtgtgtgt gtgtagtatg attatatgta tatatgtaga tgtgtgtata 6660

tatttatatg tatatatgta tatgtgagta tgtatatatg tatatgtgtg tatgtgtatg 6720
agtgtgtgta tgtatgacta tgagtgtgta tgtattgtgc aagtgtgtgt atatatgtgt 6780
gtatatgttt atgtatatgt gtattgtatg ggtgtgaatg tattatatat gtgtgtaggt 6840
acatatgtgt atatgtttat gcgtgtatat gtgtatgcat tatatgtatg tgtgtatgtg 6900
tgtatgtttt catgtgtatg tagcatatgt atatgcatgt gtgtgtctgt gtgtgtaagc 6960
atgtatttac taagaaaaaa tcagtttctc tagctaatga gtcattgaag tgaagaaacc 7020
ttacgctatc acattactat gtttccagca taaattgcaa tatcagtaag acattcccca 7080
tcataactta atggacactt agattaggtc atgcagcagt aaattatgaa agcgtctatg 7140
ctgagctgag gtgggaaatc gtctttcctg cctccttcct cctgttcttt ctcctaaagg 7200
ccctcatcct ctttctcccc ttcctttctt ttaaaatatt ctatcattga tccaagctaa 7260
ctaacccttc ccttgctcac atgtgacctg cccagaggaa tctatttata gtcaacaata 7320
acaggccaag gacatgcgaa caaatgactt tacttagccc cacttggtga atctattggg 7380
tgaataggta aggggttatt tataggagct tgactgactg gcacagctgt atctctgaaa 7440
agtcccccaa agcatcatgg gtgggaacta aggaaagccg gaaatgctgg agctggaatc 7500
ccaacctgca gtggctacat gatgaatgtg tctcctttcc tactaaatat ttaagactct 7560
gggcaggctg gttagtcttg taaaagtctt gtgggtttct ggagtcctgg gaggctcact 7620
gtccctgcag aagcttgtta ttattgtata tgaaagaaag tgctcaacac tgagtcacaa 7680
aagaagcccc cgagaatcgg tggcagaggt ggaaatagaa gaatccgatg cagttcccga 7740
ggcaagagtt gatcctctcc atggtgacaa gaaaacaaga gccacagaag cctgaaggct 7800
aagactatga cctgtgaggt tctcctctcc taggacttga atgcacttaa ctctgagggc 7860
tagcagggca gagattcagg agcgttctca gaggttgtcc cttctccttg tatgattcta 7920
ggagaagctc agtgcatagg cagaagttgc aaactctaac acattaaccc acatcctgaa 7980
ttgttttata tcaattgttt tataactaca tgttttaact tttgttttgg tttttgaatt 8040
ttgtttgttt gtttgttttc tatttttcat atgtgtgcat gtgagccaga agacaggcta 8100
agggactctg ttctctcttt ccagcatgtg ggtttcaggg atcaaagtta ggtcccaagg 8160
cttcactggg caacgggacc cttagcctct gagtcatctt cttaatcttt ttctttgaaa 8220
ctttttcatt acattgtgtc agtgcacaca cacacacaca cacacatgca tatacatcca 8280
catgcatata catccacatg ccatatgtca tggcacatgt gtggaaagaa atgggtgcct 8340
tgaggaaatc agttttcttc ttccatcacg tggatcttat gacttcaggt aacccatctc 8400
tgctccccaa gcatggctac aagcatttac ctgccaagtc atctccctgg ctcccacatt 8460
aatttataat aatacaccct ttcagcctag agagatgact cagtggttaa gaactctggc 8520
tgtgcttcag aaggttctga gttcaattcc cagcagctat aaggtggctc acaactatct 8580
ctaatgttgt tttctgtcct cttctggcat gcacttgtat gtgcagatag agtacttata 8640
tgcataaata aatataaata cattaataat cttttaaaaa gaaggaaaga ttaaaagaac 8700
aatacaccct ttcttttaag taagaaaaca tgcttagtat atagaataaa tgctggtgtt 8760

gaaaggtgaa attcagaaga aattataggt tggaaatatt tgaactcttt tgtgtcaaac 8820
caaatggtaa agatgatgtg ataataaggt aaggttttct tcctacacat atacttgttt 8880
aataaccagt ataagactat gagtgaacat aactaaaaat acatgaaaaa aaactatttt 8940
aaaaaaacat cactttcaga tgactctggt gaaactcact gataaccagc aatctctttg 9000
atagaccaga gtgtccagtc tgaagttaat tcttttgctt tattctcaaa caatgttgca 9060
gaagtatgaa ccatagtatc aagatacact gtccacatat gcacctggcc tgacttgtgt 9120

## Description

### Technical Field

The present invention relates to agents for suppressing hepatic fibrosis, methods for suppressing hepatic fibrosis, and methods for treating or preventing hepatic fibrotic diseases using the above methods.

### Background Art

Chronic fibrotic liver disease is a general name for the terminal stage of chronic inflammatory conditions that can be developed in the liver, and is accompanied by excessive tissue fibrosis. Chronic fibrotic liver disease is a group of severe diseases which often lead to death through progressive liver dysfunction. There has been no radical therapeutic method for the disease. Currently, liver transplantation therapy is used against cirrhosis; however, such therapy significantly impairs patients' quality of life (QOL), and many cases show recurrence of fibrosis in the transplanted organ. Recently, TGF-β inhibitors, angiotensin inhibitors, and such have been expected as novel therapeutic agents for the fibrotic disease; however, none of these has an established efficacy. Thus, development of new agents that can improve patients' QOL, and effectively inhibit or retard the progress of hepatic fibrosis is in strong demand.

Currently, the following are expected as candidate therapeutic agents for fibrotic liver diseases: adrenal cortical steroids, colchicine, IL-10, and the like, which suppress chronic inflammation; TGF-β inhibitors, HGF (suppressing the activity of TGF-β), endothelin inhibitors, angiotensin inhibitors, and such, which activate fibroblasts; matrix metalloproteinase (MMP) which removes collagen, and others. However, there has been no established therapeutic method using any of these (Non-Patent Document 1 [the most recent review], and Non-Patent Documents 2 and 3).

Proteoglycan (PG) is a molecule with a structure of one or more glycosaminoglycan (GAG) chains covalently linked to a protein called the core protein. The specific sugar chain structure of GAG chains is believed to be responsible for various functions of PG. Based on the type of GAG chain, PGs are roughly divided into four groups: chondroitin sulfate proteoglycan (CSPG), dermatan sulfate proteoglycan (DSPG), heparan sulfate proteoglycan (HSPG), and keratan sulfate proteoglycan (KSPG) (Non-Patent Documents 4 and 5 [reviews]). Among others, HSPG has been extensively studied because its function is greatly modified through binding with various cytokines and chemokines.

Meanwhile, chondroitin sulfate proteoglycan (CSPG) is an essential molecule in the fetal period and is abundant in organs, but it is known to decrease along with birth, growth, and aging. Surprisingly, because of the diversity of its characteristics, *the in vivo* functions of CSPG remain to be clarified. Meanwhile, there are reports describing that CSPG is increased in diseased organs of cirrhosis, pulmonary fibrosis or such (Non-Patent Documents 6 and 7). However, the significance of CSPG increase in such diseases still remains unknown. In recent years, the finding that mice that are genetically deficient in CSPG show embryonic lethality or organogenesis failure has increased the recognition that CSPG is an essential molecule in the body.

Chondroitinase is known as a bacterial enzyme having an activity of degrading chondroitin sulfates (chondroitin sulfate proteoglycans). Known therapeutic agents that use chondroitinase include therapeutic agents for disk herniation that use chondroitinase ABC (Patent Document 1), wound healing-enhancing agents that use chondroitinase AC or chondroitinase B (Patent Document 2), and therapeutic agents for scleroderma, psoriasis, keloid or pulmonary fibrosis that use chondroitinase AC or chondroitinase B (Patent Document 3).
Patent Document 1: United States Patent No. 6,001,630 specification
Patent Document 2: United States Patent No. 5,997,863 specification
Patent Document 3: International Patent Application Publication WO 2001/039795 pamphlet
Non-Patent Document 1: Bataller R & Brenner DA, J. Clin. Invest. (2005) 115:209-218
Non-Patent Document 2: Iredale JP, BMJ (2004) 327:143-147
Non-Patent Document 3: Bissell DM, Exp. Mol. Med. (2001) 33:179-190
Non-Patent Document 4: Lin X, Development (2004) 131:6009-6021
Non-Patent Document 5: Hacker U et al., Nature Rev. Cell Biol. (2005) 6:530-541
Non-Patent Document 6: Kovalski I et al., Orv Hetil. (1993) 134:2019-2026
Non-Patent Document 7: Westergren-Thorsson G et al., J Clin Invest. (1993) 92:632-637

### Disclosure of the Invention

An objective of the present invention is to provide agents that are capable of suppressing liver tissue fibrosis and useful in treating or preventing fibrotic liver diseases, therapeutic agents for fibrotic liver diseases comprising the agents as an active ingredient, and methods of screening for hepatic fibrosis-suppressing agents.

The present inventors conducted dedicated studies to develop such agents, and contemplated that excess accumulation of chondroitin sulfate proteoglycans (CSPGs) might enhance liver tissue fibrosis, although it was not considered as a cause of fibrotic liver diseases. The present inventors carried out research based on this assumption, and as a result discovered that chondroitinase ABC, a chondroitin sulfate proteoglycan-degrading enzyme, could efficiently degrade CSPGs accumulated in fibrotic livers and that fibrosis of liver tissues could be markedly suppressed by administering chondroitinase ABC *in vivo.* Furthermore, a similar therapeutic effect could be obtained by administering a recombinant peptide of a protein called ADAMTS-4 (A disintegrin and metalloproteinase with thrombospondin motifs) which has an activity of cleaving the core protein of versican (a chondroitin sulfate proteoglycan). Specifically, the present inventors demonstrated hepatic fibrosis could be suppressed by inhibiting the accumulation or biosynthesis of chondroitin sulfate proteoglycans. Accordingly, the inventors completed the present invention.

The present invention relates to hepatic fibrosis-suppressing agents, therapeutic agents for fibrotic liver diseases comprising the agents as an active ingredient, methods of screening for hepatic fibrosis-suppressing agents, and such. More specifically, the present invention provides:
[1] a hepatic fibrosis-suppressing agent comprising as an active ingredient a substance that inhibits the production or accumulation of a chondroitin sulfate proteoglycan;
[2] the agent of [1], wherein the substance has an activity of promoting the degradation of a chondroitin sulfate proteoglycan;
[3] the agent of [1], wherein the substance has an activity of inhibiting the synthesis of a chondroitin sulfate proteoglycan;
[4] the agent of [1], wherein the substance has an activity of desulfating a chondroitin sulfate proteoglycan;
[5] the agent of [1], wherein the substance has an activity of inhibiting the sulfation of a chondroitin sulfate proteoglycan;
[6] the agent of any one of [1] to [5], wherein the production or accumulation of a chondroitin sulfate proteoglycan is inhibited in liver;
[7] the agent of any one of [1] to [6], which is used for treating or preventing a fibrotic liver disease;
[8] the agent of [7], wherein the fibrotic liver disease is a chronic liver disease;
[9] the agent of [7], wherein the fibrotic liver disease is chronic hepatitis, liver fibrosis, cirrhosis, liver failure, or hepatocarcinoma;
[10] a method of screening for a hepatic fibrosis-suppressing agent, which comprises selecting from a test sample a substance with an activity of inhibiting the production or accumulation of a chondroitin sulfate proteoglycan;
[11] the screening method of [10], which comprises the step of selecting a substance with the activity of any one of
   (a) promoting the degradation of a chondroitin sulfate proteoglycan,
   (b) inhibiting the synthesis of a chondroitin sulfate proteoglycan,
   (c) desulfating a chondroitin sulfate proteoglycan, and
   (d) inhibiting the sulfation of a chondroitin sulfate proteoglycan; and
[12] the screening method of [10] or [11], wherein the hepatic fibrosis-suppressing agent is used for treating or preventing a fibrotic liver disease.
   The present invention also includes the followings:
[13] a hepatic fibrosis-suppressing agent comprising chondroitinase ABC as an active ingredient;
[14] the hepatic fibrosis-suppressing agent of [13], which is used to treat or prevent a chronic liver disease;
[15] the hepatic fibrosis-suppressing agent of [14], in which the chronic liver disease is cirrhosis; and
[16] the hepatic fibrosis-suppressing agent of any one of [13] to [15], in which chondroitinase ABC is derived from *Proteus vulgaris.*
   Further, the present invention relates to the followings:
[17] use of the agent of any one of [1] to [9] for producing a hepatic fibrosis-suppressing agent;
[18] a method of treating a hepatic fibrosis which comprises the step of administering the agent of any one of [1] to [9] to a subject (such as patients); and
[19] a composition which comprises the agent of any one of [1] to [9] and a pharmaceutically acceptable carrier.

The present invention has demonstrated that the production and accumulation of chondroitin sulfate proteoglycans is involved in the development of cirrhosis. Inhibiting the production or accumulation of chondroitin sulfate proteoglycans was shown to suppress the onset of hepatic fibrosis. Thus, therapeutic agents for fibrotic liver diseases can be provided based on this new concept. In particular, hepatic fibrosis is closely involved in chronic liver diseases such as chronic hepatitis, liver fibrosis, cirrhosis, liver failure, and hepatocarcinoma. Since the number of patients with these diseases is increasing in today's society, the new-concept therapeutic agents have great medical and industrial significances.

### Brief Description of the Drawings

Fig. 1 presents photographs showing results of CSPG, C4SPG, C6SPG, and HSPG (brown signals) detected in carbon tetrachloride-induced cirrhosis model mice. The magnification was 100 fold. Names in the parentheses represent antibody clones used in the detection.
Fig. 2 presents photographs showing results of CSPG (brown signals) detected in carbon tetrachloride-induced cirrhotic liver after treatment with PBS (chondroitinase (-)), Chase AC, Chase B, or Chase ABC. The magnification was 200 fold.
Fig. 3 presents photographs showing the effect of Chase ABC administration on reducing the level of type III collagen (red signals) in carbon tetrachloride-induced cirrhosis model mice. The magnification was 100 fold.
Fig. 4 is a graph showing fibrosis scores in carbon tetrachloride-induced cirrhosis model mice after administration of Chase ABC, Chase AC, or Chase B. *p<0.05, **p<0.01 (t test).
Fig. 5 presents photographs showing the effect of Chase ABC administration on reducing the level of type I collagen (red signals) in carbon tetrachloride-induced cirrhosis model mice. The magnification was 100 fold.
Fig. 6 is a graph showing the type III collagen-positive area (%) in carbon tetrachloride-induced cirrhosis model mice after administration of Chase ABC, Chase AC, or Chase B. *p<0.05, **p<0.01 (t test).
Fig. 7 presents photographs showing results of CSPG, C4SPG, and C6SPG (brown signals) detected in carbon tetrachloride-induced cirrhosis model mice after Chase ABC administration. The magnification was 200 fold.
Fig. 8 presents photographs showing the distribution of fibroblasts (brown signals) and changes in the distribution after Chase ABC administration in carbon tetrachloride-induced cirrhosis model mice. In the upper panels, the magnification was 100 fold. In the bottom panels, the magnification was 200 fold.
Fig. 9 presents photographs showing the distribution of macrophages (brown signals) and changes in the distribution after Chase ABC administration in carbon tetrachloride-induced cirrhosis model mice. The magnification was 200 fold.
Fig. 10 presents photographs showing the suppression of fibroblast infiltration by administering a functional ADAMTS-4 peptide. Images of an ER-TR7-staining (brown) pattern in liver tissues of the cirrhosis model are shown (upper, 100 fold; bottom, 400 fold). The images demonstrate that administration of the functional ADAMTS-4 peptide not only suppressed cellular infiltration but also suppressed pseudolobule formation.
Fig. 11 presents photographs showing the suppression of type III collagen proliferation by administering a functional ADAMTS-4 peptide. Image of a staining pattern of type III collagen (brown) in liver tissues of the cirrhosis model is shown (100 fold). The image demonstrates that administration of the functional ADAMTS-4 peptide suppressed pseudolobule formation.
Fig. 12 is a graph showing the suppression of fibrosis by administering a functional ADAMTS-4 peptide. This graph was obtained by numerically evaluating the staining pattern of type III collagen as a fibrosis score. Administration of the functional ADAMTS-4 peptide was demonstrated to significantly suppress hepatic fibrosis.

### Best Mode for Carrying Out the Invention

The present invention is further specifically described below:
Pathological conditions associated with cirrhosis, a representative chronic liver disease, include fibrosis of liver. The present inventors focused on the functions of chondroitin sulfate proteoglycans to establish that the improvement of fibrotic conditions in liver is an effective therapeutic method for cirrhosis. The inventors then suppressed the accumulation of chondroitin sulfate proteoglycans in mouse models for cirrhosis, and closely analyzed this condition to reveal that the accumulation of chondroitin sulfate proteoglycans was ameliorated in many cells, and pathological condition of cirrhosis was also improved as compared with that in the liver of wild-type mice. Specifically, the inventors discovered that inhibiting the production or accumulation of chondroitin sulfate proteoglycans facilitated the amelioration of abnormal accumulations of chondroitin sulfate proteoglycans in a liver, a factor deeply involved in cirrhosis, and thus leading to the improvement of hepatic fibrosis.

The present invention relates to hepatic fibrosis-suppressing agents, which comprise substances that inhibit the production or accumulation of chondroitin sulfate proteoglycans as active ingredients.

In the present invention, "chondroitin sulfate proteoglycans" are a type of proteoglycan and collectively refer to compounds in which proteins (core proteins) are covalently linked to chondroitin sulfate/dermatan sulfate, which are representative sulfated mucopolysaccharides. In the present invention, preferable "chondroitin sulfate proteoglycans" are human chondroitin sulfate proteoglycans. The species from which the proteoglycans are derived are not particularly limited. Proteins of nonhuman organisms (homologues, orthologues, and such) that are equivalent to the chondroitin sulfate proteoglycans are also included in the "chondroitin sulfate proteoglycans" of the present invention. For example, the present invention can be conducted as long as the species has a protein corresponding to a human chondroitin sulfate proteoglycan and equivalent to a human chondroitin sulfate proteoglycan. Furthermore, the chondroitin sulfate proteoglycans in the present invention also include so-called part-time proteoglycans, which are temporarily linked with glycosaminoglycan (GAG) chains to become proteoglycans upon inflammation or the like.

Examples of the chondroitin sulfate proteoglycans described below are aggrecan, versican, neurocan, brevican, β-glycan, decorin, biglycan, fibromodulin, and PG-Lb. However, the chondroitin sulfate proteoglycans in the present invention are not limited to these examples, and may be any substances with a chondroitin sulfate proteoglycan activity. Herein, chondroitin sulfate proteoglycan activities include, for example, cell adhesion ability and cell growth promotion. Those skilled in the art can assay chondroitin sulfate proteoglycan activities by assaying cell division and growth of tumor cells (for example, Caco-2 and HT-29 cells) in the presence of a protein containing a partial amino acid sequence of a chondroitin sulfate proteoglycan, or a protein with high homology to such a partial amino acid (typically 70% homology or higher, preferably 80% or higher, more preferably 90% or higher, and most preferably 95% or higher). Proteins with the effect of promoting cell division and growth can be evaluated as proteins with a chondroitin sulfate proteoglycan activity (Int. J. Exp. Pathol. 2005 Aug, 86(4), 219-29; and Histochem. Cell Biol. 2005 Aug, 124(2), 139-49). High homology means 50% or higher homology, preferably 70% or higher homology, more preferably 80% or higher homology, and still more preferably 90% or higher homology (for example, 95% or higher homology, or 96%, 97%, 98%, 99% or higher homology). Such homology can be determined using the mBLAST algorithm (Altschul et al., Proc. Natl. Acad. Sci. USA 1990, 87, 2264-8; Karlin and Altschul, Proc. Natl. Acad. Sci. USA 1993, 90, 5873-7).

Herein, "hepatic fibrosis" refers to fibrotic conditions in liver tissues. Such conditions include but are not limited to, for example, accumulation of extracellular matrix such as collagen and activation of fibroblasts in liver tissues.

Herein, "inhibition of production or accumulation" of chondroitin sulfate proteoglycans includes, for example, "promotion of degradation", "inhibition of synthesis", "desulfation", and "inhibition of sulfation" of chondroitin sulfate proteoglycans; however, "inhibition of production or accumulation" is not limited thereto, and it refers to a reduction or loss of the amount, function or activity of a chondroitin sulfate proteoglycan, as compared to a comparison subject. Herein, "substances that inhibit the production or accumulation" of chondroitin sulfate proteoglycans are not particularly limited. Such substances are preferably "substances with the effect of promoting the degradation of chondroitin sulfate proteoglycans", "substances with the effect of inhibiting the synthesis of chondroitin sulfate proteoglycans", "substances with the effect of desulfating chondroitin sulfate proteoglycans", or "substances with the effect of inhibiting the sulfation of chondroitin sulfate proteoglycans".

"Promotion of degradation" of chondroitin sulfate proteoglycans includes, for example, inhibition of the expression of core proteins of chondroitin sulfate proteoglycans, and a reduction in the abundance of the core proteins. Herein, "core proteins of chondroitin sulfate proteoglycans" include, for example, aggrecan, versican, neurocan, and brevican for matrix-type chondroitin sulfate proteoglycans; and β-glycan, decorin, biglycan, fibromodulin, and PG-Lb for membrane chondroitin sulfate proteoglycans. Those described above are all examples, but the core proteins are not limited to these and a wide variety of proteins may serve as chondroitin sulfate proteoglycan cores.

"Expression" includes "transcription" from genes, "translation" into polypeptides, and "suppression of degradation" of proteins. The "expression of core proteins of chondroitin sulfate proteoglycans" refers to transcription and translation of the genes encoding core proteins of chondroitin sulfate proteoglycans, or production of core proteins of chondroitin sulfate proteoglycans through transcription and translation. Furthermore, the "function of core proteins of chondroitin sulfate proteoglycans" includes, for example, their function in binding to chondroitin sulfate, and in binding with other cellular components. Those skilled in the art can appropriately evaluate (measure) the various above-mentioned functions using general methods. Specifically, the evaluation can be performed using the methods described in the Examples herein below, or using the same methods with appropriate modifications.

The "promotion of degradation" of chondroitin sulfate proteoglycans may also be an increase in the expression of enzymes that cleave or degrade chondroitin sulfate proteoglycans, or of enzymes involved in the cleavage or degradation of proteoglycans. Such enzymes include, for example, metalloproteinases (for example, ADAMTS-1, ADAMTS-4, and ADAMTS-5), chondroitinase, and calpain I, but are not limited thereto. The "promotion of degradation" may be a reduction in the abundance of chondroitin sulfate proteoglycans caused by administering all or some of the enzymes.

Alternatively, "promotion of degradation" may be achieved by administering a substance that promotes the suppression of expression of chondroitin sulfate proteoglycans. Such substances include, for example, n-butylate, diethylcarbamazepine, tunicamycin, non-steroidal estrogen, and cyclofenil diphenol, but are not limited thereto.

Preferred embodiments of the "substance with the activity of promoting degradation" include, for example, compounds (nucleic acids) selected from the group consisting of:
(a) antisense nucleic acids against transcripts of the genes encoding the core proteins of chondroitin sulfate proteoglycans, or portions thereof;
(b) nucleic acids with the ribozyme activity of specifically cleaving transcripts of genes encoding core proteins of chondroitin sulfate proteoglycans; and
(c) nucleic acids with the activity of using RNAi effect to inhibit the expression of genes encoding core proteins of chondroitin sulfate proteoglycans.

Furthermore, the "substances with the activity of promoting degradation" include, for example, compounds selected from the group consisting of:
(a) antibodies that bind to core proteins of chondroitin sulfate proteoglycans;
(b) chondroitin sulfate proteoglycan variants that are dominant-negative for core proteins of chondroitin sulfate proteoglycans; and
(c) low-molecular-weight compounds that bind to core proteins of chondroitin sulfate proteoglycans.

"Inhibition of synthesis" of chondroitin sulfate proteoglycans includes, for example, inhibition of biosynthesis of glycosaminoglycans and inhibition of enzymes involved in the synthesis of chondroitin sulfate proteoglycans, but is not limited thereto. The inhibition refers to inhibition of any of the processes of chondroitin sulfate proteoglycan synthesis.

As substances that inhibit the synthesis of chondroitin sulfate proteoglycans, substances inhibiting the biosynthesis of glycosaminoglycans include, for example, β-D-xyloside, 2-deoxy-D-glucose (2-DG), ethane-1-hydroxy-1,1-diphosphonate (ETDP), and 5-hexyl-2-deoxyuridine (HudR). Such substances inhibit the biosynthesis of glycosaminoglycans, and thereby inhibit the synthesis of chondroitin sulfate proteoglycans.

Meanwhile, enzymes involved in chondroitin synthesis include, for example, GalNAc4ST-1, GalNAc4ST-2, GALNAC4S-6ST, UA2OST, GalT-I, GalT-II, GlcAT-I, and XylosylT. The synthesis of chondroitin sulfate proteoglycans is inhibited by inhibiting such enzymes, suppressing the expression thereof, or the like.

Preferred embodiments of "substances with the activity of inhibiting synthesis" include, for example, compounds (nucleic acids) selected from the group consisting of:
(a) antisense nucleic acids against transcripts of genes encoding chondroitin sulfate proteoglycan synthetases, or portions thereof;
(b) nucleic acids with the ribozyme activity of specifically cleaving transcripts of the genes encoding chondroitin sulfate proteoglycan synthetases; and
(c) nucleic acids with the activity of using RNAi effect to inhibit the expression of genes encoding chondroitin sulfate proteoglycan synthetases.

The "substances with the activity of inhibiting synthesis" also include, for example, compounds selected from the group consisting of:
(a) antibodies that bind to chondroitin sulfate proteoglycan synthetases;
(b) chondroitin sulfate proteoglycan synthetase variants that are dominant-negative for chondroitin sulfate proteoglycan synthetases; and
(c) low-molecular-weight compounds that bind to chondroitin sulfate proteoglycan synthetases.

The "desulfation" of chondroitin sulfate proteoglycans refers to the removal of a sulfate group from chondroitin sulfate proteoglycans, and includes, for example, desulfation by an endogenous or exogenously-administered desulfation enzyme, and suppression of sulfation by a sulfation-suppressing compound, but is not limited thereto. "Desulfation" refers to the process of sulfate group removal.

Such desulfation enzymes include, for example, chondroitin-4-sulfatase and chondroitin-6-sulfatase. Sulfation-suppressing compounds include, for example, chlorate and EGF receptor antagonists.

Preferred embodiments of such "substances with desulfating activity" include, for example, compounds (nucleic acids) selected from the group consisting of:
(a) antisense nucleic acids against transcripts of genes encoding proteins that suppress chondroitin sulfate proteoglycan-desulfating enzymes, or portions thereof;
(b) nucleic acids with the ribozyme activity of specifically cleaving transcripts of the genes encoding proteins that suppress chondroitin sulfate proteoglycan-desulfating enzymes; and
(c) nucleic acids with the activity of using RNAi effect to inhibit the expression of genes encoding proteins that suppress chondroitin sulfate proteoglycan-desulfating enzymes.

The "substances with desulfating activity" also include, for example, compounds selected from the group consisting of:
(a) antibodies that bind to compounds that suppress chondroitin sulfate proteoglycan-desulfating enzymes;
(b) variants of proteins that suppress chondroitin sulfate proteoglycan-desulfating enzymes, which are dominant-negative for proteins that suppress chondroitin sulfate proteoglycan-desulfating enzymes; and
(c) low-molecular-weight compounds that bind to compounds that suppress chondroitin sulfate proteoglycan-desulfating enzymes.

Herein, "desulfation-suppressing compounds" include not only proteins but also non-proteinacious compounds such as coenzymes.

The "activity of inhibiting sulfation" of chondroitin sulfate proteoglycans includes, for example, inhibition of sulfotransferases, but is not limited thereto. The activity refers to the inhibition of sulfation in the process of chondroitin sulfate proteoglycan synthesis.

Such sulfotransferases include, for example, chondroitin
D-N-acetylgalactosamine-4-O-sulfotransferase 1 (C4ST-1), chondroitin
D-N-acetylgalactosamine-4-O-sulfotransferase 2 (C4ST-2), chondroitin
D-N-acetylgalactosamine-4-O-sulfotransferase 3 (C4ST-3), D4ST, C6ST-1, and C6ST-2.

Preferred embodiments of "substances with the activity of inhibiting sulfation" include, for example, compounds (nucleic acids) selected from the group consisting of:
(a) antisense nucleic acids against transcripts of genes encoding sulfotransferases for chondroitin sulfate proteoglycans, or portions thereof;
(b) nucleic acids with the ribozyme activity of specifically cleaving transcripts of the genes encoding sulfotransferases for chondroitin sulfate proteoglycans; and
(c) nucleic acids with the activity of using RNAi effect to inhibit the expression of genes encoding sulfotransferases for chondroitin sulfate proteoglycans.

The "substances with the activity of inhibiting sulfation" also include, for example, compounds selected from the group consisting of:
(a) antibodies that bind to sulfotransferases for chondroitin sulfate proteoglycans;
(b) sulfotransferase variants for chondroitin sulfate proteoglycans; and
(c) low-molecular-weight compounds that bind to sulfotransferases for chondroitin sulfate proteoglycans.

The above enzymes shown as examples include not only single enzymes that correspond to single genes, but also groups of enzymes that share certain characteristics. For example, chondroitinase is a collective name for enzymes such as ABC, AC, and B, whose substrate specificities or such are different, but which share the characteristics of mucopolysaccharide-degrading enzymes. For example, chondroitinase AC I eliminatively cleaves the N-acetylhexosaminide linkages of chondroitin sulfates (A, C, and E), chondroitin, chondroitin sulfate-dermatan sulfate hybrids, and hyaluronic acid, and yields oligosaccharides with Δ4-glucuronate residues at the non-reducing ends. This enzyme does not act on dermatan sulfate (chondroitin sulfate B, which has L-iduronic acid for a hexuronic acid), keratan sulfate, heparan sulfate, and heparin. Meanwhile, chondroitinase AC II eliminatively cleaves the N-acetylhexosaminide linkages of chondroitin, chondroitin sulfate A, and chondroitin sulfate C, and yields Δ4-unsaturated disaccharides (ΔDi-0S, ΔDi-4S, and ΔDi-6S). This enzyme also acts well on hyaluronic acid. The enzyme does not act on dermatan sulfate (chondroitin sulfate B), which is therefore a competitive inhibitor of the enzyme. Chondroitinase B (dermatanase) eliminatively cleaves N-acetylhexosaminide linkages to L-iduronic acids in dermatan sulfate, and yields oligosaccharides (di- and tetra-saccharides) with Δ4-hexuronate residues at the non-reducing ends. This enzyme acts on neither chondroitin sulfate A nor chondroitin sulfate C, which are free of L-iduronic acid. Dermatan, which is a derivative of dermatan sulfate in which the sulfate group is removed, does not serve as a substrate for this enzyme. This enzyme preferentially cleaves portions of dermatan sulfate in which the second of the L-iduronic acid units are sulfated. Chondroitinase ABC eliminatively cleaves N-acetylhexosaminide linkages of chondroitin sulfate A, chondroitin sulfate C, dermatan sulfate, chondroitin, and hyaluronic acid, and yields mainly disaccharides with Δ4-hexuronate groups at the non-reducing ends. This enzyme does not act on keratan sulfate, heparin, and heparan sulfate. Chondroitinases collectively refer to enzymes sharing the characteristics of mucopolysaccharide-degrading enzymes while also having different characteristics as described above, and they are not limited to chondroitinase AC I, chondroitinase AC II, chondrotinase B, and chondroitinase ABC as exemplified above.

Further, on a genomic DNA level, such groups of enzymes sharing features do not necessarily correspond to single genes. For example, both chondroitin-4-sulfatase and chondroitin-6-sulfatase can be retrieved from the public gene database Genbank as sequences referred to by multiple accession numbers (for example, Genbank Accession Nos: NT_039500 (a portion thereof is shown under Accession No: CAAA01098429 (SEQ ID NO: 68)), NT_078575, NT_039353, NW_001030904, NW_001030811, NW_001030796, and NW_000349).

The above example proteins that correspond to single genes are shown below:
Specifically, below are the accession numbers in the public gene database Genbank, nucleotide sequences, and amino acid sequences for human genes encoding:
   aggrecan, versican, neurocan, brevican, β-glycan, decorin, biglycan, fibromodulin, and PG-Lb, which are shown above as examples of chondroitin sulfate proteoglycans; ADAMTS-1, ADAMTS-4, ADAMTS-5, and calpain I, which are shown above as examples of enzymes that cleave or degrade chondroitin sulfate proteoglycans or related enzymes; GalNAc4ST-1, GalNAc4ST-2, GALNAC4S-6ST, UA2OST, GalT-I, GalT-II, GlcCAT-I, and XylosylT, which are shown above as examples of enzymes involved in chondroitin synthesis; C4ST-1, C4ST-2, C4ST-3, D4ST, C6ST-1, and C6ST-2, which are shown above as examples of sulfotransferases.

   Aggrecan (Accession No: NM_007424; nucleotide sequence: SEQ ID NO: 1; amino acid sequence: SEQ ID NO: 2)
   Versican (Accession No: BC096495; nucleotide sequence: SEQ ID NO: 3; amino acid sequence: SEQ ID NO: 4)
   Neurocan (Accession No: NM_010875; nucleotide sequence: SEQ ID NO: 5; amino acid sequence: SEQ ID NO: 6)
   Brevican (Accession No: NM_007529; nucleotide sequence: SEQ ID NO: 7; amino acid sequence: SEQ ID NO: 8)
   β-glycan (Accession No: AF039601; nucleotide sequence: SEQ ID NO: 9; amino acid sequence: SEQ ID NO: 10)
   Decorin (Accession No: NM_007833; nucleotide sequence: SEQ ID NO: 11; amino acid sequence: SEQ ID NO: 12)
   Biglycan (Accession No: BC057185; nucleotide sequence: SEQ ID NO: 13; amino acid sequence: SEQ ID NO: 14)
   Fibromodulin (Accession No: NM_021355; nucleotide sequence: SEQ ID NO: 15; amino acid sequence: SEQ ID NO: 16)
   PG-Lb (Accession No: NM_007884; nucleotide sequence: SEQ ID NO: 17; amino acid sequence: SEQ ID NO: 18)
   ADAMTS-1 (Accession No: NM_009621; nucleotide sequence: SEQ ID NO: 19; amino acid sequence: SEQ ID NO: 20)
   ADAMTS-4 (Accession No: NM_172845; nucleotide sequence: SEQ ID NO: 21; amino acid sequence: SEQ ID NO: 22)
   ADAMTS-5 (Accession No: AF140673; nucleotide sequence: SEQ ID NO: 23; amino acid sequence: SEQ ID NO: 24)
   Calpain I (Accession No: NM_007600; nucleotide sequence: SEQ ID NO: 25; amino acid sequence: SEQ ID NO: 26)
   GalNAc4ST- 1 (Accession No: NM_175140; nucleotide sequence: SEQ ID NO: 27; amino acid sequence: SEQ ID NO: 28)
   GalNAc4ST-2 (Accession No: NM_199055; nucleotide sequence: SEQ ID NO: 29; amino acid sequence: SEQ ID NO: 30)
   GALNAC4S-6ST (Accession No: NM_029935; nucleotide sequence: SEQ ID NO: 31; amino acid sequence: SEQ ID NO: 32)
   UA2OST (Accession No: NM_177387; nucleotide sequence: SEQ ID NO: 33; amino acid sequence: SEQ ID NO: 34)
   GaIT-I (Accession No: NM_016769; nucleotide sequence: SEQ ID NO: 35; amino acid sequence: SEQ ID NO: 36)
   GalT-II (Accession No: BC064767; nucleotide sequence: SEQ ID NO: 37; amino acid sequence: SEQ ID NO: 38)
   GlcAT-I (Accession No: BC058082; nucleotide sequence: SEQ ID NO: 39; amino acid sequence:
   SEQ ID NO: 40), or Accession No: NM_024256; nucleotide sequence: SEQ ID NO: 41; amino acid sequence: SEQ ID NO: 42)
   XylosylT (Accession No: NM_145828; nucleotide sequence: SEQ ID NO: 43; amino acid sequence: SEQ ID NO: 44)
   C4ST-1 (Accession No: NM_021439; nucleotide sequence: SEQ ID NO: 45; amino acid sequence: SEQ ID NO: 46)
   C4ST-2 (Accession No: NM_021528; nucleotide sequence: SEQ ID NO: 47; amino acid sequence: SEQ ID NO: 48)
   C4ST-3 (Accession No: XM_355798; nucleotide sequence: SEQ ID NO: 49; amino acid sequence: SEQ ID NO: 50)
   D4ST (Accession No: NM_028117; nucleotide sequence: SEQ ID NO: 51; amino acid sequence: SEQ ID NO: 52)
   C6ST-1 (Accession No: NM_016803; nucleotide sequence: SEQ ID NO: 53; amino acid sequence: SEQ ID NO: 54)
   C6ST-2 (Accession No: AB046929; nucleotide sequence: SEQ ID NO: 55; amino acid sequence: SEQ ID NO: 56)

In addition to the proteins listed above, the proteins of the present invention include those exhibiting high homology (typically 70% or higher, preferably 80% or higher, more preferably 90% or higher, and most preferably 95% or higher) to sequences shown in the Sequence Listing and with a function of the proteins listed above (for example, the function of binding to intracellular components). The proteins listed above are, for example, proteins comprising an amino acid sequence with an addition, deletion, substitution, or insertion of one or more amino acids in any of the amino acid sequences of SEQ ID NOs: 2, 4, 6, 8, 10,12, 14,16, 18, 20, 22, 24, 26, 28, 30, 32, 34, 36, 38, 40, 42, 44, 46, 48, 50, 52, 54, and 56, in which the number of altered amino acids is typically 30 amino acids or less, preferably ten amino acids or less, more preferably five amino acids or less, and most preferably three amino acids or less.

The above-described genes of the present invention include, for example, endogenous genes of other organisms which correspond to DNAs comprising any of the nucleotide sequences of SEQ ID NOs: 1, 3, 5, 7, 9, 11, 13, 15, 17, 19, 21, 23, 25, 27, 29, 31, 33, 35, 37, 39, 41, 43, 45, 47, 49, 51, 53, and 55 (homologues to the human genes described above, or the like).

Each of the endogenous DNAs of other organisms which correspond to DNAs comprising any of the nucleotide sequences of SEQ ID NOs: 1, 3, 5, 7, 9, 11, 13, 15, 17, 19, 21, 23, 25, 27, 29, 31, 33, 35, 37, 39, 41, 43, 45, 47, 49, 51, 53, and 55 are generally highly homologous to a DNA of any of SEQ ID NOs: 1,3,5,7,9,11,13,15,17,19,21,23,25,27,29, 31, 33, 35,-37,39, 41, 43, 45, 47, 49, 51, 53, and 55. High homology means 50% or higher homology, preferably 70% or higher homology, more preferably 80% or higher homology, and still more preferably 90% or higher homology (for example, 95% or higher, or 96%, 97%, 98%, or 99% or higher). Homology can be determined using the mBLAST algorithm (Altschul et al., Proc. Natl. Acad. Sci. USA 1990, 87, 2264-8; Karlin and Altschul, Proc. Natl. Acad. Sci. USA 1993, 90, 5873-7). When the DNAs have been isolated from the body, each of them may hybridize under stringent conditions to a DNA of SEQ ID NO: 1, 3, 5, 7, 9, 11, 13, 15, 17, 19,21, 23, 25, 27, 29, 31, 33, 35, 37, 39, 41, 43, 45, 47, 49, 51, 53, or 55. Herein, stringent conditions include, for example, "2x SSC, 0.1% SDS, 50°C", "2x SSC, 0.1% SDS, 42°C", and "1x SSC, 0.1% SDS, 37°C"; more stringent conditions include "2x SSC, 0.1% SDS, 65°C", "0.5x SSC, 0.1% SDS, 42°C", and "0.2x SSC, 0.1% SDS, 65°C".

Those skilled in the art can appropriately obtain proteins functionally equivalent to the above-described proteins from the above-described highly homologous proteins by using methods for assaying the activity of promoting the degradation of CSPGs, inhibiting the synthesis of CSPGs, desulfating CSPGs, or inhibiting the sulfation of CSPGs. Specific methods for assaying the activities are described below in a section on the screening methods of the present invention. Further, based on the nucleotide sequences of the above-described genes, those skilled in the art can appropriately obtain endogenous genes of other organisms that correspond to the above-described genes. In the present invention, the above-described proteins and genes in non-human organisms, which correspond to the above-described proteins and genes, or the above-described proteins and genes that are functionally equivalent to the above-described proteins and genes, may simply be referred to using the above-described names.

The proteins of the present invention can be prepared not only as natural proteins but also as recombinant proteins using genetic recombination techniques. The natural proteins can be prepared by, for example, methods of subjecting cell extracts (tissue extracts) that may express the above-described proteins to affinity chromatography using antibodies against the above-described proteins. On the other hand, the recombinant proteins can be prepared, for example, by culturing cells transformed with DNAs encoding the proteins described above. The above-described proteins of the present invention can be suitably used, for example, in the screening methods described herein below.

In the present invention, "nucleic acids" refer to both RNAs and DNAs. Chemically synthesized nucleic acid analogs, such as so-called "PNAs" (peptide nucleic acids), are also included in the nucleic acids of the present invention. PNAs are nucleic acids in which the fundamental backbone structure of nucleic acids, the pentose-phosphate backbone, is replaced by a polyamide backbone with glycine units. PNAs have a three-dimensional structure quite similar to that of nucleic acids.

Methods for inhibiting the expression of specific endogenous genes using antisense technology are well known to those skilled in the art. There are a number of causes for the action of antisense nucleic acids in inhibiting target gene expression, including:
inhibition of transcription initiation by triplex formation;
transcription inhibition by hybrid formation at a site with a local open loop structure generated by an RNA polymerase;
transcription inhibition by hybrid formation with the RNA being synthesized;
splicing inhibition by hybrid formation at an intron-exon junction;
splicing inhibition by hybrid formation at the site of spliceosome formation;
inhibition of transport from the nucleus to the cytoplasm by hybrid formation with mRNA; splicing inhibition by hybrid formation at the capping site or poly(A) addition site;
inhibition of translation initiation by hybrid formation at the translation initiation factor binding site;
inhibition of translation by hybrid formation at the ribosome binding site adjacent to the start codon;
inhibition of peptide chain elongation by hybrid formation in the translational region of mRNA or at the polysome binding site of mRNA; and
inhibition of gene expression by hybrid formation at the protein-nucleic acid interaction sites. Thus, antisense nucleic acids inhibit the expression of target genes by inhibiting various processes, such as transcription, splicing, and translation (Hirashima and Inoue, Shin Seikagaku Jikken Koza 2 (New Courses in Experimental Biochemistry 2), Kakusan (Nucleic Acids) IV: "Idenshi no Fukusei to Hatsugen (Gene replication and expression)", Ed. The Japanese Biochemical Society, Tokyo Kagakudojin, 1993, pp. 319-347).

The antisense nucleic acids used in the present invention may inhibit the expression and/or function of genes encoding any of the CSPG core proteins, synthetases, proteins suppressing desulfation enzymes, and sulfotransferases described above, based on any of the actions described above. In one embodiment, antisense sequences designed to be complementary to an untranslated region adjacent to the 5' end of an mRNA for a gene encoding an above-described CSPG core protein, synthetase, desulfation enzyme-suppressing protein, or sulfotransferase may be effective for inhibiting translation of the gene. Sequences complementary to a coding region or 3'-untranslated region can also be used. Thus, the antisense nucleic acids to be used in the present invention include not only nucleic acids comprising sequences antisense to the coding regions, but also nucleic acids comprising sequences antisense to untranslated regions of genes encoding the above-described CSPG core proteins, synthetases, desulfation enzyme-suppressing proteins, or sulfotransferases. Such antisense nucleic acids to be used are linked downstream of adequate promoters and are preferably linked with transcription termination signals on the 3' side. Nucleic acids thus prepared can be introduced into desired animals (cells) using known methods. The sequences of the antisense nucleic acids are preferably complementary to a gene or portion thereof encoding a CSPG core protein, synthetase, desulfation enzyme-suppressing protein, or sulfotransferase that is endogenous to the animals (cells) to be transformed with them. However, the sequences need not be perfectly complementary, as long as the antisense nucleic acids can effectively suppress expression of a gene. The transcribed RNAs preferably have 90% or higher, and most preferably 95% or higher complementarity to target gene transcripts. To effectively inhibit target gene expression using antisense nucleic acids, the antisense nucleic acids are preferably at least 15 nucleotides long, and less than 25 nucleotides long. However, the lengths of the antisense nucleic acids of the present invention are not limited to the lengths mentioned above, and they may be 100 nucleotides or more, or 500 nucleotides or more.

The antisense nucleic acids of the preset invention are not particularly limited, and can be prepared, for example, based on the nucleotide sequence of a versican gene (GenBank Accession No: BC096495; SEQ ID NO: 3), C4ST-1 (GenBank Accession No: NM_021439; SEQ ID NO: 45), C4ST-2 (GenBank Accession No: NM_021528; SEQ ID NO: 47), C4ST-3 (GenBank Accession No: XM_355798; SEQ ID NO: 49), or such.

Expression of the above-mentioned genes encoding CSPG core proteins, synthetases, desulfation enzyme-suppressing proteins, or sulfotransferases can also be inhibited using ribozymes or ribozyme-encoding DNAs. Ribozymes refer to RNA molecules with catalytic activity. There are various ribozymes with different activities. Among others, studies that focused on ribozymes functioning as RNA-cleaving enzymes have enabled the design of ribozymes that cleave RNAs in a site-specific manner. Some ribozymes have 400 or more nucleotides, such as group I intron type ribozymes and M1 RNA, which is comprised by RNase P, but others, called hammerhead and hairpin ribozymes, have a catalytic domain of about 40 nucleotides (Koizumi, M. and Otsuka, E., Tanpakushitsu Kakusan Koso (Protein, Nucleic Acid, and Enzyme) 1990, 35, 2191).

For example, the autocatalytic domain of a hammerhead ribozyme cleaves the sequence G13U14C15 at the 3' side of C15. Base pairing between U14 and A9 has been shown to be essential for this activity, and the sequence can be cleaved when C15 is substituted with A15 or U15 (Koizumi, M. et al., FEBS Lett. 1988,228,228). Restriction enzyme-like RNA-cleaving ribozymes that recognize the sequence UC, UU, or UA in target RNAs can be created by designing their substrate-binding sites to be complementary to an RNA sequence adjacent to a target site (Koizumi, M. et al., FEBS Lett. 1988, 239, 285; Koizumi, M. and Otsuka, E., Tanpakushitsu Kakusan Koso (Protein, Nucleic Acid, and Enzyme) 1990, 35, 2191; and Koizumi, M. et al., Nucl Acids Res. 1989, 17, 7059).

In addition, hairpin ribozymes are also useful for the purposes of the present invention. Such ribozymes are found in, for example, the minus strand of satellite RNAs of tobacco ringspot viruses (Buzayan, J.M., Nature 1986, 323, 349). It has been shown that target-specific RNA-cleaving ribozymes can also be created from hairpin ribozymes (Kikuchi, Y. and Sasaki, N., Nucl Acids Res. 1991, 19, 6751; and Kikuchi, Y. Kagaku to Seibutsu (Chemistry and Biology) 1992, 30, 112). Thus, the expression of the above-described genes encoding CSPG core proteins, synthetases, desulfation enzyme-suppressing proteins, or sulfotransferases can be inhibited by using ribozymes to specifically cleave the gene transcripts.

The expression of endogenous genes can also be suppressed by RNA interference (hereinafter abbreviated as "RNAi"), using double-stranded RNAs comprising a sequence the same as or similar to a target gene sequence.

A great many disease-related genes have been rapidly identified since the entire human nucleotide sequence was revealed upon the recent completion of the genome project, and currently specific gene-targeted therapies and drugs are being actively developed. Of these, the application to gene therapy of small interfering RNAs (siRNAs), which produce the effect of specific post-transcriptional suppression, has been drawing attention. RNAi is a technology currently drawing attention in which double-stranded RNAs (dsRNAs) incorporated directly into cells suppress the expression of genes with sequences homologous to the dsRNAs. In mammalian cells, RNAi can be induced using short dsRNAs (siRNAs) and has many advantages: compared to knockout mice, RNAi has a stable effect, simple experiments, low costs, and so on.

Nucleic acids with inhibitory activity based on RNAi effect are generally referred to as siRNAs or shRNAs. RNAi is a phenomenon in which, when cells or such are introduced with short double-stranded RNAs (hereinafter abbreviated as "dsRNAs") comprising sense RNAs that comprise sequences homologous to the mRNAs of a target gene, and antisense RNAs that comprise sequences homologous a sequence complementary thereto, the dsRNAs bind specifically and selectively to the target gene mRNAs, induce their disruption, and cleave the target gene, thereby effectively inhibiting (suppressing) target gene expression. For example, when dsRNAs are introduced into cells, the expression of genes with sequences homologous to the RNAs is suppressed (the genes are knocked down). As described above, RNAi can suppress the expression of target genes, and is thus drawing attention as a method applicable to gene therapy, or as a simple gene knockout method replacing conventional methods of gene disruption, which are based on complicated and inefficient homologous recombination. The RNAs to be used in RNAi are not necessarily perfectly identical to the genes or portions thereof that encode an above-described CSPG core protein, synthetase, desulfation enzyme-suppressing protein, or sulfotransferase; however, the RNAs are preferably perfectly homologous to the genes or portions thereof.

The targets of the siRNAs to be designed are not particularly limited, as long as they are genes encoding an above-described CSPG core protein, synthetase, desulfation enzyme-suppressing protein, or sulfotransferase. Any region of the gene can be a candidate for a target. For example, siRNAs may be prepared based on a nucleotide sequence of the versican gene (SEQ ID NO: 3), C4ST-1 gene (SEQ ID NO: 45), C4ST-2 gene (SEQ ID NO: 47), C4ST-3 gene (SEQ ID NO: 49), and such. More specifically, partial regions of such sequences may be used as candidates for the targets. For example, siRNAs may be prepared based on portions of the nucleotide sequences of a versican gene (SEQ ID NO: 57), C4ST-1 gene (SEQ ID NO: 58), C4ST-2 gene (SEQ ID NO: 59), C4ST-3 gene (SEQ ID NO: 60), C6ST-1 gene (SEQ ID NO: 61), C6ST-2 gene (SEQ ID NO: 62), GalNAc4ST-1 gene (SEQ ID NO: 63), GalNAc4ST-2 gene (SEQ ID NO: 64), GALNAC4S-6ST gene (SEQ ID NO: 65), or such.

The siRNAs can be introduced into cells by adopting methods of introducing cells with plasmid DNAs linked with siRNAs synthesized *in vitro* or methods that comprise annealing two RNA strands.

The two RNA molecules described above may be closed at one end or, for example, may be siRNAs with hairpin structures (shRNAs). shRNAs refer to short hairpin RNAs, which are RNA molecules with a stem-loop structure, since a portion of the single strand constitutes a strand complementary to another portion. Thus, molecules capable of forming an intramolecular RNA duplex structure are also included in the siRNAs of the present invention.

In a preferred embodiment of the present invention, the siRNAs of the present invention also include, for example, double-stranded RNAs with additions or deletions of one or a few RNAs in an siRNA which targets a specific DNA sequence (SEQ ID NOs: 57 to 65) shown herein and which can suppress the expression of versican, C4ST-1, C4ST-2, C4ST-3, or such *via* RNAi effect, as long as the double-stranded RNAs have the function of suppressing the expression of a gene encoding an above-described CSPG core protein, synthetase, desulfation enzyme-suppressing protein, or sulfotransferase.

The RNAs used in RNAi (siRNAs) do not need to be perfectly identical (homologous) to the genes encoding the above proteins or portions thereof; however, the RNAs are preferably perfectly identical (homologous).

Some details of the RNAi mechanism still remain unclear, but it is understood that an enzyme called "DICER" (a member of the RNase III nuclease family) is contacted with a double-stranded RNA and degrades it in to small fragments, called "small interfering RNAs" or "siRNAs". The double-stranded RNAs of the present invention that have RNAi effect include such double-stranded RNAs prior to being degraded by DICER. Specifically, since even long RNAs that have no RNAi effect when intact can be degraded into siRNAs which have RNAi effect in cells, the length of the double-stranded RNAs of the present invention is not particularly limited.

For example, long double-stranded RNAs covering the full-length or near full-length mRNA of a gene encoding an above-described CSPG core protein, synthetase, desulfation enzyme-suppressing protein, or sulfotransferase can be pre-digested, for example, by DICER, and then the degradation products can be used as agents of the present invention. These degradation products are expected to contain double-stranded RNA (siRNA) molecules with RNAi effect. With this method, it is not necessary to specifically select the mRNA regions expected to have RNAi effect. In other words, it is not necessary to accurately determine regions with RNAi effect in the mRNAs of the genes described above.

The above-described "double-stranded RNAs capable of suppression via RNAi effect" can be suitably prepared by those skilled in the art based on nucleotide sequences of the above-described CSPG genes encoding core proteins, synthetases, desulfation enzyme-suppressing proteins, or sulfotransferases, which are targeted by the double-stranded RNAs. For example, the double-stranded RNAs of the present invention can be prepared based on the nucleotide sequence of SEQ ID NO: 57. In other words, it is within the range of ordinary trials for those skilled in the art to select an arbitrary consecutive RNA region in an mRNA that is a transcript of the nucleotide sequence of SEQ ID NO: 57, and prepare double-stranded RNA corresponding to the region. Those skilled in the art can also use known methods to properly select siRNA sequences with stronger RNAi effect from the mRNA sequence, which is the transcript of the nucleotide sequence of SEQ ID NO: 57. When one of the strands is already identified, those skilled in the art can readily determine the nucleotide sequence of the other strand (complementary strand). Those skilled in the art can appropriately prepare siRNAs using a commercially available nucleic acid synthesizer. Alternatively, general custom synthesis services may be used to synthesize desired RNAs.

The siRNAs of the present invention are not necessarily single pairs of double-stranded RNAs directed to target sequences, but may be mixtures of multiple double-stranded RNAs directed to regions that cover the target sequence. Herein, those skilled in the art can appropriately prepare the siRNAs as nucleic acid mixtures matched to a target sequence by using a commercially available nucleic acid synthesizer or DICER enzyme. Meanwhile, general custom synthesis services may be used to synthesize desired RNAs. The siRNAs of the present invention include so-called "siRNA cocktails".

All nucleotides in the siRNAs of the present invention do not necessarily need to be ribonucleotides (RNAs). Specifically, one or more of the ribonucleotides constituting the siRNAs of the present invention may be replaced with corresponding deoxyribonucleotides. The term "corresponding" means that although the sugar moieties are structurally differently, the nucleotide residues (adenine, guanine, cytosine, or thymine (uracil)) are the same. For example, deoxyribonucleotides corresponding to ribonucleotides with adenine refer to deoxyribonucleotides with adenine. The term "or more" described above is not particularly limited, but preferably refers to a small number of about two to five ribonucleotides.

Furthermore, DNAs (vectors) capable of expressing the RNAs of the present invention are also included in the preferred embodiments of compounds capable of suppressing the expression of the genes encoding the above-described proteins of the present invention. The DNAs (vectors) capable of expressing the double-stranded RNAs of the present invention are, for example, DNAs structured such that a DNA encoding one strand of a double-stranded RNA and a DNA encoding the other strand of the double-stranded RNA are linked with promoters so that each DNA can be expressed. The above DNAs of the present invention can be appropriately prepared by those skilled in the art using standard genetic engineering techniques. More specifically, the expression vectors of the present invention can be prepared by adequately inserting DNAs encoding the RNAs of the present invention into various known expression vectors.

Furthermore, the expression-inhibiting substances of the present invention also include compounds that inhibit the expression of the above-described CSPG core proteins, synthetases, desulfation enzyme-suppressing proteins, or sulfotransferases by binding to an expression regulatory region of a gene encoding the above-described CSPG core proteins, synthetases, desulfation enzyme-suppressing proteins, or sulfotransferases (for example, a promoter region; specific examples include the nucleotide sequence of SEQ ID NO: 66, which is a promoter region of PG-Lb). Such compounds can be obtained, for example, using a fragment of a promoter DNA of the gene encoding an above-described CSPG core protein, synthetase, desulfation enzyme-suppressing protein, or sulfotransferase to perform screening methods using as an indicator the activity of binding to the DNA fragment. Those skilled in the art can appropriately determine whether compounds of interest inhibit the expression of the above-described genes encoding CSPG core proteins, synthetases, desulfation enzyme-suppressing proteins, or sulfotransferases by using known methods, for example, reporter assays and such.

Furthermore, DNAs (vectors) capable of expressing the above-described RNAs of the present invention are also included in preferred embodiments of the compounds capable of inhibiting the expression of a gene encoding an above-described CSPG core protein, synthetase, desulfation enzyme-suppressing protein, or sulfotransferase of the present invention. For example, DNAs (vectors) capable of expressing the above-described double-stranded RNAs of the present invention are structured such that a DNA encoding one strand of a double-stranded RNA and a DNA encoding the other strand of the double-stranded RNA are linked to promoters so that both can be expressed. Those skilled in the art can appropriately prepare the above-described DNAs of the present invention using standard genetic engineering techniques. More specifically, the expression vectors of the present invention can be prepared by appropriately inserting DNAs encoding the RNAs of the present invention into various known expression vectors.

Preferred embodiments of the above-described vector of the present invention include vectors expressing RNAs (siRNAs) that can suppress the expression of versican, C4ST-1, C4ST-2, C4ST-3, or the like by RNAi effect.

Antibodies that bind to the above-described CSPG core proteins, synthetases, desulfation enzyme-suppressing compounds, or sulfotransferases can be prepared by methods known to those skilled in the art. Polyclonal antibodies can be obtained, for example, by the following procedure: small animals such as rabbits are immunized with an above-described natural protein or a recombinant protein expressed in microorganisms as a fusion protein with GST, or a partial peptide thereof. Sera are obtained from these animals and purified by, for example, ammonium sulfate precipitation, Protein A or G column, DEAE ion exchange chromatography, affinity column coupled with the core protein, synthetase, desulfation enzyme-suppressing compound, or sulfotransferase for CSPGs described above, synthetic peptide, or such, to prepare antibodies. Monoclonal antibodies can be obtained by the following procedure: small animals such as mice are immunized with an above-described CSPG core protein, synthetase, desulfation enzyme-suppressing compound, or sulfotransferase, or a partial peptide thereof. Spleens are removed from the mice and crushed to isolate cells. The cells are fused with mouse myeloma cells using a reagent such as polyethylene glycol. Clones producing antibodies that bind to an above-described CSPG core protein, synthetase, desulfation enzyme-suppressing compound, or sulfotransferase are selected from among the resulting fused cells (hybridomas). The obtained hybridomas are then transplanted in the peritoneal cavities of mice, and ascites is collected from the mice. The obtained monoclonal antibodies can be purified by, for example, ammonium sulfate precipitation, Protein A or G columns, DEAE ion exchange chromatography, affinity columns coupled with an above-described CSPG core protein, synthetase, desulfation enzyme-suppressing compound, or sulfotransferase, synthetic peptides, or such.

The antibodies of the present invention are not particularly limited as long as they bind to an above-described core protein, synthetase, desulfation enzyme-suppressing compound, or sulfotransferase of the present invention. The antibodies of the present invention may be human antibodies, humanized antibodies created by gene recombination, fragments or modified products of such antibodies, in addition to the polyclonal and monoclonal antibodies described above.

The proteins of the present invention used as sensitizing antigens to prepare antibodies are not limited in terms of the animal species from which the proteins are derived. However, the proteins are preferably derived from mammals, for example, mice and humans. Human-derived proteins are particularly preferred. The human-derived proteins can be appropriately obtained by those skilled in the art using the gene or amino acid sequences disclosed herein.

In the present invention, the proteins to be used as sensitizing antigens may be whole proteins or partial peptides thereof. Such partial peptides of the proteins include, for example, amino-terminal (N) fragments and carboxyl-terminal (C) fragments of the proteins. Herein, "antibodies" refer to antibodies that react with a full-length protein or fragment thereof.

In addition to immunizing nonhuman animals with antigens to obtain the above hybridomas, human lymphocytes, for example, EB virus-infected human lymphocytes, can be sensitized *in vitro* with the proteins or with cells expressing the proteins, or with lysates thereof, and the sensitized lymphocytes can be fused with human-derived myeloma cells with the ability to divide permanently, for example, U266, to obtain hybridomas that produce desired human antibodies with binding activity to the proteins.

It is expected that antibodies against the above-described CSPG core proteins, synthetases, desulfation enzyme-suppressing compounds, or sulfotransferases of the present invention exhibit the effect of inhibiting protein expression or function by binding to the proteins. When using the prepared antibodies for human administration (antibody therapy), the antibodies are preferably human or humanized antibodies in order to reduce immunogenicity.

Furthermore, in the present invention, low-molecular-weight substances (low-molecular-weight compounds) that bind to the above-described CSPG core proteins, synthetases, desulfation enzyme-suppressing compounds, or sulfotransferases are also included in the substances capable of inhibiting the function of the above-described CSPG core proteins, synthetases, desulfation enzyme-suppressing compounds, or sulfotransferases. Such low-molecular-weight substances may be natural or artificial compounds. In general, the compounds can be produced or obtained by methods known to those skilled in the art. The compounds of the present invention can also be obtained by the screening methods described below.

In addition, the substances of the present invention capable of inhibiting the expression or function of the above-described CSPG core proteins, synthetases, desulfation enzyme-suppressing proteins, or sulfotransferases include dominant-negative mutants (dominant-negative proteins) for the above-described CSPG core proteins, synthetases, desulfation enzyme-suppressing proteins, or sulfotransferases. The "dominant-negative protein mutants for the above CSPG core proteins, synthetases, desulfation enzyme-suppressing proteins, or sulfotransferases" refer to proteins with the function of reducing or abolishing the activity of endogenous wild-type proteins by expressing the genes encoding the CSPG core proteins, synthetases, desulfation enzyme-suppressing proteins, or sulfotransferases. Such dominant-negative proteins include, for example, versican core protein mutants that competitively inhibit the linking of the wild-type versican core protein with chondroitin sulfate.

Furthermore, in the present invention, tissues and cells where the production or accumulation of chondroitin sulfate proteoglycans is inhibited are not particularly limited, but are preferably liver tissues.

Compounds that inhibit the production or accumulation of chondroitin sulfate proteoglycans are expected to serve as therapeutic or preventive agents for fibrotic liver diseases. Herein, "therapeutic or preventive" does not necessarily refer to a perfect therapeutic or preventive effect on tissues or cells with hepatic fibrosis, and may refer to a partial effect.

Herein, the fibrotic liver diseases are not particularly limited, as long as they are associated with hepatic fibrosis; however, they preferably include chronic liver diseases, such as chronic hepatitis, liver fibrosis, cirrhosis, liver failure, and hepatocarcinoma. In addition, complications of these diseases are also included in the fibrotic liver diseases of the present invention.

The hepatic fibrosis-suppressing agents of the present invention have an activity of suppressing hepatic fibrosis by inhibiting the production or accumulation of chondroitin sulfate proteoglycans which causes hepatic fibrosis. Thus, preferred embodiments of the present invention provide, for example, therapeutic agents for chronic liver diseases or cirrhosis, which comprise a hepatic fibrosis-suppressing agent of the present invention as an active ingredient.

The "hepatic fibrosis-suppressing agents" of the present invention can also be referred to as "therapeutic agents for hepatic fibrosis", "hepatic fibrosis-improving agents", or the like. Meanwhile, the "suppressing agents" of the present invention can also be referred to as "pharmaceutical agents", "pharmaceutical compositions", "therapeutic medicines", or the like.

The "treatments" of the present invention also comprise preventive effects that can suppress the onset of hepatic fibrosis in advance. The treatments are not limited to those producing a perfect therapeutic effect on cells (tissues) developing hepatic fibrosis, and the effects may be partial.

The agents of the present invention can be combined with physiologically acceptable carriers, excipients, diluents and such, and orally or parenterally administered as pharmaceutical compositions. Oral agents may be in the form of granules, powders, tablets, capsules, solutions, emulsions, suspensions, or the like. The dosage forms of parenteral agents can be selected from injections, infusions, external preparations, inhalants, suppositories, and the like. Injections include preparations for subcutaneous, intramuscular, and intraperitoneal injections, and the like. The external preparations include nasal preparations, ointments, and such. Techniques for formulating the above-described dosage forms that contain the agents of the present invention as primary ingredients are known.

For example, tablets for oral administration can be produced by compressing and shaping the agents of the present invention in combination with excipients, disintegrants, binders, lubricants, and the like. Excipients commonly used include lactose, starch, mannitol, and the like. Commonly used disintegrants include calcium carbonate, carboxymethylcellulose calcium, and the like. Binders include gum arabic, carboxymethylcellulose, and polyvinylpyrrolidone. Known lubricants include talc, magnesium stearate, and such.

Known coatings can be applied to tablets comprising the agents of the present invention to prepare enteric coated formulations or for masking. Ethylcellulose, polyoxyethylene glycol, or such can be used as a coating agent.

Meanwhile, injections can be prepared by dissolving the agents of the present invention, which are chief ingredients, together with an appropriate dispersing agent, or dissolving or dispersing the agents in a dispersion medium. Both water-based and oil-based injections can be prepared, depending on the selection of dispersion medium. When preparing water-based injections, the dispersing agent is distilled water, physiological saline, Ringer's solution or such. For oil-based injections, any of the various vegetable oils, propylene glycols, or such is used as a dispersing agent. If required, a preservative such as paraben may be added at this time. Known isotonizing agents such as sodium chloride and glucose can also be added to the injections. In addition, soothing agents such as benzalkonium chloride and procaine hydrochloride can be added.

Alternatively, the agents of the present invention can be formed into solid, liquid, or semi-solid compositions to prepare external preparations. Such solid or liquid compositions can be prepared as the same compositions as described above and then used as external preparations. The semi-solid compositions can be prepared using an appropriate solvent, to which a thickener is added if required. Water, ethyl alcohol, polyethylene glycol, and the like can be used as the solvent. Commonly used thickeners are bentonite, polyvinyl alcohol, acrylic acid, methacrylic acid, polyvinylpyrrolidone, and the like. Preservatives such as benzalkonium chloride can be added to these compositions. Alternatively, suppositories can be prepared by combining the compositions with carriers, like oil bases such as cacao butter, or aqueous gel bases such as cellulose derivatives.

When the agents of the present invention are used as gene therapy agents, the agents may be directly administered by injection, or vectors carrying the nucleic acid may be administered. Such vectors include adenovirus vectors, adeno-associated virus vectors, herpes virus vectors, vaccinia virus vectors, retroviral vectors, and lentivirus vectors. These vectors allow efficient administration.

Alternatively, the agents of the present invention can be encapsulated into phospholipid vesicles such as liposomes, and then the vesicles can be administered. Vesicles carrying siRNAs or shRNAs are introduced into given cells by lipofection. The resulting cells are then systemically administered, for example, intravenously or intraarterially. The cells can also be locally administered into tissues or such with hepatic fibrosis. siRNAs exhibit a quite superior and specific post-transcriptional suppression effect *in vitro;* however, *in vivo* they are rapidly degraded due to serum nuclease activity. Thus, their limited time *in vivo* becomes problematic, and there is therefore demand for the development of optimized and effective delivery systems. As one example, Ochiya *et al.* have reported that atelocollagen, a bio-affinity material, is a highly suitable siRNA carrier because it has the activity of protecting nucleic acids from nucleases in the body when mixed with the nucleic acids to form a complex (Ochiya, T. et al., Nat. Med. 1999, 5, 707-710; Ochiya, T. et al., Curr. Gene Ther. 2001, 1, 31-52). However, the methods for introducing drugs of the present invention are not limited thereto.

The agents of the present invention are administered to mammals including humans at required (effective) doses, within a dose range considered to be safe. Ultimately, the doses of the agents of the present invention can be appropriately determined by medical practitioners or veterinarians after considering the dosage form and administration method, and the patient's age and weight, symptoms, and the like. For example, adenoviruses are administered once a day at a dose of about 10⁶ to 10¹³ viruses every one to eight weeks, although the doses vary depending on the age, sex, symptoms, administration route, administration frequency, and dosage form.

Commercially available gene transfer kits (for example: AdenoExpressTM, Clontech) may be used to introduce siRNAs or shRNAs into target tissues or organs.

When the agents of the present invention are used, the type of disease and site to which the agents are applied are not particularly limited, as long as the disease develops hepatic fibrosis; for example, the agents are applied to chronic hepatitis, liver fibrosis, cirrhosis, liver failure, hepatocarcinoma, and such. The above diseases may occur in combination with other diseases.

In another preferred embodiment, the present invention relates to hepatic fibrosis-suppressing agents comprising chondroitinase ABC as an active ingredient.

Chondroitinase ABC is a lyase classified under enzyme number EC 4.2.2.4 (chondroitin ABC lyase). Chondroitinase ABC has an activity of removing polysaccharides that have 1,4-β-D-hexosaminide linkage, and 1,3-β-D-glucuronosyl linkage or 1,3-α-L-iduronosyl linkage by degrading them to disaccharides with a 4-deoxy-β-D-gluc-4-enuronosyl group. Chondroitinase ABC acts on and degrades chondroitin sulfate A (chondroitin-4-sulfate), chondroitin sulfate B (dermatan sulfate), chondroitin sulfate C (chondroitin-6-sulfate), and hyaluronic acid.

Chondroitinase ABC used in the present invention preferably includes, for example, bacteria of the genus *Proteus,* such as *Proteus vulgaris,* and bacteria of the genus *Bacteroides,* such as *Bacteroides stercoris,* but is not limited thereto. More preferably, chondroitinase ABC used in the present invention is derived from *Proteus vulgaris.* Chondroitinase ABC of the present invention may be a sample purified from extracts of *Proteus vulgaris* by ammonium sulfate precipitation and DEAE-cellulose chromatography (Yamagata, T. et al., J. Biol. Chem. 1968, 243, 1523-1535). The *Proteus* vulgaris-derived chondroitinase ABC of the present invention includes, for example, proteins comprising the amino acid sequence of SEQ ID NO: 9 disclosed in Japanese Patent Application Kokai Publication No. (JP-A) H06-098769 (unexamined, published Japanese patent application) and proteins encoded by the nucleotide sequence deposited under Accession No. E07183 in the international nucleotide sequence database DDBJ/EMBL/GenBank (for example, by accessing the web site of the DNA Data Bank of Japan (DDBJ): http://www.ddbj.nig.ac.jp/Welcome-j.html). It is preferred that chondroitinase ABC of the present invention at least has an activity of degrading chondroitin sulfate A (chondroitin-4-sulfate), chondroitin sulfate B (dermatan sulfate), chondroitin sulfate C (chondroitin-6-sulfate), and hyaluronic acid.

Commercially available products of chondroitinase ABC, for example, chondroitinase ABC *(Proteus vulgaris,* Seikagaku Co., Tokyo, Japan) and chondroitinase ABC *(Proteus vulgaris,* Sigma-Aldrich, Saint Louis, Missouri, USA) can be used in the present invention. Alternatively, those skilled in the art can readily produce chondroitinase ABC of the present invention by expressing a protein from chondroitinase ABC-encoding DNA (for example, DNA comprising the nucleotide sequence deposited under Accession No. E07183) using recombinant techniques. Conventional molecular biology methods, such as recombinant techniques, are described in detail, for example, in Molecular Cloning: A Laboratory Manual/Volume 3, Joseph Sambrook and David W Russell. 3rd Ed., New York, Cold Spring Harbor Laboratory Press, 2001, and so on.

The hepatic fibrosis-suppressing agents of the present invention comprising chondroitinase ABC as an active ingredient can effectively suppress liver tissue fibrosis (hepatic fibrosis). Herein, "suppressing fibrosis" means reducing or eliminating fibrotic lesions in fibrotic tissues, or retarding or inhibiting further advancement of fibrosis (suppressing the expansion of fibrotic lesions).

The degree of fibrosis in liver tissues can be evaluated by various methods known to those skilled in the art. In the most fundamental method, fibrosis may be evaluated by histologically observing fibrotic findings in liver biopsy, for example, images of fibrotic tissues emphasized by special staining (aniline blue stain, trichrome stain, silver stain, and such) in liver biopsy samples. Specifically, evaluation of fibrosis can be achieved, for example, by presenting the degree of hepatic fibrosis in each sample measured by immunohistochemical staining in terms of a fibrosis score according to Dai K, et al., World J Gactroenterol. 2005, 31, 4822-4826; or Hillebrandt S, et al., Nature Genetics 2005, 37, 835-843. Alternatively, the degree of hepatic fibrosis in liver tissues may be evaluated more simply by using hepatic fibrosis markers, such as hyaluronic acid, type I, III, and IV collagens and other collagens, fibroblasts, and macrophages. Simple tests that comprise determining the platelet count, which sensitively reflects the degree of hepatic fibrosis, can also be conveniently used. Alternatively, the degree of hepatic fibrosis can also be roughly estimated by image diagnosis of liver, such as abdominal ultrasonographic examination. Alternatively, the degree of hepatic fibrosis can also be evaluated using a measurement device (FibroScan 502, or such) for a non-invasive method that examines hepatic fibrosis based on the transient elastography technology recently developed by EchoSens (France). The degree of suppression of hepatic fibrosis by a hepatic fibrosis-suppressing agent of the present invention comprising as an active ingredient chondroitinase ABC may be determined based on evaluation of the degree of hepatic fibrosis by the methods described above.

The hepatic fibrosis-suppressing effect of hepatic fibrosis-suppressing agents of the present invention comprising chondroitinase ABC as an active ingredient is preferably accompanied by in particular, suppression of at least one of type III collagen elongation, type I collagen deposition, fibroblast accumulation, and macrophage accumulation in liver tissues, and more preferably suppression of all of them. The degree of suppression of hepatic fibrosis by the hepatic fibrosis-suppressing agents of the present invention comprising chondroitinase ABC as an active ingredient may be determined using one or more of the suppression effects described above as an indicator.

The present invention is based on a completely novel finding that chondroitinase ABC, which is an active ingredient in the hepatic fibrosis-suppressing agents of the present invention that comprise chondroitinase ABC as an active ingredient, produces a very strong hepatic fibrosis-suppressing effect, as demonstrated in the Examples described below. The hepatic fibrosis-suppressing effect of chondroitinase ABC is far superior when compared to other chondroitinases. This superior effect of chondroitinase ABC can be attributed to the fact that the type and quantity of chondroitin sulfate that is difficult to be cleaved by chondroitinase AC, chondroitinase B, or such accumulates in fibrotic liver tissues, and is for the first time successfully cleaved by only chondroitinase ABC. In recent years, various types of chondroitin sulfates have been reported, including chondroitin sulfates with varying degrees of sulfation and chondroitin sulfates having sulfate groups at different positions. Therefore, the hypothesis described above is highly convincing. However, the present invention is not limited to such hypothesis.

According to the present invention, the degree of fibrosis in liver tissues can be reduced by adding and reacting a hepatic fibrosis-suppressing agent of the present invention comprising chondroitinase ABC as an active ingredient to liver tissue samples.

According to the present invention, fibrosis of liver tissues in a subject can be effectively suppressed by administering a hepatic fibrosis-suppressing agent of the present invention comprising chondroitinase ABC as an active ingredient to the subject. There is no limitation on the administration route of the hepatic fibrosis-suppressing agents comprising chondroitinase ABC as an active ingredient; however, parenteral route is preferred. Preferred examples of such parenteral routes include intravenous, intraarterial, intraperitoneal, intrahepatic, intrarectal, and subcutaneous routes. The hepatic fibrosis-suppressing agents of the present invention comprising chondroitinase ABC as an active ingredient may be administered systemically or locally (for example, administered directly to fibrotic liver tissues).

The present invention also relates to methods for suppressing liver fibrosis in subjects, which comprise administering a hepatic fibrosis-suppressing agent of the present invention comprising chondroitinase ABC as an active ingredient.

Furthermore, the hepatic fibrosis-suppressing agents of the present invention comprising chondroitinase ABC as an active ingredient can effectively suppress liver tissue fibrosis, and thus can be used advantageously to treat and prevent fibrotic liver diseases (diseases with excessive fibrosis of liver tissues). In the present invention, more preferred examples of fibrotic liver diseases include, but are not limited to, chronic liver diseases. Chronic liver diseases generally involve hepatic fibrosis. Specifically, such chronic liver diseases of the present invention include, for example, chronic hepatitis (chronic hepatitis B, chronic hepatitis C, and such), liver fibrosis, cirrhosis, liver failure, and hepatocarcinoma. In particular, the hepatic fibrosis-suppressing agents of the present invention comprising chondroitinase ABC as an active ingredient are useful in treating and preventing cirrhosis. Herein, "cirrhosis" refers to a liver condition where the liver is extensively fibrotic and is altered into an aberrant hepatic lobular structure (pseudolobule/regenerating nodule) after losing its normal hepatic lobular structure. Cirrhosis has been divided into many types depending on the cause of disease, characteristic features of pseudolobule, and so on. Furthermore, a liver in such conditions is called a cirrhotic liver. Meanwhile, "liver fibrosis" is a condition showing aberrant fibrosis but no alteration of hepatic lobule, and is in general assumed to be a preliminary stage of cirrhosis. Specifically, cirrhosis of the present invention includes, but is not limited to, for example, viral cirrhosis, parasitic cirrhosis, toxic cirrhosis, trophopathic cirrhosis, alcoholic cirrhosis, cardiac cirrhosis, hepatic sclerosis, Charcot's cirrhosis, Todd's cirrhosis, primary biliary cirrhosis, secondary biliary cirrhosis, monolobular cirrhosis, cirrhosis resulting from chronic non-suppurative destructive cholangitis, obstructive cirrhosis, cholangiolitic cirrhosis, biliary cirrhosis, atrophic cirrhosis, postnecrotic cirrhosis, posthepatitic cirrhosis, nodular cirrhosis, mixed cirrhosis, micronodular cirrhosis, compensatory cirrhosis, non-compensatory cirrhosis, macronodular cirrhosis, septal cirrhosis, cryptogenic cirrhosis, periportal cirrhosis, and portal cirrhosis.

Preferred subjects to be administered with the hepatic fibrosis-suppressing agents of the present invention comprising chondroitinase ABC as an active ingredient are mammals, including humans, domestic animals, pets, and experimental animals. In particular, mammals (patients) with fibrotic liver diseases, for example, cirrhosis and liver fibrosis, are preferred subjects of the present invention. In another embodiment, mammals (patients) with fibrotic liver diseases that tend to progress to cirrhosis (for example, chronic non-suppurative destructive cholangitis) are also preferred subjects. The present invention reduces the degree of liver fibrosis, or retards or inhibits the progression of fibrosis by administering a hepatic fibrosis-suppressing agent of the present invention comprising chondroitinase ABC as an active ingredient to subjects. As a result, liver damages caused by fibrosis are reduced to improve the liver function or keep it stronger. Thus, the present invention can treat or prevent fibrotic liver diseases.

The dosage of the hepatic fibrosis-suppressing agents of the present invention comprising chondroitinase ABC as an active ingredient can be determined by those skilled in the art. In general, the dosage is equivalent to 10 µg to 1 mg chondroitinase ABC/1 kg body weight.

Thus, the present invention also relates to methods for treating or preventing fibrotic liver diseases (in particular, cirrhosis and such), including chronic liver diseases, which comprise administering the hepatic fibrosis-suppressing agents of the present invention comprising chondroitinase ABC as an active ingredient to subjects (for example, patients).

The hepatic fibrosis-suppressing agents of the present invention comprising chondroitinase ABC as an active ingredient may be pharmaceutical compositions appropriately comprising pharmaceutically acceptable (pharmaceutically inactive) carriers, excipients, and/or diluents, in addition to chondroitinase ABC. Such carriers, excipients, and/or diluents include, but are not limited to, for example, water, physiological saline, phosphate buffered saline, polyvinyl alcohol, polyvinylpyrrolidone, carboxyvinyl polymer, sodium alginate, water-soluble dextran, pectin, xanthan gum, gum Arabic, gelatin, agar, glycerin, propylene glycol, polyethylene glycol, vaseline, paraffin, stearyl alcohol, stearic acid, human serum albumin, mannitol, sorbitol, and lactose. The pharmaceutical compositions of the present invention may further comprise additives, such as preservatives. The pharmaceutical compositions of the present invention may further comprise other pharmacological ingredients.

The pharmaceutical composition of the present invention may be provided as oral or parenteral preparations; however they are preferably provided as parenteral preparations. Preferred parenteral preparations are liquid preparations such as liquids and suspensions. Injections are especially preferred.

The present invention also provides methods of screening for hepatic fibrosis-suppressing agents, wherein the methods comprise selecting, from test samples, substances with the activity of inhibiting the production or accumulation of chondroitin sulfate proteoglycans. Hepatic fibrosis-suppressing agents or candidate compounds for hepatic fibrosis-suppressing agents can be efficiently obtained using the screening methods of the present invention.

Preferred embodiments of the screening methods of the present invention are methods of screening for hepatic fibrosis-suppressing agents that comprise the step of selecting substances with any of the activities (a) to (d):
(a) promoting the degradation of chondroitin sulfate proteoglycans;
(b) inhibiting the synthesis of chondroitin sulfate proteoglycans;
(c) desulfating chondroitin sulfate proteoglycans; and
(d) inhibiting the sulfation of chondroitin sulfate proteoglycans.

Representative examples based on fundamental principles common in screening for these substances include methods comprising the following procedure:
A preferred procedure uses tools (1) to (3) below, and is as follows: (1) and (2) are mixed in a test tube or culture dish, and the resulting effect is simply detected using (3).
   (1) chondroitin sulfate proteoglycans (CSPGs) themselves, or glycosaminoglycans (GAG) chains, or cells synthesizing (producing) CSPGs or GAG chains
   (2) test compounds (for example, enormous compound libraries owned by pharmaceutical companies)
   (3) methods for detecting CSPG cleavage sites, the amount of CSPGs, or the amount of free glycosaminoglycans (GAGs)

Embodiments of the screening methods of the present invention are exemplified below. In the embodiments described below, the chondroitin sulfate proteoglycans, synthetases, compounds suppressing desulfation enzymes, sulfotransferases, degradation-promoting enzymes, and desulfation enzymes to be used include those derived from humans, mice, rats, and others, but are not limited thereto. Chondroitin sulfate proteoglycan portions are components such as glycosaminoglycan chains or core proteins, or portions thereof. The chondroitin sulfate proteoglycan portions are not particularly limited.

The test compounds to be used in the embodiments described below are not particularly limited, but include, for example, single compounds, such as natural compounds, organic compounds, inorganic compounds, proteins, and peptides, as well as compound libraries, expression products of gene libraries, cell extracts, cell culture supernatants, products of fermenting microorganisms, extracts of marine organisms, and plant extracts.

In the embodiments described below, the "contact" with test compounds is typically achieved by mixing the test compounds with chondroitin sulfate proteoglycans, portions thereof, synthetases, compounds suppressing desulfation enzymes, sulfotransferases, degradation-promoting enzymes, or desulfation enzymes, but the "contact" is not limited to this methods. For example, the "contact" can also be achieved by contacting test compounds with cells expressing these proteins or portions thereof.

In the embodiments described below, the "cells" include those derived from humans, mice, rats, and such, but are not limited thereto. Cells of microorganisms, such as *Escherichia coli* and yeasts, which are transformed to express the proteins used in each embodiment, can also be used. For example, the "cells that express chondroitin sulfate proteoglycans" include cells that express endogenous genes for chondroitin sulfate proteoglycans, and cells that express introduced foreign genes for chondroitin sulfate proteoglycans. Such cells that express foreign genes for chondroitin sulfate proteoglycans can typically be prepared by introducing host cells with expression vectors carrying a chondroitin sulfate proteoglycan gene as an insert. The expression vectors can be prepared using standard genetic engineering techniques.

The "chondroitin sulfate proteoglycan core proteins" described below include, for example, core proteins of matrix-type chondroitin sulfate proteoglycans, such as aggrecan, versican, neurocan, and brevican, and core proteins of membrane chondroitin sulfate proteoglycans, such as decorin, biglycan, fibromodulin, and PG-Lb. The "synthetases" include, for example, GalNAc4ST-1, GalNAc4ST-2, GALNAC4S-6ST, UA2OST, GalT-I, GalT-II, GIcAT-1, and XylosylT. The "sulfotransferases" include, for example, chondroitin D-N-acetylgalactosamine-4-O-sulfotransferase 1 (C4ST-1), chondroitin D-N-acetylgalactosamine-4-O-sulfotransferase 2 (C4ST-2), chondroitin D-N-acetylgalactosamine-4-O-sulfotransferase 3 (C4ST-3), D4ST, C6ST-1, and C6ST-2. The "degradation-promoting enzymes" include, for example, ADAMTS-1, ADAMTS-4, ADAMTS-5, chondroitinase ABC (ChABC), chondroitinase AC, chondroitinase B, and calpain I. The "desulfation enzymes" include, for example, chondroitin-4-sulfatase and chondroitin-6-sulfatase.

Embodiments of the screening methods of the present invention include methods comprising the step of selecting compounds that have the activity of promoting the degradation of chondroitin sulfate proteoglycans. An example of the above-mentioned methods of the present invention comprises the steps of:
(a) contacting test compounds with chondroitin sulfate proteoglycans or portions thereof;
(b) measuring the abundance of chondroitin sulfate proteoglycans or portions thereof; and
(c) selecting substances that reduce the abundances as compared with those determined in the absence of the test compounds.

In the above methods, first, test compounds are contacted with chondroitin sulfate proteoglycans or portions thereof.

In these methods, the amount of the chondroitin sulfate proteoglycans or portions thereof is then measured. The measurement can be conducted by methods known to those skilled in the art. For example, the amounts can be detected using labeled compounds or antibodies that bind to the chondroitin sulfate proteoglycans or portions thereof, and then measuring the amount of the label. Alternatively, the detection can be achieved by chromatography or mass spectrometry.

In these methods, compounds that reduce the abundance of the chondroitin sulfate proteoglycans or portions thereof as compared with in the absence of a test compound (the control) are then selected. Compounds resulting in a reduction can be used as therapeutic agents for hepatic fibrosis.

Below is a brief illustrative example of the methods able to assess (measure) whether a test compound has the above activity (a): the activity of promoting the degradation of chondroitin sulfate proteoglycans.
Embodiment of the methods for screening for the above activity (a) of promoting the degradation of chondroitin sulfate proteoglycans:
A CS-GAG, such as chondroitin sulfate A (CS-A), CS-B, CS-C (Seikagaku Co., ICN, Sigma, and others), or human-derived proteoglycan (BGN, ISL, and others), is prepared, and 96-well plates are coated with it at a concentration of 10 µg/ml (using known methods, such as in Kawashima, H. et al., J. Biol. Chem. 2002, 277, 12921-12930). Various test compounds are added to each well of the plates. After two hours of reaction at 37°C, changes in CS-GAG are detected.

Detection methods include, for example, the simple WFA lectin (Wisteria floribunda lectin)-binding method. Since WFA lectin binds to the GalNAc residues of CS-GAG chains, it can easily detect CS-GAGs. Chondroitinase ABC is used as a positive control for test compounds. The principle behind this use of chondroitinase ABC is that its addition degrades CS-GAG chains, making it impossible for WFA lectin to bind them. More specifically, FITC-labeled WFA lectin (EY Co.) is added to the CS-coated wells before and after mixing the test compounds, and changes in the intensity of FITC fluorescence in the wells due to the CS-GAG degradation can be quantified and digitized very simply by using detection devices, such as fluorescence plate readers or fluorescence microscopes. Compounds whose addition most reduces fluorescent values may be determined to be novel therapeutic candidate compounds that fulfill the concept of the present invention.

In an alternative detection method, anti-CS antibody (clone CS56, Seikagaku Co.) can be used to directly label CS-GAGs. As with WFA lectin, large-scale screening can be carried out simply and in very short time by adding FITC-labeled anti-CS antibody to CS-coated wells and examining changes in fluorescence value.

In more specific detection methods, GAG content is accurately quantified and digitized by simply using the plates before and after mixing of test compounds in an sGAG Assay Kit (Wieslab Co.), an ELISA Kit for Sulphanated Glycosaminoglycans (Funakoshi Co.), or such.

More specifically, the reducing ends of free GAG chains can easily be fluorescently labeled by adding 2-aminobenzamide, 2-aminopyridine (2-AB and 2-AP, respectively; LUD Co. and others), or the like to the plates before and after mixing of test compounds, which enables more specific analysis using HPLC, MALDI-MS, LC-MS, or such to determine the types of sugar chains and even the content of each type of chain. These methods, which examine the properties of candidate compounds in detail, take screening to the next level.

Other embodiments of the screening methods of the present invention include methods comprising the step of selecting substances with the activity of inhibiting the synthesis of chondroitin sulfate proteoglycans. These methods of the present invention comprise, for example, the steps of:
(a) contacting test compounds with cells expressing chondroitin sulfate proteoglycans or portions thereof, extracts of these cells, or groups of substances including those enzymes and substrates constituting the process of chondroitin sulfate proteoglycan synthesis;
(b) measuring the amount of synthesized chondroitin sulfate proteoglycans or intermediates thereof in the above-mentioned cells, cell extracts, or group of substances; and
(c) selecting compounds that reduce the amount as compared to in the absence of the test compounds.

In the above methods, test compounds are first contacted with cells expressing chondroitin sulfate proteoglycans or portions thereof, extract of these cells, or groups of substances including those enzymes and substrates that constitute the process of chondroitin sulfate proteoglycan synthesis.

Next, the amount of synthesized chondroitin sulfate proteoglycans or intermediates thereof is measured. The measurement can be performed by those skilled in the art using known methods; for example, methods using labeled antibodies, mass spectrometry, and chromatography can be used.

Further, compounds that reduce (suppress) the synthesized amount as compared with in the absence of the test compounds (the control) are selected. Compounds resulting in a reduction (suppression) can be used as therapeutic agents for hepatic fibrosis.

Below is a brief illustrative example of the methods able to assess (measure) whether a test compound has the above activity (b): the activity of inhibiting the synthesis of chondroitin sulfate proteoglycans.
Embodiment of the methods for screening for the above activity (b) of inhibiting the synthesis of chondroitin sulfate proteoglycans:
Cells and cell lines synthesizing chondroitin sulfate are known to researchers in the art. In human, for example, chondroitin sulfate is produced after 16 hours of cell culture by standard methods for culturing mononuclear cells isolated from peripheral blood collected from healthy subjects (Uhlin-Hansen, L. et al., Blood 1993, 82, 2880; etc.). Alternatively, for more convenience, there are many examples of known cell; for example, the fibroblast cell line NIH3T3 (Phillip, H.A. et al., J. Biol. Chem. 2004, 279, 48640; etc.); the renal tubule-derived cancer cell line ACHN (Kawashima, H. et al., J. Biol. Chem. 2002, 277, 12921), the renal distal tubule-derived cell line MDCK (Borges, F.T. et al., Kidney Int. 2005, 68, 1630; etc.), and the vascular endothelial cell line HUVEC (Schick, B.P. et al., Blood 2001, 97, 449; etc.). Various test compounds are added during the process of culturing such cell lines for set periods, and changes in the amount of CS-GAG before and after culture can be easily evaluated by the above-described method of (a). Compounds that suppress the increase in the amount of CS-GAG after culture (which thus reflects the amount of synthesized CS-GAG) can be easily determined to be candidate therapeutic compounds that fulfill the concept of the present invention.

As a further option, cell lines constitutively expressing the genes for CS-GAG synthetases such as GalNAc4ST-1 and XylosylT can be prepared by introducing the genes into CHO cells, L cells, or such by well-known methods. The use of such cell lines that constitutively synthesize CS-GAG allows more clear determination of candidates for therapeutic compounds.

In another embodiment, the screening methods of the present invention include methods comprising the step of selecting substances with the activity of desulfating chondroitin sulfate proteoglycans. The above methods of the present invention comprise, for example, the steps of:
(a) contacting test compounds with chondroitin sulfate proteoglycans or portions thereof;
(b) measuring the amount of sulfation in the chondroitin sulfate proteoglycans or portions thereof; and
(c) selecting substances that reduce the amount of sulfation as compared with in the absence of the test compounds.

In the above methods, test compounds are first contacted with chondroitin sulfate proteoglycans or portions thereof.

Next, the amount of sulfation in the chondroitin sulfate proteoglycans or portions thereof is measured. The measurement can be conducted using methods known to those skilled in the art. For example, the amount of sulfation can be determined by using labeled compounds or antibodies that bind to the desulfated structures remaining in the chondroitin sulfate proteoglycans or portions thereof, and measuring the amount of the label. Alternatively, the measurement can be achieved by chromatography or mass spectrometry or such.

Then, in the present methods compounds that reduce the abundances of the chondroitin sulfate proteoglycans or portions thereof as compared with in the absence of the test compounds (the control) are selected. Compounds resulting in a reduction can be used as therapeutic agents for hepatic fibrosis.

Below is a brief illustrative example of the methods able to assess (measure) whether a test compound has the above activity (c): the activity of desulfating chondroitin sulfate proteoglycans.
Embodiment of the methods for screening for the above activity (c) of desulfating chondroitin sulfate proteoglycans:
By using essentially the same method as described above in (a), human-derived proteoglycans (BGN Co., ISL Co., etc.) or such are prepared and coated on to 6-well plates at a concentration of 10 µg/ml (by known methods, such as those described in Kawashima, H. et al., J. Biol. Chem. 2002, 277, 12921-12930). Various test compounds are added to each well of the plates, and alterations in CS-GAG are detected after two hours of reaction at 37°C.

In this detection method, desulfated moieties can be easily detected using the reaction of either anti-proteoglycan Δdi4S antibody (clone: 2-B-6, which recognizes sulfated moieties at position 4) or anti-proteoglycan Δdi6S antibody (clone: 3-B-3, which recognizes sulfated moieties at position 6) (both from Seikagaku Co.) with the disaccharide structure of the desulfated fragments that remain in the core protein of proteoglycans after desulfation. Thus, FITC-labeled 2-B-6 or 3-B-3 antibody is reacted in such plates before and after mixed culture, and changes in the fluorescence value can be simply detected. Compounds whose addition increases the fluorescence intensity can be determined to be substances that promote desulfation, and are easily identified as novel candidate therapeutic compounds that fulfill the concept of the present invention.

Another embodiment of the screening methods of present invention includes methods comprising the step of selecting substances with the activity of inhibiting the sulfation of chondroitin sulfate proteoglycans. The above methods of the present invention comprise, for example, the steps of:
(a) contacting test compounds with cells expressing chondroitin sulfate proteoglycans or portions thereof, extracts of these cells, or groups of substances including those enzymes and substrates constituting the process of sulfation of chondroitin sulfate proteoglycans;
(b) measuring the activity of sulfation of chondroitin sulfate proteoglycans in the above-mentioned cells, cell extracts, or groups of substances; and
(c) selecting compounds that reduce the activity as compared with in the absence of the test compounds.

In the above methods, test compounds are first contacted with chondroitin sulfate proteoglycans or portions thereof.

Next, the amount of sulfation in the chondroitin sulfate proteoglycans or portions thereof is measured. The measurement can be conducted using methods known to those skilled in the art. For example, the amount of sulfation can be determined by using labeled compounds or antibodies that bind to the sulfated structures of the chondroitin sulfate proteoglycans or portions thereof, and measuring the amount of the label. Alternatively, the measurement can be achieved by chromatography or mass spectrometry and such.

Then, compounds that reduce the abundance of the chondroitin sulfate proteoglycans or portions thereof as compared with in the absence of the test compounds (the control) are selected. Compounds resulting in a reduction can be used as therapeutic agents for hepatic fibrosis.

Below is a brief illustrative example of the methods able to assess (measure) whether a test compound has the above activity (d): the activity of inhibiting the sulfation of chondroitin sulfate proteoglycans.
Embodiment of the methods for screening for the above activity (d) of inhibiting the sulfation of chondroitin sulfate proteoglycans:
The cells and cell lines that promote the sulfation of chondroitin sulfates are the same as described above in (c). Various test compounds are mixed during a set period of culture of such cell lines, and the degree of sulfation before and after the culture can be easily determined by, for example, using an antibody that recognizes sulfation at position 4 (clone: LY111) or an antibody that recognizes sulfation at position 6 (clone: MC21C) (both from Seikagaku Co.). Fluorescence values may be compared between before and after the culture by using fluorescently labeled antibodies. Alternatively, the same detection method as described above in (c) can be conducted using 2-B-6 or 3-B-3 antibodies before and after culture. Compounds that suppress an increase in the sulfation after cell culture (an increase in the fluorescence value for LY111 or MC21C), or compounds that promote the progression of desulfation after cell culture (an increase in the fluorescence value for 2-B-6 or 3-B-3) can be easily determined to be candidate therapeutic compounds that fulfill the concept of the present invention.

As a further option, cell lines that constitutively express sulfotransferase genes such as C4ST- 1 and C6ST- 1 can be prepared by introducing the genes into CHO cells, L cells, or such by well-known methods. The use of such cell lines that constitutively add sulfate groups allows more clear determination of candidates for therapeutic compounds.

Other preferred embodiments of the present invention are methods of screening for hepatic fibrosis-suppressing agents in which compounds that reduce the expression level of a gene encoding a CSPG core protein, synthetase, desulfation enzyme-suppressing protein, or sulfotransferase of the present invention, or compounds that increase the expression level of a gene for an enzyme that desulfates CSPGs or promotes the degradation of CSPGs, are selected; wherein the method comprises the steps of:
(a) contacting test compounds with cells expressing a gene encoding a CSPG core protein, synthetase, desulfation enzyme-suppressing protein, sulfotransferase, degradation-promoting enzyme, or desulfating enzyme;
(b) determining the expression level of the gene in the cells;
(c) comparing the expression level with that in the absence of the test compounds (the control); and
(d) selecting compounds that reduce the expression level of the gene of the CSPG core protein, synthetase, desulfation enzyme-suppressing protein, or sulfotransferase as compared with the control, or compounds that increase the expression level of the gene of the CSPG desulfating enzyme or the CSPG degradation-promoting enzyme as compared with the control.

In the above methods, test compounds are first contacted with cells expressing a gene encoding a CSPG core protein, synthetase, desulfating enzyme-suppressing protein, sulfotransferase, degradation-promoting enzyme, or desulfating enzyme.

Next, the expression level of the gene encoding the core protein, synthetase, desulfating enzyme-suppressing protein, sulfotransferase, degradation-promoting enzyme, or desulfating enzyme is measured. Herein, "expression of the gene" includes both transcription and translation. Gene expression level can be measured by methods known to those skilled in the art.

For example, mRNAs are extracted from cells expressing any one of the above-described proteins by conventional methods, and these mRNAs can be used as templates in Northern hybridization, RT-PCR, DNA arrays, or such to measure the transcription level of the gene. Alternatively, protein fractions are collected from cells expressing a gene encoding any of the above-described proteins, and expression of the protein can be detected by electrophoresis such as SDS-PAGE to measure the level of gene translation. Alternatively, the level of gene translation can be measured by detecting the expression of any of the above-described proteins by Western blotting using an antibody against the proteins. Such antibodies for use in detecting the proteins are not particularly limited, as long as they are detectable. For example, both monoclonal and polyclonal antibodies can be used.

Next, the expression level is compared with that in the absence of the test compounds (the control).

Then, when the gene encodes a CSPG core protein, synthetase, desulfating enzyme-suppressing protein, or sulfotransferase, compounds that reduce (suppress) the expression level of the gene as compared with a control are selected. The compounds resulting in a reduction (suppression) can be agents for suppressing hepatic fibrosis or candidate compounds for treating hepatic fibrosis.

Alternatively, when the gene encodes a CSPG desulfating enzyme or an enzyme promoting CSPG degradation, compounds that increase (enhance) the expression level of the gene as compared with a control are selected. Compounds resulting in an increase (enhancement) can be agents for suppressing hepatic fibrosis or candidate compounds for treating hepatic fibrosis.

An embodiment of the screening methods of the present invention is a method in which compounds that reduce the expression level of a gene encoding a CSPG core protein, synthetase, desulfation enzyme-suppressing protein, or sulfotransferase of the present invention, or compounds that increase the expression level of a gene for a CSPG degradation-promoting enzyme or a CSPG desulfating enzyme, can be selected using the expression of a reporter gene as an indicator. The above methods of the present invention comprise, for example, the steps of:
(a) contacting test compounds with cells or cell extracts containing a DNA structured such that a reporter gene is operably linked to a transcriptional regulatory region of a gene encoding a CSPG core protein, synthetase, desulfating enzyme-suppressing protein, sulfotransferase, degradation-promoting enzyme, or desulfating enzyme;
(b) measuring the expression level of the reporter gene;
(c) comparing the level with the that in the absence of the test compounds (the control); and
(d) selecting compounds that reduce the expression level of the reporter gene as compared with the control when the reporter gene is operably linked with a transcriptional regulatory region of the gene encoding a CSPG core protein, synthetase, desulfating enzyme-suppressing protein, or sulfotransferase; or selecting compounds that increase the expression level of the reporter gene as compared with the control when the reporter gene is operably linked with a transcriptional regulatory region of a gene encoding a CSPG degradation-promoting enzyme or a CSPG desulfating enzyme.

In the above methods, test compounds are first contacted with cells or cell extracts containing DNAs structured such that a reporter gene is operably linked with a transcriptional regulatory region of a gene encoding a CSPG core protein, synthetase, desulfating enzyme-suppressing protein, sulfotransferase, degradation-promoting enzyme, or desulfating enzyme.

Herein, "operably linked" means that a reporter gene is linked with a transcriptional regulatory region of a gene encoding a CSPG core protein, synthetase, desulfating enzyme-suppressing protein, sulfotransferase, degradation-promoting enzyme, or desulfating enzyme, such that expression of the reporter gene is induced upon binding of transcriptional factors to the transcriptional regulatory region. Therefore, the meaning of "operably linked" also includes cases where a reporter gene is linked with a different gene and produces a fusion protein with a different gene product, as long as expression of the fusion protein is induced upon the binding of transcriptional factors to the transcriptional regulatory region of the gene encoding the CSPG core protein, synthetase, desulfating enzyme-suppressing protein, sulfotransferase, degradation-promoting enzyme, or desulfating enzyme. Those skilled in the art can obtain the transcriptional regulatory regions of genes encoding CSPG core proteins, synthetases, desulfating enzyme-suppressing proteins, sulfotransferases, degradation-promoting enzymes, or desulfating enzymes that are present in the genome, based on the cDNA nucleotide sequences of the genes encoding the CSPG core proteins, synthetases, desulfating enzyme-suppressing proteins, sulfotransferases, degradation-promoting enzymes, or desulfating enzymes.

The reporter genes for use in these methods are not particularly limited, as long as their expression is detectable. The reporter genes include, for example, the CAT gene, the lacZ gene, the luciferase gene, and the GFP gene. The "cells containing a DNA structured such that a reporter gene is operably linked with a transcriptional regulatory region of a gene encoding a CSPG core protein, synthetase, desulfating enzyme-suppressing protein, sulfotransferase, degradation-promoting enzyme, or desulfating enzyme" include, for example, cells introduced with vectors carrying such structures as inserts. Such vectors can be prepared by methods well known to those skilled in the art. The vectors can be introduced into cells by standard methods, for example, calcium phosphate precipitation, electroporation, lipofection, and microinjection. The "cells containing a DNA structured such that a reporter gene is operably linked with a transcriptional regulatory region of a gene encoding a CSPG core protein, synthetase, desulfating enzyme-suppressing protein, sulfotransferase, degradation-promoting enzyme, or desulfating enzyme" include cells in which the structure has been integrated into the chromosomes. A DNA structure can be integrated into chromosomes by methods generally used by those skilled in the art, for example, gene transfer methods using homologous recombination.

The "cell extracts containing a DNA structured such that a reporter gene is operably linked with a transcriptional regulatory region of a gene encoding a CSPG core protein, synthetase, desulfating enzyme-suppressing protein, sulfotransferase, degradation-promoting enzyme, or desulfating enzyme" include, for example, mixtures of cell extracts included in commercially available *in vitro* transcription-translation kits and DNAs structured such that a reporter gene is operably linked with the transcriptional regulatory region of the gene encoding a CSPG core protein, synthetase, desulfating enzyme-suppressing protein, or sulfotransferase.

"Contact" can be achieved by adding test compounds to a culture medium of "cells containing a DNA structured such that a reporter gene is operably linked with a transcriptional regulatory region of a gene encoding a CSPG core protein, synthetase, desulfating enzyme-suppressing protein, sulfotransferase, degradation-promoting enzyme, or desulfating enzyme", or by adding test compounds to the above-described commercially available cell extracts containing the DNAs. When the test compound is a protein, contact may also be achieved, for example, by introducing a DNA vector expressing the protein into the cells.

In the above methods, the expression level of the reporter gene is then measured. The expression level of the reporter gene can be measured by methods known to those skilled in the art, depending on the type of the reporter gene. When the reporter gene is the CAT gene, its expression can be determined, for example, by detecting the acetylation of chloramphenicol by the gene product. When the reporter gene is the lacZ gene, its expression level can be determined by detecting the color development of chromogenic compounds due to the catalytic action of the gene expression product. Alternatively, when the reporter gene is the luciferase gene, its expression level can be determined by detecting the fluorescence of fluorogenic compounds due to the catalytic action of the gene expression product. Furthermore, when the reporter gene is the GFP gene, its expression level can be determined by detecting the fluorescence of the GFP protein.

In the above methods, the expression level of the reporter gene is then compared with that in the absence of the test compounds (the control).

In the present methods, compounds that reduce (suppress) the expression level of a reporter gene as compared with a control are then selected, where the reporter gene is operably linked with a gene encoding a CSPG core protein, synthetase, desulfating enzyme-suppressing protein, or sulfotransferase. Compounds resulting in a reduction (suppression) can be agents for suppressing hepatic fibrosis or candidate compounds for treating hepatic fibrosis.

Alternatively, when the reporter gene is operably linked with a gene encoding a CSPG degradation-promoting enzyme or CSPG desulfating enzyme, compounds that increase (enhance) the reporter gene expression level as compared with a control are selected. Compounds resulting in an increase (enhancement) can be agents for suppressing hepatic fibrosis or candidate compounds for treating hepatic fibrosis.

The hepatic fibrosis-suppressing agents that are found by the screening methods of the present invention are preferably therapeutic or preventive agents for fibrotic liver diseases.

The present invention also provides kits comprising various agents, reagents, and the like, which are used to conduct the screening methods of the present invention.

The kits of the present invention can be prepared, for example, by selecting adequate reagents from the above-described various reagents, depending on the screening method to be conducted. The kits of the present invention may contain, for example, the chondroitin sulfate proteoglycans of the present invention. The kits of the present invention may further contain various reagents, vessels, and the like to be used in the methods of the present invention. The kits may appropriately contain, for example, anti-chondroitin sulfate proteoglycan antibodies, probes, various reaction reagents, cells, culture media, control samples, buffers, and instruction manuals containing a description of how to use the kits.

Preferred embodiments of the present invention are the methods of screening for hepatic fibrosis-suppressing agents, comprising the step of detecting whether the production or accumulation of chondroitin sulfate proteoglycans is inhibited. Thus, the kits for screening for the hepatic fibrosis-suppressing agents of the present invention may contain, for example, oligonucleotides such as probes for the genes encoding CSPG core proteins, and primers to amplify certain regions of these genes; and antibodies recognizing CSPGs (anti-chondroitin sulfate proteoglycan antibodies), which can be used to detect chondroitin sulfate proteoglycans.

The above-described oligonucleotides specifically hybridize to, for example, DNAs of the genes encoding the versican core protein of the present invention. Herein, "specifically hybridize to" means that the oligonucleotides do not significantly cross-hybridize to DNAs encoding other proteins under standard hybridization conditions, and preferably under stringent hybridization conditions (for example, the conditions described in Sambrook, J. et al. "Molecular Cloning" 2nd Ed., Cold Spring Harbour Laboratory Press, New York, USA, 1989). The oligonucleotides are not necessarily perfectly complementary to the nucleotide sequences of the versican core protein genes of the present invention, as long as they allow specific hybridization.

The hybridization conditions in the present invention include, for example, conditions such as "2x SSC, 0.1% SDS, and 50°C", "2x SSC, 0.1% SDS, and 42°C", and "1x SSC, 0.1% SDS, and 37°C", and more stringent conditions such as "2x SSC, 0.1% SDS, and 65°C", "0.5x SSC, 0.1% SDS, and 42°C", and "0.2x SSC, 0.1% SDS, and 65°C". More specifically, for methods using Rapid-hyb buffer (Amersham Life Science), prehybridization is carried out at 68°C for 30 minutes or more; then a probe is added, and after one hour or more of hybrid formation at 68°C, washing is carried out three times with 2x SSC/0.1% SDS at room temperature for 20 minutes, then three times with 1xSSC/0.1% SDS at 37°C for 20 minutes, and finally twice with 1x SSC/0.1% SDS at 50°C for 20 minutes. Alternatively, for example, prehybridization is carried out in Expresshyb Hybridization Solution (CLONTECH) at 55°C for 30 minutes, and then a labeled probe is added thereto, and after one hour or more of incubation at 37-55°C, washing is carried out three times in 2x SSC/0.1% SDS at room temperature for 20 minutes and then once in 1x SSC/0.1% SDS at 37°C for 20 minutes. More stringent conditions can be achieved, for example, by increasing the temperature for prehybridization, hybridization, or second washing. For example, temperatures for prehybridization and hybridization can be 60°C, and more stringent conditions can be achieved by increasing the temperature to 68°C. In addition to conditions such as the salt concentration of the buffers and the temperature, those skilled in the art can also determine conditions using other conditions including the nucleotide sequence composition of the probe, the probe length and concentration, and reaction time.

The oligonucleotides can be used as probes and primers in the above-described screening kits of the present invention. When the oligonucleotides are used as primers, they are typically 15 to 100 bp long, and preferably 17 to 30 bp long. Primers are not particularly limited as long as they can amplify at least a portion of a DNA of the above-described genes of the present invention.

The present invention also provides therapeutic or preventive methods for fibrotic liver diseases, which comprise the step of administering the agents of the present invention to individuals (for example, to patients and such).

The individuals subjected to the therapeutic or preventive methods of the present invention are not particularly limited, as long as they are organisms that can develop a fibrotic liver disease; however, humans are preferred.

In general, administration to individuals can be achieved, for example, by methods known to those skilled in the art, such as intraarterial injections, intravenous injections, and subcutaneous injections. The administered dose varies depending on the patient's weight and age, and the administration method or such; however, those skilled in the art (medical practitioners, veterinarians, pharmacists, and the like) can appropriately select a suitable dose.

The present invention also relates to the uses of agents of the present invention in producing hepatic fibrosis-suppressing agents.

All prior-art documents cited herein are incorporated by reference herein.

### Examples

Herein below, the present invention will be specifically described with reference to Examples, but the technical scope of the present invention is not to be construed as being limited thereto.

### [Example 1] Increase in the expression level of chondroitin sulfate proteoglycans (CSPGs) in cirrhosis mouse models

In this Example, a cirrhosis mouse model was prepared using a mouse liver fibrosis model induced by carbon tetrachloride (CCl₄), which is a typical example of mouse model for chronic fibrotic liver diseases, and is examined for the accumulation of proteoglycans using immunohistochemical staining. This liver fibrosis mouse model has superior reproducibility and is widely used as an experimental cirrhosis model (Sakaida I, et al., Hepatology 2004, 40, 1304-1311).

First, carbon tetrachloride (25 µl/100 g body weight, Sigma-Aldrich) was injected into the peritoneal cavities of C57BL/6JcL mice (female, five to six weeks old, CLEA Japan Inc.) twice a week for four weeks (eight times) to induce liver fibrosis. Then, carbon tetrachloride was additionally administered twice a week for two weeks (12 times in total) to induce cirrhosis. Mice with induced cirrhosis were sacrificed, and their livers were collected (cirrhotic livers). Meanwhile, in the control experiment, livers were collected from C57BL/6JcL mice (female, CLEA Japan Inc.) of the same age not administered with carbon tetrachloride (normal livers).

Portions of second lobes from the collected livers were embedded in the OCT compound (Miles), an embedding medium for cryosectioning, and cryoblocks were prepared using liquid nitrogen. The cryoblocks were sliced into 6-µm sections using cryostat (Microm).

The resulting sections were fixed with acetone (Wako Pure Chemical Industries Ltd.) for ten minutes, and then washed with phosphate buffer. Then, as a primary antibody, an anti-chondroitin sulfate proteoglycan (CSPG) antibody (clone CS56, mouse monoclonal antibody, 10 µg/ml, Seikagaku Co.), anti-chondroitin-4-sulfate proteoglycan (C4SPG) antibody (clone LY111, mouse monoclonal antibody, 10 µg/ml, Seikagaku Co.), anti-chondroitin-6-sulfate proteoglycan (C6SPG) antibody (clone MC21C, mouse monoclonal antibody, 10 µg/ml, Seikagaku Co.), or anti-heparan sulfate proteoglycan (HSPG)/Perlecan antibody (rat monoclonal antibody, 10 µg/ml, Dainippon Pharma.) was added, and the sections were incubated at room temperature for one hour. Next, the secondary antibody reaction was conducted using a Histofine Mouse Stain Kit (Nichirei, used for mouse monoclonal antibodies) or a peroxidase-labeled anti-rat IgG (Biosource, at 1:200 dilution, used for rat monoclonal antibodies), and then DAB substrate (Nichirei) was added thereto. The samples were observed under a light microscope (Leica Microsystems). Brown signals showing visible antibody binding were confirmed.

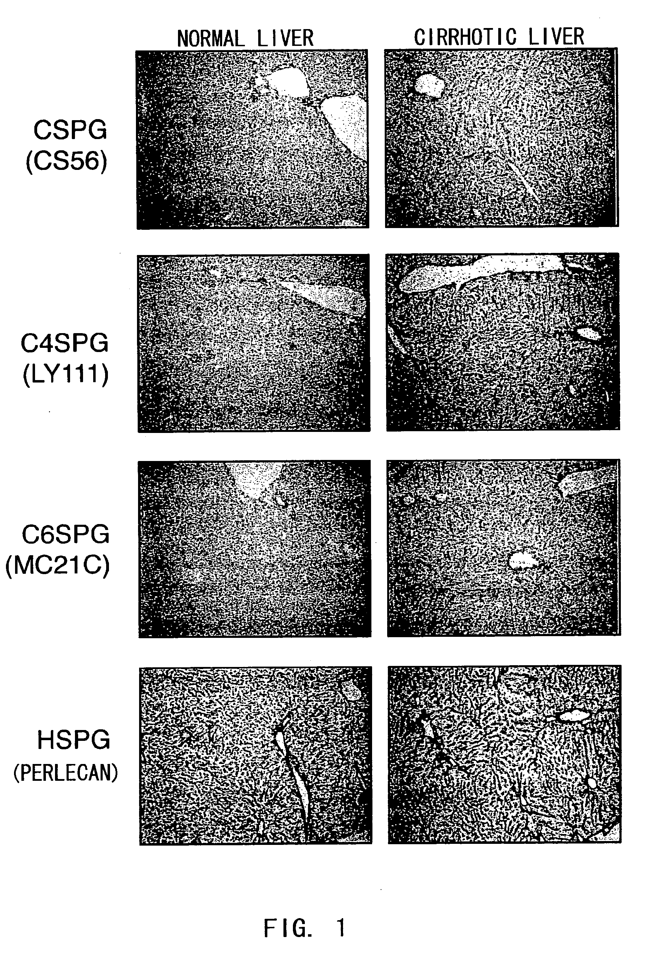

Examples of images obtained by immunostaining the liver tissues are shown in Fig. 1. In Fig. 1, the left panels correspond to normal livers, and the right panels correspond to the cirrhotic livers. In the normal livers, specifically in the livers of non-treated mice of the same age, CSPG (the top panel) and C4SPG (the second panel from the top) gave only weak positive signals within hepatic sinusoids, while C6SPG (the third panel from the top) gave no signal. Meanwhile, in the cirrhotic livers, specifically in the livers in which cirrhosis-like fibrosis was induced by carbon tetrachloride, all of CSPG (the top panel), C4SPG (the second panel from the top), and C6SPG (the third panel from the top) gave extensive positive signals mainly in sinusoids. The signals were significantly enhanced when compared to the normal livers. Heparan sulfate proteoglycan (HSPG) gave a strong positive signal in sinusoids of the normal livers. No significant difference in the signal intensity and distribution was found in the cirrhotic livers. Thus, CSPG was found to be dramatically increased in the liver of the cirrhosis model when compared with HSPG.

### [Example 2] Ability of various chondroitinases to cleave chondroitin sulfate accumulated in mouse cirrhotic livers

In this Example, each type of chondroitinase was evaluated for its ability to cleave chondroitin sulfate (CSPG) in tissue sections from cirrhotic livers. Using the procedure described below, tissue sections of the cirrhotic livers were treated with chondroitinase, and after the enzyme reaction, residual CSPG in the tissue sections, specifically CSPG that was not cleaved in the chondroitinase treatment, was detected by the immunostaining method.

First, carbon tetrachloride (25 µl/100 g body weight, Sigma-Aldrich) was injected into the peritoneal cavities of C57BL/6JcL mice (female, five to six weeks old, CLEA Japan Inc.) twice a week for four weeks (eight times) to induce liver fibrosis. Then, carbon tetrachloride was additionally administered twice a week for two weeks (12 times in total) to induce cirrhosis. Mice with induced cirrhosis were sacrificed, and their livers were collected (cirrhotic livers).

Portions of second lobes from the collected livers were embedded in the OCT compound (Miles), an embedding medium for cryosectioning, and cryoblocks were prepared using liquid nitrogen. The cryoblocks were sliced into 6-µm sections using cryostat (Microm).

The resulting sections were fixed with acetone (Wako Pure Chemical Industries Ltd.) for ten minutes, and then washed with phosphate buffer. After the sections were incubated with Tris-HCl buffer (20 mM, pH8.0) at 37°C for 15 minutes, 100 µl of chondroitinase ABC solution (Seikagaku Co., 5 U/ml), chondroitinase AC solution (Seikagaku Co., 5 U/ml), chondroitinase B solution (Seikagaku Co., 5 U/ml), or buffer alone (chondroitinase (-)) was added to the each section. The sections were covered and then incubated at 37°C for one hour.

After the enzyme reaction, an anti-CSPG antibody (clone CS56, mouse monoclonal antibody, 10 µg/ml, Seikagaku Co.) was added and the sections were incubated at room temperature for one hour. After reaction using the Histofine Mouse Stain Kit (Nichirei), DAB substrate (Nichirei) was added thereto. The samples were observed under a light microscope (Leica Microsystems). Brown signals showing visible antibody binding were confirmed.

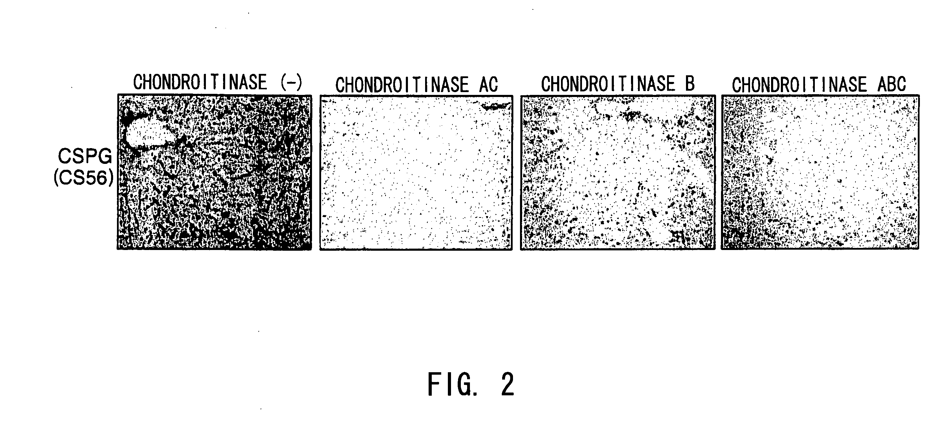

The result is shown in Fig. 2 (magnification, 200 fold). A significantly strong and extensive positive signal of CSPG (brown) was detected in the sections treated with buffer alone (specifically, sections without the enzyme treatment) (Fig. 2, the left most; chondroitinase (-)). The signal intensity was comparable to that detected in the cirrhotic liver shown in Fig. 1. Meanwhile, a positive CSPG signal was impaired and the positive area was decreased in the sections treated with chondroitinase AC as compared to the chondroitinase (-) sections (Fig. 2, the second from the left; chondroitinase AC). This suggests that indeed chondroitinase AC can cleave the CSPG accumulated in cirrhotic liver tissues. However, residual CSPG, which was not completely cleaved, was still detected in the sections treated with chondroitinase AC. In addition, a positive CSPG signal was further impaired and the positive area was also further reduced in the sections treated with chondroitinase B in comparison to the sections treated with chondroitinase AC. This suggests that chondroitinase B can cleave more of the CSPG accumulated in cirrhotic liver tissues. However, in the sections treated with chondroitinase B, residual CSPG was also detected along the pseudolobular structure. Thus, the chondroitinase AC or B treatment could not completely cleave the CSPG deposited in cirrhotic livers with advanced fibrosis.

In contrast to the results described above, in the sections treated with chondroitinase ABC, the positive CSPG signal (brown) was almost undetectable, and thus CSPG was clearly demonstrated to disappear when comparing the chondroitinase ABC-treated sections with the chondroitinase (-) sections. Specifically, chondroitinase ABC was demonstrated to be able to highly efficiently cleave CSPG deposited in lesions with advanced fibrosis such as cirrhosis.

### [Example 3] The hepatic fibrosis-suppressing effect of chondroitinase ABC (Chase ABC) in cirrhosis mouse models

Carbon tetrachloride (25 µl/100 g body weight, Sigma-Aldrich) was injected into the peritoneal cavities of C57BL/6JcL mice (female, five to six weeks old, CLEA Japan Inc.) twice a week for four weeks (eight times) to induce liver fibrosis. Then, carbon tetrachloride was additionally administered twice a week for two weeks (12 times in total).

Prior to further administration, an Alzet Osmotic Pump (Muromachi Kikai Co. Ltd.) pre-infused with 150 µl of chondroitinase ABC (Chase ABC) (20 U/ml, Sigma-Aldrich), chondroitinase AC (Chase AC) (20 U/ml, Sigma-Aldrich), chondroitinase B (Chase B) (20 U/ml, Sigma-Aldrich), or PBS alone was surgically implanted within the peritoneal cavity of each mouse. The respective groups of mice treated as described above were named Chase ABC enzyme group, Chase AC enzyme group, Chase B enzyme group, and control group. During two weeks of further administration of carbon tetrachloride, this treatment allowed constant infusion of a fixed volume of liquid from the implanted pump for all groups.

After further administration of carbon tetrachloride, mice from each group were sacrificed and their livers were collected. Cryosections of liver samples were prepared from the collected livers and analyzed by immunohistochemistry using the same procedure as described in Example 1.

In this analysis, a rabbit anti-mouse type III collagen antibody (at 1:250 dilution, Santa Cruz) was added as a primary antibody to the samples, and the samples were incubated at room temperature for one hour. Then, an alkaline phosphatase-labeled anti-rabbit IgG (at 1:200 dilution, Jackson ImmunoResearch) was added as a secondary antibody. After the sections were incubated at room temperature for 30 minutes, a chromogenic substrate for alkaline phosphatase (Vector Red, Vector Laboratory) was added thereto. The samples were observed under a light microscope (Leica Microsystems). Red signals showing visible antibody binding were confirmed.

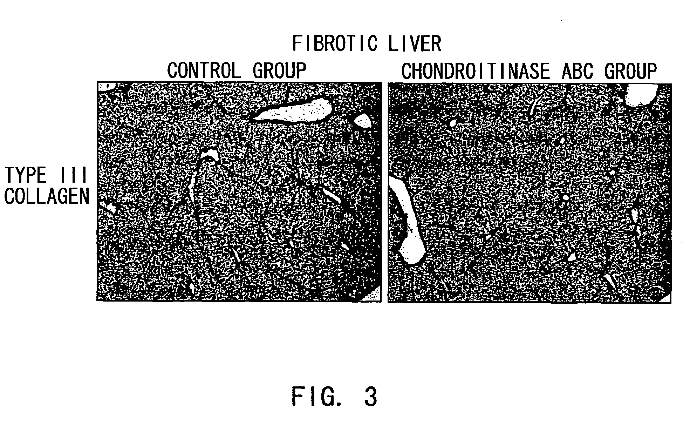

Examples of images obtained by immunostaining the liver tissues are shown in Fig. 3. It was found that type III collagen fibers detected using the rabbit anti-mouse type III collagen antibody elongated not only in general sinusoids but continuously through the central vein up to the portal vein, and pseudolobules were formed in some portions in the control group (the left panel in Fig. 3). This pattern was typical of cirrhosis. In contrast, in the Chase ABC enzyme group, elongation of type III collagen was very mild (the right panel in Fig. 3).

Based on the immunohistochemical staining carried out as described above, the degree of fibrosis of each sample was evaluated using fibrosis scores according to previous reports (Dai K, et al., World J Gactroenterol. 2005, 31, 4822-4826; Hillebrandt S, et al., Nature Genetics 2005, 37, 835-843). The evaluation criteria for fibrosis scores are as follows: score 0, normal; score 1, a small fraction of collagen is found to elongate from the central vein; score 2, collagen evidently elongates from the central vein but to the extent that it does not cover the whole liver; score 3, collagen evidently elongates from the central vein to the extent that it covers the whole liver; score 4, diffuse collagen elongation is observed in the whole liver and pseudolobules are formed.

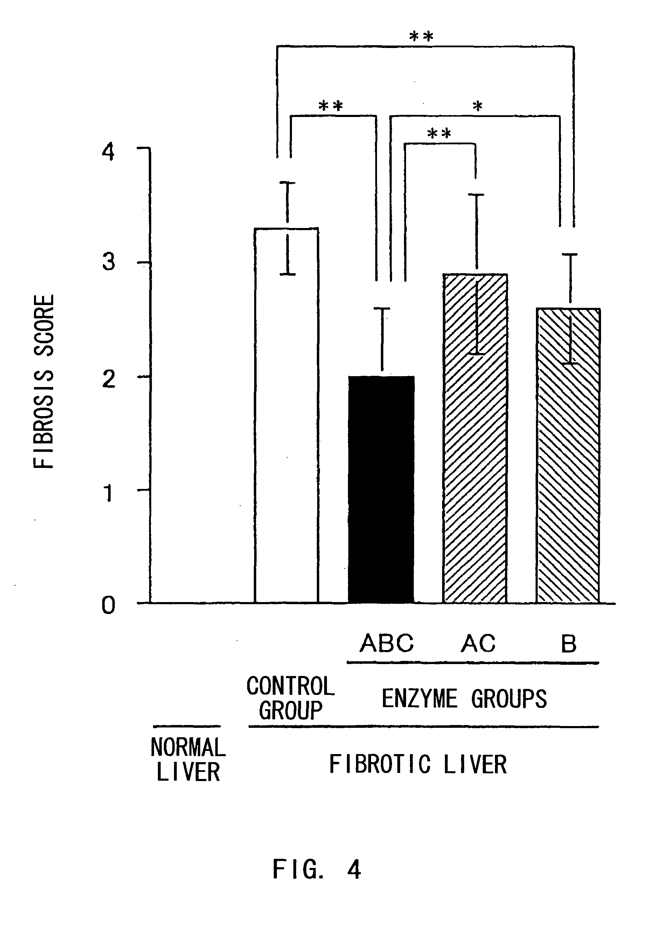

The result is shown as a graph in Fig. 4. Each bar represents mean value ± standard deviation for the fibrosis score in each group. As shown in Fig. 4, the score was 2.0 ± 0.6 (n=6) in the Chase ABC enzyme group, 2.9 ± 0.7 (n=6) in the Chase AC enzyme group, and 2.6 ± 0.5 (n=6) in the Chase B enzyme group, while it was 5.3 ± 0.4 (n=6) in the control group. When compared with the control group, reduction of fibrosis was found to be statistically significant in the Chase ABC enzyme group (p=0.00018, t test) and the Chase B enzyme group (p=0.0029, t test). In particular, the fibrosis-suppressing effect was statistically significantly higher in the Chase ABC enzyme group than in the Chase AC enzyme group (p=0.0019, t test) and Chase B enzyme group (p=0.018, t test). Meanwhile, there was no statistical significant difference between the Chase AC enzyme and Chase B enzyme groups (p=0.15, t test). Normal livers collected from normal mice that were not administered with carbon tetrachloride were also analyzed in the same way by immunohistochemistry, and the fibrosis score was found to be 0. Thus, administration of chondroitinase ABC was demonstrated to exert a clinically superior effect of suppressing hepatic fibrosis.

### [Example 4] Effect of chondroitinase ABC (Chase ABC) in suppressing deposition of type I collagen in cirrhosis mouse models

In fibrotic diseases including cirrhosis, increase of type I collagen is assumed to be the most crucial factor in the formation of lesion-constituting fibers. Thus, immunostaining experiments for type I collagen were conducted using the liver tissue samples derived from each group prepared in Example 3.

A rabbit anti-mouse type I collagen antibody (at 1:100 dilution, Rockland) was added as a primary antibody to the sections prepared in Example 3, which were derived from the control group, Chase ABC enzyme group, Chase AC enzyme group, and Chase B enzyme group. The sections were incubated at room temperature for one hour. Then, an alkaline phosphatase-labeled anti-rabbit IgG (at 1:200 dilution, Jackson ImmunoResearch) was added as a secondary antibody. After the sections were incubated at room temperature for additional 30 minutes, a chromogenic substrate for alkaline phosphatase (Vector Red, Vector Laboratory) was added thereto. The samples were observed under a light microscope (Leica Microsystems). Red signals showing visible antibody binding were confirmed.

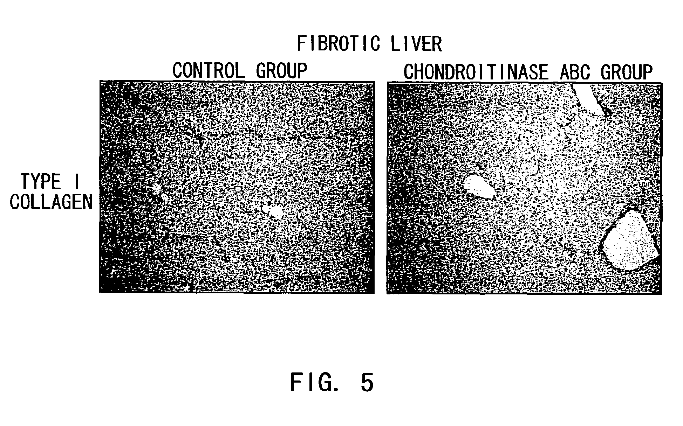

Examples of images obtained by immunostaining the liver tissues are shown in Fig. 5. As shown in Fig. 5, type I collagen was clearly detected along the formed pseudolobules in the control group. In contrast, the collagen was found only occasionally in the Chase ABC enzyme group.

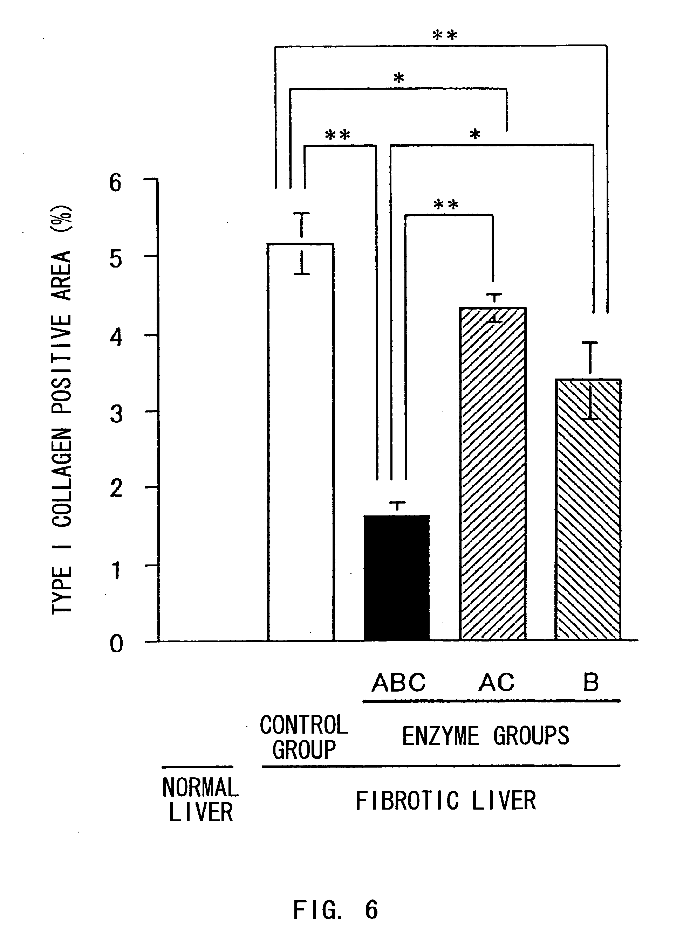

Then, based on the immunohistochemical staining carried out as described above, the type I collagen positive area was calculated using the Win ROOF image analysis software (Win ROOF, Mitani Co.). Areas of at least 15 mm² in the liver sections were measured, and the result was presented as a ratio (%) of the positive area to the liver area measured. The result is shown as a graph in Fig. 6. As also shown in Fig. 6, the percentage of type I collagen-positive area (%) was 1.61 ± 0.29% in the Chase ABC enzyme group, 4.30 ± 0.18% in the Chase AC enzyme group, 3.38 ± 0.50% in the Chase B enzyme group, and 5.16 ± 0.98% in the control group. The percentage of type I collagen-positive area (%) was significantly reduced in all the enzyme groups as compared with the control group (Chase ABC enzyme group, p=0.0018; Chase AC enzyme group, p=0.023; Chase B enzyme group, p=0.0059; all by t test). In particular, the percentage of type I collagen-positive area (%) in the Chase ABC enzyme group was also statistically significantly decreased as compared with those of the Chase AC enzyme group and the Chase B enzyme group (significant difference p=0.0008 when compared with the Chase AC enzyme group, significant difference p=0.003 when compared with the Chase B enzyme group, both by t test). Thus, the effect of Chase ABC enzyme in suppressing the deposition of type I collagen was demonstrated to be extremely strong. Meanwhile, there was no statistically significant difference between the Chase AC enzyme and Chase B enzyme groups (p=0.068, t test). The normal livers collected from normal mice that were not administered with carbon tetrachloride were also analyzed in the same way, and the percentage of type I collagen-positive area was found to be 0%. This result demonstrates that administration of chondroitinase ABC can significantly suppress the deposition of type I collagen in fibrotic livers.

### [Example 5] Effect of chondroitinase ABC (Chase ABC) in suppressing deposition of chondroitin sulfate (CSPG) in cirrhosis mouse models

To demonstrate that chondroitin sulfate (CSPG) deposited in fibrotic livers is degraded by administering Chase ABC, the liver tissue samples of the control group and the Chase ABC enzyme group prepared in Example 3 were analyzed by immunohistochemistry using anti-CSPG antibody, anti-C4SPG antibody, and anti-C6SPG antibody as primary antibodies by the same procedure as described in Example 1.

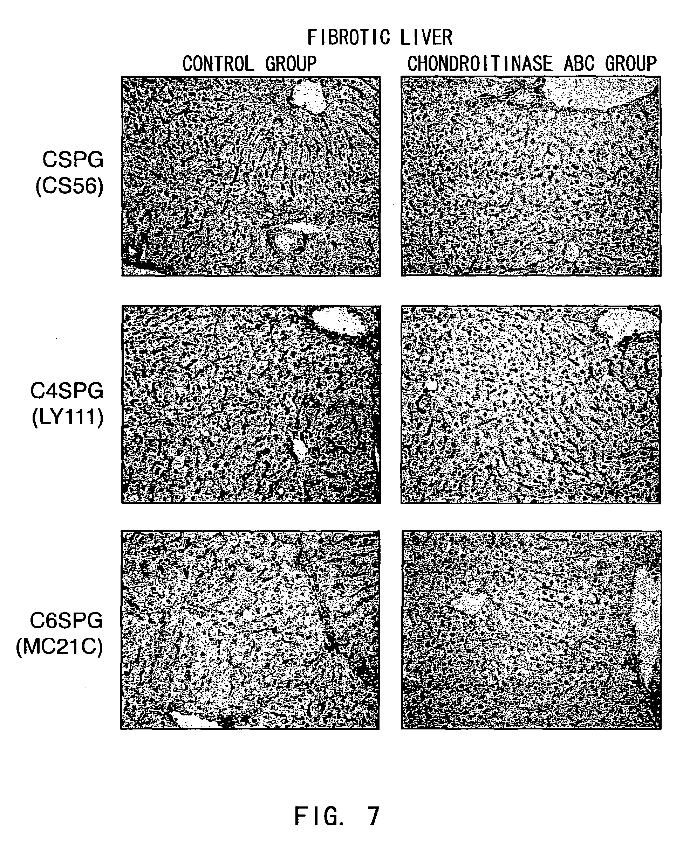

Examples of images obtained by immunostaining the liver tissues are shown in Fig. 7. In Fig. 7, the left panels correspond to the control group, and the right panels correspond to the Chase ABC enzyme group. As shown in Fig. 7, as is the case of cirrhotic livers in Example 1, all CSPG; C4SPG, and C6SPG gave clear positive signals in the control group. In contrast, evidently impaired signals were observed for all CSPG, C4SPG, and C6SPG in the Chase ABC enzyme group, as compared with the control group. In particular, the staining images for C6SPG (the bottom) showed that in the control group, a clear positive signal was detected surrounding the pseudolobules where the elongation of collagen was observed in Examples 4 and 5, while there was almost no such signal in the Chase ABC enzyme group.

The result described above demonstrates that CSPG deposited in fibrotic livers is degraded by administering chondroitinase ABC.

### [Example 6] Effect of chondroitinase ABC (Chase ABC) in suppressing accumulation of fibroblasts in cirrhosis mouse models

As collagen-producing cells that enhance fibrosis, activated fibroblasts are a key player. Excessive accumulation, settlement, and activation of fibroblasts are assumed to exacerbate the pathological condition of fibrosis. Thus, the dynamics of fibroblasts in the cirrhosis model was examined by immunohistochemistry.

First, a rat anti-mouse fibroblast antibody (clone ER-TR7, at 1:500 dilution, BMA) was added as a primary antibody to the section samples derived from the control group and the Chase ABC enzyme group prepared in Example 3, and the sections were incubated at room temperature for one hour. Then, a peroxidase-labeled anti-rat IgG (at 1:200 dilution, Biosource) was added as a secondary antibody. After 30 minutes of incubation at room temperature, DAB substrate (Nichirei) was added thereto. The samples were observed under a light microscope (Leica Microsystems). Brown signals showing visible antibody binding were confirmed. The same immunohistochemical analysis was carried out using normal livers collected from normal mice that were not administered with carbon tetrachloride.

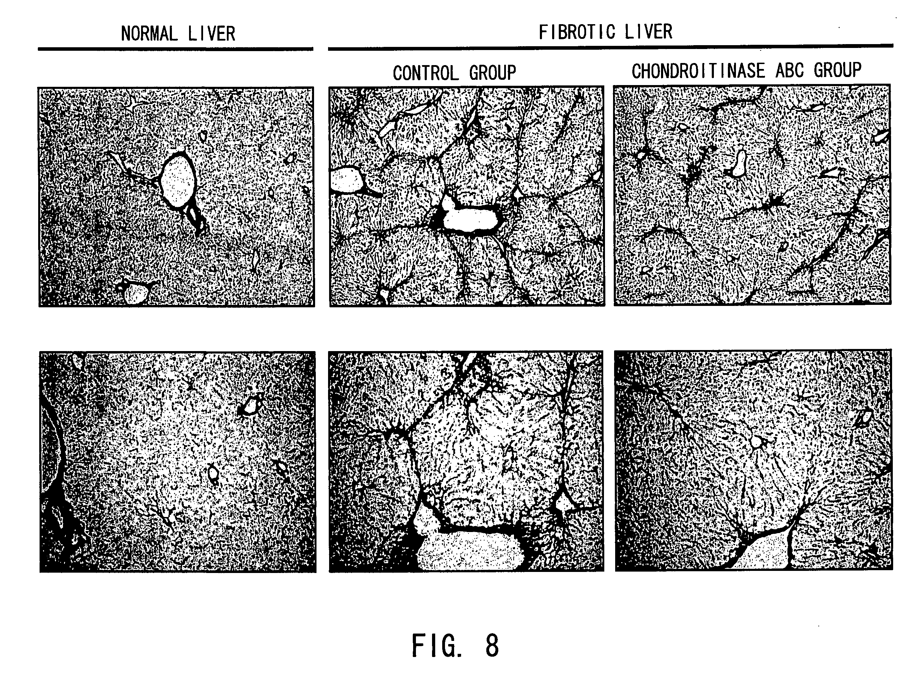

Examples of images obtained by immunostaining the liver tissues are shown in Fig. 8. In Fig. 8, the left most panels correspond to the normal group (normal livers), the middle panels correspond to the control group, and the right most panels correspond to the Chase ABC enzyme group. The upper panels are images at 100 fold magnification and the bottom panels are images at 200 fold magnification. Intense positive signals for fibroblast were distributed in areas surrounding the portal veins of liver in the non-treated normal mice. Furthermore, evident accumulation of fibroblasts along pseudolobules was seen in the control group (cirrhotic liver); however, such accumulation was very mild in the Chase ABC group. This finding indicates that excessive accumulation or settlement of fibroblasts in fibrotic livers was significantly suppressed by administering Chase ABC.

Furthermore, from the finding of this Example that fibroblasts accumulated along pseudolobules (fibrotic layer) in the cirrhotic livers and the finding of the above-described Example that a clear positive signal for CSPG was detected in areas surrounding pseudolobules in the cirrhotic livers of the control group, it is suggested that CSPG provides anchorage for fibroblasts in liver fibrosis.

### [Example 7] Effect of chondroitinase ABC (Chase ABC) in suppressing accumulation of macrophages in cirrhosis mouse models

The major cell that produces fibroblast-activating factors is assumed to be macrophage. Thus, the dynamics of macrophages in the cirrhosis model were also examined by immunohistochemistry.

First, a rat anti-mouse macrophage antibody (clone F4/80, at 1:500 dilution, BMA) was added as a primary antibody to the section samples derived from the control group and the Chase ABC enzyme group prepared in Example 3, and the sections were incubated at room temperature for one hour. Then, a peroxidase-labeled anti-rat IgG (at 1:200 dilution, Biosource) was added as a secondary antibody. After 30 minutes of incubation at room temperature, DAB substrate (Nichirei) was added thereto. The samples were observed under a light microscope (Leica Microsystems). Brown signals showing visible antibody binding were confirmed. The same immunohistochemical analysis was carried out using normal livers collected from normal mice that were not administered with carbon tetrachloride.

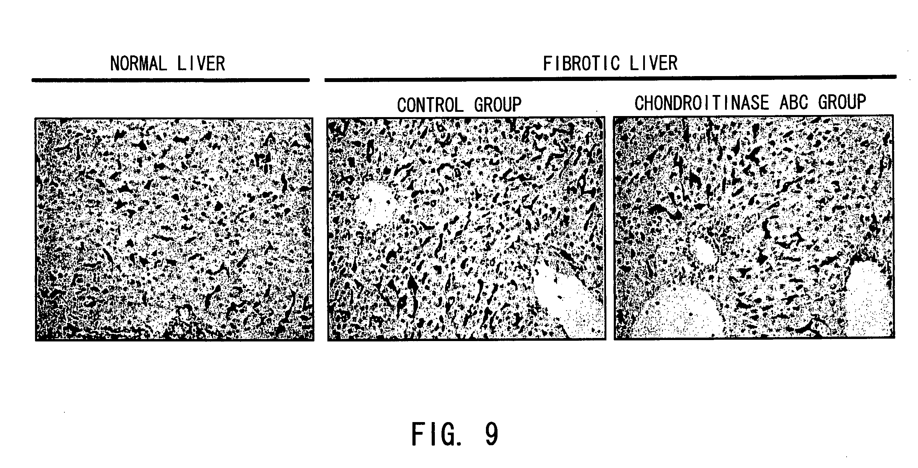

Examples of images obtained by immunostaining the liver tissues are shown in Fig. 9. In Fig. 9, the left most panels correspond to the normal group (normal liver), the middle panels correspond to the control group, and the right most panels correspond to the Chase ABC enzyme group. As shown in Fig. 9, positive signals were widely distributed in sinusoids in the normal livers, and the cells were assumed to be Kupffer cells. By contrast, the macrophage count was evidently increased in the cirrhotic livers of the control group, and many of the macrophages were detected near collagen that was found to surround pseudolobules in Examples 4 and 5. In contrast, the macrophage count was evidently reduced in the Chase ABC enzyme group as compared with the control group. Specifically, accumulation of macrophages in cirrhotic livers was demonstrated to be suppressed in the Chase ABC enzyme group. This result suggests that the progression of fibrosis was suppressed in the Chase ABC enzyme group as opposed to the formation of progressive fibrotic lesions in the control group (cirrhotic livers).

### [Example 8] Therapeutic effect (suppression of fibroblast infiltration) of administering ADAMTS-4 peptide in cirrhosis mouse models

Carbon tetrachloride-induced cirrhosis mouse models were prepared by the same induction method as described in Example 1. The sequence of the ADAMTS-4 peptide used in the therapy was NH2-DRARSCAIVEDDGLQSAFTA-COOH (SEQ ID NO: 67) (synthesized as a peptide having positions 336 to 355 of mouse ADAMTS-4, which is a domain having metalloproteinase activity, GeneWorld Ltd., 1 µg/head). Vehicle (PBS) was used in the control group. The peptide or vehicle was administered into the peritoneal cavities four times in total, as is the case for the supplementary administration of carbon tetrachloride (a total of four times from the ninth to the twelfth time). After the supplementary administration, mice from both groups were sacrificed. Sections were prepared from their liver tissues and examined by immunohistochemistry.

Activated fibroblasts are a key player as collagen-producing cells that enhance fibrosis. Excessive accumulation, settlement, and activation of fibroblasts are assumed to exacerbate the pathological condition of fibrosis. Thus, the dynamics of fibroblasts in the cirrhosis model were examined by immunohistochemistry.

A rat anti-mouse fibroblast antibody (clone ER-TR7, at 1:500 dilution, BMA) was added as a primary antibody to the section samples derived from the control group and the ADAMTS-4-treated group, and the sections were incubated at room temperature for one hour. Then, a peroxidase-labeled anti-rat IgG (at 1:200 dilution, Biosource) was added as a secondary antibody. After 30 minutes of incubation at room temperature, DAB substrate (Nichirei) was added thereto. The samples were observed under a light microscope (Leica Microsystems). Brown signals showing visible antibody binding were confirmed.

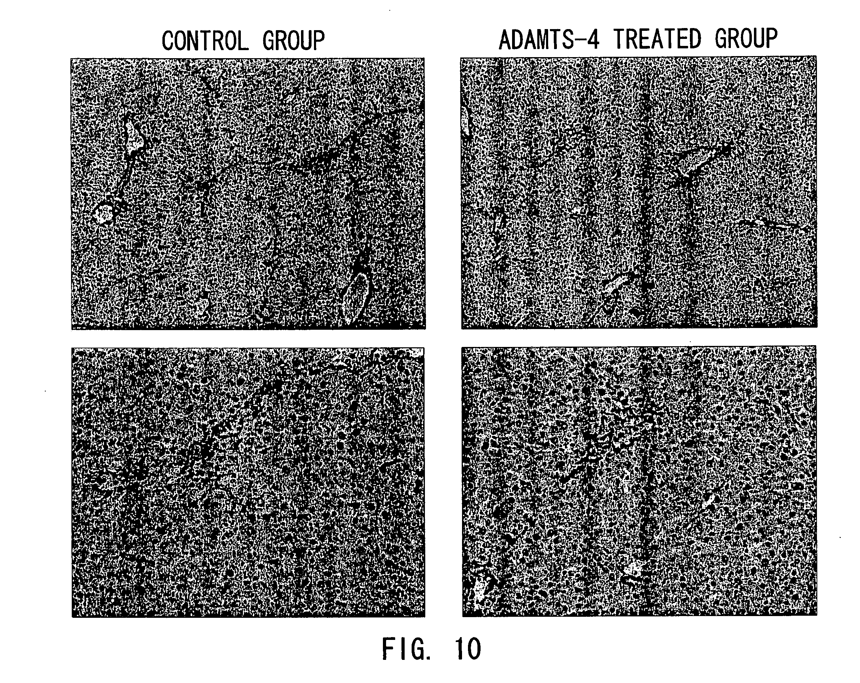

Examples of images obtained by immunostaining the liver tissues are shown in Fig. 10. In the control group (cirrhotic livers), fibroblasts were evidently demonstrated to accumulate along pseudolobules. In contrast, the degree of accumulation was very mild in the ADAMTS-4-treated group. This suggests that ADAMTS-4 administration significantly suppressed the excessive accumulation or settlement of fibroblasts in the fibrotic livers.

### [Example 9] Therapeutic effect (suppression of type III collagen) of administering the ADAMTS-4 peptide in cirrhosis mouse models

Cryosections of liver samples were prepared and analyzed by immunohistochemistry using the same procedure as described in Example 8. A rabbit anti-mouse type III collagen antibody (at 1:250 dilution, Santa Cruz) was added as a primary antibody to the samples, and the samples were incubated at room temperature for one hour. Then, an alkaline phosphatase-labeled anti-rabbit IgG (at 1:200 dilution, Jackson ImmunoResearch) was added as a secondary antibody. After the sections were incubated at room temperature for 30 minutes, a chromogenic substrate for alkaline phosphatase (Vector Red, Vector Laboratory) was added thereto. The samples were observed under a light microscope (Leica Microsystems). Red signals showing visible antibody binding were confirmed.

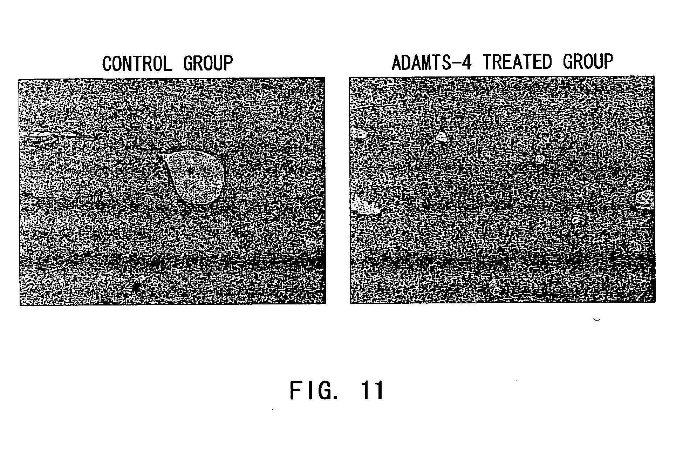

Examples of images obtained by immunostaining the liver tissues are shown in Fig. 11. It was found that type III collagen fibers detected using the rabbit anti-mouse type III collagen antibody elongated in sinusoids and continuously through the central vein up to the portal vein, and a portion of the fibers formed pseudolobules in the control group. This pattern was typical of cirrhosis. In contrast, in the ADAMTS-4 group, elongation of type III collagen was almost undetectable.

### [Example 10] Therapeutic effect (suppression in fibrosis score) of administering the ADAMTS-4 peptide in cirrhosis mouse models

Based on the immunohistochemical staining carried out in Example 9, the degree of fibrosis in each sample was evaluated using fibrosis scores according to previous reports (Dai K, et al., World J Gactroenterol. 2005, 31, 4822-4826; Hillebrandt S, et al., Nature Genetics 2005, 37, 835-843). The evaluation criteria for fibrosis scores are as follows: score 0, normal; score 1, a small fraction of collagen is found to elongate from the central vein; score 2, collagen evidently elongates from the central vein but to the extent that it does not cover the whole liver; score 3, collagen evidently elongates from the central vein to the extent that it covers the whole liver; score 4, diffuse collagen elongation is observed in the whole liver and pseudolobules are formed.

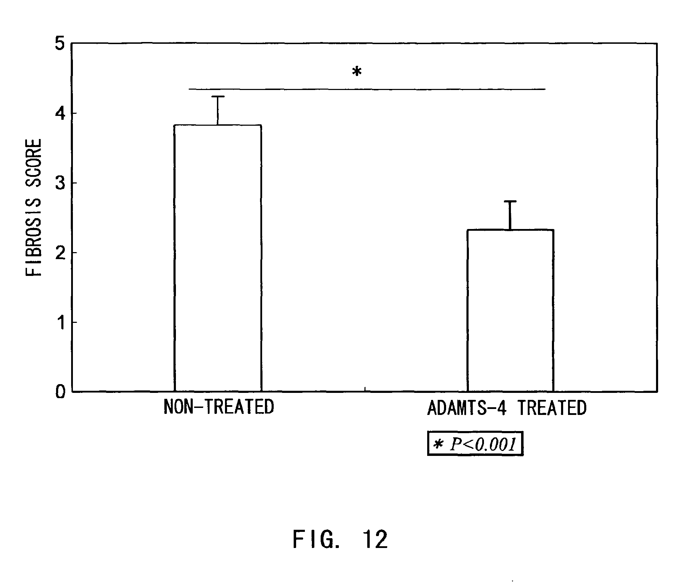

The result is shown as a graph in Fig. 12. Each bar represents mean value ± standard deviation for fibrosis score in each group. The score was 2.3 ± 0.4 (n=6) in the ADAMTS-4-treated group, while it was 3.9 ± 0.4 (n=6) in the control group. As compared with the control group, the fibrosis reduction was found to be statistically significant in the ADAMTS-4-treated group (p<0.001, t test). Thus, administration of the ADAMTS-4 peptide was demonstrated to exert a clinically superior effect of suppressing hepatic fibrosis.

### Industrial Applicability

The hepatic fibrosis-suppressing agents of the present invention comprising chondroitinase ABC as an active ingredient suppress fibrosis of liver tissues and thus are effective for treating or preventing diseases accompanied by excessive fibrosis of liver tissues (fibrotic liver diseases). The hepatic fibrosis-suppressing agents of the present invention are highly useful in suppressing hepatic fibrosis, because they are efficacious in suppressing various symptoms associated with fibrosis in various ways, such as by suppressing elongation of type III collagen, suppressing deposition of type I collagen, suppressing accumulation of fibroblasts, and suppressing accumulation of macrophages in fibrotic liver tissues. Methods for treating or preventing fibrotic liver diseases by administering the hepatic fibrosis-suppressing agents of the present invention can effectively improve fibrotic lesions through medicinal therapy, and are potentially excellent therapeutic methods that are useful for improving patients' QOL.

All publications, patents, and patent applications cited herein are incorporated herein by reference in their entirety.

## Claims

1. A hepatic fibrosis-suppressing agent comprising as an active ingredient a substance that inhibits the production or accumulation of a chondroitin sulfate proteoglycan.

2. The agent of claim 1, wherein the substance has an activity of promoting the degradation of a chondroitin sulfate proteoglycan.

3. The agent of claim 1, wherein the substance has an activity of inhibiting the synthesis of a chondroitin sulfate proteoglycan.

4. The agent of claim 1, wherein the substance has an activity of desulfating a chondroitin sulfate proteoglycan.

5. The agent of claim 1, wherein the substance has an activity of inhibiting the sulfation of a chondroitin sulfate proteoglycan.

6. The agent of any one of claims 1 to 5, wherein the production or accumulation of a chondroitin sulfate proteoglycan is inhibited in liver.

7. The agent of any one of claims 1 to 6, which is used for treating or preventing a fibrotic liver disease.

8. The agent of claim 7, wherein the fibrotic liver disease is a chronic liver disease.

9. The agent of claim 7, wherein the fibrotic liver disease is chronic hepatitis, liver fibrosis, cirrhosis, liver failure, or hepatocarcinoma.

10. A method of screening for a hepatic fibrosis-suppressing agent, which comprises selecting from a test sample a substance with an activity of inhibiting the production or accumulation of a chondroitin sulfate proteoglycan.

11. The screening method of claim 10, which comprises the step of selecting a substance with the activity of any one of:
(a) promoting the degradation of a chondroitin sulfate proteoglycan;
(b) inhibiting the synthesis of a chondroitin sulfate proteoglycan;
(c) desulfating a chondroitin sulfate proteoglycan; and
(d) inhibiting the sulfation of a chondroitin sulfate proteoglycan.

12. The screening method of claim 10 or 11, wherein the hepatic fibrosis-suppressing agent is used for treating or preventing a fibrotic liver disease.
